# EUROPEAN PATENT SPECIFICATION

(11) **EP 2 788 384 B1**
(45) Date of publication and mention of the grant of the patent: **09.08.2017**
(21) Application number: 12801700.1
(22) Date of filing: 07.12.2012
(51) Int. Cl.: C07K 16/40, A61K 39/395, A61P 3/06, A61P 9/10

(54) **AGONISTIC HUMAN LCAT ANTIGEN BINDING PROTEINS AND THEIR USE IN THERAPY**
AGONISTISCHE MENSCHLICHE LCAT-ANTIGENBINDENDE PROTEINE UND IHRE VERWENDUNG IN DER THERAPIE
PROTÉINES AGONISTES DE LIAISON À L'ANTIGÈNE LCAT HUMAIN ET LEUR UTILISATION THÉRAPEUTIQUE

(30) Priority: 08.12.2011 US 201161568448 P; 29.11.2012 US 201261731408 P
(43) Date of publication of application: 15.10.2014
(73) Proprietor: Amgen Inc., Thousand Oaks, California 91320 (US)
(72) Inventor: MEININGER, David, P., San Carlos California 94070 (US); WITTEKIND, Michael, New Canaan Connecticut 06840 (US); DELANEY, John,M., Bellevue Washington 98004 (US); ZHOU, Mingyue, Union City California 94587 (US); PIPER, Derek, E., Santa Clara California 95051 (US)
(74) Representative: Grünecker Patent- und Rechtsanwälte PartG mbB
(86) International application number: PCT/US2012/068608
(87) International publication number: WO 2013/086443

(56) References cited:
- JP-A- 11 269 199
- BANKA C L ET AL: "Localization of an Apolipoprotein A-I Epitope Critical for Activation of Lecithin-Cholesterol Acyltransferase", JOURNAL OF BIOLOGICAL CHEMISTRY, AMERICAN SOCIETY FOR BIOCHEMISTRY AND MOLECULAR BIOLOGY, US, vol. 266, no. 35, 15 December 1991 (1991-12-15), pages 23886-23892, XP002299434, ISSN: 0021-9258
- ZHU CHEN ET AL: "AAV8-Mediated Long-Term Expression of Human LCAT Significantly Improves Lipid Profiles in hCETP;LdlrMice", JOURNAL OF CARDIOVASCULAR TRANSLATIONAL RESEARCH, SPRINGER US, BOSTON, vol. 4, no. 6, 6 August 2011 (2011-08-06), pages 801-810, XP019979792, ISSN: 1937-5395, DOI: 10.1007/S12265-011-9309-8

### SEQUENCE LISTING

<110> AMGEN INC.
<120> HUMAN LCAT ANTIGEN BINDING PROTEINS AND THEIR USE IN THERAPY
<130> A-1678-WO-PCT
<140>
   <141>
<150> 61/731,408
   <151> 2012-11-29
<150> 61/568,448
   <151> 2011-12-08
<160> 169
<170> PatentIn version 3.5
<210> 1
   <211> 416
   <212> PRT
   <213> Homo sapiens
<400> 1
   Phe Trp Leu Leu Asn Val Leu Phe Pro Pro His Thr Thr Pro Lys Ala
   1               5                   10                  15
   Glu Leu Ser Asn His Thr Arg Pro Val Ile Leu Val Pro Gly Cys Leu
               20                  25                  30
   Gly Asn Gln Leu Glu Ala Lys Leu Asp Lys Pro Asp Val Val Asn Trp
           35                  40                  45
   Met Cys Tyr Arg Lys Thr Glu Asp Phe Phe Thr Ile Trp Leu Asp Leu
       50                  55                  60
   Asn Met Phe Leu Pro Leu Gly Val Asp Cys Trp Ile Asp Asn Thr Arg
   65                  70                  75                  80
   Val Val Tyr Asn Arg Ser Ser Gly Leu Val Ser Asn Ala Pro Gly Val
                   85                  90                  95
   Gln Ile Arg Val Pro Gly Phe Gly Lys Thr Tyr Ser Val Glu Tyr Leu
               100                 105                 110
   Asp Ser Ser Lys Leu Ala Gly Tyr Leu His Thr Leu Val Gln Asn Leu
           115                 120                 125
   Val Asn Asn Gly Tyr Val Arg Asp Glu Thr Val Arg Ala Ala Pro Tyr
       130                 135                 140
   Asp Trp Arg Leu Glu Pro Gly Gln Gln Glu Glu Tyr Tyr Arg Lys Leu
   145 150 155 160
   Ala Gly Leu Val Glu Glu Met His Ala Ala Tyr Gly Lys Pro Val Phe
   165 170 175
   Leu Ile Gly His Ser Leu Gly Cys Leu His Leu Leu Tyr Phe Leu Leu
   180 185 190
   Arg Gln Pro Gln Ala Trp Lys Asp Arg Phe Ile Asp Gly Phe Ile Ser
   195 200 205
   Leu Gly Ala Pro Trp Gly Gly Ser Ile Lys Pro Met Leu Val Leu Ala
   210 215 220
   Ser Gly Asp Asn Gln Gly Ile Pro Ile Met Ser Ser Ile Lys Leu Lys
   225 230 235 240
   Glu Glu Gln Arg Ile Thr Thr Thr Ser Pro Trp Met Phe Pro Ser Arg
   245 250 255
   Met Ala Trp Pro Glu Asp His Val Phe Ile Ser Thr Pro Ser Phe Asn
   260 265 270
   Tyr Thr Gly Arg Asp Phe Gln Arg Phe Phe Ala Asp Leu His Phe Glu
   275 280 285
   Glu Gly Trp Tyr Met Trp Leu Gln Ser Arg Asp Leu Leu Ala Gly Leu
   290 295 300
   Pro Ala Pro Gly Val Glu Val Tyr Cys Leu Tyr Gly Val Gly Leu Pro
   305 310 315 320
   Thr Pro Arg Thr Tyr Ile Tyr Asp His Gly Phe Pro Tyr Thr Asp Pro
   325 330 335
   Val Gly Val Leu Tyr Glu Asp Gly Asp Asp Thr Val Ala Thr Arg Ser
   340 345 350
   Thr Glu Leu Cys Gly Leu Trp Gln Gly Arg Gln Pro Gln Pro Val His
   355 360 365
   Leu Leu Pro Leu His Gly Ile Gln His Leu Asn Met Val Phe Ser Asn
   370 375 380
   Leu Thr Leu Glu His Ile Asn Ala Ile Leu Leu Gly Ala Tyr Arg Gln
   385 390 395 400
   Gly Pro Pro Ala Ser Pro Thr Ala Ser Pro Glu Pro Pro Pro Pro Glu
   405 410 415
<210> 2
   <211> 416
   <212> PRT
   <213> Homo sapiens
<400> 2
   Phe Trp Leu Leu Asn Val Leu Phe Pro Pro His Thr Thr Pro Lys Ala
   1 5 10 15
   Glu Leu Ser Asn His Thr Arg Pro Val Ile Leu Val Pro Gly Cys Leu
   20 25 30
   Gly Asn Gln Leu Glu Ala Lys Leu Asp Lys Pro Asp Val Val Asn Trp
   35 40 45
   Met Cys Tyr Arg Lys Thr Glu Asp Phe Phe Thr Ile Trp Leu Asp Leu
   50 55 60
   Asn Met Phe Leu Pro Leu Gly Val Asp Cys Trp Ile Asp Asn Thr Arg
   65 70 75 80
   Val Val Tyr Asn Arg Ser Ser Gly Leu Val Ser Asn Ala Pro Gly Val
   85 90 95
   Gln Ile Arg Val Pro Gly Phe Gly Lys Thr Tyr Ser Val Glu Tyr Leu
   100 105 110
   Asp Ser Ser Lys Leu Ala Gly Tyr Leu His Thr Leu Val Gln Asn Leu
   115 120 125
   Val Asn Asn Gly Tyr Val Arg Asp Glu Thr Val Arg Ala Ala Pro Tyr
   130 135 140
   Asp Trp Arg Leu Glu Pro Gly Gln Gln Glu Glu Tyr Tyr Arg Lys Leu
   145 150 155 160
   Ala Gly Leu Val Glu Glu Met His Ala Ala Tyr Gly Lys Pro Val Phe
   165 170 175
   Leu Ile Gly His Ser Leu Gly Cys Leu His Leu Leu Tyr Phe Leu Leu
   180 185 190
   Arg Gln Pro Gln Ala Trp Lys Asp Arg Phe Ile Asp Gly Phe Ile Ser
   195 200 205
   Leu Gly Ala Pro Trp Gly Gly Ser Ile Lys Pro Met Leu Val Leu Ala
       210                 215                 220
   Ser Gly Asp Asn Gln Gly Ile Pro Ile Met Ser Ser Ile Lys Leu Lys
   225                 230                 235                 240
   Glu Glu Gln Arg Ile Thr Thr Thr Ser Pro Trp Met Phe Pro Ser Arg
                   245                 250                 255
   Met Ala Trp Pro Glu Asp His Val Phe Ile Ser Thr Pro Ser Phe Asn
               260                 265                 270
   Tyr Thr Gly Arg Asp Phe Gln Arg Phe Phe Ala Asp Leu His Phe Glu
           275                 280                 285
   Glu Gly Trp Tyr Met Trp Leu Gln Ser Arg Asp Leu Leu Ala Gly Leu
       290                 295                 300
   Pro Ala Pro Gly Val Glu Val Tyr Cys Leu Tyr Gly Val Gly Leu Pro
   305                 310                 315                 320
   Thr Pro Arg Thr Tyr Ile Tyr Asp His Gly Phe Pro Tyr Thr Asp Pro
                   325                 330                 335
   Val Gly Val Leu Tyr Glu Asp Gly Asp Asp Thr Val Ala Thr Arg Ser
               340                 345                 350
   Thr Glu Leu Cys Gly Leu Trp Gln Gly Arg Gln Pro Gln Pro Val His
           355                 360                 365
   Leu Leu Pro Leu His Gly Ile Gln His Leu Asn Met Val Phe Ser Asn
       370                 375                 380
   Leu Thr Leu Glu His Ile Asn Ala Ile Leu Leu Gly Ala Tyr Arg Gln
   385                 390                 395                 400
   Gly Pro Pro Ala Ser Pro Thr Ala Ser Pro Glu Pro Pro Pro Pro Glu
                   405                 410                 415
<210> 3
   <211> 416
   <212> PRT
   <213> Macaca fascicularis
<400> 3
   Phe Trp Leu Leu Asn Val Leu Phe Pro Pro His Thr Thr Pro Lys Ala
   1               5                   10                  15
   Glu Leu Ser Asn His Thr Arg Pro Val Ile Leu Val Pro Gly Cys Leu
               20                  25                  30
   Gly Asn Gln Leu Glu Ala Lys Leu Asp Lys Pro Asp Val Val Asn Trp
           35                  40                  45
   Met Cys Tyr Arg Lys Thr Glu Asp Phe Phe Thr Ile Trp Leu Asp Leu
       50                  55                  60
   Asn Met Phe Leu Pro Leu Gly Val Asp Cys Trp Ile Asp Asn Thr Arg
   65                  70                  75                  80
   Val Val Tyr Asn Arg Ser Ser Gly Leu Val Ser Asn Ala Pro Gly Val
                   85                  90                  95
   Gln Ile Arg Val Pro Gly Phe Gly Lys Thr Tyr Ser Val Glu Tyr Leu
               100                 105                 110
   Asp Ser Ser Lys Leu Ala Gly Tyr Leu His Thr Leu Val Gln Asn Leu
           115                 120                 125
   Val Asn Asn Gly Tyr Val Arg Asp Glu Thr Val Arg Ala Ala Pro Tyr
       130                 135                 140
   Asp Trp Arg Leu Glu Pro Gly Gln Gln Glu Glu Tyr Tyr His Lys Leu
   145                 150                 155                 160
   Ala Gly Leu Val Glu Glu Met His Ala Ala Tyr Gly Lys Pro Val Phe
                   165                 170                 175
   Leu Ile Gly His Ser Leu Gly Cys Leu His Leu Leu Tyr Phe Leu Leu
               180                 185                 190
   Arg Gln Pro Gln Ala Trp Lys Asp Arg Phe Ile Asp Gly Phe Ile Ser
           195                 200                 205
   Leu Gly Ala Pro Trp Gly Gly Ser Ile Lys Pro Met Leu Val Leu Ala
       210                 215                 220
   Ser Gly Asp Asn Gln Gly Ile Pro Ile Met Ser Ser Ile Lys Leu Lys
   225                 230                 235                 240
   Glu Glu Gln Arg Ile Thr Thr Thr Ser Pro Trp Met Phe Pro Ser Arg
                   245                 250                 255
   Met Ala Trp Pro Glu Asp His Val Phe Ile Ser Thr Pro Ser Phe Asn
               260                 265                 270
   Tyr Thr Gly Arg Asp Phe Gln Arg Phe Phe Ala Asp Leu His Phe Glu
   275 280 285
   Glu Gly Trp Tyr Met Trp Leu Gln Ser Arg Asp Leu Leu Ala Gly Leu
   290 295 300
   Pro Ala Pro Gly Val Glu Val Tyr Cys Leu Tyr Gly Val Gly Leu Pro
   305 310 315 320
   Thr Pro Arg Thr Tyr Ile Tyr Asp His Gly Phe Pro Tyr Thr Asp Pro
   325 330 335
   Val Asp Val Leu Tyr Glu Asp Gly Asp Asp Thr Val Ala Thr Arg Ser
   340 345 350
   Thr Glu Leu Cys Gly Leu Trp Gln Gly Arg Gln Pro Gln Pro Val His
   355 360 365
   Leu Leu Pro Leu Arg Gly Ile Gln His Leu Asn Met Val Phe Ser Asn
   370 375 380
   Gln Thr Leu Glu His Ile Asn Ala Ile Leu Leu Gly Ala Tyr Arg Gln
   385 390 395 400
   Gly Pro Pro Ala Ser Pro Thr Ala Ser Pro Glu Pro Pro Pro Pro Glu
   405 410 415
<210> 4
   <211> 414
   <212> PRT
   <213> Mus musculus
<400> 4
   Phe Trp Leu Leu Asn Val Leu Phe Pro Pro His Thr Thr Pro Lys Ala
   1               5                   10                  15
   Glu Leu Ser Asn His Thr Arg Pro Val Ile Leu Val Pro Gly Cys Leu
               20                  25                  30
   Gly Asn Arg Leu Glu Ala Lys Leu Asp Lys Pro Asp Val Val Asn Trp
           35                  40                  45
   Met Cys Tyr Arg Lys Thr Glu Asp Phe Phe Thr Ile Trp Leu Asp Phe
       50                  55                  60
   Asn Leu Phe Leu Pro Leu Gly Val Asp Cys Trp Ile Asp Asn Thr Arg
   65                  70                  75                  80
   Ile Val Tyr Asn His Ser Ser Gly Arg Val Ser Asn Ala Pro Gly Val
   85 90 95
   Gln Ile Arg Val Pro Gly Phe Gly Lys Thr Glu Ser Val Glu Tyr Val
   100 105 110
   Asp Asp Asn Lys Leu Ala Gly Tyr Leu His Thr Leu Val Gln Asn Leu
   115 120 125
   Val Asn Asn Gly Tyr Val Arg Asp Glu Thr Val Arg Ala Ala Pro Tyr
   130 135 140
   Asp Trp Arg Leu Ala Pro His Gln Gln Asp Glu Tyr Tyr Lys Lys Leu
   145 150 155 160
   Ala Gly Leu Val Glu Glu Met Tyr Ala Ala Tyr Gly Lys Pro Val Phe
   165 170 175
   Leu Ile Gly His Ser Leu Gly Cys Leu His Val Leu His Phe Leu Leu
   180 185 190
   Arg Gln Pro Gln Ser Trp Lys Asp His Phe Ile Asp Gly Phe Ile Ser
   195 200 205
   Leu Gly Ala Pro Trp Gly Gly Ser Ile Lys Ala Met Arg Ile Leu Ala
   210 215 220
   Ser Gly Asp Asn Gln Gly Ile Pro Ile Leu Ser Asn Ile Lys Leu Lys
   225 230 235 240
   Glu Glu Gln Arg Ile Thr Thr Thr Ser Pro Trp Met Leu Pro Ala Pro
   245 250 255
   His Val Trp Pro Glu Asp His Val Phe Ile Ser Thr Pro Asn Phe Asn
   260 265 270
   Tyr Thr Val Gln Asp Phe Glu Arg Phe Phe Thr Asp Leu His Phe Glu
   275 280 285
   Glu Gly Trp His Met Phe Leu Gln Ser Arg Asp Leu Leu Glu Arg Leu
   290 295 300
   Pro Ala Pro Gly Val Glu Val Tyr Cys Leu Tyr Gly Val Gly Arg Pro
   305 310 315 320
   Thr Pro His Thr Tyr Ile Tyr Asp His Asn Phe Pro Tyr Lys Asp Pro
   325 330 335
   Val Ala Ala Leu Tyr Glu Asp Gly Asp Asp Thr Val Ala Thr Arg Ser
               340                 345                 350
   Thr Glu Leu Cys Gly Gln Trp Gln Gly Arg Gln Ser Gln Pro Val His
           355                 360                 365
   Leu Leu Pro Met Asn Glu Thr Asp His Leu Asn Met Val Phe Ser Asn
       370                 375                 380
   Lys Thr Leu Glu His Ile Asn Ala Ile Leu Leu Gly Ala Tyr Arg Thr
   385                 390                 395                 400
   Pro Lys Ser Pro Ala Ala Ser Pro Ser Pro Pro Pro Pro Glu
                   405                 410
<210> 5
   <211> 416
   <212> PRT
   <213> Oryctolagus cuniculus
<400> 5
   Phe Trp Leu Leu Asn Val Leu Phe Pro Pro His Thr Thr Pro Lys Ala
   1               5                   10                  15
   Glu Leu Ser Asn His Thr Arg Pro Val Ile Leu Val Pro Gly Cys Leu
               20                  25                  30
   Gly Asn Gln Leu Glu Ala Lys Leu Asp Lys Pro Ser Val Val Asn Trp
           35                  40                  45
   Met Cys Tyr Arg Lys Thr Glu Asp Phe Phe Thr Ile Trp Leu Asp Leu
       50                  55                  60
   Asn Met Phe Leu Pro Leu Gly Val Asp Cys Trp Ile Asp Asn Thr Arg
   65                  70                  75                  80
   Val Val Tyr Asn Arg Ser Ser Gly Arg Val Val Ile Ser Pro Gly Val
                   85                  90                  95
   Gln Ile Arg Val Pro Gly Phe Gly Lys Thr Tyr Ser Val Glu Tyr Leu
               100                 105                 110
   Asp Asn Asn Lys Leu Ala Gly Tyr Met His Thr Leu Val Gln Asn Leu
           115                 120                 125
   Val Asn Asn Gly Tyr Val Arg Asp Glu Thr Val Arg Ala Ala Pro Tyr
       130                 135                 140
   Asp Trp Arg Leu Glu Pro Ser Gln Gln Glu Glu Tyr Tyr Gly Lys Leu
   145 150 155 160
   Ala Gly Leu Val Glu Glu Met His Ala Ala Tyr Gly Lys Pro Val Phe
   165 170 175
   Leu Ile Gly His Ser Leu Gly Cys Leu His Leu Leu Tyr Phe Leu Leu
   180 185 190
   Arg Gln Pro Gln Ser Trp Lys Asp Arg Phe Ile Asp Gly Phe Ile Ser
   195 200 205
   Leu Gly Ala Pro Trp Gly Gly Ser Ile Lys Pro Met Leu Val Leu Ala
   210 215 220
   Ser Gly Asp Asn Gln Gly Ile Pro Leu Met Ser Ser Ile Lys Leu Arg
   225 230 235 240
   Glu Glu Gln Arg Ile Thr Thr Thr Ser Pro Trp Met Phe Pro Ser Gln
   245 250 255
   Gly Val Trp Pro Glu Asp His Val Phe Ile Ser Thr Pro Ser Phe Asn
   260 265 270
   Tyr Thr Gly Arg Asp Phe Lys Arg Phe Phe Glu Asp Leu His Phe Glu
   275 280 285
   Glu Gly Trp Tyr Met Trp Leu Gln Ser Arg Asp Leu Leu Ala Gly Leu
   290 295 300
   Pro Ala Pro Gly Val Glu Val Tyr Cys Leu Tyr Gly Ile Gly Leu Pro
   305 310 315 320
   Thr Pro His Thr Tyr Ile Tyr Asp His Gly Phe Pro Tyr Thr Asp Pro
   325 330 335
   Val Gly Val Leu Tyr Glu Asp Gly Asp Asp Thr Val Ala Thr Ser Ser
   340 345 350
   Thr Asp Leu Cys Gly Leu Trp Arg Gly Arg Gln Pro Gln Pro Val His
   355 360 365
   Leu Leu Pro Leu His Glu Thr Glu His Leu Asn Met Val Phe Ser Asn
   370 375 380
   Gln Thr Leu Glu His Ile Asn Ala Ile Leu Leu Gly Ala Tyr Arg Ser
   385 390 395 400
   Gly Thr Pro Ala Ser Pro Thr Ala Ser Pro Gly Ser Pro Pro Pro Glu
   405 410 415
<210> 6
   <211> 326
   <212> PRT
   <213> Artificial Sequence
<220>
   <221> source
   <223> /note="Description of Artificial Sequence: Synthetic polypeptide"
<400> 6
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Asn Phe Gly Thr Gln Thr
   65 70 75 80
   Tyr Thr Cys Asn Val Asp His Lys Pro Ser Asn Thr Lys Val Asp Lys
   85 90 95
   Thr Val Glu Arg Lys Cys Cys Val Glu Cys Pro Pro Cys Pro Ala Pro
   100 105 110
   Pro Val Ala Gly Pro Ser Val Phe Leu Phe Pro Pro Lys Pro Lys Asp
   115 120 125
   Thr Leu Met Ile Ser Arg Thr Pro Glu Val Thr Cys Val Val Val Asp
   130 135 140
   Val Ser His Glu Asp Pro Glu Val Gln Phe Asn Trp Tyr Val Asp Gly
   145 150 155 160
   Val Glu Val His Asn Ala Lys Thr Lys Pro Arg Glu Glu Gln Phe Asn
   165 170 175
   Ser Thr Phe Arg Val Val Ser Val Leu Thr Val Val His Gln Asp Trp
   180 185 190
   Leu Asn Gly Lys Glu Tyr Lys Cys Lys Val Ser Asn Lys Gly Leu Pro
   195 200 205
   Ala Pro Ile Glu Lys Thr Ile Ser Lys Thr Lys Gly Gln Pro Arg Glu
   210 215 220
   Pro Gln Val Tyr Thr Leu Pro Pro Ser Arg Glu Glu Met Thr Lys Asn
   225 230 235 240
   Gln Val Ser Leu Thr Cys Leu Val Lys Gly Phe Tyr Pro Ser Asp Ile
   245 250 255
   Ala Val Glu Trp Glu Ser Asn Gly Gln Pro Glu Asn Asn Tyr Lys Thr
   260 265 270
   Thr Pro Pro Met Leu Asp Ser Asp Gly Ser Phe Phe Leu Tyr Ser Lys
   275 280 285
   Leu Thr Val Asp Lys Ser Arg Trp Gln Gln Gly Asn Val Phe Ser Cys
   290 295 300
   Ser Val Met His Glu Ala Leu His Asn His Tyr Thr Gln Lys Ser Leu
   305 310 315 320
   Ser Leu Ser Pro Gly Lys
   325
<210> 7
   <211> 105
   <212> PRT
   <213> Artificial Sequence
<220>
   <221> source
   <223> /note="Description of Artificial Sequence: Synthetic polypeptide"
<400> 7
   Gln Pro Lys Ala Ala Pro Ser Val Thr Leu Phe Pro Pro Ser Ser Glu
   1 5 10 15
   Glu Leu Gln Ala Asn Lys Ala Thr Leu Val Cys Leu Ile Ser Asp Phe
   20 25 30
   Tyr Pro Gly Ala Val Thr Val Ala Trp Lys Ala Asp Ser Ser Pro Val
   35 40 45
   Lys Ala Gly Val Glu Thr Thr Thr Pro Ser Lys Gln Ser Asn Asn Lys
   50 55 60
   Tyr Ala Ala Ser Ser Tyr Leu Ser Leu Thr Pro Glu Gln Trp Lys Ser
   65 70 75 80
   His Arg Ser Tyr Ser Cys Gln Val Thr His Glu Gly Ser Thr Val Glu
   85 90 95
   Lys Thr Val Ala Pro Thr Glu Cys Ser
   100 105
<210> 8
   <211> 106
   <212> PRT
   <213> Artificial Sequence
<220>
   <221> source
   <223> /note="Description of Artificial Sequence: Synthetic polypeptide"
<400> 8
   Thr Val Ala Ala Pro Ser Val Phe Ile Phe Pro Pro Ser Asp Glu Gln
   1 5 10 15
   Leu Lys Ser Gly Thr Ala Ser Val Val Cys Leu Leu Asn Asn Phe Tyr
   20 25 30
   Pro Arg Glu Ala Lys Val Gln Trp Lys Val Asp Asn Ala Leu Gln Ser
   35 40 45
   Gly Asn Ser Gln Glu Ser Val Thr Glu Gln Asp Ser Lys Asp Ser Thr
   50 55 60
   Tyr Ser Leu Ser Ser Thr Leu Thr Leu Ser Lys Ala Asp Tyr Glu Lys
   65 70 75 80
   His Lys Val Tyr Ala Cys Glu Val Thr His Gln Gly Leu Ser Ser Pro
   85 90 95
   Val Thr Lys Ser Phe Asn Arg Gly Glu Cys
   100 105
<210> 9
   <211> 452
   <212> PRT
   <213> Artificial Sequence
<220>
   <221> source
   <223> /note="Description of Artificial Sequence: Synthetic polypeptide"
<400> 9
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 10
   <211> 452
   <212> PRT
   <213> Artificial Sequence
<220>
   <221> source
   <223> /note="Description of Artificial Sequence: Synthetic polypeptide"
<400> 10
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Gln Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 11
   <211> 452
   <212> PRT
   <213> Artificial Sequence
<220>
   <221> source
   <223> /note="Description of Artificial Sequence: Synthetic polypeptide"
<400> 11
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asp Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 12
   <211> 452
   <212> PRT
   <213> Artificial Sequence
<220>
   <221> source
   <223> /note="Description of Artificial Sequence: Synthetic polypeptide"
<400> 12
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Glu Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 13
   <211> 452
   <212> PRT
   <213> Artificial Sequence
<220>
   <221> source
   <223> /note="Description of Artificial Sequence: Synthetic polypeptide"
<400> 13
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Ala Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
       450
<210> 14
   <211> 452
   <212> PRT
   <213> Artificial Sequence
<220>
   <221> source
   <223> /note="Description of Artificial Sequence: Synthetic polypeptide"
<400> 14
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Ser Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
       450
<210> 15
   <211> 452
   <212> PRT
   <213> Artificial Sequence
<220>
   <221> source
   <223> /note="Description of Artificial Sequence: Synthetic polypeptide"
<400> 15
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Thr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
       450
<210> 16
   <211> 452
   <212> PRT
   <213> Artificial Sequence
<220>
   <221> source
   <223> /note="Description of Artificial Sequence: Synthetic polypeptide"
<400> 16
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Val Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 17
   <211> 452
   <212> PRT
   <213> Artificial Sequence
<220>
   <221> source
   <223> /note="Description of Artificial Sequence: Synthetic polypeptide"
<400> 17
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 18
   <211> 452
   <212> PRT
   <213> Artificial Sequence
<220>
   <221> source
   <223> /note="Description of Artificial Sequence: Synthetic polypeptide"
<400> 18
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Val Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 19
   <211> 452
   <212> PRT
   <213> Artificial Sequence
<220>
   <221> source
   <223> /note="Description of Artificial Sequence: Synthetic polypeptide"
<400> 19
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 20
   <211> 452
   <212> PRT
   <213> Artificial Sequence
<220>
   <221> source
   <223> /note="Description of Artificial Sequence: Synthetic polypeptide"
<400> 20
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Thr Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 21
   <211> 452
   <212> PRT
   <213> Artificial Sequence
<220>
   <221> source
   <223> /note="Description of Artificial Sequence: Synthetic polypeptide"
<400> 21
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Thr Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
       450
<210> 22
   <211> 452
   <212> PRT
   <213> Artificial Sequence
<220>
   <221> source
   <223> /note="Description of Artificial Sequence: Synthetic polypeptide"
<400> 22
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Ile
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
       450
<210> 23
   <211> 452
   <212> PRT
   <213> Artificial Sequence
<220>
   <221> source
   <223> /note="Description of Artificial Sequence: Synthetic polypeptide"
<400> 23
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Ile Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 24
   <211> 452
   <212> PRT
   <213> Artificial Sequence
<220>
   <221> source
   <223> /note="Description of Artificial Sequence: Synthetic polypeptide"
<400> 24
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Thr Cys Ser Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
               195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
   450
<210> 25
   <211> 452
   <212> PRT
   <213> Artificial Sequence
<220>
   <221> source
   <223> /note="Description of Artificial Sequence: Synthetic polypeptide"
<400> 25
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Thr Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
   450
<210> 26
   <211> 452
   <212> PRT
   <213> Artificial Sequence
<220>
   <221> source
   <223> /note="Description of Artificial Sequence: Synthetic polypeptide"
<400> 26
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Ile
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 27
   <211> 452
   <212> PRT
   <213> Artificial Sequence
<220>
   <221> source
   <223> /note="Description of Artificial Sequence: Synthetic polypeptide"
<400> 27
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Ile Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 28
   <211> 452
   <212> PRT
   <213> Artificial Sequence
<220>
   <221> source
   <223> /note="Description of Artificial Sequence: Synthetic polypeptide"
<400> 28
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Thr Cys Thr Arg Val
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 29
   <211> 452
   <212> PRT
   <213> Artificial Sequence
<220>
   <221> source
   <223> /note="Description of Artificial Sequence: Synthetic polypeptide"
<400> 29
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Ser Arg Ile
   100 105 110
   Met Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 30
   <211> 452
   <212> PRT
   <213> Artificial Sequence
<220>
   <221> source
   <223> /note="Description of Artificial Sequence: Synthetic polypeptide"
<400> 30
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Ser Arg Val
   100 105 110
   Ile Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
   435 440 445
   Ser Pro Gly Lys
   450
<210> 31
   <211> 452
   <212> PRT
   <213> Artificial Sequence
<220>
   <221> source
   <223> /note="Description of Artificial Sequence: Synthetic polypeptide"
<400> 31
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Thr Arg Ile
   100 105 110
   Met Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 32
   <211> 452
   <212> PRT
   <213> Artificial Sequence
<220>
   <221> source
   <223> /note="Description of Artificial Sequence: Synthetic polypeptide"
<400> 32
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Thr Arg Val
               100                 105                 110
   Ile Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 33
   <211> 452
   <212> PRT
   <213> Artificial Sequence
<220>
   <221> source
   <223> /note="Description of Artificial Sequence: Synthetic polypeptide"
<400> 33
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Ile
   100 105 110
   Ile Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 34
   <211> 452
   <212> PRT
   <213> Artificial Sequence
<220>
   <221> source
   <223> /note="Description of Artificial Sequence: Synthetic polypeptide"
<400> 34
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Thr Arg Ile
   100 105 110
   Met Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 35
   <211> 452
   <212> PRT
   <213> Artificial Sequence
<220>
   <221> source
   <223> /note="Description of Artificial Sequence: Synthetic polypeptide"
<400> 35
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Thr Arg Val
   100 105 110
   Ile Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Asn Phe Gly Thr Gln Thr Tyr Thr
           195                 200                 205
   Cys Asn Val Asp His Lys Pro Ser Asn Thr Lys Val Asp Lys Thr Val
       210                 215                 220
   Glu Arg Lys Cys Cys Val Glu Cys Pro Pro Cys Pro Ala Pro Pro Val
   225                 230                 235                 240
   Ala Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Gln Phe Asn Trp Tyr Val Asp Gly Val Glu
           275                 280                 285
   Val His Asn Ala Lys Thr Lys Pro Arg Glu Glu Gln Phe Asn Ser Thr
       290                 295                 300
   Phe Arg Val Val Ser Val Leu Thr Val Val His Gln Asp Trp Leu Asn
   305                 310                 315                 320
   Gly Lys Glu Tyr Lys Cys Lys Val Ser Asn Lys Gly Leu Pro Ala Pro
                   325                 330                 335
   Ile Glu Lys Thr Ile Ser Lys Thr Lys Gly Gln Pro Arg Glu Pro Gln
               340                 345                 350
   Val Tyr Thr Leu Pro Pro Ser Arg Glu Glu Met Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 36
   <211> 452
   <212> PRT
   <213> Artificial Sequence
<220>
   <221> source
   <223> /note="Description of Artificial Sequence: Synthetic polypeptide"
<400> 36
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Thr Cys Ser Arg Ile
               100                 105                 110
   Ile Asp Val Trp Gly Gln Gly Thr Thr Val Thr Val Ser Ser Ala Ser
           115                 120                 125
   Thr Lys Gly Pro Ser Val Phe Pro Leu Ala Pro Cys Ser Arg Ser Thr
       130                 135                 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 37
   <211> 452
   <212> PRT
   <213> Artificial Sequence
<220>
   <221> source
   <223> /note="Description of Artificial Sequence: Synthetic polypeptide"
<400> 37
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Thr Arg Ile
   100 105 110
   Ile Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 38
   <211> 452
   <212> PRT
   <213> Artificial Sequence
<220>
   <221> source
   <223> /note="Description of Artificial Sequence: Synthetic polypeptide"
<400> 38
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Thr Arg Ile
   100 105 110
   Ile Asp Val Trp Gly Gln Gly Thr Thr Val Thr Val Ser Ser Ala Ser
   115 120 125
   Thr Lys Gly Pro Ser Val Phe Pro Leu Ala Pro Cys Ser Arg Ser Thr
   130 135 140
   Ser Glu Ser Thr Ala Ala Leu Gly Cys Leu Val Lys Asp Tyr Phe Pro
   145 150 155 160
   Glu Pro Val Thr Val Ser Trp Asn Ser Gly Ala Leu Thr Ser Gly Val
   165 170 175
   His Thr Phe Pro Ala Val Leu Gln Ser Ser Gly Leu Tyr Ser Leu Ser
   180 185 190
   Ser Val Val Thr Val Pro Ser Ser Asn Phe Gly Thr Gln Thr Tyr Thr
   195 200 205
   Cys Asn Val Asp His Lys Pro Ser Asn Thr Lys Val Asp Lys Thr Val
   210 215 220
   Glu Arg Lys Cys Cys Val Glu Cys Pro Pro Cys Pro Ala Pro Pro Val
   225 230 235 240
   Ala Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Gln Phe Asn Trp Tyr Val Asp Gly Val Glu
   275 280 285
   Val His Asn Ala Lys Thr Lys Pro Arg Glu Glu Gln Phe Asn Ser Thr
   290 295 300
   Phe Arg Val Val Ser Val Leu Thr Val Val His Gln Asp Trp Leu Asn
   305 310 315 320
   Gly Lys Glu Tyr Lys Cys Lys Val Ser Asn Lys Gly Leu Pro Ala Pro
   325 330 335
   Ile Glu Lys Thr Ile Ser Lys Thr Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Met Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 39
   <211> 445
   <212> PRT
   <213> Artificial Sequence
<220>
   <221> source
   <223> /note="Description of Artificial Sequence: Synthetic polypeptide"
<400> 39
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Asn Arg Leu Arg Ser Asp Asp Thr Ala Val Tyr Tyr Cys
   85 90 95
   Ala Arg Gly Arg Trp Glu Leu Tyr Ala Phe Asp Ile Trp Gly Gln Gly
   100 105 110
   Thr Met Val Thr Val Ser Ser Ala Ser Thr Lys Gly Pro Ser Val Phe
   115 120 125
   Pro Leu Ala Pro Cys Ser Arg Ser Thr Ser Glu Ser Thr Ala Ala Leu
   130 135 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Asn Phe Gly Thr Gln Thr Tyr Thr Cys Asn Val Asp His Lys Pro
           195                 200                 205
   Ser Asn Thr Lys Val Asp Lys Thr Val Glu Arg Lys Cys Cys Val Glu
       210                 215                 220
   Cys Pro Pro Cys Pro Ala Pro Pro Val Ala Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Gln
               260                 265                 270
   Phe Asn Trp Tyr Val Asp Gly Val Glu Val His Asn Ala Lys Thr Lys
           275                 280                 285
   Pro Arg Glu Glu Gln Phe Asn Ser Thr Phe Arg Val Val Ser Val Leu
       290                 295                 300
   Thr Val Val His Gln Asp Trp Leu Asn Gly Lys Glu Tyr Lys Cys Lys
   305                 310                 315                 320
   Val Ser Asn Lys Gly Leu Pro Ala Pro Ile Glu Lys Thr Ile Ser Lys
                   325                 330                 335
   Thr Lys Gly Gln Pro Arg Glu Pro Gln Val Tyr Thr Leu Pro Pro Ser
               340                 345                 350
   Arg Glu Glu Met Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Met Leu Asp Ser Asp Gly
   385 390 395 400
   Ser Phe Phe Leu Tyr Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Lys
   435 440 445
<210> 40
   <211> 214
   <212> PRT
   <213> Artificial Sequence
<220>
   <221> source
   <223> /note="Description of Artificial Sequence: Synthetic polypeptide"
<400> 40
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Asn Ser Arg Asp Asn Ile Gly Asn His
                   85                  90                  95
   Gln Val Phe Gly Gly Gly Thr Lys Leu Thr Val Leu Gly Gln Pro Lys
               100                 105                 110
   Ala Ala Pro Ser Val Thr Leu Phe Pro Pro Ser Ser Glu Glu Leu Gln
           115                 120                 125
   Ala Asn Lys Ala Thr Leu Val Cys Leu Ile Ser Asp Phe Tyr Pro Gly
       130                 135                 140
   Ala Val Thr Val Ala Trp Lys Ala Asp Ser Ser Pro Val Lys Ala Gly
   145                 150                 155                 160
   Val Glu Thr Thr Thr Pro Ser Lys Gln Ser Asn Asn Lys Tyr Ala Ala
                   165                 170                 175
   Ser Ser Tyr Leu Ser Leu Thr Pro Glu Gln Trp Lys Ser His Arg Ser
               180                 185                 190
   Tyr Ser Cys Gln Val Thr His Glu Gly Ser Thr Val Glu Lys Thr Val
           195                 200                 205
   Ala Pro Thr Glu Cys Ser
       210
<210> 41
   <211> 214
   <212> PRT
   <213> Artificial Sequence
<220>
   <221> source
   <223> /note="Description of Artificial Sequence: Synthetic polypeptide"
<400> 41
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Gly Ser Arg Asp Asn Ile Gly Asn His
                   85                  90                  95
   Gln Val Phe Gly Gly Gly Thr Lys Leu Thr Val Leu Gly Gln Pro Lys
               100                 105                 110
   Ala Ala Pro Ser Val Thr Leu Phe Pro Pro Ser Ser Glu Glu Leu Gln
           115                 120                 125
   Ala Asn Lys Ala Thr Leu Val Cys Leu Ile Ser Asp Phe Tyr Pro Gly
   130 135 140
   Ala Val Thr Val Ala Trp Lys Ala Asp Ser Ser Pro Val Lys Ala Gly
   145 150 155 160
   Val Glu Thr Thr Thr Pro Ser Lys Gln Ser Asn Asn Lys Tyr Ala Ala
   165 170 175
   Ser Ser Tyr Leu Ser Leu Thr Pro Glu Gln Trp Lys Ser His Arg Ser
   180 185 190
   Tyr Ser Cys Gln Val Thr His Glu Gly Ser Thr Val Glu Lys Thr Val
   195 200 205
   Ala Pro Thr Glu Cys Ser
   210
<210> 42
   <211> 214
   <212> PRT
   <213> Artificial Sequence
<220>
   <221> source
   <223> /note="Description of Artificial Sequence: Synthetic polypeptide"
<400> 42
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Asn Ser Arg Asp Asn Val Gly Asn His
                   85                  90                  95
   Gln Val Phe Gly Gly Gly Thr Lys Leu Thr Val Leu Gly Gln Pro Lys
               100                 105                 110
   Ala Ala Pro Ser Val Thr Leu Phe Pro Pro Ser Ser Glu Glu Leu Gln
           115                 120                 125
   Ala Asn Lys Ala Thr Leu Val Cys Leu Ile Ser Asp Phe Tyr Pro Gly
       130                 135                 140
   Ala Val Thr Val Ala Trp Lys Ala Asp Ser Ser Pro Val Lys Ala Gly
   145                 150                 155                 160
   Val Glu Thr Thr Thr Pro Ser Lys Gln Ser Asn Asn Lys Tyr Ala Ala
                   165                 170                 175
   Ser Ser Tyr Leu Ser Leu Thr Pro Glu Gln Trp Lys Ser His Arg Ser
               180                 185                 190
   Tyr Ser Cys Gln Val Thr His Glu Gly Ser Thr Val Glu Lys Thr Val
           195                 200                 205
   Ala Pro Thr Glu Cys Ser
       210
<210> 43
   <211> 214
   <212> PRT
   <213> Artificial Sequence
<220>
   <221> source
   <223> /note="Description of Artificial Sequence: Synthetic polypeptide"
<400> 43
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Gly Ser Arg Asp Asn Val Gly Asn His
                   85                  90                  95
   Gln Val Phe Gly Gly Gly Thr Lys Leu Thr Val Leu Gly Gln Pro Lys
                   100                 105                 110
   Ala Ala Pro Ser Val Thr Leu Phe Pro Pro Ser Ser Glu Glu Leu Gln
           115                 120                 125
   Ala Asn Lys Ala Thr Leu Val Cys Leu Ile Ser Asp Phe Tyr Pro Gly
       130                 135                 140
   Ala Val Thr Val Ala Trp Lys Ala Asp Ser Ser Pro Val Lys Ala Gly
   145                 150                 155                 160
   Val Glu Thr Thr Thr Pro Ser Lys Gln Ser Asn Asn Lys Tyr Ala Ala
                   165                 170                 175
   Ser Ser Tyr Leu Ser Leu Thr Pro Glu Gln Trp Lys Ser His Arg Ser
               180                 185                 190
   Tyr Ser Cys Gln Val Thr His Glu Gly Ser Thr Val Glu Lys Thr Val
           195                 200                 205
   Ala Pro Thr Glu Cys Ser
       210
<210> 44
   <211> 216
   <212> PRT
   <213> Artificial Sequence
<220>
   <221> source
   <223> /note="Description of Artificial Sequence: Synthetic polypeptide"
<400> 44
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Gly Ser
               20                  25                  30
   Tyr Leu Thr Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Met Tyr Tyr Cys Gln Gln Tyr Gly Gly Ser Pro
                   85                  90                  95
   Pro Phe Thr Phe Gly Pro Gly Thr Lys Val Asp Ile Lys Arg Thr Val
               100                 105                 110
   Ala Ala Pro Ser Val Phe Ile Phe Pro Pro Ser Asp Glu Gln Leu Lys
           115                 120                 125
   Ser Gly Thr Ala Ser Val Val Cys Leu Leu Asn Asn Phe Tyr Pro Arg
       130                 135                 140
   Glu Ala Lys Val Gln Trp Lys Val Asp Asn Ala Leu Gln Ser Gly Asn
   145                 150                 155                 160
   Ser Gln Glu Ser Val Thr Glu Gln Asp Ser Lys Asp Ser Thr Tyr Ser
                   165                 170                 175
   Leu Ser Ser Thr Leu Thr Leu Ser Lys Ala Asp Tyr Glu Lys His Lys
               180                 185                 190
   Val Tyr Ala Cys Glu Val Thr His Gln Gly Leu Ser Ser Pro Val Thr
           195                 200                 205
   Lys Ser Phe Asn Arg Gly Glu Cys
       210                 215
<210> 45
   <211> 126
   <212> PRT
   <213> Artificial Sequence
<220>
   <221> source
   <223> /note="Description of Artificial Sequence: Synthetic polypeptide"
<400> 45
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser
           115                 120                 125
<210> 46
   <211> 126
   <212> PRT
   <213> Artificial Sequence
<220>
   <221> source
   <223> /note="Description of Artificial Sequence: Synthetic polypeptide"
<400> 46
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Gln Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser
           115                 120                 125
<210> 47
   <211> 126
   <212> PRT
   <213> Artificial Sequence
<220>
   <221> source
   <223> /note="Description of Artificial Sequence: Synthetic polypeptide"
<400> 47
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asp Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser
   115 120 125
<210> 48
   <211> 126
   <212> PRT
   <213> Artificial Sequence
<220>
   <221> source
   <223> /note="Description of Artificial Sequence: Synthetic polypeptide"
<400> 48
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Glu Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser
   115 120 125
<210> 49
   <211> 126
   <212> PRT
   <213> Artificial Sequence
<220>
   <221> source
   <223> /note="Description of Artificial Sequence: Synthetic polypeptide"
<400> 49
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Ala Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser
   115 120 125
<210> 50
   <211> 126
   <212> PRT
   <213> Artificial Sequence
<220>
   <221> source
   <223> /note="Description of Artificial Sequence: Synthetic polypeptide"
<400> 50
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Ser Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser
           115                 120                 125
<210> 51
   <211> 126
   <212> PRT
   <213> Artificial Sequence
<220>
   <221> source
   <223> /note="Description of Artificial Sequence: Synthetic polypeptide"
<400> 51
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Thr Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser
   115 120 125
<210> 52
   <211> 126
   <212> PRT
   <213> Artificial Sequence
<220>
   <221> source
   <223> /note="Description of Artificial Sequence: Synthetic polypeptide"
<400> 52
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Val Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser
           115                 120                 125
<210> 53
   <211> 126
   <212> PRT
   <213> Artificial Sequence
<220>
   <221> source
   <223> /note="Description of Artificial Sequence: Synthetic polypeptide"
<400> 53
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Tyr Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Val
               100                 105                 110
   Met Asp Val Trp Gly Gln Gly Thr Thr Val Thr Val Ser Ser
           115                 120                 125
<210> 54
   <211> 126
   <212> PRT
   <213> Artificial Sequence
<220>
   <221> source
   <223> /note="Description of Artificial Sequence: Synthetic polypeptide"
<400> 54
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Val Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser
   115 120 125
<210> 55
   <211> 126
   <212> PRT
   <213> Artificial Sequence
<220>
   <221> source
   <223> /note="Description of Artificial Sequence: Synthetic polypeptide"
<400> 55
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Met Asp Val Trp Gly Gln Gly Thr Thr Val Thr Val Ser Ser
   115 120 125
<210> 56
   <211> 126
   <212> PRT
   <213> Artificial Sequence
<220>
   <221> source
   <223> /note="Description of Artificial Sequence: Synthetic polypeptide"
<400> 56
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Ser Arg Val
   100 105 110
   Met Asp Val Trp Gly Gln Gly Thr Thr Val Thr Val Ser Ser
   115 120 125
<210> 57
   <211> 126
   <212> PRT
   <213> Artificial Sequence
<220>
   <221> source
   <223> /note="Description of Artificial Sequence: Synthetic polypeptide"
<400> 57
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Thr Arg Val
   100 105 110
   Met Asp Val Trp Gly Gln Gly Thr Thr Val Thr Val Ser Ser
   115 120 125
<210> 58
   <211> 126
   <212> PRT
   <213> Artificial Sequence
<220>
   <221> source
   <223> /note="Description of Artificial Sequence: Synthetic polypeptide"
<400> 58
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ser Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Ile
                   100                 105                 110
   Met Asp Val Trp Gly Gln Gly Thr Thr Val Thr Val Ser Ser
           115                 120                 125
<210> 59
   <211> 126
   <212> PRT
   <213> Artificial Sequence
<220>
   <221> source
   <223> /note="Description of Artificial Sequence: Synthetic polypeptide"
<400> 59
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ser Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Ile Asp Val Trp Gly Gln Gly Thr Thr Val Thr Val Ser Ser
   115 120 125
<210> 60
   <211> 126
   <212> PRT
   <213> Artificial Sequence
<220>
   <221> source
   <223> /note="Description of Artificial Sequence: Synthetic polypeptide"
<400> 60
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Ser Arg Val
   100 105 110
   Met Asp Val Trp Gly Gln Gly Thr Thr Val Thr Val Ser Ser
   115 120 125
<210> 61
   <211> 126
   <212> PRT
   <213> Artificial Sequence
<220>
   <221> source
   <223> /note="Description of Artificial Sequence: Synthetic polypeptide"
<400> 61
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Thr Arg Val
   100 105 110
   Met Asp Val Trp Gly Gln Gly Thr Thr Val Thr Val Ser Ser
   115 120 125
<210> 62
   <211> 126
   <212> PRT
   <213> Artificial Sequence
<220>
   <221> source
   <223> /note="Description of Artificial Sequence: Synthetic polypeptide"
<400> 62
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Ile
   100 105 110
   Met Asp Val Trp Gly Gln Gly Thr Thr Val Thr Val Ser Ser
   115 120 125
<210> 63
   <211> 126
   <212> PRT
   <213> Artificial Sequence
<220>
   <221> source
   <223> /note="Description of Artificial Sequence: Synthetic polypeptide"
<400> 63
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Ser Arg Val
   100 105 110
   Ile Asp Val Trp Gly Gln Gly Thr Thr Val Thr Val Ser Ser
   115 120 125
<210> 64
   <211> 126
   <212> PRT
   <213> Artificial Sequence
<220>
   <221> source
   <223> /note="Description of Artificial Sequence: Synthetic polypeptide"
<400> 64
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Thr Arg Val
   100 105 110
   Met Asp Val Trp Gly Gln Gly Thr Thr Val Thr Val Ser Ser
   115 120 125
<210> 65
   <211> 126
   <212> PRT
   <213> Artificial Sequence
<220>
   <221> source
   <223> /note="Description of Artificial Sequence: Synthetic polypeptide"
<400> 65
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Ser Arg Ile
   100 105 110
   Met Asp Val Trp Gly Gln Gly Thr Thr Val Thr Val Ser Ser
   115 120 125
<210> 66
   <211> 126
   <212> PRT
   <213> Artificial Sequence
<220>
   <221> source
   <223> /note="Description of Artificial Sequence: Synthetic polypeptide"
<400> 66
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Thr Cys Ser Arg Val
               100                 105                 110
   Ile Asp Val Trp Gly Gln Gly Thr Thr Val Thr Val Ser Ser
           115                 120                 125
<210> 67
   <211> 126
   <212> PRT
   <213> Artificial Sequence
<220>
   <221> source
   <223> /note="Description of Artificial Sequence: Synthetic polypeptide"
<400> 67
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Thr Arg Ile
               100                 105                 110
   Met Asp Val Trp Gly Gln Gly Thr Thr Val Thr Val Ser Ser
           115                 120                 125
<210> 68
   <211> 126
   <212> PRT
   <213> Artificial Sequence
<220>
   <221> source
   <223> /note="Description of Artificial Sequence: Synthetic polypeptide"
<400> 68
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Thr Arg Val
               100                 105                 110
   Ile Asp Val Trp Gly Gln Gly Thr Thr Val Thr Val Ser Ser
           115                 120                 125
<210> 69
   <211> 126
   <212> PRT
   <213> Artificial Sequence
<220>
   <221> source
   <223> /note="Description of Artificial Sequence: Synthetic polypeptide"
<400> 69
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Ser Cys Ser Arg Ile
               100                 105                 110
   Ile Asp Val Trp Gly Gln Gly Thr Thr Val Thr Val Ser Ser
           115                 120                 125
<210> 70
   <211> 126
   <212> PRT
   <213> Artificial Sequence
<220>
   <221> source
   <223> /note="Description of Artificial Sequence: Synthetic polypeptide"
<400> 70
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Thr Arg Ile
   100 105 110
   Met Asp Val Trp Gly Gln Gly Thr Thr Val Thr Val Ser Ser
   115 120 125
<210> 71
   <211> 126
   <212> PRT
   <213> Artificial Sequence
<220>
   <221> source
   <223> /note="Description of Artificial Sequence: Synthetic polypeptide"
<400> 71
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Thr Arg Val
   100 105 110
   Ile Asp Val Trp Gly Gln Gly Thr Thr Val Thr Val Ser Ser
   115 120 125
<210> 72
   <211> 126
   <212> PRT
   <213> Artificial Sequence
<220>
   <221> source
   <223> /note="Description of Artificial Sequence: Synthetic polypeptide"
<400> 72
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Ala Ser Ile Ser Ser Gly
               20                  25                  30
   Gly Tyr Asn Trp Ala Trp Ile Arg Gln His Pro Gly Lys Gly Leu Glu
           35                  40                  45
   Trp Ile Gly Tyr Ile Tyr Tyr Ser Gly Ser Thr Tyr Tyr Asn Pro Ser
       50                  55                  60
   Leu Lys Ser Arg Val Thr Ile Ser Val Asp Thr Ser Lys Asn Gln Phe
   65                  70                  75                  80
   Ser Leu Lys Leu Ser Ser Val Thr Ala Ala Asp Thr Ala Val Tyr Tyr
                   85                  90                  95
   Cys Ala Arg Glu Arg Gly Tyr Cys Ser Ser Thr Thr Cys Ser Arg Ile
               100                 105                 110
   Ile Asp Val Trp Gly Gln Gly Thr Thr Val Thr Val Ser Ser
           115                 120                 125
<210> 73
   <211> 126
   <212> PRT
   <213> Artificial Sequence
<220>
   <221> source
   <223> /note="Description of Artificial Sequence: Synthetic polypeptide"
<400> 73
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Ser Cys Thr Arg Ile
   100 105 110
   Ile Asp Val Trp Gly Gln Gly Thr Thr Val Thr Val Ser Ser
   115 120 125
<210> 74
   <211> 126
   <212> PRT
   <213> Artificial Sequence
<220>
   <221> source
   <223> /note="Description of Artificial Sequence: Synthetic polypeptide"
<400> 74
   Gln Val Gln Leu Gln Glu Ser Gly Pro Gly Leu Val Lys Pro Ser Gln
   1 5 10 15
   Thr Leu Ser Leu Thr Cys Thr Val Ser Gly Ala Ser Ile Ser Ser Gly
   20 25 30
   Gly Tyr Asn Trp Ala Trp Ile Arg Gln His Pro Gly Lys Gly Leu Glu
   35 40 45
   Trp Ile Gly Tyr Ile Tyr Tyr Ser Gly Ser Thr Tyr Tyr Asn Pro Ser
   50 55 60
   Leu Lys Ser Arg Val Thr Ile Ser Val Asp Thr Ser Lys Asn Gln Phe
   65 70 75 80
   Ser Leu Lys Leu Ser Ser Val Thr Ala Ala Asp Thr Ala Val Tyr Tyr
   85 90 95
   Cys Ala Arg Glu Arg Gly Tyr Cys Ser Ser Thr Thr Cys Thr Arg Ile
   100 105 110
   Ile Asp Val Trp Gly Gln Gly Thr Thr Val Thr Val Ser Ser
   115 120 125
<210> 75
   <211> 119
   <212> PRT
   <213> Artificial Sequence
<220>
   <221> source
   <223> /note="Description of Artificial Sequence: Synthetic polypeptide"
<400> 75
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Asn Arg Leu Arg Ser Asp Asp Thr Ala Val Tyr Tyr Cys
                   85                  90                  95
   Ala Arg Gly Arg Trp Glu Leu Tyr Ala Phe Asp Ile Trp Gly Gln Gly
               100                 105                 110
   Thr Met Val Thr Val Ser Ser
           115
<210> 76
   <211> 109
   <212> PRT
   <213> Artificial Sequence
<220>
   <221> source
   <223> /note="Description of Artificial Sequence: Synthetic polypeptide"
<400> 76
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Asn Ser Arg Asp Asn Ile Gly Asn His
   85 90 95
   Gln Val Phe Gly Gly Gly Thr Lys Leu Thr Val Leu Gly
   100 105
<210> 77
   <211> 109
   <212> PRT
   <213> Artificial Sequence
<220>
   <221> source
   <223> /note="Description of Artificial Sequence: Synthetic polypeptide"
<400> 77
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Ser Ser Gly Asn Thr Ala Ser Leu Thr Ile Thr Gly Ala Gln Ala Glu
   65 70 75 80
   Asp Glu Ala Asp Tyr Tyr Cys Gly Ser Arg Asp Asn Ile Gly Asn His
   85 90 95
   Gln Val Phe Gly Gly Gly Thr Lys Leu Thr Val Leu Gly
   100 105
<210> 78
   <211> 109
   <212> PRT
   <213> Artificial Sequence
<220>
   <221> source
   <223> /note="Description of Artificial Sequence: Synthetic polypeptide"
<400> 78
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Asn Ser Arg Asp Asn Val Gly Asn His
                   85                  90                  95
   Gln Val Phe Gly Gly Gly Thr Lys Leu Thr Val Leu Gly
               100                 105
<210> 79
   <211> 109
   <212> PRT
   <213> Artificial Sequence
<220>
   <221> source
   <223> /note="Description of Artificial Sequence: Synthetic polypeptide"
<400> 79
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Ser Ser Gly Asn Thr Ala Ser Leu Thr Ile Thr Gly Ala Gln Ala Glu
   65                  70                  75                  80
   Asp Glu Ala Asp Tyr Tyr Cys Gly Ser Arg Asp Asn Val Gly Asn His
                   85                  90                  95
   Gln Val Phe Gly Gly Gly Thr Lys Leu Thr Val Leu Gly
               100                 105
<210> 80
   <211> 110
   <212> PRT
   <213> Artificial Sequence
<220>
   <221> source
   <223> /note="Description of Artificial Sequence: Synthetic polypeptide"
<400> 80
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Gly Ser
               20                  25                  30
   Tyr Leu Thr Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Met Tyr Tyr Cys Gln Gln Tyr Gly Gly Ser Pro
                   85                  90                  95
   Pro Phe Thr Phe Gly Pro Gly Thr Lys Val Asp Ile Lys Arg
               100                 105                 110
<210> 81
   <211> 7
   <212> PRT
   <213> Artificial Sequence
<220>
   <221> source
   <223> /note="Description of Artificial Sequence: Synthetic peptide"
<400> 81
   Ser Gly Gly Tyr Asn Trp Ser
       1               5
<210> 82
   <211> 7
   <212> PRT
   <213> Artificial Sequence
<220>
   <221> source
   <223> /note="Description of Artificial Sequence: Synthetic peptide"
<400> 82
   Ser Gly Gly Tyr Gln Trp Ser
   1               5
<210> 83
   <211> 7
   <212> PRT
   <213> Artificial Sequence
<220>
   <221> source
   <223> /note="Description of Artificial Sequence: Synthetic peptide"
<400> 83
   Ser Gly Gly Tyr Asp Trp Ser
   1               5
<210> 84
   <211> 7
   <212> PRT
   <213> Artificial Sequence
<220>
   <221> source
   <223> /note="Description of Artificial Sequence: Synthetic peptide"
<400> 84
   Ser Gly Gly Tyr Glu Trp Ser
   1               5
<210> 85
   <211> 7
   <212> PRT
   <213> Artificial Sequence
<220>
   <221> source
   <223> /note="Description of Artificial Sequence: Synthetic peptide"
<400> 85
   Ser Gly Gly Tyr Ala Trp Ser
   1               5
<210> 86
   <211> 7
   <212> PRT
   <213> Artificial Sequence
<220>
   <221> source
   <223> /note="Description of Artificial Sequence: Synthetic peptide"
<400> 86
   Ser Gly Gly Tyr Ser Trp Ser
   1               5
<210> 87
   <211> 7
   <212> PRT
   <213> Artificial Sequence
<220>
   <221> source
   <223> /note="Description of Artificial Sequence: Synthetic peptide"
<400> 87
   Ser Gly Gly Tyr Thr Trp Ser
   1               5
<210> 88
   <211> 7
   <212> PRT
   <213> Artificial Sequence
<220>
   <221> source
   <223> /note="Description of Artificial Sequence: Synthetic peptide"
<400> 88
   Ser Gly Gly Tyr Val Trp Ser
   1               5
<210> 89
   <211> 7
   <212> PRT
   <213> Artificial Sequence
<220>
   <221> source
   <223> /note="Description of Artificial Sequence: Synthetic peptide"
<400> 89
   Ser Gly Gly Tyr Tyr Trp Ser
   1               5
<210> 90
   <211> 7
   <212> PRT
   <213> Artificial Sequence
<220>
   <221> source
   <223> /note="Description of Artificial Sequence: Synthetic peptide"
<400> 90
   Ser Gly Gly Tyr Asn Trp Val
   1               5
<210> 91
   <211> 7
   <212> PRT
   <213> Artificial Sequence
<220>
   <221> source
   <223> /note="Description of Artificial Sequence: Synthetic peptide"
<400> 91
   Ser Gly Gly Tyr Asn Trp Ala
   1 5
<210> 92
   <211> 5
   <212> PRT
   <213> Artificial Sequence
<220>
   <221> source
   <223> /note="Description of Artificial Sequence: Synthetic peptide"
<400> 92
   Gly Tyr Tyr Met His
   1 5
<210> 93
   <211> 16
   <212> PRT
   <213> Artificial Sequence
<220>
   <221> source
   <223> /note="Description of Artificial Sequence: Synthetic peptide"
<400> 93
   Tyr Ile Tyr Tyr Ser Gly Ser Thr Tyr Tyr Asn Pro Ser Leu Lys Ser
   1 5 10 15
<210> 94
   <211> 17
   <212> PRT
   <213> Artificial Sequence
<220>
   <221> source
   <223> /note="Description of Artificial Sequence: Synthetic peptide"
<400> 94
   Trp Ile Asn Pro Asn Ser Gly Gly Thr Asn Tyr Ala Gln Lys Phe Gln
   1 5 10 15
   Gly
<210> 95
   <211> 16
   <212> PRT
   <213> Artificial Sequence
<220>
   <221> source
   <223> /note="Description of Artificial Sequence: Synthetic peptide"
<400> 95
   Glu Arg Gly Tyr Cys Ser Ser Thr Ser Cys Ser Arg Val Met Asp Val
   1 5 10 15
<210> 96
   <211> 16
   <212> PRT
   <213> Artificial Sequence
<220>
   <221> source
   <223> /note="Description of Artificial Sequence: Synthetic peptide"
<400> 96
   Glu Arg Gly Tyr Cys Ser Ser Thr Thr Cys Ser Arg Val Met Asp Val
   1 5 10 15
<210> 97
   <211> 16
   <212> PRT
   <213> Artificial Sequence
<220>
   <221> source
   <223> /note="Description of Artificial Sequence: Synthetic peptide"
<400> 97
   Glu Arg Gly Tyr Cys Ser Ser Thr Ser Cys Thr Arg Val Met Asp Val
   1 5 10 15
<210> 98
   <211> 16
   <212> PRT
   <213> Artificial Sequence
<220>
   <221> source
   <223> /note="Description of Artificial Sequence: Synthetic peptide"
<400> 98
   Glu Arg Gly Tyr Cys Ser Ser Thr Ser Cys Ser Arg Ile Met Asp Val
   1 5 10 15
<210> 99
   <211> 16
   <212> PRT
   <213> Artificial Sequence
<220>
   <221> source
   <223> /note="Description of Artificial Sequence: Synthetic peptide"
<400> 99
   Glu Arg Gly Tyr Cys Ser Ser Thr Ser Cys Ser Arg Val Ile Asp Val
   1               5                   10                  15
<210> 100
   <211> 16
   <212> PRT
   <213> Artificial Sequence
<220>
   <221> source
   <223> /note="Description of Artificial Sequence: Synthetic peptide"
<400> 100
   Glu Arg Gly Tyr Cys Ser Ser Thr Thr Cys Thr Arg Val Met Asp Val
   1               5                   10                  15
<210> 101
   <211> 16
   <212> PRT
   <213> Artificial Sequence
<220>
   <221> source
   <223> /note="Description of Artificial Sequence: Synthetic peptide"
<400> 101
   Glu Arg Gly Tyr Cys Ser Ser Thr Thr Cys Ser Arg Ile Met Asp Val
   1               5                   10                  15
<210> 102
   <211> 16
   <212> PRT
   <213> Artificial Sequence
<220>
   <221> source
   <223> /note="Description of Artificial Sequence: Synthetic peptide"
<400> 102
   Glu Arg Gly Tyr Cys Ser Ser Thr Thr Cys Ser Arg Val Ile Asp Val
   1               5                   10                  15
<210> 103
   <211> 16
   <212> PRT
   <213> Artificial Sequence
<220>
   <221> source
   <223> /note="Description of Artificial Sequence: Synthetic peptide"
<400> 103
   Glu Arg Gly Tyr Cys Ser Ser Thr Ser Cys Thr Arg Ile Met Asp Val
   1 5 10 15
<210> 104
   <211> 16
   <212> PRT
   <213> Artificial Sequence
<220>
   <221> source
   <223> /note="Description of Artificial Sequence: Synthetic peptide"
<400> 104
   Glu Arg Gly Tyr Cys Ser Ser Thr Ser Cys Thr Arg Val Ile Asp Val
   1 5 10 15
<210> 105
   <211> 16
   <212> PRT
   <213> Artificial Sequence
<220>
   <221> source
   <223> /note="Description of Artificial Sequence: Synthetic peptide"
<400> 105
   Glu Arg Gly Tyr Cys Ser Ser Thr Ser Cys Ser Arg Ile Ile Asp Val
   1 5 10 15
<210> 106
   <211> 16
   <212> PRT
   <213> Artificial Sequence
<220>
   <221> source
   <223> /note="Description of Artificial Sequence: Synthetic peptide"
<400> 106
   Glu Arg Gly Tyr Cys Ser Ser Thr Thr Cys Thr Arg Ile Met Asp Val
   1 5 10 15
<210> 107
   <211> 16
   <212> PRT
   <213> Artificial Sequence
<220>
   <221> source
   <223> /note="Description of Artificial Sequence: Synthetic peptide"
<400> 107
   Glu Arg Gly Tyr Cys Ser Ser Thr Thr Cys Thr Arg Val Ile Asp Val
   1 5 10 15
<210> 108
   <211> 16
   <212> PRT
   <213> Artificial Sequence
<220>
   <221> source
   <223> /note="Description of Artificial Sequence: Synthetic peptide"
<400> 108
   Glu Arg Gly Tyr Cys Ser Ser Thr Thr Cys Ser Arg Ile Ile Asp Val
   1 5 10 15
<210> 109
   <211> 16
   <212> PRT
   <213> Artificial Sequence
<220>
   <221> source
   <223> /note="Description of Artificial Sequence: Synthetic peptide"
<400> 109
   Glu Arg Gly Tyr Cys Ser Ser Thr Ser Cys Thr Arg Ile Ile Asp Val
   1 5 10 15
<210> 110
   <211> 16
   <212> PRT
   <213> Artificial Sequence
<220>
   <221> source
   <223> /note="Description of Artificial Sequence: Synthetic peptide"
<400> 110
   Glu Arg Gly Tyr Cys Ser Ser Thr Thr Cys Thr Arg Ile Ile Asp Val
   1 5 10 15
<210> 111
   <211> 10
   <212> PRT
   <213> Artificial Sequence
<220>
   <221> source
   <223> /note="Description of Artificial Sequence: Synthetic peptide"
<400> 111
   Gly Arg Trp Glu Leu Tyr Ala Phe Asp Ile
   1 5 10
<210> 112
   <211> 11
   <212> PRT
   <213> Artificial Sequence
<220>
   <221> source
   <223> /note="Description of Artificial Sequence: Synthetic peptide"
<400> 112
   Gln Gly Asp Ser Leu Arg Ser Tyr Tyr Ala Ser
   1 5 10
<210> 113
   <211> 12
   <212> PRT
   <213> Artificial Sequence
<220>
   <221> source
   <223> /note="Description of Artificial Sequence: Synthetic peptide"
<400> 113
   Arg Ala Ser Gln Ser Val Ser Gly Ser Tyr Leu Thr
   1 5 10
<210> 114
   <211> 7
   <212> PRT
   <213> Artificial Sequence
<220>
   <221> source
   <223> /note="Description of Artificial Sequence: Synthetic peptide"
<400> 114
   Gly Lys Asn Asn Arg Pro Ser
   1 5
<210> 115
   <211> 7
   <212> PRT
   <213> Artificial Sequence
<220>
   <221> source
   <223> /note="Description of Artificial Sequence: Synthetic peptide"
<400> 115
   Gly Ala Ser Ser Arg Ala Thr
   1 5
<210> 116
   <211> 11
   <212> PRT
   <213> Artificial Sequence
<220>
   <221> source
   <223> /note="Description of Artificial Sequence: Synthetic peptide"
<400> 116
   Asn Ser Arg Asp Asn Ile Gly Asn His Gln Val
   1               5                   10
<210> 117
   <211> 11
   <212> PRT
   <213> Artificial Sequence
<220>
   <221> source
   <223> /note="Description of Artificial Sequence: Synthetic peptide"
<400> 117
   Gly Ser Arg Asp Asn Ile Gly Asn His Gln Val
   1               5                   10
<210> 118
   <211> 11
   <212> PRT
   <213> Artificial Sequence
<220>
   <221> source
   <223> /note="Description of Artificial Sequence: Synthetic peptide"
<400> 118
   Asn Ser Arg Asp Asn Val Gly Asn His Gln Val
   1               5                   10
<210> 119
   <211> 11
   <212> PRT
   <213> Artificial Sequence
<220>
   <221> source
   <223> /note="Description of Artificial Sequence: Synthetic peptide"
<400> 119
   Gly Ser Arg Asp Asn Val Gly Asn His Gln Val
   1               5                   10
<210> 120
   <211> 10
   <212> PRT
   <213> Artificial Sequence
<220>
   <221> source
   <223> /note="Description of Artificial Sequence: Synthetic peptide"
<400> 120
   Gln Gln Tyr Gly Gly Ser Pro Pro Phe Thr
   1               5                   10
<210> 121
   <211> 7
   <212> PRT
   <213> Artificial Sequence
<220>
   <221> source
   <223> /note="Description of Artificial Sequence: Synthetic consensus peptide"
<220>
   <221> VARIANT
   <222> (5)..(5)
   <223> /replace="Asp" or "Glu" or "Gln" or "Ser" or "Thr" or "Val" or "Tyr"
<220>
   <221> misc_feature
   <222> (5)..(5)
   <223> /note="Residue given in the sequence has no preference with respect to the annotations for said position"
<400> 121
   Ser Gly Gly Tyr Ala Trp Ser
   1               5
<210> 122
   <211> 981
   <212> DNA
   <213> Artificial Sequence
<220>
   <221> source
   <223> /note="Description of Artificial Sequence: Synthetic polynucleotide"
<400> 122
   gctagcacca agggcccatc ggtcttcccc ctggcgccct gctccaggag cacctccgag 60
   agcacagcgg ccctgggctg cctggtcaag gactacttcc ccgaaccggt gacggtgtcg 120
   tggaactcag gcgctctgac cagcggcgtg cacaccttcc cagctgtcct acagtcctca 180
   ggactctact ccctcagcag cgtggtgacc gtgccctcca gcaacttcgg cacccagacc 240
   tacacctgca acgtagatca caagcccagc aacaccaagg tggacaagac agttgagcgc 300
   aaatgttgtg tcgagtgccc accgtgccca gcaccacctg tggcaggacc gtcagtcttc 360
   ctcttccccc caaaacccaa ggacaccctc atgatctccc ggacccctga ggtcacgtgc 420
   gtggtggtgg acgtgagcca cgaagacccc gaggtccagt tcaactggta cgtggacggc 480
   gtggaggtgc ataatgccaa gacaaagcca cgggaggagc agttcaacag cacgttccgt 540
   gtggtcagcg tcctcaccgt tgtgcaccag gactggctga acggcaagga gtacaagtgc 600
   aaggtctcca acaaaggcct cccagccccc atcgagaaaa ccatctccaa aaccaaaggg 660
   cagccccgag aaccacaggt gtacaccctg cccccatccc gggaggagat gaccaagaac 720
   caggtcagcc tgacctgcct ggtcaaaggc ttctacccca gcgacatcgc cgtggagtgg 780
   gagagcaatg ggcagccgga gaacaactac aagaccacac ctcccatgct ggactccgac 840
   ggctccttct tcctctacag caagctcacc gtggacaaga gcaggtggca gcaggggaac 900
   gtcttctcat gctccgtgat gcatgaggct ctgcacaacc actacacgca gaagagcctc 960
   tccctgtctc cgggtaaatg a 981
<210> 123
   <211> 318
   <212> DNA
   <213> Artificial Sequence
<220>
   <221> source
   <223> /note="Description of Artificial Sequence: Synthetic polynucleotide"
<400> 123
   ggtcagccca aggctgcccc ctcggtcact ctgttcccgc cctcctctga ggagcttcaa 60
   gccaacaagg ccacactggt gtgtctcata agtgacttct acccgggagc cgtgacagtg 120
   gcctggaagg cagatagcag ccccgtcaag gcgggagtgg agaccaccac accctccaaa 180
   caaagcaaca acaagtacgc ggccagcagc tatctgagcc tgacgcctga gcagtggaag 240
   tcccacagaa gctacagctg ccaggtcacg catgaaggga gcaccgtgga gaagacagtg 300
   gcccctacag aatgttca 318
<210> 124
   <211> 324
   <212> DNA
   <213> Artificial Sequence
<220>
   <221> source
   <223> /note="Description of Artificial Sequence: Synthetic polynucleotide"
<400> 124
   cgtacggtgg ctgcaccatc tgtcttcatc ttcccgccat ctgatgagca gttgaaatct 60
   ggaactgcct ctgttgtgtg cctgctgaat aacttctatc ccagagaggc caaagtacag 120
   tggaaggtgg ataacgccct ccaatcgggt aactcccagg agagtgtcac agagcaggac 180
   agcaaggaca gcacctacag cctcagcagc accctgacgc tgagcaaagc agactacgag 240
   aaacacaaag tctacgcctg cgaagtcacc catcagggcc tgagctcgcc cgtcacaaag 300
   agcttcaaca ggggagagtg ttag 324
<210> 125
   <211> 378
   <212> DNA
   <213> Artificial Sequence
<220>
   <221> source
   <223> /note="Description of Artificial Sequence: Synthetic polynucleotide"
<400> 125
   caggtgcagc tgcaggagtc gggcccagga ctggtgaagc cttcacagac cctgtccctc 60
   acctgcactg tctctggtgc ctccattagc agtggtggtt acaactggag ctggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 126
   <211> 378
   <212> DNA
   <213> Artificial Sequence
<220>
   <221> source
   <223> /note="Description of Artificial Sequence: Synthetic polynucleotide"
<400> 126
   caggtgcagc tgcaggagtc gggcccagga ctggtgaagc cttcacagac cctgtccctc 60
   acctgcactg tctctggtgc ctccattagc agtggtggtt accagtggag ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 127
   <211> 378
   <212> DNA
   <213> Artificial Sequence
<220>
   <221> source
   <223> /note="Description of Artificial Sequence: Synthetic polynucleotide"
<400> 127
   caggtgcagc tgcaggagtc gggcccagga ctggtgaagc cttcacagac cctgtccctc 60
   acctgcactg tctctggtgc ctccattagc agtggtggtt acgactggtc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 128
   <211> 378
   <212> DNA
   <213> Artificial Sequence
<220>
   <221> source
   <223> /note="Description of Artificial Sequence: Synthetic polynucleotide"
<400> 128
   caggtgcagc tgcaggagtc gggcccagga ctggtgaagc cttcacagac cctgtccctc 60
   acctgcactg tctctggtgc ctccattagc agtggtggtt acgagtggtc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 129
   <211> 378
   <212> DNA
   <213> Artificial Sequence
<220>
   <221> source
   <223> /note="Description of Artificial Sequence: Synthetic polynucleotide"
<400> 129
   caggtgcagc tgcaggagtc gggcccagga ctggtgaagc cttcacagac cctgtccctc 60
   acctgcactg tctctggtgc ctccattagc agtggtggtt acgcttggtc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 130
   <211> 378
   <212> DNA
   <213> Artificial Sequence
<220>
   <221> source
   <223> /note="Description of Artificial Sequence: Synthetic polynucleotide"
<400> 130
   caggtgcagc tgcaggagtc gggcccagga ctggtgaagc cttcacagac cctgtccctc 60
   acctgcactg tctctggtgc ctccattagc agtggtggtt acagttggtc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 131
   <211> 378
   <212> DNA
   <213> Artificial Sequence
<220>
   <221> source
   <223> /note="Description of Artificial Sequence: Synthetic polynucleotide"
<400> 131
   caggtgcagc tgcaggagtc gggcccagga ctggtgaagc cttcacagac cctgtccctc 60
   acctgcactg tctctggtgc ctccattagc agtggtggtt acacctggtc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 132
   <211> 378
   <212> DNA
   <213> Artificial Sequence
<220>
   <221> source
   <223> /note="Description of Artificial Sequence: Synthetic polynucleotide"
<400> 132
   caggtgcagc tgcaggagtc gggcccagga ctggtgaagc cttcacagac cctgtccctc 60
   acctgcactg tctctggtgc ctccattagc agtggtggtt acgtctggtc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 133
   <211> 378
   <212> DNA
   <213> Artificial Sequence
<220>
   <221> source
   <223> /note="Description of Artificial Sequence: Synthetic polynucleotide"
<400> 133
   caggtgcagc tgcaggagtc gggcccagga ctggtgaagc cttcacagac cctgtccctc 60
   acctgcactg tctctggtgc ctccattagc agtggtggtt actactggag ctggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 134
   <211> 378
   <212> DNA
   <213> Artificial Sequence
<220>
   <221> source
   <223> /note="Description of Artificial Sequence: Synthetic polynucleotide"
<400> 134
   caggtgcagc tgcaggagtc gggcccagga ctggtgaagc cttcacagac cctgtccctc 60
   acctgcactg tctctggtgc ctccattagc agtggtggtt acaactgggt ctggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 135
   <211> 378
   <212> DNA
   <213> Artificial Sequence
<220>
   <221> source
   <223> /note="Description of Artificial Sequence: Synthetic polynucleotide"
<400> 135
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatgg acgtctgggg ccaagggacc 360
   acggtcaccg tctcctca 378
<210> 136
   <211> 378
   <212> DNA
   <213> Artificial Sequence
<220>
   <221> source
   <223> /note="Description of Artificial Sequence: Synthetic polynucleotide"
<400> 136
   caggtgcagc tgcaggagtc gggcccagga ctggtgaagc cttcacagac cctgtccctc 60
   acctgcactg tctctggtgc ctccattagc agtggtggtt acaactggag ctggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cacctgttct agggttatgg acgtctgggg ccaagggacc 360
   acggtcaccg tctcctca 378
<210> 137
   <211> 378
   <212> DNA
   <213> Artificial Sequence
<220>
   <221> source
   <223> /note="Description of Artificial Sequence: Synthetic polynucleotide"
<400> 137
   caggtgcagc tgcaggagtc gggcccagga ctggtgaagc cttcacagac cctgtccctc 60
   acctgcactg tctctggtgc ctccattagc agtggtggtt acaactggag ctggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgtact agggttatgg acgtctgggg ccaagggacc 360
   acggtcaccg tctcctca 378
<210> 138
   <211> 378
   <212> DNA
   <213> Artificial Sequence
<220>
   <221> source
   <223> /note="Description of Artificial Sequence: Synthetic polynucleotide"
<400> 138
   caggtgcagc tgcaggagtc gggcccagga ctggtgaagc cttcacagac cctgtccctc 60
   acctgcactg tctctggtgc ctccattagc agtggtggtt acaactggag ctggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct aggattatgg acgtctgggg ccaagggacc 360
   acggtcaccg tctcctca 378
<210> 139
   <211> 378
   <212> DNA
   <213> Artificial Sequence
<220>
   <221> source
   <223> /note="Description of Artificial Sequence: Synthetic polynucleotide"
<400> 139
   caggtgcagc tgcaggagtc gggcccagga ctggtgaagc cttcacagac cctgtccctc 60
   acctgcactg tctctggtgc ctccattagc agtggtggtt acaactggag ctggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct agggttatag acgtctgggg ccaagggacc 360
   acggtcaccg tctcctca 378
<210> 140
   <211> 378
   <212> DNA
   <213> Artificial Sequence
<220>
   <221> source
   <223> /note="Description of Artificial Sequence: Synthetic polynucleotide"
<400> 140
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cacctgttct agggttatgg acgtctgggg ccaagggacc 360
   acggtcaccg tctcctca 378
<210> 141
   <211> 378
   <212> DNA
   <213> Artificial Sequence
<220>
   <221> source
   <223> /note="Description of Artificial Sequence: Synthetic polynucleotide"
<400> 141
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgtact agggttatgg acgtctgggg ccaagggacc 360
   acggtcaccg tctcctca 378
<210> 142
   <211> 378
   <212> DNA
   <213> Artificial Sequence
<220>
   <221> source
   <223> /note="Description of Artificial Sequence: Synthetic polynucleotide"
<400> 142
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct aggattatgg acgtctgggg ccaagggacc 360
   acggtcaccg tctcctca 378
<210> 143
   <211> 378
   <212> DNA
   <213> Artificial Sequence
<220>
   <221> source
   <223> /note="Description of Artificial Sequence: Synthetic polynucleotide"
<400> 143
   caggtgcagc tgcaggagtc gggcccagga ctggtgaagc cttcacagac cctgtccctc    60
   acctgcactg tctctggtgc ctccattagc agtggtggtt acaactgggc ttggatccgc   120
   cagcacccag ggaagggcct ggagtggatt gggtacatct attacagtgg gagcacctac   180
   tacaacccgt ccctcaagag tcgagttacc atatcagtag acacgtctaa gaaccagttc   240
   tccctgaagc tgagctctgt gactgccgcg gacacggccg tgtattactg tgcgagagag   300
   aggggatatt gtagtagtac cagctgttct agggttatag acgtctgggg ccaagggacc   360
   acggtcaccg tctcctca                                                 378
<210> 144
   <211> 378
   <212> DNA
   <213> Artificial Sequence
<220>
   <221> source
   <223> /note="Description of Artificial Sequence: Synthetic polynucleotide"
<400> 144
   caggtgcagc tgcaggagtc gggcccagga ctggtgaagc cttcacagac cctgtccctc    60
   acctgcactg tctctggtgc ctccattagc agtggtggtt acaactgggc ttggatccgc   120
   cagcacccag ggaagggcct ggagtggatt gggtacatct attacagtgg gagcacctac   180
   tacaacccgt ccctcaagag tcgagttacc atatcagtag acacgtctaa gaaccagttc   240
   tccctgaagc tgagctctgt gactgccgcg gacacggccg tgtattactg tgcgagagag   300
   aggggatatt gtagtagtac cacctgtact agggttatgg acgtctgggg ccaagggacc   360
   acggtcaccg tctcctca                                                 378
<210> 145
   <211> 378
   <212> DNA
   <213> Artificial Sequence
<220>
   <221> source
   <223> /note="Description of Artificial Sequence: Synthetic polynucleotide"
<400> 145
   caggtgcagc tgcaggagtc gggcccagga ctggtgaagc cttcacagac cctgtccctc      60
   acctgcactg tctctggtgc ctccattagc agtggtggtt acaactgggc ttggatccgc     120
   cagcacccag ggaagggcct ggagtggatt gggtacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagag     300
   aggggatatt gtagtagtac cacctgttct aggattatgg acgtctgggg ccaagggacc     360
   acggtcaccg tctcctca                                                   378
<210> 146
   <211> 378
   <212> DNA
   <213> Artificial Sequence
<220>
   <221> source
   <223> /note="Description of Artificial Sequence: Synthetic polynucleotide"
<400> 146
   caggtgcagc tgcaggagtc gggcccagga ctggtgaagc cttcacagac cctgtccctc      60
   acctgcactg tctctggtgc ctccattagc agtggtggtt acaactgggc ttggatccgc     120
   cagcacccag ggaagggcct ggagtggatt gggtacatct attacagtgg gagcacctac     180
   tacaacccgt ccctcaagag tcgagttacc atatcagtag acacgtctaa gaaccagttc     240
   tccctgaagc tgagctctgt gactgccgcg gacacggccg tgtattactg tgcgagagag     300
   aggggatatt gtagtagtac cacctgttct agggttatag acgtctgggg ccaagggacc     360
   acggtcaccg tctcctca                                                   378
<210> 147
   <211> 378
   <212> DNA
   <213> Artificial Sequence
<220>
   <221> source
   <223> /note="Description of Artificial Sequence: Synthetic polynucleotide"
<400> 147
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgtact aggattatgg acgtctgggg ccaagggacc 360
   acggtcaccg tctcctca 378
<210> 148
   <211> 378
   <212> DNA
   <213> Artificial Sequence
<220>
   <221> source
   <223> /note="Description of Artificial Sequence: Synthetic polynucleotide"
<400> 148
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgtact agggttatag acgtctgggg ccaagggacc 360
   acggtcaccg tctcctca 378
<210> 149
   <211> 378
   <212> DNA
   <213> Artificial Sequence
<220>
   <221> source
   <223> /note="Description of Artificial Sequence: Synthetic polynucleotide"
<400> 149
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgttct aggattatag acgtctgggg ccaagggacc 360
   acggtcaccg tctcctca 378
<210> 150
   <211> 378
   <212> DNA
   <213> Artificial Sequence
<220>
   <221> source
   <223> /note="Description of Artificial Sequence: Synthetic polynucleotide"
<400> 150
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cacctgtact aggattatgg acgtctgggg ccaagggacc 360
   acggtcaccg tctcctca 378
<210> 151
   <211> 378
   <212> DNA
   <213> Artificial Sequence
<220>
   <221> source
   <223> /note="Description of Artificial Sequence: Synthetic polynucleotide"
<400> 151
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cacctgtact agggttatag acgtctgggg ccaagggacc 360
   acggtcaccg tctcctca 378
<210> 152
   <211> 378
   <212> DNA
   <213> Artificial Sequence
<220>
   <221> source
   <223> /note="Description of Artificial Sequence: Synthetic polynucleotide"
<400> 152
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cacctgttct aggattatag acgtctgggg ccaagggacc 360
   acggtcaccg tctcctca 378
<210> 153
   <211> 378
   <212> DNA
   <213> Artificial Sequence
<220>
   <221> source
   <223> /note="Description of Artificial Sequence: Synthetic polynucleotide"
<400> 153
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cagctgtact aggattatag acgtctgggg ccaagggacc 360
   acggtcaccg tctcctca 378
<210> 154
   <211> 378
   <212> DNA
   <213> Artificial Sequence
<220>
   <221> source
   <223> /note="Description of Artificial Sequence: Synthetic polynucleotide"
<400> 154
   caggtgcagc tgcaggagtc gggcccagga ctggtgaagc cttcacagac cctgtccctc 60
   acctgcactg tctctggtgc ctccattagc agtggtggtt acaactgggc ttggatccgc 120
   cagcacccag ggaagggcct ggagtggatt gggtacatct attacagtgg gagcacctac 180
   tacaacccgt ccctcaagag tcgagttacc atatcagtag acacgtctaa gaaccagttc 240
   tccctgaagc tgagctctgt gactgccgcg gacacggccg tgtattactg tgcgagagag 300
   aggggatatt gtagtagtac cacctgtact aggattatag acgtctgggg ccaagggacc 360
   acggtcaccg tctcctca 378
<210> 155
   <211> 357
   <212> DNA
   <213> Artificial Sequence
<220>
   <221> source
   <223> /note="Description of Artificial Sequence: Synthetic polynucleotide"
<400> 155
   caggtgcagc tggtgcagtc tggggctgag gtgaagaagc ctggggcctc agtgaaggtc 60
   tcctgcaagg cttctggata caccttcacc ggctactata tgcactgggt gcgacaggcc 120
   cctggacaag ggcttgagtg gatgggatgg atcaacccca acagtggtgg cacaaactat 180
   gcacagaagt ttcagggcag ggtcaccatg accagggaca cgtccatcag cacagcctac 240
   atggagctga acaggctgag atctgacgac acggccgtgt attactgtgc gagagggagg 300
   tgggagcttt atgcttttga tatctggggc caagggacaa tggtcaccgt ctcttca 357
<210> 156
   <211> 327
   <212> DNA
   <213> Artificial Sequence
<220>
   <221> source
   <223> /note="Description of Artificial Sequence: Synthetic polynucleotide"
<400> 156
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagttcagg aaacacagct tccttgacca tcactggggc tcaggcggaa 240
   gatgaggctg actattactg taactcccgg gacaacattg gtaaccatca ggtgttcggc 300
   ggagggacca agctgaccgt cctaggt 327
<210> 157
   <211> 327
   <212> DNA
   <213> Artificial Sequence
<220>
   <221> source
   <223> /note="Description of Artificial Sequence: Synthetic polynucleotide"
<400> 157
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagttcagg aaacacagct tccttgacca tcactggggc tcaggcggaa 240
   gatgaggctg actattactg tggctcccgg gacaacattg gtaaccatca ggtgttcggc 300
   ggagggacca agctgaccgt cctaggt 327
<210> 158
   <211> 327
   <212> DNA
   <213> Artificial Sequence
<220>
   <221> source
   <223> /note="Description of Artificial Sequence: Synthetic polynucleotide"
<400> 158
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagttcagg aaacacagct tccttgacca tcactggggc tcaggcggaa 240
   gatgaggctg actattactg taactcccgg gacaacgttg gtaaccatca ggtgttcggc 300
   ggagggacca agctgaccgt cctaggt 327
<210> 159
   <211> 327
   <212> DNA
   <213> Artificial Sequence
<220>
   <221> source
   <223> /note="Description of Artificial Sequence: Synthetic polynucleotide"
<400> 159
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagttcagg aaacacagct tccttgacca tcactggggc tcaggcggaa 240
   gatgaggctg actattactg tggctcccgg gacaacgttg gtaaccatca ggtgttcggc 300
   ggagggacca agctgaccgt cctaggt 327
<210> 160
   <211> 330
   <212> DNA
   <213> Artificial Sequence
<220>
   <221> source
   <223> /note="Description of Artificial Sequence: Synthetic polynucleotide"
<400> 160
   gaaattgtgt tgacgcagtc tccaggcacc ctgtctttgt ctccagggga aagagccacc    60
   ctctcctgca gggccagtca gagtgttagc ggcagctact taacctggta ccagcagaaa   120
   cctggccagg ctcccaggct cctcatctat ggtgcatcca gcagggccac tggcatccca   180
   gacaggttca gtggcagtgg gtctgggaca gacttcactc tcaccatcag cagactggag   240
   cctgaagatt ttgcaatgta ttactgtcag cagtatggtg gctcacctcc attcactttc   300
   ggccctggga ccaaagtgga tatcaaacgt                                    330
<210> 161
   <211> 422
   <212> PRT
   <213> Homo sapiens
<400> 161
   Phe Trp Leu Phe Asp Val Leu Phe Pro Pro His Thr Thr Pro Lys Ala
   1               5                   10                  15
   Glu Leu Ser Asn His Thr Arg Pro Val Ile Leu Val Pro Gly Cys Leu
               20                  25                  30
   Gly Asn Gln Leu Glu Ala Lys Leu Asp Lys Pro Asp Val Val Asn Trp
           35                  40                  45
   Met Cys Tyr Arg Lys Thr Glu Asp Phe Phe Thr Ile Trp Leu Asp Leu
       50                  55                  60
   Asn Met Phe Leu Pro Leu Gly Val Asp Cys Trp Ile Asp Asn Thr Arg
   65                  70                  75                  80
   Val Val Tyr Asn Arg Ser Ser Gly Leu Val Ser Asn Ala Pro Gly Val
                   85                  90                  95
   Gln Ile Arg Val Pro Gly Phe Gly Lys Thr Tyr Ser Val Glu Tyr Leu
               100                 105                 110
   Asp Ser Ser Lys Leu Ala Gly Tyr Leu His Thr Leu Val Gln Asn Leu
           115                 120                 125
   Val Asn Asn Gly Tyr Val Arg Asp Glu Thr Val Arg Ala Ala Pro Tyr
       130                 135                 140
   Asp Trp Arg Leu Glu Pro Gly Gln Gln Glu Glu Tyr Tyr Arg Lys Leu
   145                 150                 155                 160
   Ala Gly Leu Val Glu Glu Met His Ala Ala Tyr Gly Lys Pro Val Phe
                   165                 170                 175
   Leu Ile Gly His Ser Leu Gly Cys Leu His Leu Leu Tyr Phe Leu Leu
               180                 185                 190
   Arg Gln Pro Gln Ala Trp Lys Asp Arg Phe Ile Asp Gly Phe Ile Ser
           195                 200                 205
   Leu Gly Ala Pro Trp Gly Gly Ser Ile Lys Pro Met Leu Val Leu Ala
       210                 215                 220
   Ser Gly Asp Asn Gln Gly Ile Pro Ile Met Ser Ser Ile Lys Leu Lys
   225                 230                 235                 240
   Glu Glu Gln Arg Ile Thr Thr Thr Ser Pro Trp Met Phe Pro Ser Arg
                   245                 250                 255
   Met Ala Trp Pro Glu Asp His Val Phe Ile Ser Thr Pro Ser Phe Asn
               260                 265                 270
   Tyr Thr Gly Arg Asp Phe Gln Arg Phe Phe Ala Asp Leu His Phe Glu
           275                 280                 285
   Glu Gly Trp Tyr Met Trp Leu Gln Ser Arg Asp Leu Leu Ala Gly Leu
       290                 295                 300
   Pro Ala Pro Gly Val Glu Val Tyr Cys Leu Tyr Gly Val Gly Leu Pro
   305                 310                 315                 320
   Thr Pro Arg Thr Tyr Ile Tyr Asp His Gly Phe Pro Tyr Thr Asp Pro
                   325                 330                 335
   Val Gly Val Leu Tyr Glu Asp Gly Asp Asp Thr Val Ala Thr Arg Ser
               340                 345                 350
   Thr Glu Leu Cys Gly Leu Trp Gln Gly Arg Gln Pro Gln Pro Val His
           355                 360                 365
   Leu Leu Pro Leu His Gly Ile Gln His Leu Asn Met Val Phe Ser Asn
       370                 375                 380
   Leu Thr Leu Glu His Ile Asn Ala Ile Leu Leu Gly Ala Tyr Arg Gln
   385                 390                 395                 400
   Gly Pro Pro Ala Ser Pro Thr Ala Ser Pro Glu Pro Pro Pro Pro Glu
                   405                 410                 415
   Glu Asn Leu Tyr Phe Gln
               420
<210> 162
   <211> 213
   <212> PRT
   <213> Artificial Sequence
<220>
   <221> source
   <223> /note="Description of Artificial Sequence: Synthetic polypeptide"
<400> 162
   Ser Tyr Glu Leu Thr Gln Pro Pro Ser Val Ser Val Ser Pro Gly Gln
   1               5                   10                  15
   Thr Ala Ser Ile Thr Cys Ser Gly Asp Lys Leu Gly Asn Lys Phe Thr
               20                  25                  30
   Ser Trp Tyr Gln Arg Lys Pro Gly Gln Ser Pro Val Leu Val Ile Tyr
           35                  40                  45
   Gln Asp Thr Lys Arg Pro Ser Gly Ile Pro Glu Arg Phe Ser Gly Ser
       50                  55                  60
   Thr Ser Gly Asn Thr Ala Thr Leu Thr Ile Ser Gly Thr Gln Ala Met
   65                  70                  75                  80
   Asp Glu Ala Asp Tyr Tyr Cys Gln Ala Trp Asp Ser Ser Thr Ala Trp
                   85                  90                  95
   Val Phe Gly Gly Gly Thr Lys Leu Glu Val Leu Gly Gln Pro Lys Ala
               100                 105                 110
   Ala Pro Ser Val Thr Leu Phe Pro Pro Ser Ser Glu Glu Leu Gln Ala
           115                 120                 125
   Asn Lys Ala Thr Leu Val Cys Leu Ile Ser Asp Phe Tyr Pro Gly Ala
       130                 135                 140
   Val Thr Val Ala Trp Lys Ala Asp Ser Ser Pro Val Lys Ala Gly Val
   145                 150                 155                 160
   Glu Thr Thr Thr Pro Ser Lys Gln Ser Asn Asn Lys Tyr Ala Ala Ser
                   165                 170                 175
   Ser Tyr Leu Ser Leu Thr Pro Glu Gln Trp Lys Ser His Lys Ser Tyr
               180                 185                 190
   Ser Cys Gln Val Thr His Glu Gly Ser Thr Val Glu Lys Thr Val Ala
           195                 200                 205
   Pro Thr Glu Cys Ser
       210
<210> 163
   <211> 238
   <212> PRT
   <213> Artificial Sequence
<220>
   <221> source
   <223> /note="Description of Artificial Sequence: Synthetic polypeptide"
<400> 163
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Asn Lys Phe Tyr Glu Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asp Ser Leu Arg Ala Glu Asp Thr Ala Val Tyr Tyr Cys
                   85                  90                  95
   Ala Arg Glu Gly Ala Ala Val Arg Ser Phe Tyr Tyr Ser Tyr Tyr Gly
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Ser Ser Lys Ser Thr
       130                 135                 140
   Ser Gly Gly Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser His Ser
               180                 185                 190
   Ser Val Val Thr Val Pro Ser Ser Ser Leu Gly Thr Gln Thr Tyr Ile
           195                 200                 205
   Cys Asn Val Asn His Lys Pro Ser Asn Thr Lys Val Asp Lys Lys Val
       210                 215                 220
   Glu Pro Lys Ser Cys Ala Ala Ala Glu Asn Leu Tyr Phe Gln
   225                 230                 235
<210> 164
   <211> 422
   <212> PRT
   <213> Homo sapiens
<400> 164
   Phe Trp Leu Leu Asn Val Leu Phe Pro Pro His Thr Thr Pro Lys Ala
   1               5                   10                  15
   Glu Leu Ser Asn His Thr Arg Pro Val Ile Leu Val Pro Gly Tyr Leu
               20                  25                  30
   Gly Asn Gln Leu Glu Ala Lys Leu Asp Lys Pro Asp Val Val Asn Trp
           35                  40                  45
   Met Cys Tyr Arg Lys Thr Glu Asp Phe Phe Thr Ile Trp Leu Asp Leu
       50                  55                  60
   Asn Met Phe Leu Pro Leu Gly Val Asp Cys Trp Ile Asp Asn Thr Arg
   65                  70                  75                  80
   Val Val Tyr Asn Arg Ser Ser Gly Leu Val Ser Asn Ala Pro Gly Val
                   85                  90                  95
   Gln Ile Arg Val Pro Gly Phe Gly Lys Thr Tyr Ser Val Glu Tyr Leu
               100                 105                 110
   Asp Ser Ser Lys Leu Ala Gly Tyr Leu His Thr Leu Val Gln Asn Leu
           115                 120                 125
   Val Asn Asn Gly Tyr Val Arg Asp Glu Thr Val Arg Ala Ala Pro Tyr
       130                 135                 140
   Asp Trp Arg Leu Glu Pro Gly Gln Gln Glu Glu Tyr Tyr Arg Lys Leu
   145                 150                 155                 160
   Ala Gly Leu Val Glu Glu Met His Ala Ala Tyr Gly Lys Pro Val Phe
                   165                 170                 175
   Leu Ile Gly His Ser Leu Gly Cys Leu His Leu Leu Tyr Phe Leu Leu
               180                 185                 190
   Arg Gln Pro Gln Ala Trp Lys Asp Arg Phe Ile Asp Gly Phe Ile Ser
           195                 200                 205
   Leu Gly Ala Pro Trp Gly Gly Ser Ile Lys Pro Met Leu Val Leu Ala
       210                 215                 220
   Ser Gly Asp Asn Gln Gly Ile Pro Ile Met Ser Ser Ile Lys Leu Lys
   225 230 235 240
   Glu Glu Gln Arg Ile Thr Thr Thr Ser Pro Trp Met Phe Pro Ser Arg
   245 250 255
   Met Ala Trp Pro Glu Asp His Val Phe Ile Ser Thr Pro Ser Phe Asn
   260 265 270
   Tyr Thr Gly Arg Asp Phe Gln Arg Phe Phe Ala Asp Leu His Phe Glu
   275 280 285
   Glu Gly Trp Tyr Met Trp Leu Gln Ser Arg Asp Leu Leu Ala Gly Leu
   290 295 300
   Pro Ala Pro Gly Val Glu Val Tyr Cys Leu Tyr Gly Val Gly Leu Pro
   305 310 315 320
   Thr Pro Arg Thr Tyr Ile Tyr Asp His Gly Phe Pro Tyr Thr Asp Pro
   325 330 335
   Val Gly Val Leu Tyr Glu Asp Gly Asp Asp Thr Val Ala Thr Arg Ser
   340 345 350
   Thr Glu Leu Cys Gly Leu Trp Gln Gly Arg Gln Pro Gln Pro Val His
   355 360 365
   Leu Leu Pro Leu His Gly Ile Gln His Leu Asn Met Val Phe Ser Asn
   370 375 380
   Leu Thr Leu Glu His Ile Asn Ala Ile Leu Leu Gly Ala Tyr Arg Gln
   385 390 395 400
   Gly Pro Pro Ala Ser Pro Thr Ala Ser Pro Glu Pro Pro Pro Pro Glu
   405 410 415
   Glu Asn Leu Tyr Phe Gln
   420
<210> 165
   <211> 211
   <212> PRT
   <213> Artificial Sequence
<220>
   <221> source
   <223> /note="Description of Artificial Sequence: Synthetic polypeptide"
<400> 165
   Ser Tyr Glu Val Thr Gln Pro Pro Ser Val Ser Val Ser Pro Gly Gln
   1               5                   10                  15
   Thr Ala Ser Ile Thr Cys Ser Gly Asp Lys Leu Gly Asp Lys Asn Ala
               20                  25                  30
   Cys Trp Tyr Gln Gln Arg Pro Gly Gln Ser Pro Val Leu Val Ile Tyr
           35                  40                  45
   Gln Asn Ser Lys Arg Pro Ser Gly Ile Pro Glu Arg Phe Ser Gly Ser
       50                  55                  60
   Asn Ser Gly Asn Thr Ala Thr Leu Thr Ile Ser Gly Thr Gln Ala Met
   65                  70                  75                  80
   Asp Glu Ala Asp Tyr Tyr Cys Gln Ala Trp Asp Ser Ser Thr Val Phe
                   85                  90                  95
   Gly Gly Gly Thr Arg Leu Glu Val Leu Gly Gln Pro Lys Ala Ala Pro
               100                 105                 110
   Ser Val Thr Leu Phe Pro Pro Ser Ser Glu Glu Leu Gln Ala Asn Lys
           115                 120                 125
   Ala Thr Leu Val Cys Leu Ile Ser Asp Phe Tyr Pro Gly Ala Val Thr
       130                 135                 140
   Val Ala Trp Lys Ala Asp Ser Ser Pro Val Lys Ala Gly Val Glu Thr
   145                 150                 155                 160
   Thr Thr Pro Ser Lys Gln Ser Asn Asn Lys Tyr Ala Ala Ser Ser Tyr
                   165                 170                 175
   Leu Ser Leu Thr Pro Glu Gln Trp Lys Ser His Lys Ser Tyr Ser Cys
               180                 185                 190
   Gln Val Thr His Glu Gly Ser Thr Val Glu Lys Thr Val Ala Pro Thr
           195                 200                 205
   Glu Cys Ser
       210
<210> 166
   <211> 236
   <212> PRT
   <213> Artificial Sequence
<220>
   <221> source
   <223> /note="Description of Artificial Sequence: Synthetic polypeptide"
<400> 166
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Arg Ser Tyr
               20                  25                  30
   Ala Met Asn Trp Val Arg Gln Ala Pro Gly Lys Gly Leu Glu Trp Val
           35                  40                  45
   Ser Thr Ile Ser Gly Ser Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Leu Ser Ser Leu Arg Ala Glu Asp Thr Ala Val Tyr Tyr Cys
                   85                  90                  95
   Ala Lys Gly Ile Ala Ala Ala Asp Tyr Tyr Tyr Tyr Tyr Gly Met Asp
               100                 105                 110
   Val Trp Gly Gln Gly Thr Thr Val Thr Val Ser Ser Ala Ser Thr Lys
           115                 120                 125
   Gly Pro Ser Val Phe Pro Leu Ala Pro Ser Ser Lys Ser Thr Ser Gly
       130                 135                 140
   Gly Thr Ala Ala Leu Gly Cys Leu Val Lys Asp Tyr Phe Pro Glu Pro
   145                 150                 155                 160
   Val Thr Val Ser Trp Asn Ser Gly Ala Leu Thr Ser Gly Val His Thr
                   165                 170                 175
   Phe Pro Ala Val Leu Gln Ser Ser Gly Leu Tyr Ser His Ser Ser Val
               180                 185                 190
   Val Thr Val Pro Ser Ser Ser Leu Gly Thr Gln Thr Tyr Ile Cys Asn
           195                 200                 205
   Val Asn His Lys Pro Ser Asn Thr Lys Val Asp Lys Lys Val Glu Pro
       210                 215                 220
   Lys Ser Cys Ala Ala Ala Glu Asn Leu Tyr Phe Gln
   225                 230                 235
<210> 167
   <211> 5
   <212> PRT
   <213> Artificial Sequence
<220>
   <221> source
   <223> /note="Description of Artificial Sequence: Synthetic peptide"
<400> 167
   Cys Pro Ser Cys Pro
   1               5
<210> 168
   <211> 5
   <212> PRT
   <213> Artificial Sequence
<220>
   <221> source
   <223> /note="Description of Artificial Sequence: Synthetic peptide"
<400> 168
   Cys Pro Pro Cys Pro
   1               5
<210> 169
   <211> 6
   <212> PRT
   <213> Artificial Sequence
<220>
   <221> source
   <223> /note="Description of Artificial Sequence: Synthetic 6xHis tag"
<400> 169
   His His His His His His
   1               5

## Description

The present invention relates to an agonist antigen binding protein that specifically binds to human Lecithin-cholesterol acyltransferase polypeptide, a nucleic acid molecule encoding the antigen binding protein and a method for preparing the antigen binding protein. The invention further relates to a pharmaceutical composition comprising the antigen binding protein and therapeutic uses. Furthermore, the invention relates to an agonsit antigen binding protein that competes with the above antigen binding protein for binding to a human Lecithin-choleserol acyltransferase polypeptide of SEQ ID NO: 1.

### BACKGROUND

Over 50 million Americans have cardiovascular problems, and many other countries face high and increasing rates of cardiovascular disease. It is the number one cause of death and disability in the United States and most European countries. By the time that heart problems are detected, the underlying cause, atherosclerosis, is usually quite advanced, having progressed for decades.

Atherosclerosis is a polygenic complex disease of mammals characterized by the deposits or plaques of lipids and other blood derivatives in the arterial walls (aorta, coronary arteries, and carotid). These plaques can be calcified to a greater or lesser extent according to the progression of the process. They are also associated with the accumulation of fatty deposits consisting mainly of cholesterol esters in the arteries. Cholesterol accumulates in the foam cells of the arterial wall, thereby narrowing the lumen and decreasing the flow of blood. This is accompanied by a thickening of the arterial wall, with hypertrophy of the smooth muscle, the appearance of foam cells and the accumulation of the fibrous tissue. Hypercholesterolemia can therefore result in very serious cardiovascular pathologies such as infarction, peripheral vascular disease, stroke, sudden death, cardiac decompensation, cerebral vascular accidents and the like.

The cholesterol is carried in the blood by various lipoproteins including the very low-density lipoprotein (VLDL), the low-density lipoproteins (LDL) and the high-density lipoproteins (HDL). The VLDL is synthesized in the liver and is converted to LDL in the blood, which makes it possible to supply the peripheral tissues with cholesterol. In contrast, the HDL captures cholesterol molecules from the peripheral tissues and transports them to the liver where they are converted to bile acids and excreted. The development of atherosclerosis and the risk of coronary heart disease (CHD) inversely correlate to the levels of HDL in the serum. Gordon et al. (1989) N. Engl. J. Med. 321: 1311; Goldbourt et al. (1997) Thromb Vasc. Biol. 17: 107. Low HDL cholesterols often occur in the context of central obesity, diabetes and other features of the metabolic syndrome. Goldbourt et al., supra. It has been suggested that low HDL cholesterol levels are associated with an increased risk of CHD, while high concentrations of HDL have a protective effect against the development of premature atherosclerosis. Gordon et al. (1986) Circulation 74: 1217. Studies demonstrated that the risk for developing clinical atherosclerosis in men drops 2-3% with every 1 mg/dL increase in the concentration of HDL in plasma. Gordon et al. (1989) N. Engl. J. Med. 321: 1311. It has been established that concentrations of LDL cholesterol can be reduced by treatment with statins, inhibitors of the cholesterols biosynthesis enzyme 3-hydroxyl-3-methylglutary Coenzyme A reductase and thereby this treatment has been used as a successful approach for reducing the risk for atherosclerosis where the primary indication is high LDL level. However, it remains unclear whether statins are beneficial for patients whose primary lipid abnormality is low HDL cholesterol.

Lecithin-cholesterol acyltransferase (LCAT) is an enzyme which catalyses the esterification of free cholesterol by the transfer of an acyl group from phosphatidylcholine onto 3-hydroxyl group of the cholesterol, forming cholesteryl ester and lysophosphatidylcholine. McLean et al. (1986) Proc. Natl. Acad. Sci. 83: 2335 and McLean et al. (1986) Nucleic Acids Res. 14(23): 9397. LCAT is synthesized in the liver and secreted into the plasma, where it is combined with HDL, called anti-atherogenic lipoproteins. These HDL particles have the capacity to accept the excess cholesterol, which is then esterified by LCAT in the HDL particles. The cholesteryl ester molecules in the HDL particles are either transported to the liver directly through SR-BI receptor, or transferred to apoB-containing lipoproteins, including very low density lipoproteins (VLDL) and LDL, mediated by CETP, and then transported to the liver through LDL-receptor pathway. This mechanism, called reverse cholesterol transport (Glomset (1968) J. Lipid Res. 9:155), allows the removal of excess cholesterol from the body, and therefore is involved in the prevention of atherogenesis. LCAT plays a key role in this process by creating a gradient of free cholesterol between the plasma membranes and the circulating lipoproteins in favor of cholesterol efflux from peripheral tissues and cell membranes into the plasma compartment.

JP 11 269199 A (1999-10-05) describes a protein having Lecithin-cholesterol acyltransferase activity and its potential use as a preventive/therapeutic agent for diseases such as arteriosclerosis, hyperlipidemia and hypercaloric diseases. Presently, however, there are no approved drugs that directly increase the reverse cholesterol pathway. Instead, the approaches taken to increase HDL involve an increase in the synthesis and secretion of apoA-I, the major protein in HDL and/or a decrease in HDL catabolism. Several agents increase HDLc levels. See Linsel-Nitschke P, et al., Nat Rev Drug Discov. 2005; 4(3): 193-205. Gemfibrozil is an example of such a drug and is a member of a class of drugs called fibrates, which are fibric acid derivatives (bezafibrate, fenofibrate, gemfibrozil and clofibrate) that are thought to act on the liver. This family of compounds cause a significant lowering of plasma triglyceride levels and elevate HDLc. The typical clinical use of fibrates is in patients with hypertriglyceridemia, low HDLc and combined hyperlipidemia. The mechanism of action of fibrates involves the induction of certain apolipoproteins and enzymes involved in VLDL and HDL metabolism. See Staels B, et al., Diabetes 2005, 54:2460-2470 and Meyers C. D., et al., Curr Opin Cardiol. 2005; 20(4):307-12.

Nicotinic acid (niacin), a water-soluble vitamin, has also been used in the treatment of cardiovascular disease. It has a lipid lowering profile similar to fibrates and may target the liver. Niacin is reported to act by increasing apoA-I by selectively decreasing hepatic removal of HDL apoA-I. Niacin, however, does not increase the selective hepatic uptake of cholesteryl esters. See Meyers C D, et al., Curr Opin Cardiol. 2005; 20(4):307-12.

The steroids dexamethasone, prednisone, and estrogen activate the apoA-I gene, increase apoA-I and HDL cholesterol, reduce lipoprotein B, and reduce LDL. The attendant side effects of such steroids limit their chronic use in atherosclerosis.

There thus remains a need for other options for treating and preventing diseases associated with elevated cholesterol levels, such as cardiovascular disease, atherosclerosis, inflammatory disease and thrombosis related diseases associated with high serum cholesterol.

### SUMMARY

The subject matter of the invention is as set out in the appended claims. In particular:

In one aspect, the invention relates to an agonist antigen binding protein that specifically binds to a human Lecithin-cholesterol acyltransferase polypeptide, wherein the antigen binding protein is a human antibody.

In a further aspect, the invention relates to agonist antigen binding protein that competes with the above mentioned antigen binding protein for binding to a human Lecithin-cholesterol acyltransferase polypeptide of SEQ ID NO: 1

In a further aspect, the invention relates to a pharmaceutical composition comprising at least one of the above mentioned antigen binding proteins.

A further aspect of the invention relates to a nucleic acid sequence molecule encoding any one of the above mentioned antigen binding proteins.

In an additional aspect, the inevtnion relates to a method of making any one of the above mentioned antigen binding proteins.

In yet a further aspect, the invention relates to the above mentioned antigenbinding proteins for use in therapy.

Antigen binding proteins that activate the enzyme LCAT are described herein. The ability to develop of antigen binding proteins that can act as agonists of LCAT is surprising because it would not have been expected that an antigen binding protein such as an antibody or antibody fragment would be capable of activating an enzyme. Given the role that LCAT plays in cholesterol transport via HDL particles, the antigen binding proteins that are provided have utility in therapeutic methods in which it is useful to modulate HDL particle size, increase plasma levels of HDL-C, and increase reverse cholesterol transport. The antigen binding proteins thus have utility in the treatment and prevention of a variety of diseases, including atherosclerosis, various cardiovascular diseases and cholesterol-related disorders, inflammatory conditions, thrombosis-related conditions, metabolic syndrome, diabetes and insulin-resistance. The LCAT antigen binding proteins are also useful in treating the consequences, symptoms, and/or pathology associated with either genetic or acquired LCAT deficiency and chronic kidney disease (CKD).

In a first embodiment, antigen binding proteins that bind to a human LCAT polypeptide are provided,wherein the antigen binding protein is a human antibody.

In a second embodiment, the antigen binding protein o binds to human LCAT having the sequence shown in SEQ ID NO:1 and/or that shown in SEQ ID NO:2. The antigen binding protein binds to either of these human LCAT proteins in their active form.

In a third embodiment, the antigen binding protein of any of the foregoing four embodiments has one or more of the following characteristics:
(a) binds to the human LCAT polypeptide of SEQ ID NO:1 with a K_{D} of less than 50 nM; and
(b) binds to human LCAT with K_{D} of less than 50 nM and cyno LCAT with K_{D} of less than 500 nM.

In a fourth embodiment, the antigen binding protein of any of the foregoing embodiments comprises
(a) one or more heavy chain complementary determining regions (CDRHs) selected from the group consisting of:
   i. a CDRH1 selected from the group consisting of SEQ ID NO:81-92;
   ii. a CDRH2 selected from the group consisting of SEQ ID NO:93-94;
   iii. a CDRH3 selected from the group consisting of SEQ ID NO:95-111; and
   iv. a CDRH of (i), (ii) or (iii) that contains one or more amino acid substitutions, deletions or insertions totaling no more than 4 amino acids;
(b) one or more light chain complementary determining regions (CDRLs) selected from the group consisting of:
   i. a CDRL1 selected from the group consisting of SEQ ID NO: 112-113;
   ii. a CDRL2 selected from the group consisting of SEQ ID NO: 114-115;
   iii. a CDRL3 selected from the group consisting of SEQ ID NO:116-120; and
   iv. a CDRL of (i), (ii) or (iii) that contains one or more amino acid substitutions, deletions or insertions totaling no more than 4 amino acids; or
(c) one or more CDRHs of (a) and one or more CDRLs of (b).

In a fifth embodiment, the antigen binding protein comprises a CDRH3 and a CDRL3 as described for the fourth embodiment.

In a sixth embodiment, the CDRH3 of the antigen binding protein comprises SEQ ID NO:95 and the CDRL3 of the antigen binding protein comprises SEQ ID NO:116, or the CDRH3 comprises SEQ ID NO: 111 and the CDRL3 comprises SEQ ID NO: 120.

In a seventh embodiment, an isolated antigen binding protein of any of the foregoing embodiments comprises
(a) a CDRH selected from the group consisting of
   i. a CDRH1 selected from the group consisting of SEQ ID NO:81-92;
   ii. a CDRH2 selected from the group consisting of SEQ ID NO:93-94; and
   iii. a CDRH3 selected from the group consisting of SEQ ID NO:95-111;
(b) a CDRL selected from the group consisting of
   i. a CDRL1 selected from the group consisting of SEQ ID NO: 112-113;
   ii. a CDRL2 selected from the group consisting of SEQ ID NO: 114-115; and
   iii. a CDRL3 selected from the group consisting of SEQ ID NO: 116-120; or
(c) one or more CDRHs of (a) and one or more CDRLs of (b).

In an eighth embodiment, the antigen binding protein comprises
(a) a CDRH1 of SEQ ID NO:81, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(b) a CDRH1 of SEQ ID NO:82, a CDRH2 of SEQ ID NO:93, a CDRH3 of SEQ ID NO:95, a CDRL1 of SEQ ID NO: 112, a CDRL2 of SEQ ID NO:114, and a CDRL3 of SEQ ID NO:116;
(c) a CDRH1 of SEQ ID NO:83, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(d) a CDRH1 of SEQ ID NO:84, a CDRH2 of SEQ ID NO:93, a CDRH3 of SEQ ID NO:95, a CDRL1 of SEQ ID NO:112, a CDRL2 of SEQ ID NO:114, and a CDRL3 of SEQ ID NO:116;
(e) a CDRH1 of SEQ ID NO:85, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(f) a CDRH1 of SEQ ID NO:86, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(g) a CDRH1 of SEQ ID NO:87, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(h) a CDRH1 of SEQ ID NO:88, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(i) a CDRH1 of SEQ ID NO:89, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(j) a CDRH1 of SEQ ID NO:90, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(k) a CDRH1 of SEQ ID NO:91, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(l) a CDRH1 of SEQ ID NO:81, a CDRH2 of SEQ ID NO:93, a CDRH3 of SEQ ID NO:96, a CDRL1 of SEQ ID NO: 112, a CDRL2 of SEQ ID NO: 114, and a CDRL3 of SEQ ID NO:116;
(m) a CDRH1 of SEQ ID NO:81, a CDRH2 of SEQ ID NO:93, a CDRH3 of SEQ ID NO:97, a CDRL1 of SEQ ID NO: 112, a CDRL2 of SEQ ID NO: 114, and a CDRL3 of SEQ ID NO:116;
(n) a CDRH1 of SEQ ID NO:81, a CDRH2 of SEQ ID NO:93, a CDRH3 of SEQ ID NO:98, a CDRL1 of SEQ ID NO: 112, a CDRL2 of SEQ ID NO: 114, and a CDRL3 of SEQ ID NO:116;
(o) a CDRH1 of SEQ ID NO:81, a CDRH2 of SEQ ID NO:93, a CDRH3 of SEQ ID NO:99, a CDRL1 of SEQ ID NO: 112, a CDRL2 of SEQ ID NO:114, and a CDRL3 of SEQ ID NO:116; or
(p) a CDRH1 of SEQ ID NO:92, a CDRH2 of SEQ ID NO:94, a CDRH3 of SEQ ID NO: 111, a CDRL1 of SEQ ID NO: 113, a CDRL2 of SEQ ID NO: 115, and a CDRL3 of SEQ ID NO: 120.

In an eleventh embodiment, the antigen binding protein comprises
(a) a CDRH1 of SEQ ID NO:91, a CDRH2 of SEQ ID NO:93, a CDRH3 of SEQ ID NO:96, a CDRL1 of SEQ ID NO: 112, a CDRL2 of SEQ ID NO: 114, and a CDRL3 of SEQ ID NO: 116;
(b) a CDRH1 of SEQ ID NO:91, a CDRH2 of SEQ ID NO:93, a CDRH3 of SEQ ID NO:97, a CDRL1 of SEQ ID NO: 112, a CDRL2 of SEQ ID NO: 114, and a CDRL3 of SEQ ID NO: 116;
(c) a CDRH1 of SEQ ID NO:91, a CDRH2 of SEQ ID NO:93, a CDRH3 of SEQ ID NO:98, a CDRL1 of SEQ ID NO: 112, a CDRL2 of SEQ ID NO: 114, and a CDRL3 of SEQ ID NO: 116;
(d) a CDRH1 of SEQ ID NO:91, a CDRH2 of SEQ ID NO:93, a CDRH3 of SEQ ID NO:99, a CDRL1 of SEQ ID NO: 112, a CDRL2 of SEQ ID NO: 114, and a CDRL3 of SEQ ID NO:116;
(e) a CDRH1 of SEQ ID NO:91, a CDRH2 of SEQ ID NO:93, a CDRH3 of SEQ ID NO: 100, a CDRL1 of SEQ ID NO: 112, a CDRL2 of SEQ ID NO: 114, and a CDRL3 of SEQ ID NO:116;
(f) a CDRH1 of SEQ ID NO:91, a CDRH2 of SEQ ID NO:93, a CDRH3 of SEQ ID NO:101, a CDRL1 of SEQ ID NO: 112, a CDRL2 of SEQ ID NO: 114, and a CDRL3 of SEQ ID NO:116;
(g) a CDRH1 of SEQ ID NO:91, a CDRH2 of SEQ ID NO:93, a CDRH3 of SEQ ID NO: 102, a CDRL1 of SEQ ID NO: 112, a CDRL2 of SEQ ID NO: 114, and a CDRL3 of SEQ ID NO:116;
(h) a CDRH1 of SEQ ID NO:91, a CDRH2 of SEQ ID NO:93, a CDRH3 of SEQ ID NO: 103, a CDRL1 of SEQ ID NO: 112, a CDRL2 of SEQ ID NO: 114, and a CDRL3 of SEQ ID NO:116;
(i) a CDRH1 of SEQ ID NO:91, a CDRH2 of SEQ ID NO:93, a CDRH3 of SEQ ID NO: 104, a CDRL1 of SEQ ID NO: 112, a CDRL2 of SEQ ID NO: 114, and a CDRL3 of SEQ ID NO:116;
(j) a CDRH1 of SEQ ID NO:91, a CDRH2 of SEQ ID NO:93, a CDRH3 of SEQ ID NO: 105, a CDRL1 of SEQ ID NO: 112, a CDRL2 of SEQ ID NO: 114, and a CDRL3 of SEQ ID NO: 116;
(k) a CDRH1 of SEQ ID NO:91, a CDRH2 of SEQ ID NO:93, a CDRH3 of SEQ ID NO: 106, a CDRL1 of SEQ ID NO: 112, a CDRL2 of SEQ ID NO: 114, and a CDRL3 of SEQ ID NO: 116;
(l) a CDRH1 of SEQ ID NO:91, a CDRH2 of SEQ ID NO:93, a CDRH3 of SEQ ID NO: 107, a CDRL1 of SEQ ID NO: 112, a CDRL2 of SEQ ID NO: 114, and a CDRL3 of SEQ ID NO:116;
(m) a CDRH1 of SEQ ID NO:91, a CDRH2 of SEQ ID NO:93, a CDRH3 of SEQ ID NO: 108, a CDRL1 of SEQ ID NO: 112, a CDRL2 of SEQ ID NO: 114, and a CDRL3 of SEQ ID NO:116;
(n) a CDRH1 of SEQ ID NO:91, a CDRH2 of SEQ ID NO:93, a CDRH3 of SEQ ID NO: 109, a CDRL1 of SEQ ID NO: 112, a CDRL2 of SEQ ID NO: 114, and a CDRL3 of SEQ ID NO:116; or
(o) a CDRH1 of SEQ ID NO:91, a CDRH2 of SEQ ID NO:93, a CDRH3 of SEQ ID NO: 110, a CDRL1 of SEQ ID NO: 112, a CDRL2 of SEQ ID NO: 114, and a CDRL3 of SEQ ID NO:116.

In a ninth embodiment, the antigen binding protein comprises
(a) a CDRH1 of SEQ ID NO:91, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO: 117;
(b) a CDRH1 of SEQ ID NO:91, a CDRH2 of SEQ ID NO:93, a CDRH3 of SEQ ID NO:96, a CDRL1 of SEQ ID NO: 112, a CDRL2 of SEQ ID NO: 114, and a CDRL3 of SEQ ID NO:117;
(c) a CDRH1 of SEQ ID NO:91, a CDRH2 of SEQ ID NO:93, a CDRH3 of SEQ ID NO:97, a CDRL1 of SEQ ID NO: 112, a CDRL2 of SEQ ID NO: 114, and a CDRL3 of SEQ ID NO: 117;
(d) a CDRH1 of SEQ ID NO:91, a CDRH2 of SEQ ID NO:93, a CDRH3 of SEQ ID NO:98, a CDRL1 of SEQ ID NO: 112, a CDRL2 of SEQ ID NO: 114, and a CDRL3 of SEQ ID NO: 117;
(e) a CDRH1 of SEQ ID NO:91, a CDRH2 of SEQ ID NO:93, a CDRH3 of SEQ ID NO:99, a CDRL1 of SEQ ID NO: 112, a CDRL2 of SEQ ID NO: 114, and a CDRL3 of SEQ ID NO: 117;
(f) a CDRH1 of SEQ ID NO:91, a CDRH2 of SEQ ID NO:93, a CDRH3 of SEQ ID NO: 100, a CDRL1 of SEQ ID NO: 112, a CDRL2 of SEQ ID NO: 114, and a CDRL3 of SEQ ID NO: 117;
(g) a CDRH1 of SEQ ID NO:91, a CDRH2 of SEQ ID NO:93, a CDRH3 of SEQ ID NO:101, a CDRL1 of SEQ ID NO: 112, a CDRL2 of SEQ ID NO: 114, and a CDRL3 of SEQ ID NO: 117;
(h) a CDRH1 of SEQ ID NO:91, a CDRH2 of SEQ ID NO:93, a CDRH3 of SEQ ID NO: 102, a CDRL1 of SEQ ID NO: 112, a CDRL2 of SEQ ID NO: 114, and a CDRL3 of SEQ ID NO: 117;
(i) a CDRH1 of SEQ ID NO:91, a CDRH2 of SEQ ID NO:93, a CDRH3 of SEQ ID NO: 103, a CDRL1 of SEQ ID NO: 112, a CDRL2 of SEQ ID NO: 114, and a CDRL3 of SEQ ID NO: 117;
(j) a CDRH1 of SEQ ID NO:91, a CDRH2 of SEQ ID NO:93, a CDRH3 of SEQ ID NO: 104, a CDRL1 of SEQ ID NO: 112, a CDRL2 of SEQ ID NO: 114, and a CDRL3 of SEQ ID NO: 117;
(k) a CDRH1 of SEQ ID NO:91, a CDRH2 of SEQ ID NO:93, a CDRH3 of SEQ ID NO: 105, a CDRL1 of SEQ ID NO: 112, a CDRL2 of SEQ ID NO: 114, and a CDRL3 of SEQ ID NO: 117;
(l) a CDRH1 of SEQ ID NO:91, a CDRH2 of SEQ ID NO:93, a CDRH3 of SEQ ID NO: 106, a CDRL1 of SEQ ID NO: 112, a CDRL2 of SEQ ID NO: 114, and a CDRL3 of SEQ ID NO:117;
(m) a CDRH1 of SEQ ID NO:91, a CDRH2 of SEQ ID NO:93, a CDRH3 of SEQ ID NO: 107, a CDRL1 of SEQ ID NO: 112, a CDRL2 of SEQ ID NO: 114, and a CDRL3 of SEQ ID NO: 117;
(n) a CDRH1 of SEQ ID NO:91, a CDRH2 of SEQ ID NO:93, a CDRH3 of SEQ ID NO: 108, a CDRL1 of SEQ ID NO: 112, a CDRL2 of SEQ ID NO: 114, and a CDRL3 of SEQ ID NO: 117;
(o) a CDRH1 of SEQ ID NO:91, a CDRH2 of SEQ ID NO:93, a CDRH3 of SEQ ID NO: 109, a CDRL1 of SEQ ID NO: 112, a CDRL2 of SEQ ID NO: 114, and a CDRL3 of SEQ ID NO: 117; or
(p) a CDRH1 of SEQ ID NO:91, a CDRH2 of SEQ ID NO:93, a CDRH3 of SEQ ID NO: 110, a CDRL1 of SEQ ID NO: 112, a CDRL2 of SEQ ID NO: 114, and a CDRL3 of SEQ ID NO: 117.

In a tenthembodiment, the antigen binding protein comprises
(a) a CDRH1 of SEQ ID NO:91, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO: 118;
(b) a CDRH1 of SEQ ID NO:91, a CDRH2 of SEQ ID NO:93, a CDRH3 of SEQ ID NO:96, a CDRL1 of SEQ ID NO: 112, a CDRL2 of SEQ ID NO: 114, and a CDRL3 of SEQ ID NO:118;
(c) a CDRH1 of SEQ ID NO:91, a CDRH2 of SEQ ID NO:93, a CDRH3 of SEQ ID NO:97, a CDRL1 of SEQ ID NO: 112, a CDRL2 of SEQ ID NO: 114, and a CDRL3 of SEQ ID NO:118;
(d) a CDRH1 of SEQ ID NO:91, a CDRH2 of SEQ ID NO:93, a CDRH3 of SEQ ID NO:98, a CDRL1 of SEQ ID NO: 112, a CDRL2 of SEQ ID NO: 114, and a CDRL3 of SEQ ID NO:118;
(e) a CDRH1 of SEQ ID NO:91, a CDRH2 of SEQ ID NO:93, a CDRH3 of SEQ ID NO:99, a CDRL1 of SEQ ID NO: 112, a CDRL2 of SEQ ID NO: 114, and a CDRL3 of SEQ ID NO:118;
(f) a CDRH1 of SEQ ID NO:91, a CDRH2 of SEQ ID NO:93, a CDRH3 of SEQ ID NO: 100, a CDRL1 of SEQ ID NO: 112, a CDRL2 of SEQ ID NO: 114, and a CDRL3 of SEQ ID NO:118;
(g) a CDRH1 of SEQ ID NO:91, a CDRH2 of SEQ ID NO:93, a CDRH3 of SEQ ID NO:101, a CDRL1 of SEQ ID NO:112, a CDRL2 of SEQ ID NO:114, and a CDRL3 of SEQ ID NO:118;
(h) a CDRH1 of SEQ ID NO:91, a CDRH2 of SEQ ID NO:93, a CDRH3 of SEQ ID NO: 102, a CDRL1 of SEQ ID NO: 112, a CDRL2 of SEQ ID NO: 114, and a CDRL3 of SEQ ID NO:118;
(i) a CDRH1 of SEQ ID NO:91, a CDRH2 of SEQ ID NO:93, a CDRH3 of SEQ ID NO: 103, a CDRL1 of SEQ ID NO: 112, a CDRL2 of SEQ ID NO: 114, and a CDRL3 of SEQ ID NO:118;
(j) a CDRH1 of SEQ ID NO:91, a CDRH2 of SEQ ID NO:93, a CDRH3 of SEQ ID NO: 104, a CDRL1 of SEQ ID NO: 112, a CDRL2 of SEQ ID NO: 114, and a CDRL3 of SEQ ID NO:118;
(k) a CDRH1 of SEQ ID NO:91, a CDRH2 of SEQ ID NO:93, a CDRH3 of SEQ ID NO: 105, a CDRL1 of SEQ ID NO: 112, a CDRL2 of SEQ ID NO: 114, and a CDRL3 of SEQ ID NO:118;
(l) a CDRH1 of SEQ ID NO:91, a CDRH2 of SEQ ID NO:93, a CDRH3 of SEQ ID NO:106, a CDRL1 of SEQ ID NO: 112, a CDRL2 of SEQ ID NO: 114, and a CDRL3 of SEQ ID NO:118;
(m) a CDRH1 of SEQ ID NO:91, a CDRH2 of SEQ ID NO:93, a CDRH3 of SEQ ID NO: 107, a CDRL1 of SEQ ID NO: 112, a CDRL2 of SEQ ID NO: 114, and a CDRL3 of SEQ ID NO:118;
(n) a CDRH1 of SEQ ID NO:91, a CDRH2 of SEQ ID NO:93, a CDRH3 of SEQ ID NO: 108, a CDRL1 of SEQ ID NO: 112, a CDRL2 of SEQ ID NO: 114, and a CDRL3 of SEQ ID NO:118;
(o) a CDRH1 of SEQ ID NO:91, a CDRH2 of SEQ ID NO:93, a CDRH3 of SEQ ID NO: 109, a CDRL1 of SEQ ID NO: 112, a CDRL2 of SEQ ID NO: 114, and a CDRL3 of SEQ ID NO:118; or
(p) a CDRH1 of SEQ ID NO:91, a CDRH2 of SEQ ID NO:93, a CDRH3 of SEQ ID NO: 110, a CDRL1 of SEQ ID NO: 112, a CDRL2 of SEQ ID NO: 114, and a CDRL3 of SEQ ID NO:118.

In an eleventh embodiment, the antigen binding protein comprises
(a) a CDRH1 of SEQ ID NO:91, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:119;
(b) a CDRH1 of SEQ ID NO:91, a CDRH2 of SEQ ID NO:93, a CDRH3 of SEQ ID NO:96, a CDRL1 of SEQ ID NO: 112, a CDRL2 of SEQ ID NO:114, and a CDRL3 of SEQ ID NO:119;
(c) a CDRH1 of SEQ ID NO:91, a CDRH2 of SEQ ID NO:93, a CDRH3 of SEQ ID NO:97, a CDRL1 of SEQ ID NO: 112, a CDRL2 of SEQ ID NO:114, and a CDRL3 of SEQ ID NO:119;
(d) a CDRH1 of SEQ ID NO:91, a CDRH2 of SEQ ID NO:93, a CDRH3 of SEQ ID NO:98, a CDRL1 of SEQ ID NO: 112, a CDRL2 of SEQ ID NO:114, and a CDRL3 of SEQ ID NO:119;
(e) a CDRH1 of SEQ ID NO:91, a CDRH2 of SEQ ID NO:93, a CDRH3 of SEQ ID NO:99, a CDRL1 of SEQ ID NO: 112, a CDRL2 of SEQ ID NO: 114, and a CDRL3 of SEQ ID NO:119;
(f) a CDRH1 of SEQ ID NO:91, a CDRH2 of SEQ ID NO:93, a CDRH3 of SEQ ID NO: 100, a CDRL1 of SEQ ID NO: 112, a CDRL2 of SEQ ID NO: 114, and a CDRL3 of SEQ ID NO: 119;
(g) a CDRH1 of SEQ ID NO:91, a CDRH2 of SEQ ID NO:93, a CDRH3 of SEQ ID NO:101, a CDRL1 of SEQ ID NO:112, a CDRL2 of SEQ ID NO:114, and a CDRL3 of SEQ ID NO: 119;
(h) a CDRH1 of SEQ ID NO:91, a CDRH2 of SEQ ID NO:93, a CDRH3 of SEQ ID NO: 102, a CDRL1 of SEQ ID NO: 112, a CDRL2 of SEQ ID NO: 114, and a CDRL3 of SEQ ID NO: 119;
(i) a CDRH1 of SEQ ID NO:91, a CDRH2 of SEQ ID NO:93, a CDRH3 of SEQ ID NO: 103, a CDRL1 of SEQ ID NO: 112, a CDRL2 of SEQ ID NO: 114, and a CDRL3 of SEQ ID NO: 119;
(j) a CDRH1 of SEQ ID NO:91, a CDRH2 of SEQ ID NO:93, a CDRH3 of SEQ ID NO: 104, a CDRL1 of SEQ ID NO: 112, a CDRL2 of SEQ ID NO: 114, and a CDRL3 of SEQ ID NO: 119;
(k) a CDRH1 of SEQ ID NO:91, a CDRH2 of SEQ ID NO:93, a CDRH3 of SEQ ID NO: 105, a CDRL1 of SEQ ID NO: 112, a CDRL2 of SEQ ID NO: 114, and a CDRL3 of SEQ ID NO: 119;
(l) a CDRH1 of SEQ ID NO:91, a CDRH2 of SEQ ID NO:93, a CDRH3 of SEQ ID NO:106, a CDRL1 of SEQ ID NO: 112, a CDRL2 of SEQ ID NO: 114, and a CDRL3 of SEQ ID NO: 119;
(m) a CDRH1 of SEQ ID NO:91, a CDRH2 of SEQ ID NO:93, a CDRH3 of SEQ ID NO: 107, a CDRL1 of SEQ ID NO: 112, a CDRL2 of SEQ ID NO: 114, and a CDRL3 of SEQ ID NO: 119;
(n) a CDRH1 of SEQ ID NO:91, a CDRH2 of SEQ ID NO:93, a CDRH3 of SEQ ID NO: 108, a CDRL1 of SEQ ID NO: 112, a CDRL2 of SEQ ID NO: 114, and a CDRL3 of SEQ ID NO: 119;
(o) a CDRH1 of SEQ ID NO:91, a CDRH2 of SEQ ID NO:93, a CDRH3 of SEQ ID NO: 109, a CDRL1 of SEQ ID NO: 112, a CDRL2 of SEQ ID NO: 114, and a CDRL3 of SEQ ID NO: 119; or
(p) a CDRH1 of SEQ ID NO:91, a CDRH2 of SEQ ID NO:93, a CDRH3 of SEQ ID NO: 110, a CDRL1 of SEQ ID NO:112, a CDRL2 of SEQ ID NO: 114, and a CDRL3 of SEQ ID NO: 119.

In a twelfth embodiment, the antigen binding protein of any of the foregoing embodiments comprises a heavy chain variable region (VH) and/or a light chain variable region (VL), wherein
(a) the VH has at least 90% sequence identity with the amino acid sequence selected from the group consisting of SEQ ID NO:45-75; and
(b) the VL has at least 90% sequence identity with the amino acid sequence selected from the group consisting of SEQ ID NO:76-80.

In a thirteenthembodiment, the antigen binding protein of any of the foregoing embodiments comprises a heavy chain variable region (VH) and/or a light chain variable region (VL), wherein
(a) the VH has at least 90% sequence identity with the amino acid sequence of SEQ ID NO:45 and the VL has at least 90% sequence identity with the amino acid sequence of SEQ ID NO:76; or
(b) the VH has at least 90% sequence identity with the amino acid sequence of SEQ ID NO:75 and the VL has at least 90% sequence identity with the amino acid sequence of SEQ ID NO:80.

In a fourteenth embodiment, the antigen binding protein of any of the foregoing embodiments comprises a heavy chain variable region (VH) and a light chain variable region (VL), wherein
(a) the VH comprises the amino acid sequence of SEQ ID NO:45 and the VL comprises the amino acid sequence of SEQ ID NO:76;
(b) the VH comprises the amino acid sequence of SEQ ID NO:46 and the VL comprises the amino acid sequence of SEQ ID NO:76;
(c) the VH comprises the amino acid sequence of SEQ ID NO:47 and the VL comprises the amino acid sequence of SEQ ID NO:76;
(d) the VH comprises the amino acid sequence of SEQ ID NO:48 and the VL comprises the amino acid sequence of SEQ ID NO:76;
(e) the VH comprises the amino acid sequence of SEQ ID NO:49 and the VL comprises the amino acid sequence of SEQ ID NO:76;
(f) the VH comprises the amino acid sequence of SEQ ID NO:50 and the VL comprises the amino acid sequence of SEQ ID NO:76;
(g) the VH comprises the amino acid sequence of SEQ ID NO:51 and the VL comprises the amino acid sequence of SEQ ID NO:76;
(h) the VH comprises the amino acid sequence of SEQ ID NO:52 and the VL comprises the amino acid sequence of SEQ ID NO:76;
(i) the VH comprises the amino acid sequence of SEQ ID NO:53 and the VL comprises the amino acid sequence of SEQ ID NO:76;
(j) the VH comprises the amino acid sequence of SEQ ID NO:54 and the VL comprises the amino acid sequence of SEQ ID NO:76;
(k) the VH comprises the amino acid sequence of SEQ ID NO:55 and the VL comprises the amino acid sequence of SEQ ID NO:76;
(l) the VH comprises the amino acid sequence of SEQ ID NO:56 and the VL comprises the amino acid sequence of SEQ ID NO:76;
(m) the VH comprises the amino acid sequence of SEQ ID NO:57 and the VL comprises the amino acid sequence of SEQ ID NO:76;
(n) the VH comprises the amino acid sequence of SEQ ID NO:58 and the VL comprises the amino acid sequence of SEQ ID NO:76;
(o) the VH comprises the amino acid sequence of SEQ ID NO:59 and the VL comprises the amino acid sequence of SEQ ID NO:76; or
(p) the VH comprises the amino acid sequence of SEQ ID NO:75 and the VL comprises the amino acid sequence of SEQ ID NO:80.

In a fifteenth embodiment, the antigen binding protein of any of the foregoing embodiments comprises a heavy chain variable region (VH) and a light chain variable region (VL), wherein
(a) the VH comprises the amino acid sequence of SEQ ID NO:60 and the VL comprises the amino acid sequence of SEQ ID NO:76;
(b) the VH comprises the amino acid sequence of SEQ ID NO:61 and the VL comprises the amino acid sequence of SEQ ID NO:76;
(c) the VH comprises the amino acid sequence of SEQ ID NO:62 and the VL comprises the amino acid sequence of SEQ ID NO:76;
(d) the VH comprises the amino acid sequence of SEQ ID NO:63 and the VL comprises the amino acid sequence of SEQ ID NO:76;
(e) the VH comprises the amino acid sequence of SEQ ID NO:64 and the VL comprises the amino acid sequence of SEQ ID NO:76;
(f) the VH comprises the amino acid sequence of SEQ ID NO:65 and the VL comprises the amino acid sequence of SEQ ID NO:76;
(g) the VH comprises the amino acid sequence of SEQ ID NO:66 and the VL comprises the amino acid sequence of SEQ ID NO:76;
(h) the VH comprises the amino acid sequence of SEQ ID NO:67 and the VL comprises the amino acid sequence of SEQ ID NO:76;
(i) the VH comprises the amino acid sequence of SEQ ID NO:68 and the VL comprises the amino acid sequence of SEQ ID NO:76;
(j) the VH comprises the amino acid sequence of SEQ ID NO:69 and the VL comprises the amino acid sequence of SEQ ID NO:76;
(k) the VH comprises the amino acid sequence of SEQ ID NO:70 and the VL comprises the amino acid sequence of SEQ ID NO:76;
(l) the VH comprises the amino acid sequence of SEQ ID NO:71 and the VL comprises the amino acid sequence of SEQ ID NO:76;
(m) the VH comprises the amino acid sequence of SEQ ID NO:72 and the VL comprises the amino acid sequence of SEQ ID NO:76;
(n) the VH comprises the amino acid sequence of SEQ ID NO:73 and the VL comprises the amino acid sequence of SEQ ID NO:76; or
(o) the VH comprises the amino acid sequence of SEQ ID NO:74 and the VL comprises the amino acid sequence of SEQ ID NO:76.

In a sixteenth embodiment, the antigen binding protein of any of the foregoing embodiments comprises a heavy chain variable region (VH) and a light chain variable region (VL), wherein
(a) the VH comprises the amino acid sequence of SEQ ID NO:55 and the VL comprises the amino acid sequence of SEQ ID NO:77;
(b) the VH comprises the amino acid sequence of SEQ ID NO:60 and the VL comprises the amino acid sequence of SEQ ID NO:77;
(c) the VH comprises the amino acid sequence of SEQ ID NO:61 and the VL comprises the amino acid sequence of SEQ ID NO:77;
(d) the VH comprises the amino acid sequence of SEQ ID NO:62 and the VL comprises the amino acid sequence of SEQ ID NO:77;
(e) the VH comprises the amino acid sequence of SEQ ID NO:63 and the VL comprises the amino acid sequence of SEQ ID NO:77;
(f) the VH comprises the amino acid sequence of SEQ ID NO:64 and the VL comprises the amino acid sequence of SEQ ID NO:77;
(g) the VH comprises the amino acid sequence of SEQ ID NO:65 and the VL comprises the amino acid sequence of SEQ ID NO:77;
(h) the VH comprises the amino acid sequence of SEQ ID NO:66 and the VL comprises the amino acid sequence of SEQ ID NO:77;
(i) the VH comprises the amino acid sequence of SEQ ID NO:67 and the VL comprises the amino acid sequence of SEQ ID NO:77;
(j) the VH comprises the amino acid sequence of SEQ ID NO:68 and the VL comprises the amino acid sequence of SEQ ID NO:77;
(k) the VH comprises the amino acid sequence of SEQ ID NO:69 and the VL comprises the amino acid sequence of SEQ ID NO:77;
(l) the VH comprises the amino acid sequence of SEQ ID NO:70 and the VL comprises the amino acid sequence of SEQ ID NO:77;
(m) the VH comprises the amino acid sequence of SEQ ID NO:71 and the VL comprises the amino acid sequence of SEQ ID NO:77;
(n) the VH comprises the amino acid sequence of SEQ ID NO:72 and the VL comprises the amino acid sequence of SEQ ID NO:77;
(o) the VH comprises the amino acid sequence of SEQ ID NO:73 and the VL comprises the amino acid sequence of SEQ ID NO:77; or
(p) the VH comprises the amino acid sequence of SEQ ID NO:74 and the VL comprises the amino acid sequence of SEQ ID NO:77.

In a seventeenth embodiment, the antigen binding protein of any of the foregoing embodiments comprises a heavy chain variable region (VH) and a light chain variable region (VL), wherein
(a) the VH comprises the amino acid sequence of SEQ ID NO:55 and the VL comprises the amino acid sequence of SEQ ID NO:78;
(b) the VH comprises the amino acid sequence of SEQ ID NO:60 and the VL comprises the amino acid sequence of SEQ ID NO:78;
(c) the VH comprises the amino acid sequence of SEQ ID NO:61 and the VL comprises the amino acid sequence of SEQ ID NO:78;
(d) the VH comprises the amino acid sequence of SEQ ID NO:62 and the VL comprises the amino acid sequence of SEQ ID NO:78;
(e) the VH comprises the amino acid sequence of SEQ ID NO:63 and the VL comprises the amino acid sequence of SEQ ID NO:78;
(f) the VH comprises the amino acid sequence of SEQ ID NO:64 and the VL comprises the amino acid sequence of SEQ ID NO:78;
(g) the VH comprises the amino acid sequence of SEQ ID NO:65 and the VL comprises the amino acid sequence of SEQ ID NO:78;
(h) the VH comprises the amino acid sequence of SEQ ID NO:66 and the VL comprises the amino acid sequence of SEQ ID NO:78;
(i) the VH comprises the amino acid sequence of SEQ ID NO:67 and the VL comprises the amino acid sequence of SEQ ID NO:78;
(j) the VH comprises the amino acid sequence of SEQ ID NO:68 and the VL comprises the amino acid sequence of SEQ ID NO:78;
(k) the VH comprises the amino acid sequence of SEQ ID NO:69 and the VL comprises the amino acid sequence of SEQ ID NO:78;
(l) the VH comprises the amino acid sequence of SEQ ID NO:70 and the VL comprises the amino acid sequence of SEQ ID NO:78;
(m) the VH comprises the amino acid sequence of SEQ ID NO:71 and the VL comprises the amino acid sequence of SEQ ID NO:78;
(n) the VH comprises the amino acid sequence of SEQ ID NO:72 and the VL comprises the amino acid sequence of SEQ ID NO:78;
(o) the VH comprises the amino acid sequence of SEQ ID NO:73 and the VL comprises the amino acid sequence of SEQ ID NO:78; or
(p) the VH comprises the amino acid sequence of SEQ ID NO:74 and the VL comprises the amino acid sequence of SEQ ID NO:78.

In a eighteenth embodiment, the antigen binding protein of any of the foregoing embodiments comprises a heavy chain variable region (VH) and a light chain variable region (VL), wherein
(a) the VH comprises the amino acid sequence of SEQ ID NO:55 and the VL comprises the amino acid sequence of SEQ ID NO:79;
(b) the VH comprises the amino acid sequence of SEQ ID NO:60 and the VL comprises the amino acid sequence of SEQ ID NO:79;
(c) the VH comprises the amino acid sequence of SEQ ID NO:61 and the VL comprises the amino acid sequence of SEQ ID NO:79;
(d) the VH comprises the amino acid sequence of SEQ ID NO:62 and the VL comprises the amino acid sequence of SEQ ID NO:79;
(e) the VH comprises the amino acid sequence of SEQ ID NO:63 and the VL comprises the amino acid sequence of SEQ ID NO:79;
(f) the VH comprises the amino acid sequence of SEQ ID NO:64 and the VL comprises the amino acid sequence of SEQ ID NO:79;
(g) the VH comprises the amino acid sequence of SEQ ID NO:65 and the VL comprises the amino acid sequence of SEQ ID NO:79;
(h) the VH comprises the amino acid sequence of SEQ ID NO:66 and the VL comprises the amino acid sequence of SEQ ID NO:79;
(i) the VH comprises the amino acid sequence of SEQ ID NO:67 and the VL comprises the amino acid sequence of SEQ ID NO:79;
(j) the VH comprises the amino acid sequence of SEQ ID NO:68 and the VL comprises the amino acid sequence of SEQ ID NO:79;
(k) the VH comprises the amino acid sequence of SEQ ID NO:69 and the VL comprises the amino acid sequence of SEQ ID NO:79;
(l) the VH comprises the amino acid sequence of SEQ ID NO:70 and the VL comprises the amino acid sequence of SEQ ID NO:79;
(m) the VH comprises the amino acid sequence of SEQ ID NO:71 and the VL comprises the amino acid sequence of SEQ ID NO:79;
(n) the VH comprises the amino acid sequence of SEQ ID NO:72 and the VL comprises the amino acid sequence of SEQ ID NO:79;
(o) the VH comprises the amino acid sequence of SEQ ID NO:73 and the VL comprises the amino acid sequence of SEQ ID NO:79; or
(p) the VH comprises the amino acid sequence of SEQ ID NO:74 and the VL comprises the amino acid sequence of SEQ ID NO:79.

In a nineteenth embodiment, the antigen binding protein of any of the foregoing embodiments comprises a heavy chain (HC) and a light chain (LC), wherein
(a) the HC has at least 90% sequence identity with the amino acid sequence of SEQ ID NO:9 and the LC has at least 90% sequence identity with the amino acid sequence of SEQ ID NO:40; or
(b) the HC has at least 90% sequence identity with the amino acid sequence of SEQ ID NO:39 and the LC has at least 90% sequence identity with the amino acid sequence of SEQ ID NO:44.

In a twentieth embodiment, the antigen binding protein is such that
(a) the HC comprises the amino acid sequence of SEQ ID NO:9 and the LC comprises the amino acid sequence of SEQ ID NO:40;
(b) the HC comprises the amino acid sequence of SEQ ID NO: 10 and the LC comprises the amino acid sequence of SEQ ID NO:40;
(c) the HC comprises the amino acid sequence of SEQ ID NO: 11 and the LC comprises the amino acid sequence of SEQ ID NO:40;
(d) the HC comprises the amino acid sequence of SEQ ID NO: 12 and the LC comprises the amino acid sequence of SEQ ID NO:40;
(e) the HC comprises the amino acid sequence of SEQ ID NO: 13 and the LC comprises the amino acid sequence of SEQ ID NO:40;
(f) the HC comprises the amino acid sequence of SEQ ID NO: 14 and the LC comprises the amino acid sequence of SEQ ID NO:40;
(g) the HC comprises the amino acid sequence of SEQ ID NO: 15 and the LC comprises the amino acid sequence of SEQ ID NO:40;
(h) the HC comprises the amino acid sequence of SEQ ID NO:16 and the LC comprises the amino acid sequence of SEQ ID NO:40;
(i) the HC comprises the amino acid sequence of SEQ ID NO: 17 and the LC comprises the amino acid sequence of SEQ ID NO:40;
(j) the HC comprises the amino acid sequence of SEQ ID NO: 18 and the LC comprises the amino acid sequence of SEQ ID NO:40;
(k) the HC comprises the amino acid sequence of SEQ NO: 19 and the LC comprises the amino acid sequence of SEQ ID NO:40;
(l) the HC comprises the amino acid sequence of SEQ ID NO:20 and the LC comprises the amino acid sequence of SEQ NO:40;
(m) the HC comprises the amino acid sequence of SEQ ID NO:21 and the LC comprises the amino acid sequence of SEQ NO:40;
(n) the HC comprises the amino acid sequence of SEQ NO:22 and the LC comprises the amino acid sequence of SEQ ID NO:40;
(o) the HC comprises the amino acid sequence of SEQ ID NO:23 and the LC comprises the amino acid sequence of SEQ ID NO:40; or
(p) the HC has comprises the amino acid sequence of SEQ ID NO:39 and the LC comprises the amino acid sequence of SEQ ID NO:44.

In a twenty-first embodiment, the antigen binding protein is such that
(a) the HC comprises the amino acid sequence of SEQ ID NO:24 and the LC comprises the amino acid sequence of SEQ ID NO:40;
(b) the HC comprises the amino acid sequence of SEQ ID NO:25 and the LC comprises the amino acid sequence of SEQ ID NO:40;
(c) the HC comprises the amino acid sequence of SEQ ID NO:26 and the LC comprises the amino acid sequence of SEQ ID NO:40;
(d) the HC comprises the amino acid sequence of SEQ ID NO:27 and the LC comprises the amino acid sequence of SEQ ID NO:40;
(e) the HC comprises the amino acid sequence of SEQ ID NO:28 and the LC comprises the amino acid sequence of SEQ ID NO:40;
(f) the HC comprises the amino acid sequence of SEQ NO:29 and the LC comprises the amino acid sequence of SEQ ID NO:40;
(g) the HC comprises the amino acid sequence of SEQ NO:30 and the LC comprises the amino acid sequence of SEQ ID NO:40;
(h) the HC comprises the amino acid sequence of SEQ NO:31 and the LC comprises the amino acid sequence of SEQ ID NO:40;
(i) the HC comprises the amino acid sequence of SEQ NO:32 and the LC comprises the amino acid sequence of SEQ ID NO:40;
(j) the HC comprises the amino acid sequence of SEQ ID NO:33 and the LC comprises the amino acid sequence of SEQ ID NO:40;
(k) the HC comprises the amino acid sequence of SEQ ID NO:34 and the LC comprises the amino acid sequence of SEQ ID NO:40;
(l) the HC comprises the amino acid sequence of SEQ ID NO:35 and the LC comprises the amino acid sequence of SEQ ID NO:40;
(m) the HC comprises the amino acid sequence of SEQ ID NO:36 and the LC comprises the amino acid sequence of SEQ ID NO:40;
(n) the HC comprises the amino acid sequence of SEQ ID NO:37 and the LC comprises the amino acid sequence of SEQ ID NO:40; or
(o) the HC comprises the amino acid sequence of SEQ ID NO:38 and the LC comprises the amino acid sequence of SEQ ID NO:40.

In a twenty-fifth embodiment, the antigen binding protein is such that
(a) the HC comprises the amino acid sequence of SEQ ID NO: 19 and the LC comprises the amino acid sequence of SEQ ID NO:41;
(b) the HC comprises the amino acid sequence of SEQ ID NO:24 and the LC comprises the amino acid sequence of SEQ ID NO:41;
(c) the HC comprises the amino acid sequence of SEQ ID NO:25 and the LC comprises the amino acid sequence of SEQ ID NO:41;
(d) the HC comprises the amino acid sequence of SEQ ID NO:26 and the LC comprises the amino acid sequence of SEQ ID NO:41;
(e) the HC comprises the amino acid sequence of SEQ ID NO:27 and the LC comprises the amino acid sequence of SEQ ID NO:41;
(f) the HC comprises the amino acid sequence of SEQ ID NO:28 and the LC comprises the amino acid sequence of SEQ ID NO:41;
(g) the HC comprises the amino acid sequence of SEQ ID NO:29 and the LC comprises the amino acid sequence of SEQ ID NO:41;
(h) the HC comprises the amino acid sequence of SEQ ID NO:30 and the LC comprises the amino acid sequence of SEQ ID NO:41;
(i) the HC comprises the amino acid sequence of SEQ ID NO:31 and the LC comprises the amino acid sequence of SEQ ID NO:41;
(j) the HC comprises the amino acid sequence of SEQ ID NO:32 and the LC comprises the amino acid sequence of SEQ ID NO:41;
(k) the HC comprises the amino acid sequence of SEQ ID NO:33 and the LC comprises the amino acid sequence of SEQ ID NO:41;
(l) the HC comprises the amino acid sequence of SEQ ID NO:34 and the LC comprises the amino acid sequence of SEQ ID NO:41;
(m) the HC comprises the amino acid sequence of SEQ ID NO:35 and the LC comprises the amino acid sequence of SEQ ID NO:41;
(n) the HC comprises the amino acid sequence of SEQ ID NO:36 and the LC comprises the amino acid sequence of SEQ ID NO:41;
(o) the HC comprises the amino acid sequence of SEQ ID NO:37 and the LC comprises the amino acid sequence of SEQ ID NO:41; or
(p) the HC comprises the amino acid sequence of SEQ ID NO:38 and the LC comprises the amino acid sequence of SEQ ID NO:41.

In a twenty-second embodiment, the antigen binding protein is such that
(a) the HC comprises the amino acid sequence of SEQ ID NO: 19 and the LC comprises the amino acid sequence of SEQ ID NO:42;
(b) the HC comprises the amino acid sequence of SEQ ID NO:24 and the LC comprises the amino acid sequence of SEQ ID NO:42;
(c) the HC comprises the amino acid sequence of SEQ ID NO:25 and the LC comprises the amino acid sequence of SEQ ID NO:42;
(d) the HC comprises the amino acid sequence of SEQ ID NO:26 and the LC comprises the amino acid sequence of SEQ ID NO:42;
(e) the HC comprises the amino acid sequence of SEQ ID NO:27 and the LC comprises the amino acid sequence of SEQ ID NO:42;
(f) the HC comprises the amino acid sequence of SEQ ID NO:28 and the LC comprises the amino acid sequence of SEQ ID NO:42;
(g) the HC comprises the amino acid sequence of SEQ ID NO:29 and the LC comprises the amino acid sequence of SEQ ID NO:42;
(h) the HC comprises the amino acid sequence of SEQ ID NO:30 and the LC comprises the amino acid sequence of SEQ ID NO:42;
(i) the HC comprises the amino acid sequence of SEQ ID NO:31 and the LC comprises the amino acid sequence of SEQ ID NO:42;
(j) the HC comprises the amino acid sequence of SEQ ID NO:32 and the LC comprises the amino acid sequence of SEQ ID NO:42;
(k) the HC comprises the amino acid sequence of SEQ ID NO:33 and the LC comprises the amino acid sequence of SEQ ID NO:42;
(l) the HC comprises the amino acid sequence of SEQ ID NO:34 and the LC comprises the amino acid sequence of SEQ ID NO:42;
(m) the HC comprises the amino acid sequence of SEQ ID NO:35 and the LC comprises the amino acid sequence of SEQ ID NO:42;
(n) the HC comprises the amino acid sequence of SEQ ID NO:36 and the LC comprises the amino acid sequence of SEQ ID NO:42;
(o) the HC comprises the amino acid sequence of SEQ ID NO:37 and the LC comprises the amino acid sequence of SEQ ID NO:42; or
(p) the HC comprises the amino acid sequence of SEQ ID NO:38 and the LC comprises the amino acid sequence of SEQ ID NO:42.

In a twenty-third embodiment, the antigen binding protein is such that
(a) the HC comprises the amino acid sequence of SEQ ID NO: 19 and the LC comprises the amino acid sequence of SEQ ID NO:43;
(b) the HC comprises the amino acid sequence of SEQ ID NO:24 and the LC comprises the amino acid sequence of SEQ ID NO:43;
(c) the HC comprises the amino acid sequence of SEQ ID NO:25 and the LC comprises the amino acid sequence of SEQ ID NO:43;
(d) the HC comprises the amino acid sequence of SEQ ID NO:26 and the LC comprises the amino acid sequence of SEQ ID NO:43;
(e) the HC comprises the amino acid sequence of SEQ ID NO:27 and the LC comprises the amino acid sequence of SEQ ID NO:43;
(f) the HC comprises the amino acid sequence of SEQ ID NO:28 and the LC comprises the amino acid sequence of SEQ ID NO:43;
(g) the HC comprises the amino acid sequence of SEQ ID NO:29 and the LC comprises the amino acid sequence of SEQ ID NO:43;
(h) the HC comprises the amino acid sequence of SEQ ID NO:30 and the LC comprises the amino acid sequence of SEQ ID NO:43;
(i) the HC comprises the amino acid sequence of SEQ ID NO:31 and the LC comprises the amino acid sequence of SEQ ID NO:43;
(j) the HC comprises the amino acid sequence of SEQ ID NO:32 and the LC comprises the amino acid sequence of SEQ ID NO:43;
(k) the HC comprises the amino acid sequence of SEQ ID NO:33 and the LC comprises the amino acid sequence of SEQ ID NO:43;
(l) the HC comprises the amino acid sequence of SEQ ID NO:34 and the LC comprises the amino acid sequence of SEQ ID NO:43;
(m) the HC comprises the amino acid sequence of SEQ ID NO:35 and the LC comprises the amino acid sequence of SEQ ID NO:43;
(n) the HC comprises the amino acid sequence of SEQ ID NO:36 and the LC comprises the amino acid sequence of SEQ ID NO:43;
(o) the HC comprises the amino acid sequence of SEQ ID NO:37 and the LC comprises the amino acid sequence of SEQ ID NO:43; or
(p) the HC comprises the amino acid sequence of SEQ ID NO:38 and the LC comprises the amino acid sequence of SEQ ID NO:43.

In a twenty-fourth embodiment, antigen binding proteins that compete with the antigen binding protein of any of the foregoing fifteen embodiments for binding to a human LCAT polypeptide of SEQ ID NO:1 are provided.

In a twenty-fifth embodiment, the antigen binding protein
(a) competes with an antigen binding protein comprising: a CDRH1 of SEQ ID NO:81, a CDRH2 of SEQ ID NO:93, a CDRH3 of SEQ ID NO:95, a CDRL1 of SEQ ID NO: 112, a CDRL2 of SEQ ID NO: 114, and a CDRL3 of SEQ ID NO:116;
(b) competes with an antigen binding protein comprising: a CDRH1 of SEQ ID NO:92, a CDRH2 of SEQ ID NO:94, a CDRH3 of SEQ ID NO:111, a CDRL1 of SEQ ID NO: 113, a CDRL2 of SEQ ID NO: 115, and a CDRL3 of SEQ ID NO: 120;
(c) competes with an antigen binding protein comprising a VH of SEQ ID NO:45 and a VL of SEQ ID NO:76; or
(d) competes with an antigen binding protein comprising a VH of SEQ ID NO:75 and a VL of SEQ ID NO:80.

In a twenty-sixthembodiment, the antigen binding protein binds to amino acids located within a region of an LCAT polypeptide consisting of SEQ ID NO: 1, wherein the region is from amino acid 255 to amino acid 389 of SEQ ID NO: 1, inclusive.

In a twenty-seventh embodiment, the antigen binding protein binds to amino acids located within a region of an LCAT polypeptide consisting of SEQ ID NO: 1, wherein the region is from amino acid 255 to amino acid 374 of SEQ ID NO:1, inclusive.

In a twenty-eighth embodiment, the antigen binding protein binds to amino acids within one or more of the following regions:
(a) the region from amino acid 255 to amino acid 262 of SEQ ID NO: 1, inclusive;
(b) the region from amino acid 315 to amino acid 321 of SEQ ID NO: 1, inclusive; and
(c) the region from amino acid 341 to amino acid 374 of SEQ ID NO: 1, inclusive. In a further embodiment, the antigen binding protein binds to amino acids within regions (a), (b) and (c).

In a twenty-ninth embodiment, the antigen binding protein binds to amino acids within one or more of the following regions:
(a) the region from amino acid 255 to amino acid 262 of SEQ ID NO: 1, inclusive;
(b) the region from amino acid 315 to amino acid 321 of SEQ ID NO: 1, inclusive;
(c) the region from amino acid 341 to amino acid 343 of SEQ ID NO: 1, inclusive; and
(d) the region from amino acid 350 to amino acid 374 of SEQ ID NO: 1, inclusive. In yet another embodiment, the antigen binding protein binds to amino acids within regions (a), (b), (c) and (d).

In a thirtieth embodiment, the antigen binding protein binds to
at least 12 of the amino acids selected from the group consisting of S255, R256, M257, A258, W259, P260, Y315, V317, G318, L319, P320, T321, Y341, E342, D343, T350, R351, E354, L355, C356, G357, L358, Q360, R362, V367, H368, L369, P371, H373 and G374 of SEQ ID NO: 1.

In a thirty-first embodiment, the antigen binding protein binds toat least 15 of the amino acids selected from the group consisting of amino acids R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 and H389 of SEQ ID NO: 1.

In a thirty-second embodiment, the antigen binding protein binds to amino acids located within a region of an LCAT polypeptide consisting of SEQ ID NO: 1, wherein
(a) the region is from amino acid 315 to amino acid 399, inclusive, or
(b) the region is from amino acid 343 to amino acid 399, inclusive.

In a thirty-third embodiment, the antigen binding protein binds to amino acids within one or more of the following regions:
(a) the region from amino acid 350 to amino acid 375 of SEQ ID NO:1, inclusive; and
(b) the region from amino acid 385 to amino acid 399 of SEQ ID NO: 1, inclusive. In another embodiment, the antigen binding protein binds to amino acids within regions (a) and (b).

In a thirty-fourth embodiment, the antigen binding protein binds to amino acids within one or more of the following regions:
(a) the region from amino acid 315 to amino acid 317 of SEQ ID NO:1, inclusive;
(b) the region from amino acid 350 to amino acid 375 of SEQ ID NO: 1, inclusive; and
(c) the region from amino acid 385 to amino acid 399 of SEQ IDNO:1, inclusive. In another embodiment, the antigen binding protein binds to amino acids within regions (a), (b) and (c).

In a thirty-fifth embodiment, the antigen binding protein binds to at least 12 of the amino acids selected from the group consisting of Y315, V317, D343, T350, R351, E354, G357, Q360, G361, Q365, P366, V367, H368, L369, L370, P371, L372, H373, I375, L385, E388, H389, A392, L395, G396, A397, Y398 and R399 of SEQ ID NO: 1.

In a thirty-sixth embodiment, the antigen binding protein of binds to at least 12 of the amino acids selected from the group consisting of Y315, V317, E342, D343, T350, R351, E354, G357, Q360, G361, P364, P366, V367, H368, L369, L370, P371, L372, H373, L385, E388, H389, A392, L395, G396, A397, Y398 and R399 of SEQ ID NO:1.

In a thirty-seventh embodiment, the antigen binding protein binds to an epitope, wherein the epitope is located within
(a) amino acid 255 to amino acid 389 of SEQ ID NO: 1;
(b) amino acids 315 to amino acid 399 of SEQ ID NO:1; or
(c) amino acids 342 to amino acid 399 of SEQ ID NO: 1. In certain embodiments, the epitope is a conformational and/or discontinuous epitope.

In a thirty-eighthembodiment, the antigen binding protein of any of the foregoing embodiments has one or more of the following characteristics:
(a) is a monoclonal antibody, a human antibody, a humanized antibody, a chimeric antibody, or a multispecific antibody;
(b) is of the IgGI, IgG2, IgG3, or the IgG4 type;
(c) is a Fab fragment, a Fab' fragment, a F(ab')2 fragment, or an Fv fragment;
(d) is a diabody, a single chain antibody, a domain antibody, or a nanobody;
(e) is derived from a mammalian source selected from mouse, rat, camelid or rabbit; or
(f) is labeled.

In a thirty-ninth embodiment, pharmaceutical compositions comprising at least one antigen binding protein according to any of the foregoing embodiments are provided.

In a fortieth embodiment, nucleic acid molecules encoding the antigen binding protein of any of embodiments one through twenty-five are provided, as are vectors comprising such nucleic acids and host cells comprising such nucleic acids and vectors.

In a forty-first embodiment, antigen binding proteins of any of embodiments one to twenty-six are provided for use in therapy or for the preparation of a medicament.

In a forty-second embodiment, the antigen binding proteins of any of embodiments one to twenty-six are used to decrease cholesterol levels or to increase HDL-C serum levels in a subject.

In forty-third embodiment, antigen binding proteins of any of embodiments one to twenty-six are used in treating or preventing a condition selected from the group consisting of a cholesterol-related disorder, a cardiovascular disease, an inflammatory condition, a thrombosis-related condition, a blood disorder, anemia, chronic kidney disease and a condition associated with LCAT deficiency. In certain embodiments
(a) the cardiovascular disease or cholesterol related disorder is selected from the group consisting of arteriosclerosis, atherosclerosis, stroke, ischemia, peripheral vascular disease, coronary artery disease, coronary heart disease, myocardial infarction, high blood pressure, hypercholerolemia, metabolic syndrome, dyslipidemia, diabetes, insulin-resistance and Alzheimer's disease;
(b) the inflammatory disease is selected from the group consisting of arthritis, sepsis, septic shock, asthma, a chronic pulmonary inflammatory disease, lupus and an inflammatory bowel disease; and
(c) the thrombosis related condition is selected from the group consisting of a myocardial infarction, deep vein thrombosis, embolism, thrombocytopic purpura and microangiopathy.

In a forty-fourth embodiment, the antigen binding protein of any one of embodiments one to twenty six are used or administered in combination with a further active agent selected from the group consisting of a statin, a CETP inhibitor, a cardiovascular agent, a cholesterol lowering agent, an ACE inhibitor, an ACAT inhibitor, an aldosterone antagonist, an alpha blocker, an angiotensin II receptor antagonist, an anti-arrhythmic agent, an apoA-1 agent, a beta blocker, a bile acid sequesterant, a calcium-channel blocker, a dyslipidemia agent, and endothelin receptor antagonist, an LCAT activator, a fibrate, an PPAR agonist, a squalene epoxidase inhibitor, an anti-inflammatory agent, an anti-thrombosis agent, an anti-diabetic agent and a cytokine.

### BRIEF DESCRIPTION OF THE DRAWINGS

FIGS. 1A and 1B show plots of LCAT activation by antibody 27C3 (FIG. 1A) and antibody 27C3(S42A) (FIG. 1B) in both human and cynomolgus monkey plasma.
FIG. 2 is a plot showing the ability of the antibody 27C3(S42A) to increase HDL-C plasma levels in cynomolgus monkeys, with the HDL-raising efficacy lasting 30 days.
FIGS. 3A and 3B are schematic representations of the LCAT/27C3 Fab/22A9 Fab ternary complex and the LCAT/18E5 Fab/25B7 Fab ternary complex, respectively, as determined by x-ray crystallography.

### DETAILED DESCRIPTION

Unless otherwise defined herein, scientific and technical terms used in connection with the present application shall have the meanings that are commonly understood by those of ordinary skill in the art. Further, unless otherwise required by context, singular terms shall include pluralities and plural terms shall include the singular.

Generally, nomenclatures used in connection with, and techniques of, cell and tissue culture, molecular biology, immunology, microbiology, genetics and protein and nucleic acid chemistry and hybridization described herein are those well known and commonly used in the art. The methods and techniques of the present application are generally performed according to conventional methods well known in the art and as described in various general and more specific references that are cited and discussed throughout the present specification unless otherwise indicated. *See, e.g.,* Sambrook et al., Molecular Cloning: A Laboratory Manual, 3rd ed., Cold Spring Harbor Laboratory Press, Cold Spring Harbor, N.Y. (2001), Ausubel et al., Current Protocols in Molecular Biology, Greene Publishing Associates (1992), and Harlow and Lane Antibodies: A Laboratory Manual Cold Spring Harbor Laboratory Press, Cold Spring Harbor, N.Y. (1990). Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well known and commonly used in the art. Standard techniques can be used for chemical syntheses, chemical analyses, pharmaceutical preparation, formulation, and delivery, and treatment of patients.

The terminology used herein is for the purpose of describing particular embodiments only, and is not intended to limit the scope of the disclosed, which is defined solely by the claims.

Other than in the operating examples, or where otherwise indicated, all numbers expressing quantities of ingredients or reaction conditions used herein should be understood as modified in all instances by the term "about." The term "about" when used in connection with percentages may mean ±1%.

### Definitions

As used herein, "lecithin-cholesterol acyltransferase," or "LCAT," refers to an enzyme that can be found naturally in the blood serum of mammals, including humans, that catalyzes the synthesis of cholesterol esters and lysolecithin from phosphatidylcholine and unesterified cholesterol present in plasma lipoproteins. The enzyme is naturally produced primarily by the liver. The amino acid sequence of one specific example of a mature human LCAT polypeptide is set forth in SEQ ID NO: 1. The amino acid sequence of another mature human LCAT polypeptide is set forth in SEQ ID NO:2 (see GenBank Accession No. AAB34898). An exemplary mature LCAT protein from cynomolgus monkey has the amino acid sequence as shown in SEQ ID NO:3. The amino acid sequence of a mature LCAT from mouse is shown in SEQ ID NO:4 (see, GenBank Accession No. NP 032516; see also C. H. Warden et al. (1989) J. Biol. Chem. 264:21573-81). The amino acid sequence of a mature LCAT protein from rabbit is provided in SEQ ID NO:5 (see GenBank NP_001075659). The term LCAT as used herein also includes naturally occurring alleles (e.g., naturally occurring allelic forms of human LCAT).

Mutant forms of human LCAT having the amino acid sequence set forth in SEQ ID NO:1 are sometimes referred to herein in the form XNY, where N refers to the position of the amino acid in the sequence set forth in SEQ ID NO: 1, X refers to the amino acid naturally occurring at position N in the wild-type LCAT (i.e., as in SEQ ID NO:1) and X refers to the amino acid substituted at position N and thus the amino acid present in the mutant LCAT. Thus, for instance, the designation C31Y LCAT is a shorthand form for a mutant LCAT in which the cysteine at position 31 of SEQ ID NO:1 has been replaced by a tyrosine. As another example, the designation L4F, N5D LCAT refers to a double mutant in which the leucine at position 4 and the asparagine at position 5 of SEQ ID NO:1 have been replaced with phenylalanine and aspartic acid, respectively.

The amount of LCAT or LCAT activity in the serum can be determined in various ways. The mass of LCAT can be determined by a competitive double antibody radioimmunoassay, or ELISA. Routine methods also are known for measuring absolute LCAT activity in the serum and for measuring the more informative cholesterol esterification rate. See, e.g., J. J. Albers et al. Methods in Enzymol. 129:763-783 (1986) and M. P. T. Gillett and J. S. Owens, Chapter 7b, pp. 187-201, in Lipoprotein Analysis--A Practical Approach, C. A. Converse and E. R. Skinner, eds. LCAT activity can be determined by measuring the conversion of radiolabeled cholesterol to cholesteryl ester after incubation of the enzyme and radiolabeled lecithin-cholesterol liposome substrates containing apo A-I. Endogenous cholesterol esterification rate can be determined by measuring the rate of conversion of labeled cholesterol to cholesteryl ester after incubation of fresh plasma that is labeled with a trace amount of radioactive cholesterol by equilibration with a ¹⁴C cholesterol-albumin mixture at 4° C. A detailed discussion of LCAT assays is provided in the Examples.

An "antigen binding protein" as used herein means any protein that specifically binds a specified target antigen, such as an LCAT polypeptide (e.g., a human LCAT polypeptide such as provided in SEQ ID NO:1 or 2). The term encompasses intact antibodies that comprise at least two full-length heavy chains and two full-length light chains, as well as derivatives, variants, fragments, and mutations thereof, examples of which include Fab, Fab', F(ab')₂, and Fv fragments. An antigen binding protein also includes domain antibodies such as nanobodies and single-chain antibodies as described further below.

In general, an LCAT antigen binding protein is said to "specifically bind" its target antigen LCAT when the antigen binding protein exhibits essentially background binding to non-LCAT molecules. An antigen binding protein that specifically binds LCAT may, however, cross-react with LCAT polypeptides from different species. Typically, an LCAT antigen binding protein specifically binds human LCAT when the dissociation constant (K_{D}) is ≤10⁻⁷ M as measured via a surface plasma resonance technique (e.g., BIACore, GE-Healthcare Uppsala, Sweden). A method for determining binding affinities using such techniques is described in Example 3. An LCAT antigen binding protein specifically binds human LCAT with "high affinity" when the K_{D} is ≤5x 10⁻⁸ M, and with "very high affinity" when the K_{D} is ≤5x 10⁻⁹ M, again as measured using methods described in Example 3.

"Antigen binding region" means a protein, or a portion of a protein, that specifically binds a specified antigen. For example, that portion of an antigen binding protein that contains the amino acid residues that interact with an antigen and confer on the antigen binding protein its specificity and affinity for the antigen is referred to as "antigen binding region." An antigen binding region typically includes one or more "complementary binding regions" ("CDRs"). Certain antigen binding regions also include one or more "framework" regions. A "CDR" is an amino acid sequence that contributes to antigen binding specificity and affinity. "Framework" regions can aid in maintaining the proper conformation of the CDRs to promote binding between the antigen binding region and an antigen.

A "recombinant protein", including a recombinant LCAT antigen binding protein, is a protein made using recombinant techniques, *i.e*., through the expression of a recombinant nucleic acid as described herein. Methods and techniques for the production of recombinant proteins are well known in the art.

The term "antibody" refers to an intact immunoglobulin of any isotype, or a fragment thereof that can compete with the intact antibody for specific binding to the target antigen, and includes, for instance, chimeric, humanized, fully human, and bispecific antibodies. An "antibody" as such is a species of an antigen binding protein. An intact antibody generally will comprise at least two full-length heavy chains and two full-length light chains, but in some instances may include fewer chains such as antibodies naturally occurring in camelids which may comprise only heavy chains. Antibodies may be derived solely from a single source, or may be "chimeric," that is, different portions of the antibody may be derived from two different antibodies as described further below. The antigen binding proteins, antibodies, or binding fragments may be produced in hybridomas, by recombinant DNA techniques, or by enzymatic or chemical cleavage of intact antibodies. Unless otherwise indicated, the term "antibody" includes, in addition to antibodies comprising two full-length heavy chains and two full-length light chains, derivatives, variants, fragments, and mutations thereof, examples of which include Fab, Fab', F(ab')₂, Fv fragments, domain antibodies such as Nanobodies® and single-chain antibodies as described in more detail below.

The term "light chain" as used with respect to an antibody or fragments thereof includes a full-length light chain and fragments thereof having sufficient variable region sequence to confer binding specificity. A full-length light chain includes a variable region domain, V_{L}, and a constant region domain, C_{L}. The variable region domain of the light chain is at the amino-terminus of the polypeptide. Light chains include kappa chains and lambda chains.

The term "heavy chain" as used with respect to an antibody or fragment thereof includes a full-length heavy chain and fragments thereof having sufficient variable region sequence to confer binding specificity. A full-length heavy chain includes a variable region domain, V_{H}, and three constant region domains, C_{H}1, C_{H}2, and C_{H}3. The V_{H} domain is at the amino-terminus of the polypeptide, and the C_{H} domains are at the carboxyl-terminus, with the C_{H}3 being closest to the carboxy-terminus of the polypeptide. Heavy chains may be of any isotype, including IgG (including IgG1, IgG2, IgG3 and IgG4 subtypes), IgA (including IgA1 and IgA2 subtypes), IgM and IgE.

The term "immunologically functional fragment" (or simply "fragment") of an antibody or immunoglobulin chain (heavy or light chain), as used herein, is an antigen binding protein comprising a portion (regardless of how that portion is obtained or synthesized) of an antibody that lacks at least some of the amino acids present in a full-length chain but which is capable of specifically binding to an antigen. Such fragments are biologically active in that they bind specifically to the target antigen and can compete with other antigen binding proteins, including intact antibodies, for specific binding to a given epitope. In one aspect, such a fragment will retain at least one CDR present in the full-length light or heavy chain, and in some embodiments will comprise a single heavy chain and/or light chain or portion thereof. These biologically active fragments may be produced by recombinant DNA techniques, or may be produced by enzymatic or chemical cleavage of antigen binding proteins, including intact antibodies. Immunologically functional immunoglobulin fragments include, but are not limited to, Fab, Fab', F(ab')₂, Fv, domain antibodies and single-chain antibodies, and may be derived from any mammalian source, including but not limited to human, mouse, rat, camelids or rabbit. It is contemplated further that a functional portion of the antigen binding proteins disclosed herein, for example, one or more CDRs, could be covalently bound to a second protein or to a small molecule to create a therapeutic agent directed to a particular target in the body, possessing bifunctional therapeutic properties, or having a prolonged serum half-life.

An "Fab fragment" is comprised of one light chain and the C_{H}1 and variable regions of one heavy chain. The heavy chain of a Fab molecule cannot form a disulfide bond with another heavy chain molecule.

An "Fc" region contains two heavy chain fragments comprising the C_{H}1 and C_{H}2 domains of an antibody. The two heavy chain fragments are held together by two or more disulfide bonds and by hydrophobic interactions of the C_{H}3 domains.

An "Fab' fragment" contains one light chain and a portion of one heavy chain that contains the V_{H} domain and the C_{H}1 domain and also the region between the C_{H}1 and C_{H}2 domains, such that an interchain disulfide bond can be formed between the two heavy chains of two Fab' fragments to form an F(ab')₂ molecule.

An "F(ab')₂ fragment" contains two light chains and two heavy chains containing a portion of the constant region between the C_{H}1 and C_{H}2 domains, such that an interchain disulfide bond is formed between the two heavy chains. A F(ab')₂ fragment thus is composed of two Fab' fragments that are held together by a disulfide bond between the two heavy chains.

The "Fv region" comprises the variable regions from both the heavy and light chains, but lacks the constant regions.

"Single-chain antibodies" are Fv molecules in which the heavy and light chain variable regions have been connected by a flexible linker to form a single polypeptide chain, which forms an antigen-binding region. Single chain antibodies are discussed in detail in International Patent Application Publication No. WO 88/01649 and United States Patent Nos. 4,946,778 and No. 5,260,203.

A "domain antibody" is an immunologically functional immunoglobulin fragment containing only the variable region of a heavy chain or the variable region of a light chain. Examples of domain antibodies include Nanobodies®. In some instances, two or more V_{H} regions are covalently joined with a peptide linker to create a bivalent domain antibody. The two V_{H} regions of a bivalent domain antibody may target the same or different antigens.

A "bivalent antigen binding protein" or "bivalent antibody" comprises two antigen binding regions. In some instances, the two binding regions have the same antigen specificities. Bivalent antigen binding proteins and bivalent antibodies may be bispecific, *see*, *infra.*

A multispecific antigen binding protein" or "multispecific antibody" is one that targets more than one antigen or epitope.

A "bispecific," "dual-specific" or "bifunctional" antigen binding protein or antibody is a hybrid antigen binding protein or antibody, respectively, having two different antigen binding sites. Bispecific antigen binding proteins and antibodies are a species of multispecific antigen binding protein or multispecific antibody and may be produced by a variety of methods including, but not limited to, fusion of hybridomas or linking of Fab' fragments. *See, e.g.,* Songsivilai and Lachmann, 1990, Clin. Exp. Immunol. 79:315-321; Kostelny et al., 1992, J. Immunol. 148:1547-1553. The two binding sites of a bispecific antigen binding protein or antibody will bind to two different epitopes, which may reside on the same or different protein targets.

The term "compete" when used in the context of antigen binding proteins (*e.g*., antibodies) that compete for the same epitope means competition between antigen binding proteins is determined by an assay in which the antigen binding protein (*e.g*., antibody or immunologically functional fragment thereof) under test prevents or inhibits specific binding of a reference antigen binding protein to a common antigen (*e.g*., LCAT or a fragment thereof). Numerous types of competitive binding assays can be used, for example: solid phase direct or indirect radioimmunoassay (RIA), solid phase direct or indirect enzyme immunoassay (EIA), sandwich competition assay (*see, e.g.,* Stahli et al., 1983, Methods in Enzymology 9:242-253); solid phase direct biotin-avidin EIA (*see, e.g.,* Kirkland et al., 1986, J. Immunol. 137:3614-3619) solid phase direct labeled assay, solid phase direct labeled sandwich assay (*see*, *e.g.*, Harlow and Lane, 1988, Antibodies, A Laboratory Manual, Cold Spring Harbor Press); solid phase direct label RIA using I-125 label (*see, e.g.,* Morel et al., 1988, Molec. Immunol. 25:7-15); solid phase direct biotin-avidin EIA (*see, e.g.,* Cheung, et al., 1990, Virology 176:546-552); and direct labeled RIA (Moldenhauer et al., 1990, Scand. J. Immunol. 32:77-82). Typically, such an assay involves the use of purified antigen bound to a solid surface or cells bearing either of these, an unlabelled test antigen binding protein and a labeled reference antigen binding protein. Competitive inhibition is measured by determining the amount of label bound to the solid surface or cells in the presence of the test antigen binding protein. Usually the test antigen binding protein is present in excess. Antigen binding proteins identified by competition assay (competing antigen binding proteins) include antigen binding proteins binding to the same epitope as the reference antigen binding proteins and antigen binding proteins binding to an adjacent epitope sufficiently proximal to the epitope bound by the reference antigen binding protein for steric hindrance to occur. Additional details regarding methods for determining competitive binding are provided in the examples herein. For instance, competition is determined according to the BIAcore assay method described in **Example 8.** Usually, when a competing antigen binding protein is present in excess, it will inhibit specific binding of a reference antigen binding protein to a common antigen by at least 40%, 45%, 50%, 55%, 60%, 65%, 70% or 75%. In some instances, binding is inhibited by at least 80%, 85%, 90%, 95%, or 97% or more.

The term "antigen" refers to a molecule or a portion of a molecule capable of being bound by a selective binding agent, such as an antigen binding protein (including, *e.g*., an antibody), and additionally capable of being used in an animal to produce antibodies capable of binding to that antigen. An antigen may possess one or more epitopes that are capable of interacting with different antigen binding proteins, *e.g*., antibodies.

The term "epitope" is the portion of a molecule that is bound by an antigen binding protein (for example, an antibody). The term includes any determinant capable of specifically binding to an antigen binding protein, such as an antibody. An epitope can be contiguous or non-contiguous (discontinuous) (e.g., in a polypeptide, amino acid residues that are not contiguous to one another in the polypeptide sequence but that within in context of the molecule are bound by the antigen binding protein). A conformational epitope is an epitope that exists within the conformation of an active protein but is not present in a denatured protein. In certain embodiments, epitopes may be mimetic in that they comprise a three dimensional structure that is similar to an epitope used to generate the antigen binding protein, yet comprise none or only some of the amino acid residues found in that epitope used to generate the antigen binding protein. Most often, epitopes reside on proteins, but in some instances may reside on other kinds of molecules, such as nucleic acids. Epitope determinants may include chemically active surface groupings of molecules such as amino acids, sugar side chains, phosphoryl or sulfonyl groups, and may have specific three dimensional structural characteristics, and/or specific charge characteristics. Generally, antigen binding proteins specific for a particular target antigen will preferentially recognize an epitope on the target antigen in a complex mixture of proteins and/or macromolecules.

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) must be addressed by a particular mathematical model or computer program (*i.e.*, an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073.

In calculating percent identity, the sequences being compared are aligned in a way that gives the largest match between the sequences. The computer program used to determine percent identity is the GCG program package, which includes GAP (Devereux et al., 1984, Nucl. Acid Res. 12:387; Genetics Computer Group, University of Wisconsin, Madison, WI). The computer algorithm GAP is used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. In certain embodiments, a standard comparison matrix (*see*, Dayhoff et al., 1978, Atlas of Protein Sequence and Structure 5:345-352 for the PAM 250 comparison matrix; Henikoff et al., 1992, Proc. Natl. Acad. Sci. U.S.A. 89:10915-10919 for the BLOSUM 62 comparison matrix) is also used by the algorithm.

Recommended parameters for determining percent identity for polypeptides or nucleotide sequences using the GAP program are the following:
Algorithm: Needleman et al., 1970, J. Mol. Biol. 48:443-453;
Comparison matrix: BLOSUM 62 from Henikoff *et al*., 1992, *supra*;
Gap Penalty: 12 (but with no penalty for end gaps)
Gap Length Penalty: 4
Threshold of Similarity: 0

Certain alignment schemes for aligning two amino acid sequences may result in matching of only a short region of the two sequences, and this small aligned region may have very high sequence identity even though there is no significant relationship between the two full-length sequences. Accordingly, the selected alignment method (GAP program) can be adjusted if so desired to result in an alignment that spans at least 50 contiguous amino acids of the target polypeptide.

As used herein, "substantially pure" means that the described species of molecule is the predominant species present, that is, on a molar basis it is more abundant than any other individual species in the same mixture. A substantially pure molecule is a composition wherein the object species comprises at least 50% (on a molar basis) of all macromolecular species present. A substantially pure composition will comprise at least 80%, 85%, 90%, 95%, or 99% of all macromolecular species present in the composition. The object species is purified to essential homogeneity wherein contaminating species cannot be detected in the composition by conventional detection methods and thus the composition consists of a single detectable macromolecular species.

The term "treating" refers to any indicia of success in the treatment or amelioration of an injury, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being. The treatment or amelioration of symptoms can be based on objective or subjective parameters; including the results of a physical examination, neuropsychiatric exams, and/or a psychiatric evaluation. For example, certain means presented herein successfully treat cardiovascular disease such as atherosclerosis by decreasing the incidence of cardiovascular disease, causing remission of cardiovascular disease and/or ameliorating a symptom associated with cardiovascular disease.

An "effective amount" is generally an amount sufficient to reduce the severity and/or frequency of symptoms, eliminate the symptoms and/or underlying cause, prevent the occurrence of symptoms and/or their underlying cause, and/or improve or remediate the damage that results from or is associated with cancer. In some embodiments, the effective amount is a therapeutically effective amount or a prophylactically effective amount. A "therapeutically effective amount" is an amount sufficient to remedy a disease state (e.g. atherosclerosis) or symptoms, particularly a state or symptoms associated with the disease state, or otherwise prevent, hinder, retard or reverse the progression of the disease state or any other undesirable symptom associated with the disease in any way whatsoever. A "prophylactically effective amount" is an amount of a pharmaceutical composition that, when administered to a subject, will have the intended prophylactic effect, e.g., preventing or delaying the onset (or reoccurrence) of cancer, or reducing the likelihood of the onset (or reoccurrence) of cancer or cancer symptoms. The full therapeutic or prophylactic effect does not necessarily occur by administration of one dose, and may occur only after administration of a series of doses. Thus, a therapeutically or prophylactically effective amount may be administered in one or more administrations.

"Atherosclerosis" refers to a condition characterized by the hardening and/or narrowing of the arteries caused by the buildup of athermatous plaque inside the arterial walls. The atheromatous plaque is divided in three components, (1) the atheroma, a nodular accumulation of a soft flaky material at the center of large plaques, composed of macrophages nearest the lumen of the artery; (2) underlying areas of cholesterol crystals; (3) calcification at the outer base of more advanced lesions. Indicators of atherosclerosis include, for example, the development of plaques in the arteries, their calcification, the extent of which can be determined by Sudan IV staining, or the development of foam cells in arteries. The narrowing of the arteries can be determined by coronary angioplasty, ultrafast CT, or ultrasound.

The term "inflammatory disease" or "inflammatory condition" as used herein, means any disease in which an excessive or unregulated inflammatory response leads to excessive inflammatory symptoms, host tissue damage, or loss of tissue function. The inflammatory response is typically due to inflammatory cell activation. The term "inflammatory cell activation," as used herein, means the induction by a stimulus (including, but not limited to, cytokines, antigens or auto-antibodies) of a proliferative cellular response, the production of soluble mediators (including but not limited to cytokines, oxygen radicals, enzymes, prostanoids, or vasoactive amines), or cell surface expression of new or increased numbers of mediators (including, but not limited to, major histocompatability antigens or cell adhesion molecules) in inflammatory cells (including but not limited to monocytes, macrophages, T lymphocytes, B Lymphocytes, granulocytes, polymorphonuclear leukocytes, mast cells, basophils, eosinophils, dendritic cells, and endothelial cells). It will be appreciated by persons skilled in the art that the activation of one or a combination of these phenotypes in these cells can contribute to the initiation, perpetuation, or exacerbation of an inflammatory condition. Additionally, the term "autoimmune disease," as used herein, means any group of disorders in which tissue injury is associated with humoral or cell-mediated responses to the body's own constituents. The term "allergic disease," as used herein, means any symptoms, tissue damage, or loss of tissue function resulting from allergy. The term "arthritic disease," as used herein, means any of a large family of diseases that are characterized by inflammatory lesions of the joints attributable to a variety of etiologies. The term "dermatitis," as used herein, means any of a large family of diseases of the skin that are characterized by inflammation of the skin attributable to a variety of etiologies. The term "transplant rejection," as used herein, means any immune reaction directed against grafted tissue (including organ and cell (e.g., bone marrow)), characterized by a loss of function of the grafted and surrounding tissues, pain, swelling, leukocytosis and thrombocytopenia.

"Thrombosis" and "thrombosis-related disorder" refer to abnormal thrombus formation that causes obstruction of blood vessels and conditions associated with such obstruction. Blood vessels operate under significant shear stresses that are a function of blood flow shear rate. Frequently, there is damage to small blood vessels and capillaries. When these vessels are damaged, hemostasis is triggered to stop the bleeding. Under typical circumstances, such an injury is dealt with through a sequence of events commonly referred to as the "thrombus formation". Thrombus formation is dependent upon platelet adhesion, activation and aggregation and the coagulation cascade that culminates in the conversion of soluble fibrinogen to insoluble fibrin clot. Thrombus formation at site of wound prevents extravasation of blood components. Subsequently, wound healing and clot dissolution occurs and blood vessel integrity and flow is restored.

The term "HDL" refers to the high-density lipoproteins.

The term "LDL", as used herein, means the low-density lipoproteins.

The term "VLDL" refers to the very low density lipoproteins.

The term "increase" and other related terms such as "elevate," when used in the context of LCAT activity or HDL levels or other measures or consequences of LCAT activity, refers to an increase relative to the level existing prior to administration of an LCAT binding protein. Thus, for instance, an increase in the level of HDL in a patient's serum means that the HDL level in the patient's serum is increased after the LCAT antigen binding protein has been administered to the patient relative to the level before the binding protein was administered.

The term "polynucleotide" or "nucleic acid" includes both single-stranded and double-stranded nucleotide polymers. The nucleotides comprising the polynucleotide can be ribonucleotides or deoxyribonucleotides or a modified form of either type of nucleotide. The modifications include base modifications such as bromouridine and inosine derivatives, ribose modifications such as 2',3'-dideoxyribose, and internucleotide linkage modifications such as phosphorothioate, phosphorodithioate, phosphoroselenoate, phosphorodiselenoate, phosphoroanilothioate, phoshoraniladate and phosphoroamidate.

The term "oligonucleotide" means a polynucleotide comprising 200 or fewer nucleotides. For instance, oligonucleotides are 10 to 60 bases in length, or 12, 13, 14, 15, 16, 17, 18, 19, or 20 to 40 nucleotides in length. Oligonucleotides may be single stranded or double stranded, *e.g*., for use in the construction of a mutant gene. Oligonucleotides may be sense or antisense oligonucleotides. An oligonucleotide can include a label, including a radiolabel, a fluorescent label, a hapten or an antigenic label, for detection assays. Oligonucleotides may be used, for example, as PCR primers, cloning primers or hybridization probes.

An "isolated nucleic acid molecule" means a DNA or RNA of genomic, mRNA, cDNA, or synthetic origin or some combination thereof which is not associated with all or a portion of a polynucleotide in which the isolated polynucleotide is found in nature, or is linked to a polynucleotide to which it is not linked in nature. For purposes of this disclosure, it should be understood that "a nucleic acid molecule comprising" a particular nucleotide sequence does not encompass intact chromosomes. Isolated nucleic acid molecules "comprising" specified nucleic acid sequences may include, in addition to the specified sequences, coding sequences for up to ten or even up to twenty other proteins or portions thereof, or may include operably linked regulatory sequences that control expression of the coding region of the recited nucleic acid sequences, and/or may include vector sequences.

Unless specified otherwise, the left-hand end of any single-stranded polynucleotide sequence discussed herein is the 5' end; the left-hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA transcript that are 5' to the 5' end of the RNA transcript are referred to as "upstream sequences;" sequence regions on the DNA strand having the same sequence as the RNA transcript that are 3' to the 3' end of the RNA transcript are referred to as "downstream sequences."

The term "control sequence" refers to a polynucleotide sequence that can affect the expression and processing of coding sequences to which it is ligated. The nature of such control sequences may depend upon the host organism. Control sequences for prokaryotes may include a promoter, a ribosomal binding site, and a transcription termination sequence. For example, control sequences for eukaryotes may include promoters comprising one or a plurality of recognition sites for transcription factors, transcription enhancer sequences, and transcription termination sequences. "Control sequences" can include leader sequences and/or fusion partner sequences.

The term "vector" means any molecule or entity (*e.g*., nucleic acid, plasmid, bacteriophage or virus) used to transfer protein coding information into a host cell.

The term "expression vector" or "expression construct" refers to a vector that is suitable for transformation of a host cell and contains nucleic acid sequences that direct and/or control (in conjunction with the host cell) expression of one or more heterologous coding regions operatively linked thereto. An expression construct may include, but is not limited to, sequences that affect or control transcription, translation, and, if introns are present, affect RNA splicing of a coding region operably linked thereto.

As used herein, "operably linked" means that the components to which the term is applied are in a relationship that allows them to carry out their inherent functions under suitable conditions. For example, a control sequence in a vector that is "operably linked" to a protein coding sequence is ligated thereto so that expression of the protein coding sequence is achieved under conditions compatible with the transcriptional activity of the control sequences.

The term "host cell" means a cell that has been transformed with a nucleic acid sequence and thereby expresses a gene of interest. The term includes the progeny of the parent cell, whether or not the progeny is identical in morphology or in genetic make-up to the original parent cell, so long as the gene of interest is present.

The terms "polypeptide" or "protein" are used interchangeably herein to refer to a polymer of amino acid residues. The terms also apply to amino acid polymers in which one or more amino acid residues is an analog or mimetic of a corresponding naturally occurring amino acid, as well as to naturally occurring amino acid polymers. The terms can also encompass amino acid polymers that have been modified, e.g., by the addition of carbohydrate residues to form glycoproteins, or phosphorylated. Polypeptides and proteins can be produced by a naturally-occurring and non-recombinant cell; or it is produced by a genetically-engineered or recombinant cell, and comprise molecules having the amino acid sequence of the native protein, or molecules having deletions from, additions to, and/or substitutions of one or more amino acids of the native sequence. The terms "polypeptide" and "protein" specifically encompass LCAT antigen-binding proteins, antibodies, or sequences that have deletions from, additions to, and/or substitutions of one or more amino acids of an antigen-binding protein. The term "polypeptide fragment" refers to a polypeptide that has an amino-terminal deletion, a carboxyl-terminal deletion, and/or an internal deletion as compared with the full-length protein. Such fragments may also contain modified amino acids as compared with the full-length protein. For instance, fragments are about five to 500 amino acids long. Furthermore, fragments may be at least 5, 6, 8, 10, 14, 20, 50, 70, 100, 110, 150, 200, 250, 300, 350, 400, or 450 amino acids long. Useful polypeptide fragments include immunologically functional fragments of antibodies, including binding domains. In the case of an LCAT antibody, useful fragments include but are not limited to a CDR region, a variable domain of a heavy or light chain, a portion of an antibody chain or just its variable region including two CDRs, and the like.

The term "isolated protein" means that a subject protein (1) is free of at least some other proteins with which it would normally be found, (2) is essentially free of other proteins from the same source, *e.g*., from the same species, (3) is expressed by a cell from a different species, (4) has been separated from at least about 50 percent of polynucleotides, lipids, carbohydrates, or other materials with which it is associated in nature, (5) is operably associated (by covalent or noncovalent interaction) with a polypeptide with which it is not associated in nature, or (6) does not occur in nature. Typically, an "isolated protein" constitutes at least about 5%, at least about 10%, at least about 25%, or at least about 50% of a given sample. Genomic DNA, cDNA, mRNA or other RNA, of synthetic origin, or any combination thereof may encode such an isolated protein. Preferably, the isolated protein is substantially free from proteins or polypeptides or other contaminants that are found in its natural environment that would interfere with its therapeutic, diagnostic, prophylactic, research or other use.

A "variant" of a polypeptide (*e.g*., an antigen binding protein such as an antibody) comprises an amino acid sequence wherein one or more amino acid residues are inserted into, deleted from and/or substituted into the amino acid sequence relative to another polypeptide sequence. Variants include fusion proteins.

A "derivative" of a polypeptide is a polypeptide (*e.g*., an antigen binding protein such as an antibody) that has been chemically modified in some manner distinct from insertion, deletion, or substitution variants, *e.g*., *via* conjugation to another chemical moiety.

The term "naturally occurring" as used throughout the specification in connection with biological materials such as polypeptides, nucleic acids, host cells, and the like, refers to materials which are found in nature.

"Amino acid" includes its normal meaning in the art. The twenty naturally-occurring amino acids and their abbreviations follow conventional usage. *See*, Immunology-A Synthesis, 2nd Edition, (E. S. Golub and D. R. Green, eds.), Sinauer Associates: Sunderland, Mass. (1991). Stereoisomers (*e.g.*, D-amino acids) of the twenty conventional amino acids, unnatural amino acids such as [alpha]-, [alpha]-disubstituted amino acids, N-alkyl amino acids, and other unconventional amino acids may also be suitable components for polypeptides and are included in the phrase "amino acid." Examples of unconventional amino acids include: 4-hydroxyproline, [gamma]-carboxyglutamate, [epsilon]-N,N,N-trimethyllysine, [epsilon]-N-acetyllysine, O-phosphoserine, N-acetylserine, N-formylmethionine, 3-methylhistidine, 5-hydroxylysine, [sigma]-N-methylarginine, and other similar amino acids and imino acids (*e.g*., 4-hydroxyproline). In the polypeptide notation used herein, the left-hand direction is the amino terminal direction and the right-hand direction is the carboxyl-terminal direction, in accordance with standard usage and convention.

A "subject" or "patient" as used herein can be any mammal. In a typical embodiment, the subject or patient is a human.

### Overview

Agonist antigen binding proteins that bind LCAT, including human LCAT (hLCAT) are provided herein. In one embodiment, the human LCAT has the sequence as such as set forth in SEQ ID NO: 1. In another embodiment, the human LCAT has the sequence as set forth in SEQ ID NO:2. The identification of antigen binding proteins that can act as agonists of LCAT is a significant advance for several reasons. It is believed that the antibodies and other antigen binding proteins that are provided herein are the first to be developed that are capable of activating LCAT. In fact, there are only a very limited number of antibodies known in the art that can activate any enzyme. Furthermore, it is surprising that a large protein such as an antibody can activate a relatively small enzyme such as LCAT; the skilled person would have expected that a large antibody would block access to the enzyme and thus function as an inhibitor rather than an agonist. Moreover, as described in greater detail in the Examples, some of the agonist antigen binding proteins that are provided bind to a region of LCAT that is distinct from antibodies that inhibit LCAT. The antigen binding proteins that are provided thus bind a unique and important region of LCAT. Finally, it has been found that agonist LCAT antigen binding proteins can be prepared by immunizing a host organism with an activated form of LCAT. Although not intending to be bound by any particular theory, it is believed that antibodies prepared by such methods bind and/or stabilize the enzyme in an activated form, thereby resulting in an increase in enzyme activity.

The antigen binding proteins provided are polypeptides into which one or more complementary determining regions (CDRs), as described herein, are embedded and/or joined. In some antigen binding proteins, the CDRs are embedded into a "framework" region, which orients the CDR(s) such that the proper antigen binding properties of the CDR(s) are achieved. Certain antigen binding proteins described herein are antibodies or are derived from antibodies. In other antigen binding proteins, the CDR sequences are embedded in a different type of protein scaffold. The various structures are further described below.

The antigen binding proteins that are described herein have a variety of utilities. The antigen binding proteins, for instance, are useful in specific binding assays, affinity purification of LCAT, and in screening assays to identify other agonists of LCAT activity. Other uses for the antigen binding proteins include, for example, diagnosis of LCAT-associated diseases or conditions and screening assays to determine the presence or absence of LCAT. Given that the antigen binding proteins that are provided are agonists, the LCAT antigen binding proteins have value in therapeutic methods in which it is useful to modulate HDL particle size, increase plasma levels of HDL-C, and increase reverse cholesterol transport. Accordingly, the antigen binding proteins have utility in the treatment and prevention of atherosclerosis, various cardiovascular diseases and cholesterol-related disorders, inflammatory conditions, thrombosis-related conditions, metabolic syndrome, diabetes and insulin-resistance. The LCAT antigen binding proteins are also useful in treating the consequences, symptoms, and/or pathology associated with either genetic or acquired LCAT deficiency and chronic kidney disease (CKD).

### LCAT Antigen Binding Proteins

### General Features

A variety of selective binding agents useful for modulating the activity of LCAT are provided. These agents include, for instance, antigen binding proteins that contain an antigen binding domain (e.g., single chain antibodies, domain antibodies, and polypeptides with an antigen binding region) and specifically bind to a LCAT polypeptide, in particular human LCAT. Some of the agents, for example, are useful in enhancing the activity of LCAT, and can activate one or more activities associated with LCAT.

In general the antigen binding proteins that are provided typically comprise one or more CDRs as described herein.(e.g., 1, 2, 3, 4, 5 or 6). In some instances, the antigen binding protein comprises (a) a polypeptide structure and (b) one or more CDRs that are inserted into and/or joined to the polypeptide structure. The polypeptide structure can take a variety of different forms. For example, it can be, or comprise, the framework of a naturally occurring antibody, or fragment or variant thereof, or may be completely synthetic in nature. Examples of various polypeptide structures are further described below.

In certain embodiments, the polypeptide structure of the antigen binding proteins is an antibody or is derived from an antibody. Accordingly, examples of certain antigen binding proteins that are provided include, but are not limited to, monoclonal antibodies, bispecific antibodies, minibodies, domain antibodies such as Nanobodies®, synthetic antibodies (sometimes referred to herein as "antibody mimetics"), chimeric antibodies, humanized antibodies, human antibodies, antibody fusions (sometimes referred to as "antibody conjugates"), and portions or fragments of each, respectively. In some instances, the antigen binding protein is an immunological fragment of a complete antibody (e.g., a Fab, a Fab', a F(ab')₂, or a scFv).

The antigen binding proteins as provided herein specifically bind to a human LCAT. In a specific embodiment, the antigen binding protein specifically binds to human LCAT comprising or consisting of the amino acid sequence of SEQ ID NO: 1.

The antigen binding proteins that are provided are agonists and typically have one, two, three, four, five or all six of the following characteristics:
(a) ability to increase the enzymatic activity of LCAT, where the activity can be measured, for example, by the methods described herein (see, e.g., **Example 1**). The increase can be at least 10, 25, 50, 100, 200, 300, 400% or more relative to the activity of LCAT of SEQ ID NO:1 under comparable conditions. In some embodiments, the increase is at least two- or three-fold higher as compared to LCAT of SEQ ID NO:1.
(b) ability when administered to a patient to elevate HDL levels in vivo;
(c) ability to increase plasma levels of apoA-I;
(d) ability to increase the plasma levels of LCAT protein (e.g., by 10, 20, 30, 40, 50, 60, 70, 80, 90, 100, or 200% or more);
(e) ability to decrease plasma levels of triglyceride (TG); and
(f) ability to promote reverse cholesterol transport.

In one embodiment, an LCAT antigen binding protein has one or more of the following activities:
(a) binds human LCAT such that K_{D} is ≤200 nM, is ≤150 nM, is ≤100 nM , is ≤50 nM, is ≤10 nM, is ≤5 nM, is ≤2 nM, or is ≤1 nM (e..g, as determined by BiaCore such as described in Example 3);
(b) has a half-life in human serum of at least 3 days;
(c) binds human LCAT of SEQ ID NO:1 and cyno LCAT of SEQ ID NO:3;
(d) binds human LCAT of SEQ ID NO:1 with K_{D} ≤10 nM and binds cyno LCAT of SEQ ID NO:3 with K_{D} ≤500 nM;
(e) binds human LCAT of SEQ ID NO:1 with K_{D} ≤10 nM and binds cyno LCAT of SEQ ID NO:3 with K_{D} ≤100 nM;
(f) binds human LCAT of SEQ ID NO:1 with K_{D} ≤10 nM and binds cyno LCAT of SEQ ID NO:3 with K_{D} ≤10 nM; and
(g) binds human LCAT of SEQ ID NO:1 but does not bind cyno LCAT of SEQ ID NO:3.

Some antigen binding proteins that are provided have an on-rate (kₐ) for LCAT of at least 10⁴/ M x seconds, at least 10⁵/M x seconds, or at least 10⁶/M x seconds as measured, for instance, as described in the examples below. Certain antigen binding proteins that are provided have a slow dissociation rate or off-rate. Some antigen binding proteins, for instance, have a k_{d} (off-rate) of 1x 10⁻² s⁻¹, or 1x 10⁻³ s⁻¹, or 1x 10⁻⁴ s⁻¹, or 1x 10⁻⁵ s⁻¹. In certain embodiments, the antigen binding protein has a K_{D} (equilibrium binding affinity) of less than 25 pM, 50 pM, 100 pM, 500 pM, 1 nM, 5 nM, 10 nM, 25 nM or 50 nM.

In another embodiment, an antigen-binding protein is provided having a half-life of at least one day *in vitro* or *in vivo* (*e.g*., when administered to a human subject). In one embodiment, the antigen binding protein has a half-life of at least three days. In various other embodiments, the antigen binding protein has a half-life of 4, 5, 6, 7, 8, 9, 10, 15, 20, 25, 30, 40, 50, or 60 days or longer. In another embodiment, the antigen binding protein is derivatized or modified such that it has a longer half-life as compared to the underivatized or unmodified antibody. In another embodiment, the antigen binding protein contains point mutations to increase serum half life. Further details regarding such mutant and derivatized forms are provided below.

### LCAT Antigen Binding Proteins with Naturally Occurring Antibody Structure

Some of the antigen binding proteins that are provided have the structure typically associated with naturally occurring antibodies. The structural units of these antibodies typically comprise one or more tetramers, each composed of two identical couplets of polypeptide chains, though some species of mammals also produce antibodies having only a single heavy chain. In a typical antibody, each pair or couplet includes one full-length "light" chain (in certain embodiments, about 25 kDa) and one full-length "heavy" chain (in certain embodiments, about 50-70 kDa). Each individual immunoglobulin chain is composed of several "immunoglobulin domains", each consisting of roughly 90 to 110 amino acids and expressing a characteristic folding pattern. These domains are the basic units of which antibody polypeptides are composed. The amino-terminal portion of each chain typically includes a variable domain that is responsible for antigen recognition. The carboxy-terminal portion is more conserved evolutionarily than the other end of the chain and is referred to as the "constant region" or "C region". Human light chains generally are classified as kappa and lambda light chains, and each of these contains one variable domain and one constant domain. Heavy chains are typically classified as mu, delta, gamma, alpha, or epsilon chains, and these define the antibody's isotype as IgM, IgD, IgG, IgA, and IgE, respectively. IgG has several subtypes, including, but not limited to, IgG1, IgG2, IgG3, and IgG4. IgM subtypes include IgM, and IgM2. IgA subtypes include IgA1 and IgA2. In humans, the IgA and IgD isotypes contain four heavy chains and four light chains; the IgG and IgE isotypes contain two heavy chains and two light chains; and the IgM isotype contains five heavy chains and five light chains. The heavy chain C region typically comprises one or more domains that may be responsible for effector function. The number of heavy chain constant region domains will depend on the isotype. IgG heavy chains, for example, each contain three C region domains known as C_{H}1, C_{H}2 and C_{H}3. The antibodies that are provided can have any of these isotypes and subtypes. In certain embodiments, the LCAT antibody is of the IgG1, IgG2, or IgG4 subtype.

In full-length light and heavy chains, the variable and constant regions are joined by a "J" region of about twelve or more amino acids, with the heavy chain also including a "D" region of about ten more amino acids. See, e.g. Fundamental Immunology, 2nd ed., Ch. 7 (Paul, W., ed.) 1989, New York: Raven Press. The variable regions of each light/heavy chain pair typically form the antigen binding site.

One example of an IgG2 heavy constant domain of an exemplary LCAT monoclonal antibody has the amino acid sequence:

One example of a lambda light chain constant domain of an exemplary LCAT monoclonal antibody has the amino acid sequence:

An example of a kappa light chain constant domain of an exemplary LCAT monoclonal antibody has the amino acid sequence:

For the antibodies provided herein, the variable regions of immunoglobulin chains generally exhibit the same overall structure, comprising relatively conserved framework regions (FR) joined by three hypervariable regions, more often called "complementarity determining regions" or CDRs. The CDRs from the two chains of each heavy chain/light chain pair mentioned above typically are aligned by the framework regions to form a structure that binds specifically with a specific epitope on LCAT. From N-terminal to C-terminal, naturally-occurring light and heavy chain variable regions both typically conform with the following order of these elements: FR1, CDR1, FR2, CDR2, FR3, CDR3 and FR4. A numbering system has been devised for assigning numbers to amino acids that occupy positions in each of these domains. This numbering system is defined in Kabat Sequences of Proteins of Immunological Interest (1987 and 1991, NIH, Bethesda, Md.), or Chothia & Lesk, 1987, J. Mol. Biol. 196:901-917; Chothia et al., 1989, Nature 342:878-883.

The various heavy chain and light chain variable regions provided herein are depicted in **TABLE 2**. Each of these variable regions may be attached to the above heavy and light chain constant regions to form a complete antibody heavy and light chain, respectively. Further, each of the so generated heavy and light chain sequences may be combined to form a complete antibody structure. It should be understood that the heavy chain and light chain variable regions provided herein can also be attached to other constant domains having different sequences than the exemplary sequences listed above.

Specific examples of some of the full length heavy and light chains of the antibodies that are provided and their corresponding amino acid sequences are summarized in **TABLE 1**.

**TABLE 1: Exemplary Heavy and Light Chains**

| Designation | SEQ ID NO: | Amino Acid Sequence |
|---|---|---|
| H1 | 9 | |
| H2 | 10 | |
| | | |
| H3 | 11 | |
| | | |
| H4 | 12 | |
| | | |
| H5 | 13 | |
| | | |
| H6 | 14 | |
| | | |
| H7 | 15 | |
| | | |
| H8 | 16 | |
| | | |
| H9 | 17 | |
| | | |
| H10 | 18 | |
| | | |
| H11 | 19 | |
| H12 | 20 | |
| H13 | 21 | |
| | | |
| H14 | 22 | |
| H15 | 23 | |
| | | |
| H16 | 24 | |
| H17 | 25 | |
| | | |
| H18 | 26 | |
| H19 | 27 | |
| H20 | 28 | |
| | | |
| H21 | 29 | |
| H22 | 30 | |
| | | |
| H23 | 31 | |
| H24 | 32 | |
| | | |
| H25 | 33 | |
| H26 | 34 | |
| | | |
| H27 | 35 | |
| H28 | 36 | |
| H29 | 37 | |
| | | |
| H30 | 38 | |
| H31 | 39 | |
| L1 | 40 | |
| L2 | 41 | |
| L3 | 42 | |
| | | |
| L4 | 43 | |
| L5 | 44 | |

Each of the exemplary heavy chains (H1, H2, H3 through H12) listed in **TABLE 1** can be combined with any of the exemplary light chains shown in **TABLE 1** (L1 or L2) to form an antibody. Examples of such combinations include H1 combined with either of L1 or L2; H2 combined with either of L1 or L2; H3 combined with either of L1 or L2, and so on. In some instances, the antibodies include at least one heavy chain and one light chain from those listed in **TABLE 1**. In other embodiments, the antibodies comprise two different heavy chains and two different light chains listed in **TABLE 1**. In still other instances, the antibodies contain two identical light chains and two identical heavy chains. As an example, an antibody or immunologically functional fragment may include two H1 heavy chains and two L1 light chains, or two H2 heavy chains and two L2 light chains and other similar combinations of pairs of light chains and pairs of heavy chains as listed in **TABLE 1**.

Other antigen binding proteins that are provided are variants of antibodies formed by combination of the heavy and light chains shown in **TABLE 1** and comprise light and/or heavy chains that each have at least 70%, 75%, 80%, 85%, 90%, 95%, 97% or 99% identity to the amino acid sequences of these chains. In some instances, such antibodies include at least one heavy chain and one light chain, whereas in other instances the variant forms contain two identical light chains and two identical heavy chains

### Variable Domains of Exemplary LCAT Antigen Binding Proteins

Also provided are antigen binding proteins that contain a heavy chain variable region selected from the group consisting of V_{H}1, V_{H}2, V_{H}3, V_{H}4, V_{H}5, V_{H}6, V_{H}7, V_{H}8, V_{H}9, V_{H}10, V_{H}11, and V_{H}12 and/or a light chain variable region selected from the group consisting of V_{L}1, or V_{L}2, as shown in **TABLE 2** below, and immunologically functional fragments, derivatives, muteins and variants of these light chain and heavy chain variable regions.

Antigen binding proteins of this type can generally be designated by the formula " V_{H}x/ V_{L}y," where "x" corresponds to the number of heavy chain variable regions and "y" corresponds to the number of the light chain variable regions (in general, x and y are each 1 or 2) as listed in TABLE 2. However, the LCAT antigen binding proteins can also include a single light chain variable domain or a single heavy chain variable domain, provided the individual domain can bind an LCAT polypeptide (e.g., SEQ ID NO:1).

**TABLE 2: Exemplary V_{H} and V_{L} Chains**

| Designation | SEQ ID NO: | Amino Acid Sequence |
|---|---|---|
| V_{H}1 | 45 | |
| V_{H}2 | 46 | |
| V_{H}3 | 47 | |
| V_{H}4 | 48 | |
| V_{H}5 | 49 | |
| V_{H}6 | 50 | |
| V_{H}7 | 51 | |
| V_{H}8 | 52 | |
| V_{H}9 | 53 | |
| V_{H}10 | 54 | |
| V_{H}11 | 55 | |
| V_{H}12 | 56 | |
| V_{H}13 | 57 | |
| V_{H}14 | 58 | |
| V_{H}15 | 59 | |
| V_{H}16 | 60 | |
| V_{H}17 | 61 | |
| V_{H}18 | 62 | |
| V_{H}19 | 63 | |
| V_{H}20 | 64 | |
| V_{H}21 | 65 | |
| V_{H}22 | 66 | |
| V_{H}23 | 67 | |
| V_{H}24 | 68 | |
| V_{H}25 | 69 | |
| V_{H}26 | 70 | |
| V_{H}27 | 71 | |
| V_{H}28 | 72 | |
| V_{H}29 | 73 | |
| V_{H}30 | 74 | |
| V_{H}31 | 75 | |
| V_{L}1 | 76 | |
| V_{L}2 | 77 | |
| V_{L}3 | 78 | |
| V_{L}4 | 79 | |
| V_{L}5 | 80 | |

Each of the heavy chain variable regions listed in **TABLE 2** may be combined with any of the light chain variable regions shown in **TABLE 2** to form an antigen binding protein. Examples of such combinations include V_{H}1 combined with either of V_{L}1 or V_{L}2; V_{H}2 combined with either of V_{L}1 or V_{L}2; V_{H}3 combined with either of V_{L}1, or V_{L}2, and so on.

In some embodiments, the antigen binding protein includes at least one heavy chain variable region and/or one light chain variable region from those listed in **TABLE 2**. In some instances, the antigen binding protein includes at least two different heavy chain variable regions and/or light chain variable regions from those listed in **TABLE 2**. An example of such an antigen binding protein comprises (a) one V_{H}1, and (b) one of V_{H}2, V_{H}3, V_{H}4, V_{H}5, V_{H}6, V_{H}7, V_{H}8, V_{H}9, V_{H}10, V_{H}11 or V_{H}12. Another example comprises (a) one V_{H}2, and (b) one of V_{H}1, V_{H}3, V_{H}4, V_{H}5, V_{H}6, V_{H}7, V_{H}8, V_{H}9, V_{H}10, V_{H}11 or V_{H}12. Yet another example comprises (a) one V_{H}3, and (b) one of V_{H}1, V_{H}2, V_{H}4, V_{H}5, V_{H}6, V_{H}7, V_{H}8, V_{H}9, V_{H}10, V_{H}11, or V_{H}12 etc.

The various combinations of heavy chain variable regions may be combined with any of the various combinations of light chain variable regions.

In certain embodiments, the antigen binding protein contains two identical light chain variable regions and/or two identical heavy chain variable regions. As an example, the antigen binding protein may be an antibody or immunologically functional fragment that includes two light chain variable regions and two heavy chain variable regions in combinations of pairs of light chain variable regions and pairs of heavy chain variable regions as listed in **TABLE 2**.

Some antigen binding proteins that are provided comprise a heavy chain variable domain comprising a sequence of amino acids that differs from the sequence of a heavy chain variable domain selected from V_{H}1, V_{H}2, V_{H}3, V_{H}4, V_{H}5, V_{H}6, V_{H}7, V_{H}8, V_{H}9, V_{H}10, V_{H}11 or V_{H}12 at only 1, 2, 3, 4, 5, 6, 7, 8, 9, 10, 11, 12, 13, 14 or 15 amino acid residues, wherein each such sequence difference is independently either a deletion, insertion or substitution of one amino acid, with the deletions, insertions and/or substitutions resulting in no more than 15 amino acid changes relative to the foregoing variable domain sequences. The heavy chain variable region in some antigen binding proteins comprises a sequence of amino acids that has at least 70%, 75%, 80%, 85%, 90%, 95%, 97% or 99% sequence identity to the amino acid sequences of the heavy chain variable region of V_{H}1, V_{H}2, V_{H}3, V_{H}4, V_{H}5, V_{H}6, V_{H}7, V_{H}8, V_{H}9, V_{H}10, V_{H}11 or V_{H}12.

Certain antigen binding proteins comprise a light chain variable domain comprising a sequence of amino acids that differs from the sequence of a light chain variable domain selected from V_{L}1 or V_{L}2 at only 1, 2, 3, 4, 5, 6, 7, 8, 9, 10, 11, 12, 13, 14 or 15 amino acid residues, wherein each such sequence difference is independently either a deletion, insertion or substitution of one amino acid, with the deletions, insertions and/or substitutions resulting in no more than 15 amino acid changes relative to the foregoing variable domain sequences. The light chain variable region in some antigen binding proteins comprises a sequence of amino acids that has at least 70%, 75%, 80%, 85%, 90%, 95%, 97% or 99% sequence identity to the amino acid sequences of the light chain variable region of V_{L}1 or V_{L}2.

### CDRs of Exemplary LCAT Antigen Binding Proteins

The antigen binding proteins disclosed herein are polypeptides into which one or more CDRs are grafted, inserted and/or joined. An antigen binding protein can have 1, 2, 3, 4, 5 or 6 CDRs. An antigen binding protein thus can have, for example, one heavy chain CDR1 ("CDRH1"), and/or one heavy chain CDR2 ("CDRH2"), and/or one heavy chain CDR3 ("CDRH3"), and/or one light chain CDR1 ("CDRL1"), and/or one light chain CDR2 ("CDRL2"), and/or one light chain CDR3 ("CDRL3"). Some antigen binding proteins include both a CDRH3 and a CDRL3. Specific heavy and light chain CDRs are identified in **TABLES 3A and 3B**, respectively.

Complementarity determining regions (CDRs) and framework regions (FR) of a given antibody may be identified using the system described by Kabat et al. in Sequences of Proteins of Immunological Interest, 5th Ed., US Dept. of Health and Human Services, PHS, NIH, NIH Publication no. 91-3242, 1991. Certain antibodies that are disclosed herein comprise one or more amino acid sequences that are identical or have substantial sequence identity to the amino acid sequences of one or more of the CDRs presented in **TABLE 3A** (CDRHs) and **TABLE 3B** (CDRLs).

**TABLE 3A: Exemplary CDRH Sequences**

| Designation | Amino Acid Sequence | SEQ ID NO: |
|---|---|---|
| CDRH1-1 | SGGYNWS | 81 |
| CDRH1-2 | SGGYQWS | 82 |
| CDRH1-3 | SGGYDWS | 83 |
| CDRH1-4 | SGGYEWS | 84 |
| CDRH1-5 | SGGYAWS | 85 |
| CDRH1-6 | SGGYSWS | 86 |
| CDRH1-7 | SGGYTWS | 87 |
| CDRH1-8 | SGGYVWS | 88 |
| CDRH1-9 | SGGYYWS | 89 |
| CDRH1-10 | SGGYNWV | 90 |
| CDRH1-11 | SGGYNWA | 91 |
| CDRH1-12 | GYYMH | 92 |
| | | |
| CDRH2-1 | YIYYSGSTYYNPSLKS | 93 |
| CDRH2-2 | WINPNSGGTNYAQKFQG | 94 |
| | | |
| CDRH3-1 | ERGYCSSTSCSRVMDV | 95 |
| CDRH3-2 | ERGYCSSTTCSRVMDV | 96 |
| CDRH3-3 | ERGYCSSTSCTRVMDV | 97 |
| CDRH3-4 | ERGYCSSTSCSRIMDV | 98 |
| CDRH3-5 | ERGYCSSTSCSRVIDV | 99 |
| CDRH3-6 | ERGYCSSTTCTRVMDV | 100 |
| CDRH3-7 | ERGYCSSTTCSRIMDV | 101 |
| CDRH3-8 | ERGYCSSTTCSRVIDV | 102 |
| CDRH3-9 | ERGYCSSTSCTRIMDV | 103 |
| CDRH3-10 | ERGYCSSTSCTRVIDV | 104 |
| CDRH3-11 | ERGYCSSTSCSRIIDV | 105 |
| CDRH3-12 | ERGYCSSTTCTRIMDV | 106 |
| CDRH3-13 | ERGYCSSTTCTRVIDV | 107 |
| CDRH3-14 | ERGYCSSTTCSRIIDV | 108 |
| CDRH3-15 | ERGYCSSTSCTRIIDV | 109 |
| CDRH3-16 | ERGYCSSTTCTRIIDV | 110 |
| CDRH3-17 | GRWELYAFDI | 111 |

**TABLE 3B: Exemplary CDRL Sequences**

| Designation | Amino Acid Sequence | SEQ ID NO: |
|---|---|---|
| CDRL1-1 | QGDSLRSYYAS | 112 |
| CDRL1-2 | RASQSVSGSYLT | 113 |
| | | |
| CDRL2-1 | GKNNRPS | 114 |
| CDRL2-2 | GASSRAT | 115 |
| | | |
| CDRL3-1 | NSRDNIGNHQV | 116 |
| CDRL3-2 | GSRDNIGNHQV | 117 |
| CDRL3-3 | NSRDNVGNHQV | 118 |
| CDRL3-4 | GSRDNVGNHQV | 119 |
| CDRL3-5 | QQYGGSPPFT | 120 |

The structure and properties of CDRs within a naturally occurring antibody has been described, *supra.* Briefly, in a traditional antibody, the CDRs are embedded within a framework in the heavy and light chain variable region where they constitute the regions responsible for antigen binding and recognition. A variable region comprises at least three heavy or light chain CDRs, *see, supra* (Kabat et al., 1991, Sequences of Proteins of Immunological Interest, Public Health Service N.I.H., Bethesda, MD; *see* also Chothia and Lesk, 1987, J. Mol. Biol. 196:901-917; Chothia et al., 1989, Nature 342: 877-883), within a framework region (designated framework regions 1-4, FR1, FR2, FR3, and FR4, by Kabat *et al*., 1991, *supra*; *see* also Chothia and Lesk, 1987, *supra*). The CDRs provided herein, however, may not only be used to define the antigen binding domain of a traditional antibody structure, but may be embedded in a variety of other polypeptide structures, as described herein.

In one embodiment, the CDRs provided are (A) a CDRH selected from the group consisting of (i) a CDRH1 selected from the group consisting of SEQ ID NO:81-92; (ii) a CDRH2 selected from the group consisting of SEQ ID NO:93-94; (iii) a CDRH3 selected from the group consisting of SEQ ID NO:95-111; and (iv) a CDRH of (i), (ii) and (iii) that contains one or more amino acid substitutions, deletions or insertions of no more than five, four, three, two, or one amino acids; (B) a CDRL selected from the group consisting of (i) a CDRL1 selected from the group consisting of SEQ ID NO: 112-113 (ii) a CDRL2 selected from the group consisting of SEQ ID NO:114-115; (iii) a CDRL3 selected from the group consisting of SEQ ID NO:116-120; and (iv) a CDRL of (i), (ii) and (iii) that contains one or more amino acid substitutions, deletions or insertions of no more than five, four, three, two, or one amino acids amino acids.

In another embodiment, an antigen binding protein includes 1, 2, 3, 4, 5, or 6 variant forms of the CDRs listed in **TABLES 3A and 3B**, each having at least 80%, 85%, 90%, 95%, 96%, 97%, 98%, or 99% sequence identity to a CDR sequence listed in **TABLES 3A and 3B**. Some antigen binding proteins include 1, 2, 3, 4, 5, or 6 of the CDRs listed in **TABLES 3A and 3B**, each or collectively differing by no more than 1, 2, 3, 4 or 5 amino acids from the CDRs listed in these tables.

### Exemplary Antigen Binding Proteins

The sequence information for specific antibodies prepared and identified as described in the Examples below is summarized in **TABLE 4**. Thus, in an embodiment, an antigen binding protein is an antibody with the CDR, variable domain and/or light and heavy chain sequences as specified in one of the rows of **TABLE 4**.

**TABLE 4:**

| Ref. No. | Full Heavy SEQ ID NO: | Full Light SEQ ID NO: | Variable Heavy SEQ ID NO: | Variable Light SEQ ID NO: | CDRH1 SEQ ID NO: | CDRH2 SEQ ID NO: | CDRH3 SEQ D NO: | CDRL1 SEQ ID NO: | CDRL2 SEQ ID NO: | CDRL3 SEQ ID NO: |
|---|---|---|---|---|---|---|---|---|---|---|
| 27C3 | 9 | 40 | 45 | 76 | 81 | 93 | 95 | 112 | 114 | 116 |
| 27C3(N40Q) | 10 | 40 | 46 | 76 | 82 | 93 | 95 | 112 | 114 | 116 |
| 27C3(N40D) | 11 | 40 | 47 | 76 | 83 | 93 | 95 | 112 | 114 | 116 |
| 27C3(N40E) | 12 | 40 | 48 | 76 | 84 | 93 | 95 | 112 | 114 | 116 |
| 27C3(N40A) | 13 | 40 | 49 | 76 | 85 | 93 | 95 | 112 | 114 | 116 |
| 27C3(N40S) | 14 | 40 | 50 | 76 | 86 | 93 | 95 | 112 | 114 | 116 |
| 27C3(N40T) | 15 | 40 | 51 | 76 | 87 | 93 | 95 | 112 | 114 | 116 |
| 27C3(N40V) | 16 | 40 | 52 | 76 | 88 | 93 | 95 | 112 | 114 | 116 |
| 27C3(N40Y) | 17 | 40 | 53 | 76 | 89 | 93 | 95 | 112 | 114 | 116 |
| 27C3(S42V) | 18 | 40 | 54 | 76 | 90 | 93 | 95 | 112 | 114 | 116 |
| 27C3(S42A) | 19 | 40 | 55 | 76 | 91 | 93 | 95 | 112 | 114 | 116 |
| 27C3(S131T) | 20 | 40 | 56 | 76 | 81 | 93 | 96 | 112 | 114 | 116 |
| 27C3(S133T) | 21 | 40 | 57 | 76 | 81 | 93 | 97 | 112 | 114 | 116 |
| 27C3(V135I) | 22 | 40 | 58 | 76 | 81 | 93 | 98 | 112 | 114 | 116 |
| 27C3(M136I) | 23 | 40 | 59 | 76 | 81 | 93 | 99 | 112 | 114 | 116 |
| 27C3(S42A,S131T) | 24 | 40 | 60 | 76 | 91 | 93 | 96 | 112 | 114 | 116 |
| 27C3(S42A,S133T) | 25 | 40 | 61 | 76 | 91 | 93 | 97 | 112 | 114 | 116 |
| 27C3(S42A,V135I) | 26 | 40 | 62 | 76 | 91 | 93 | 98 | 112 | 114 | 116 |
| 27C3(S42A,M136I) | 27 | 40 | 63 | 76 | 91 | 93 | 99 | 112 | 114 | 116 |
| 27C3(S42A,S131T,S133T) | 28 | 40 | 64 | 76 | 91 | 93 | 100 | 112 | 114 | 116 |
| 27C3(S42A,S131T,V135I) | 29 | 40 | 65 | 76 | 91 | 93 | 101 | 112 | 114 | 116 |
| 27C3(S42A,S131T,M136I) | 30 | 40 | 66 | 76 | 91 | 93 | 102 | 112 | 114 | 116 |
| 27C3(S42A,S133T,V135I) | 31 | 40 | 67 | 76 | 91 | 93 | 103 | 112 | 114 | 116 |
| 27C3(S42A,S133T,M136I) | 32 | 40 | 68 | 76 | 91 | 93 | 104 | 112 | 114 | 116 |
| 27C3(S42A,V135I,M136I) | 33 | 40 | 69 | 76 | 91 | 93 | 105 | 112 | 114 | 116 |
| 27C3(S42A,S131T,S133T,V135I) | 34 | 40 | 70 | 76 | 91 | 93 | 106 | 112 | 114 | 116 |
| 27C3(S42A,S131T,S133T,M136I) | 35 | 40 | 71 | 76 | 91 | 93 | 107 | 112 | 114 | 116 |
| 27C3(S42A,S131T,V135I,M136I) | 36 | 40 | 72 | 76 | 91 | 93 | 108 | 112 | 114 | 116 |
| 27C3(S42A,S133T,V135I,M136I) | 37 | 40 | 73 | 76 | 91 | 93 | 109 | 112 | 114 | 116 |
| 27C3(S42A,S131T,S133T,V135I,M136I) | 38 | 40 | 74 | 76 | 91 | 93 | 110 | 112 | 114 | 116 |
| 27C3(S42A/N107G) | 19 | 41 | 55 | 77 | 91 | 93 | 95 | 112 | 114 | 117 |
| 27C3(S42A,S131T/N107G) | 24 | 41 | 60 | 77 | 91 | 93 | 96 | 112 | 114 | 117 |
| 27C3(S42A,S133T/N107G) | 25 | 41 | 61 | 77 | 91 | 93 | 97 | 112 | 114 | 117 |
| 27C3(S42A,V135I/N107G) | 26 | 41 | 62 | 77 | 91 | 93 | 98 | 112 | 114 | 117 |
| 27C3(S42A,M136I/N107G) | 27 | 41 | 63 | 77 | 91 | 93 | 99 | 112 | 114 | 117 |
| 27C3(S42A,S131T,S133T/N107G) | 28 | 41 | 64 | 77 | 91 | 93 | 100 | 112 | 114 | 117 |
| 27C3(S42A,S131T,V135I/N107G) | 29 | 41 | 65 | 77 | 91 | 93 | 101 | 112 | 114 | 117 |
| 27C3(S42A,S131T,M136I/N107G) | 30 | 41 | 66 | 77 | 91 | 93 | 102 | 112 | 114 | 117 |
| 27C3(S42A,S133T,V135I/N107G) | 31 | 41 | 67 | 77 | 91 | 93 | 103 | 112 | 114 | 117 |
| 27C3(S42A,S133T,M136I/N107G) | 32 | 41 | 68 | 77 | 91 | 93 | 104 | 112 | 114 | 117 |
| 27C3(S42A,V135I,M136I/N107G) | 33 | 41 | 69 | 77 | 91 | 93 | 105 | 112 | 114 | 117 |
| 27C3(S42A,S131T,S133T,V135I/N107G) | 34 | 41 | 70 | 77 | 91 | 93 | 106 | 112 | 114 | 117 |
| 27C3(S42A,S131T,S133T,M136I/N107G) | 35 | 41 | 71 | 77 | 91 | 93 | 107 | 112 | 114 | 117 |
| 27C3(S42A,S131T,V135I,M136I/N107G) | 36 | 41 | 72 | 77 | 91 | 93 | 108 | 112 | 114 | 117 |
| 27C3(S42A,S133T,V135I,M136I/N107G) | 37 | 41 | 73 | 77 | 91 | 93 | 109 | 112 | 114 | 117 |
| 27C3(S42A,S131T,S133T,V135I,M136I/N107G) | 38 | 41 | 74 | 77 | 91 | 93 | 110 | 112 | 114 | 117 |
| 27C3(S42A/I112V) | 19 | 42 | 55 | 78 | 91 | 93 | 95 | 112 | 114 | 118 |
| 27C3(S42A,S131T/I112V) | 24 | 42 | 60 | 78 | 91 | 93 | 96 | 112 | 114 | 118 |
| 27C3(S42A,S133T/I112V) | 25 | 42 | 61 | 78 | 91 | 93 | 97 | 112 | 114 | 118 |
| 27C3(S42A,V135I/I112V) | 26 | 42 | 62 | 78 | 91 | 93 | 98 | 112 | 114 | 118 |
| 27C3(S42A,M136I/I112V) | 27 | 42 | 63 | 78 | 91 | 93 | 99 | 112 | 114 | 118 |
| 27C3(S42A,S131T,S133T/I112V) | 28 | 42 | 64 | 78 | 91 | 93 | 100 | 112 | 114 | 118 |
| 27C3(S42A,S131T,V135I/I112V) | 29 | 42 | 65 | 78 | 91 | 93 | 101 | 112 | 114 | 118 |
| 27C3(S42A,S131T,M136I/I112V) | 30 | 42 | 66 | 78 | 91 | 93 | 102 | 112 | 114 | 118 |
| 27C3(S42A,S133T,V135I/I112V) | 31 | 42 | 67 | 78 | 91 | 93 | 103 | 112 | 114 | 118 |
| 27C3(S42A,S133T,M136I/I112V) | 32 | 42 | 68 | 78 | 91 | 93 | 104 | 112 | 114 | 118 |
| 27C3(S42A,V135I,M136I/I112V) | 33 | 42 | 69 | 78 | 91 | 93 | 105 | 112 | 114 | 118 |
| 27C3(S42A,S131T,S133T,V135I/I112V) | 34 | 42 | 70 | 78 | 91 | 93 | 106 | 112 | 114 | 118 |
| 27C3(S42A,S131T,S133T,M1361/I112V) | 35 | 42 | 71 | 78 | 91 | 93 | 107 | 112 | 114 | 118 |
| 27C3(S42A,S131T,V135I,M136I/I112V) | 36 | 42 | 72 | 78 | 91 | 93 | 108 | 112 | 114 | 118 |
| 27C3(S42A,S133T,V135I,M136I/I112V) | 37 | 42 | 73 | 78 | 91 | 93 | 109 | 112 | 114 | 118 |
| 27C3(S42A,S131T,S133T,V135I,M136I/I112V) | 38 | 42 | 74 | 78 | 91 | 93 | 110 | 112 | 114 | 118 |
| 27C3(S42A/N107G,I112V) | 19 | 43 | 55 | 79 | 91 | 93 | 95 | 112 | 114 | 119 |
| 27C3(S42A,S131T/N107G,I112V) | 24 | 43 | 60 | 79 | 91 | 93 | 96 | 112 | 114 | 119 |
| 27C3(S42A,S133T/N107G,I112V) | 25 | 43 | 61 | 79 | 91 | 93 | 97 | 112 | 114 | 119 |
| 27C3(S42A,V135I/N107G,I112V) | 26 | 43 | 62 | 79 | 91 | 93 | 98 | 112 | 114 | 119 |
| 27C3(S42A,M136I/N107G,I112V) | 27 | 43 | 63 | 79 | 91 | 93 | 99 | 112 | 114 | 119 |
| 27C3(S42A,S131T,S133T/N107G,I112V) | 28 | 43 | 64 | 79 | 91 | 93 | 100 | 112 | 114 | 119 |
| 27C3(S42A,S131T,V135I/N107G,I112V) | 29 | 43 | 65 | 79 | 91 | 93 | 101 | 112 | 114 | 119 |
| 27C3(S42A,S131T,M136I/N107G,I112V) | 30 | 43 | 66 | 79 | 91 | 93 | 102 | 112 | 114 | 119 |
| 27C3(S42A,S133T,V135I/N107G,I112V) | 31 | 43 | 67 | 79 | 91 | 93 | 103 | 112 | 114 | 119 |
| 27C3(S42A,S133T,M136I/N107G,I112V) | 32 | 43 | 68 | 79 | 91 | 93 | 104 | 112 | 114 | 119 |
| 27C3(S42A, V135I,M136I/N107G,I112V) | 33 | 43 | 69 | 79 | 91 | 93 | 105 | 112 | 114 | 119 |
| 27C3(S42A,S131T,S133T,V135I/N107G,I112V) | 34 | 43 | 70 | 79 | 91 | 93 | 106 | 112 | 114 | 119 |
| 27C3(S42A,S131T,S133T,M136I/N107G,I112V) | 35 | 43 | 71 | 79 | 91 | 93 | 107 | 112 | 114 | 119 |
| 27C3(S42A,S131T,V135I,M136I/N107G,I112V) | 36 | 43 | 72 | 79 | 91 | 93 | 108 | 112 | 114 | 119 |
| 27C3(S42A,S133T,V135I,M136I/N107G,I112V) | 37 | 43 | 73 | 79 | 91 | 93 | 109 | 112 | 114 | 119 |
| 27C3(S42A,S131T,S133T,V135I,M136I/N107G,I112V) | 38 | 43 | 74 | 79 | 91 | 93 | 110 | 112 | 114 | 119 |
| 18E5 | 39 | 44 | 75 | 80 | 92 | 94 | 111 | 113 | 115 | 120 |

In various other embodiments, the antigen binding protein is derived from such antibodies. For instance, in one embodiment, the antigen binding protein comprises 1, 2, 3, 4, 5 or all 6 of the CDRs listed in one of the rows for any particular antibody listed in **TABLE 4**. In another embodiment, an antigen binding protein includes 1, 2, 3, 4, 5, or 6 variant forms of the CDRs listed in one of the rows for an antibody in **TABLE 4**, each CDR having at least 80%, 85%, 90%, 95%, 96%, 97%, 98%, or 99% sequence identity to a CDR sequence listed in **TABLE 4**. Some antigen binding proteins include 1, 2, 3, 4, 5, or 6 of the CDRs listed in one of the rows of TABLE 4, each differing by no more than 1, 2, 3, 4 or 5 amino acids from the CDRs listed in these tables. In another embodiment, the antigen binding protein comprises all 6 of the CDRS listed in a row of TABLE 4 and the total number of amino acid changes to the CDRs collectively is no more than 1, 2, 3, 4, or 5 amino acids.

Some antigen binding proteins comprise a variable light domain and a variable heavy domain as listed in one of the rows for one of the antibodies listed in **TABLE 4**. In some instances, the antigen binding protein comprises two identical variable light domains and two identical variable heavy domains from one of the antibodies listed in **TABLE 4**. Some antigen binding proteins that are provided comprise a variable light domain and a variable heavy domain as listed in one of the rows for one of the antibodies listed in **TABLE 4**, except that one or both of the domains differs from the sequence specified in the table at only 1, 2, 3, 4, 5, 6, 7, 8, 9, 10, 11, 12, 13, 14 or 15 amino acid residues, wherein each such sequence difference is independently either a single amino acid deletion, insertion or substitution, with the deletions, insertions and/or substitutions resulting in no more than 1, 2, 3, 4, 5, 6, 7, 8, 9, 10, 11, 12, 13, 14 or 15 amino acid changes relative to the variable domain sequences specified in **TABLE 4**. Other antigen binding proteins also comprise a variable light domain and a variable heavy domain as listed in one of the rows for one of the antibodies listed in **TABLE 4**, except that one or both of the domains differs from the sequence specified in the table in that the heavy chain variable domain and/or light chain variable domain comprises or consists of a sequence of amino acids that has at least 70%, 75%, 80%, 85%, 90%, 95%, 96%, 97%, 98%, or 99% sequence identity to the amino acid sequences of the heavy chain variable domain or light chain variable domain sequences as specified in **TABLE 4**.

In one embodiment, the antigen binding protein consists just of a variable light or variable heavy domain from an antibody listed in **TABLE 4**. In still another embodiment, the antigen binding protein comprises two or more of the same variable heavy domains or two or more of the same variable light domains from those listed in **TABLE 4**. Such domain antibodies can be fused together or joined via a linker as described in greater detail below. The domain antibodies can also be fused or linked to one or more molecules to extend the half-life (e.g., PEG or albumin).

In another embodiment, the antigen binding protein comprises a full length light chain and a full length heavy chain as listed in one of the rows for one of the antibodies listed in **TABLE 4**. Some antigen binding proteins that are provided comprise a full length light chain and a full length heavy chain as listed in one of the rows for one of the antibodies listed in **TABLE 4**, except that one or both of the chains differs from the sequence specified in the table at only 1, 2, 3, 4, 5, 6, 7, 8, 9, 10, 11, 12, 13, 14 or 15 amino acid residues, wherein each such sequence difference is independently either a single amino acid deletion, insertion or substitution, with the deletions, insertions and/or substitutions resulting in no more than 1, 2, 3, 4, 5, 6, 7, 8, 9, 10, 11, 12, 13, 14 or 15 amino acid changes relative to the full length sequences specified in **TABLE 4**. Other antigen binding proteins also comprise a full length light chain and a full length heavy chain as listed in one of the rows for one of the antibodies listed in **TABLE 4**, except that one or both of the chains differs from the sequence specified in the table in that the light chain and/or heavy chain comprises or consists of a sequence of amino acids that has at least 70%, 75%, 80%, 85%, 90%, 95%, 96%, 97%, 98%, or 99% sequence identity to the amino acid sequences of the light chain or heavy chain sequences as specified in **TABLE 4**.

In another embodiment, the antigen binding protein consists of a just a light or a heavy chain polypeptide as set forth in **TABLE 4**.

In still another embodiment, antigen-binding proteins containing the CDRs, variable domains and/or full length sequences listed in **TABLE 4** is a monoclonal antibody, a multispecific antibody, or an antibody fragment of the foregoing. In another embodiment, the antibody fragment of the isolated antigen-binding proteins provided herein is a Fab fragment, a Fab' fragment, an F(ab')₂ fragment, an Fv fragment, a diabody, or a single chain antibody molecule based upon an antibody with the sequences as listed in **TABLE 4**.

In a further embodiment, the isolated antigen binding protein provided herein is a human antibody with the sequences as set forth in **TABLE 4** and is of the IgG1-, IgG2- IgG3- or IgG4-type.

In yet another embodiment, the isolated antigen-binding protein provided in **TABLE 4** can be coupled to a labeling group and can compete for binding to LCAT with an antigen binding protein of one of the isolated antigen-binding proteins provided herein.

Certain antigen binding proteins comprise the CDR, variable domain and/or full length sequences as specified in one of the rows of **TABLE 4** and are further characterized in having one or more of the following characteristics:
(a) binds human LCAT such that K_{D} is ≤200 nM, is ≤150 nM, is ≤100 nM , is ≤50 nM, is ≤10 nM, is ≤5 nM, is ≤2 nM, or is ≤1 nM (e..g, as determined by BiaCore such as described in **Example 3**);
(b) has a half-life in human serum of at least 3 days;
(c) binds human LCAT of SEQ ID NO:1 and cyno LCAT of SEQ ID NO:3;
(d) binds human LCAT of SEQ ID NO:1 with K_{D} ≤10 nM and binds cyno LCAT of SEQ ID NO:3 with K_{D} ≤500 nM;
(e) binds human LCAT of SEQ ID NO:1 with K_{D} ≤10 nM and binds cyno LCAT of SEQ ID NO:3 with K_{D} ≤100 nM;
(f) binds human LCAT of SEQ ID NO:1 with K_{D} ≤10 nM and binds cyno LCAT of SEQ ID NO:3 with K_{D} ≤10 nM; and
(g) binds human LCAT of SEQ ID NO:1 but does not bind cyno LCAT of SEQ ID NO:3.

### Binding Regions of Antigen Binding Proteins

As described in greater detail in **Examples 9-12** below, the region(s) of human LCAT protein (e.g., SEQ ID NO:1) to which the 27C3 and 18E5 antibodies bind was determined by x-ray crystallography and by calculation of solvent exposure differences determined from the x-ray crystallography data. The skilled person will understand that in one embodiment the LCAT antigen binding proteins that are provided can bind additional amino acids in addition to the particular amino acids listed below or bind to amino acids in regions in addition to the particular region(s) described below. In another embodiment, the LCAT binding protein binds only to the specified amino acids or to amino acids in the specified regions. In an embodiment, the regions and/or amino acids bound correspond to the regions and/or amino acids when the LCAT protein is in an active conformation.

### 27C3 Binding

LCAT amino acids involved in binding or within 5 angstroms of a 27C3 Fab include the following: S255, R256, M257, A258, W259, P260, Y315, V317, G318, L319, P320, T321, Y341, E342, D343, T350, R351, E354, L355, C356, G357, L358, Q360, R362, V367, H368, L369, P371, H373 and G374 of SEQ ID NO:1.

The solvent exposure difference analysis (see **Example 11**) identified very similar amino acids from LCAT as being at the interface with the 27C3 Fab; these amino acids include the following: R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 and H389 of SEQ ID NO: 1.

Thus, in an embodiment, antigen binding proteins that are provided herein bind to the same region(s) or amino acids of LCAT as antibody 27C3. Accordingly, in one embodiment, an LCAT antigen binding protein binds to amino acids located within a region of an LCAT polypeptide extending from amino acid 255 to amino acid 389 of SEQ ID NO:1, inclusive. In another embodiment, the LCAT antigen binding protein binds to amino acids located within a region from amino acid 255 to amino acid 374 of SEQ ID NO:1, inclusive.

Certain LCAT antigen binding proteins bind to amino acids within one, two or all three of the following regions: (a) the region from amino acid 255 to amino acid 262 of SEQ ID NO:1, inclusive; (b) the region from amino acid 315 to amino acid 321 of SEQ ID NO:1, inclusive; and (c) the region from amino acid 341 to amino acid 374 of SEQ ID NO:1, inclusive.

Still other LCAT antigen binding proteins bind to amino acids within one, two, three or all four of the following regions: (a) the region from amino acid 255 to amino acid 262 of SEQ ID NO: 1, inclusive; (b) the region from amino acid 315 to amino acid 321 of SEQ ID NO:1, inclusive; (c) the region from amino acid 341 to amino acid 343 of SEQ ID NO:1, inclusive; and (d) the region from amino acid 350 to amino acid 374 of SEQ ID NO:1, inclusive.

In one embodiment, the LCAT antigen binding protein binds to, or residues of the antigen binding protein are within 5 angstroms of, at least 10, 11, 12, 13, 14, 15, 16, 17, 18, 19, 20, 21, 22, 23, 24, 25, 26, 27, 28, 29 or all 30 of the amino acids selected from the group consisting of S255, R256, M257, A258, W259, P260, Y315, V317, G318, L319, P320, T321, Y341, E342, D343, T350, R351, E354, L355, C356, G357, L358, Q360, R362, V367, H368, L369, P371, H373 and G374 of SEQ ID NO:1. In one embodiment, the distance between the LCAT antigen binding protein and the residues of LCAT is determined by x-ray crystallography as described in **Example 9**.

In yet other embodiments, an LCAT antigen binding protein binds to, or residues of the binding protein are within 5 angstroms of, at least 10, 11, 12, 13, 14, 15, 16, 17, 18, 19, 20, 21, 22, 23, 24, 25, 26, 27, 28, 29 or all 30 of the amino acids selected from the group consisting of amino acids R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 and H389 of SEQ ID NO:1. In one embodiment, the distance between the residues of the LCAT antigen binding protein and the residues of LCAT is determined from solvent accessible surface area differences as described in **Example 11**.

In another embodiment, the LCAT antigen binding protein binds to an epitope located within amino acid 255 to amino acid 389 of SEQ ID NO:1, inclusive. Other LCAT antigen binding proteins bind to an epitope between amino acid 255 and amino acid 374, inclusive. In some of these embodiments, the epitope is a conformational and/or discontinuous epitope.

### 18E5 Binding

LCAT amino acids involved in binding or within 5 angstroms of an 18E5 Fab include the following: Y315, V317, D343, T350, R351, E354, G357, Q360, G361, Q365, P366, V367, H368, L369, L370, P371, L372, H373, I375, L385, E388, H389, A392, L395, G396, A397, Y398 and R399 of SEQ ID NO:1.

The solvent exposure difference analysis (see **Example 11**) identified very similar amino acids from LCAT as being at the interface with the 27C3 Fab; these amino acids include the following: Y315, V317, E342, D343, T350, R351, E354, G357, Q360, G361, P364, P366, V367, H368, L369, L370, P371, L372, H373, L385, E388, H389, A392, L395, G396, A397, Y398 and R399 of SEQ ID NO:1.

Thus, in an embodiment, LCAT antigen binding proteins that are provided herein bind to the same region(s) or amino acids of LCAT as antibody 18E5. Accordingly, in one aspect, an LCAT antigen binding protein binds to amino acids located within a region of an LCAT polypeptide extending from amino acid 315 to amino acid 399 of SEQ ID NO:1, inclusive. In another embodiment, the LCAT antigen binding protein binds to amino acids located within a region from amino acid 342 to amino acid 399 of SEQ ID NO:1, inclusive.

Certain LCAT antigen binding proteins bind to amino acids within one or both of the following regions: (a) the region from amino acid 350 to amino acid 375 of SEQ ID NO:1, inclusive; and (b) the region from amino acid 385 to amino acid 399 of SEQ ID NO:1, inclusive.

Other LCAT antigen binding proteins bind to amino acids within one, two or all three of the following regions: (a) the region from amino acid 315 to amino acid 317 of SEQ ID NO:1, inclusive; (b) the region from amino acid 350 to amino acid 375 of SEQ ID NO:1, inclusive; and (c) the region from amino acid 385 to amino acid 399 of SEQ ID NO:1, inclusive.

In still other embodiments, an LCAT antigen binding protein binds within one, two, three or all four of the following regions: (a) the region from amino acid 315 to amino acid 317 of SEQ ID NO:1, inclusive, (b) the region from amino acid 342 to amino acid 343 of SEQ ID NO:1, inclusive, (c) the region from amino acid 350 to amino acid 375 of SEQ ID NO:1, inclusive, and (d) the region from amino acid 385 to amino acid 399 of SEQ ID NO:1, inclusive.

In various embodiments, an LCAT antigen binding protein binds to, or residues of the binding protein are within 5 angstroms of, at least 10, 11, 12, 13, 14, 15, 16, 17, 18, 19, 20, 21, 22, 23, 24, 25, 26, 27 or all 28 amino acids selected from the group consisting of Y315, V317, D343, T350, R351, E354, G357, Q360, G361, Q365, P366, V367, H368, L369, L370, P371, L372, H373, I375, L385, E388, H389, A392, L395, G396, A397, Y398 and R399 of SEQ ID NO:1. The distance between the residues of the LCAT antigen binding protein and the residues of LCAT is determined by x-ray crystallography as described in **Example 10**.

In other embodiments, an LCAT antigen binding protein binds to, or residues of the binding protein are within 5 angstroms of, at least 10, 11, 12, 13, 14, 15, 16, 17, 18, 19, 20, 21, 22, 23, 24, 25, 26, 27 or all 28 amino acids selected from the group consisting of Y315, V317, E342, D343, T350, R351, E354, G357, Q360, G361, P364, P366, V367, H368, L369, L370, P371, L372, H373, L385, E388, H389, A392, L395, G396, A397, Y398 and R399 of **SEQ ID NO:1**. In one embodiment, the distance between the residues of the LCAT antigen binding protein and the residues of LCAT is determined from solvent accessible surface area differences as described in **Example 11**.

In another aspect, the LCAT antigen binding protein binds to an epitope located within amino acid 315 to amino acid 399 of SEQ ID NO:1, inclusive. Other LCAT antigen binding proteins bind to an epitope between amino acid 343 and amino acid 399 of SEQ ID NO:1, inclusive. In some of these embodiments, the epitope is a conformational and/or discontinuous epitope.

### Common Binding Features of the 27C3 and 18E5 Antibodies

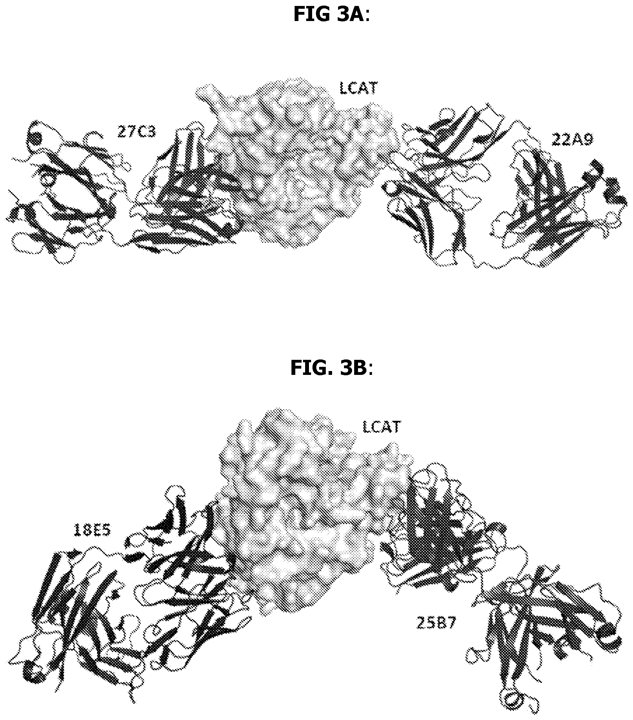

The x-ray crystal structure results for antibodies 27C3 and 18E5 demonstrate that these two agonist antibodies bind to a very similar region of the LCAT protein. X-ray crystal structures were also determined for two inhibitory antibodies when complexed with human LCAT. Interestingly, the agonist antibodies 27C3 and 18E5 bind on the opposite side of LCAT as compared to the two inhibitory antibodies (see **Example 12** and **FIGS. 3A and 3B**). In particular, the 27C3 and 18E5 antibodies bind behind D345 of the catalytic triad. It thus appears that there are two very distinct binding regions depending upon whether the antibody is an agonist or antagonist.

The amino acids of human LCAT that are within 5 angstroms of residues from both the 27C3 and 18E5 antibodies include: Y315, V317, D343, T350, R351, E354, G357, Q360, V367, H368, L369, P371 and H373 of SEQ ID NO:1.

Thus, in an embodiment, an LCAT antigen binding protein as provided herein binds to the same region(s) or amino acids of LCAT as are bound by both antibody 27C3 and 18E5. Accordingly, in one embodiment, an LCAT antigen binding protein binds to amino acids located within a region of an LCAT polypeptide extending from amino acid 315 to amino acid 373 of SEQ ID NO:1, inclusive.

Certain LCAT antigen binding proteins bind to amino acids within one or both of the following regions: (a) the region from amino acid 315 to amino acid 317 of SEQ ID NO:1, inclusive; and (b) the region from amino acid 343 to amino acid 373 of SEQ ID NO:1, inclusive.

Other LCAT antigen binding proteins bind to amino acids within one or both of the following regions: (a) the region from amino acid 315 to amino acid 317 of SEQ ID NO:1, inclusive; and (b) the region from amino acid 350 to amino acid 373 of SEQ ID NO:1, inclusive.

In various embodiments, an LCAT antigen binding protein binds to, or residues of the binding protein are within 5 angstroms of, at least 3, 4, 5, 6, 7, 8, 9, 10, 11, 12 or all 13 of the amino acids selected from the group consisting of Y315, V317, D343, T350, R351, E354, G357, Q360, V367, H368, L369, P371 and H373 of SEQ ID NO:1. The distance between the residues of the LCAT antigen binding protein and the residues of LCAT is determined by x-ray crystallography as described in **Examples 9 and 10.**

In another embodiment, the LCAT antigen binding protein binds to an epitope located within amino acid 315 to amino acid 373 of SEQ ID NO:1, inclusive. Other LCAT antigen binding proteins bind to an epitope between amino acid 343 and amino acid 373 of SEQ ID NO:1, inclusive. In some of these embodiments, the epitope is a conformational and/or discontinurous epitope.

### Competing Antigen Binding Proteins

In another embodiment, antigen binding proteins are provided that compete with one of the exemplified antibodies or functional fragments for described above for specific binding to a human LCAT (e.g., SEQ ID NO:1). Such antigen binding proteins may bind to the same epitope as one of the antigen binding proteins described herein, or to an overlapping epitope. Antigen binding proteins and fragments that compete with the exemplified antigen binding proteins are expected to show similar functional properties. The exemplified antigen binding proteins and fragments include those described above, including those with the heavy and light chains, variable region domains and CDRs included in **TABLES 1, 2, 3**, **and 4.** Thus, as a specific example, the antigen binding proteins that are provided include those that compete with an antibody having:
(a) all 6 of the CDRs listed for any antibody listed in **TABLE 4**;
(b) a VH and a VL listed for any antibody listed in **TABLE 4**; or
(c) two light chains and two heavy chains as specified for any antibody listed in **TABLE 4**.
Competition is determined by a BIAcore assay as described in **Example 8**.

### Monoclonal Antibodies

The antigen binding proteins that are provided include monoclonal antibodies that bind to LCAT. Monoclonal antibodies may be produced using any technique known in the art, *e.g*., by immortalizing spleen cells harvested from the transgenic animal after completion of the immunization schedule. The spleen cells can be immortalized using any technique known in the art, *e.g*., by fusing them with myeloma cells to produce hybridomas. Myeloma cells for use in hybridoma-producing fusion procedures preferably are non-antibody-producing, have high fusion efficiency, and enzyme deficiencies that render them incapable of growing in certain selective media which support the growth of only the desired fused cells (hybridomas). Examples of suitable cell lines for use in mouse fusions include Sp-20, P3-X63/Ag8, P3-X63-Ag8.653, NS1/1.Ag 4 1, Sp210-Ag14, FO, NSO/U, MPC-11, MPC11-X45-GTG 1.7 and S194/5XXO Bul; examples of cell lines used in rat fusions include R210.RCY3, Y3-Ag 1.2.3, IR983F and 4B210. Other cell lines useful for cell fusions are U-266, GM1500-GRG2, LICR-LON-HMy2 and UC729-6.

In some instances, a hybridoma cell line is produced by immunizing an animal (*e.g.,* a transgenic animal having human immunoglobulin sequences) with an LCAT immunogen; harvesting spleen cells from the immunized animal; fusing the harvested spleen cells to a myeloma cell line, thereby generating hybridoma cells; establishing hybridoma cell lines from the hybridoma cells, and identifying a hybridoma cell line that produces an antibody that binds an LCAT polypeptide. Such hybridoma cell lines, and anti-LCAT monoclonal antibodies produced by them, are aspects of the present application.

Monoclonal antibodies secreted by a hybridoma cell line can be purified using any technique known in the art. Hybridomas or mAbs may be further screened to identify mAbs with particular properties, such as the ability to increase LCAT activity. Examples of such screens are provided in the Examples below.

### Chimeric and Humanized Antibodies

Chimeric and humanized antibodies based upon the foregoing sequences are also provided. Monoclonal antibodies for use as therapeutic agents may be modified in various ways prior to use. One example is a chimeric antibody, which is an antibody composed of protein segments from different antibodies that are covalently joined to produce functional immunoglobulin light or heavy chains or immunologically functional portions thereof. Generally, a portion of the heavy chain and/or light chain is identical with or homologous to a corresponding sequence in antibodies derived from a particular species or belonging to a particular antibody class or subclass, while the remainder of the chain(s) is/are identical with or homologous to a corresponding sequence in antibodies derived from another species or belonging to another antibody class or subclass. For methods relating to chimeric antibodies, *see,* for example, United States Patent No. 4,816,567; and Morrison et al., 1985, Proc. Natl. Acad. Sci. USA 81:6851-6855. CDR grafting is described, for example, in United States Patent No. 6,180,370, No. 5,693,762, No. 5,693,761, No. 5,585,089, and No. 5,530,101.

Generally, the goal of making a chimeric antibody is to create a chimera in which the number of amino acids from the intended patient species is maximized. One example is the "CDR-grafted" antibody, in which the antibody comprises one or more complementarity determining regions (CDRs) from a particular species or belonging to a particular antibody class or subclass, while the remainder of the antibody chain(s) is/are identical with or homologous to a corresponding sequence in antibodies derived from another species or belonging to another antibody class or subclass. For use in humans, the variable region or selected CDRs from a rodent antibody often are grafted into a human antibody, replacing the naturally-occurring variable regions or CDRs of the human antibody.

One useful type of chimeric antibody is a "humanized" antibody. Generally, a humanized antibody is produced from a monoclonal antibody raised initially in a non-human animal. Certain amino acid residues in this monoclonal antibody, typically from non-antigen recognizing portions of the antibody, are modified to be homologous to corresponding residues in a human antibody of corresponding isotype. Humanization can be performed, for example, using various methods by substituting at least a portion of a rodent variable region for the corresponding regions of a human antibody (*see, e.g.,* United States Patent No. 5,585,089, and No. 5,693,762; Jones et al., 1986, Nature 321:522-525; Riechmann et al., 1988, Nature 332:323-27; Verhoeyen et al., 1988, Science 239:1534-1536).

In one embodiment, the CDRs of the light and heavy chain variable regions of the antibodies provided herein (*see,* **TABLES 3A and 3B**) are grafted to framework regions (FRs) from antibodies from the same, or a different, phylogenetic species. For example, the CDRs of the heavy and light chain variable regions V_{H}1, V_{H}2, V_{H}3, V_{H}4, V_{H}5, V_{H}6, V_{H}7, V_{H}8, V_{H}9, V_{H}10, V_{H}11, V_{H}12 and/or V_{L}1, and V_{L}2 can be grafted to consensus human FRs. To create consensus human FRs, FRs from several human heavy chain or light chain amino acid sequences may be aligned to identify a consensus amino acid sequence. In other embodiments, the FRs of a heavy chain or light chain disclosed herein are replaced with the FRs from a different heavy chain or light chain. In one aspect, rare amino acids in the FRs of the heavy and light chains of LCAT antibodies are not replaced, while the rest of the FR amino acids are replaced. A "rare amino acid" is a specific amino acid that is in a position in which this particular amino acid is not usually found in an FR. Alternatively, the grafted variable regions from the one heavy or light chain may be used with a constant region that is different from the constant region of that particular heavy or light chain as disclosed herein. In other embodiments, the grafted variable regions are part of a single chain Fv antibody.

In certain embodiments, constant regions from species other than human can be used along with the human variable region(s) to produce hybrid antibodies.

### Fully Human Antibodies

Fully human LCAT antibodies are also provided. Methods are available for making fully human antibodies specific for a given antigen without exposing human beings to the antigen ("fully human antibodies"). One specific means provided for implementing the production of fully human antibodies is the "humanization" of the mouse humoral immune system. Introduction of human immunoglobulin (Ig) loci into mice in which the endogenous Ig genes have been inactivated is one means of producing fully human monoclonal antibodies (mAbs) in mouse, an animal that can be immunized with any desirable antigen. Using fully human antibodies can minimize the immunogenic and allergic responses that can sometimes be caused by administering mouse or mouse-derived mAbs to humans as therapeutic agents.

Fully human antibodies can be produced by immunizing transgenic animals (usually mice) that are capable of producing a repertoire of human antibodies in the absence of endogenous immunoglobulin production. Antigens for this purpose typically have six or more contiguous amino acids, and optionally are conjugated to a carrier, such as a hapten. *See, e.g.,* Jakobovits et al., 1993, Proc. Natl. Acad. Sci. USA 90:2551-2555; Jakobovits et al., 1993, Nature 362:255-258; and Bruggermann et al., 1993, Year in Immunol. 7:33. In one example of such a method, transgenic animals are produced by incapacitating the endogenous mouse immunoglobulin loci encoding the mouse heavy and light immunoglobulin chains therein, and inserting into the mouse genome large fragments of human genome DNA containing loci that encode human heavy and light chain proteins. Partially modified animals, which have less than the full complement of human immunoglobulin loci, are then cross-bred to obtain an animal having all of the desired immune system modifications. When administered an immunogen, these transgenic animals produce antibodies that are immunospecific for the immunogen but have human rather than murine amino acid sequences, including the variable regions. For further details of such methods, *see,* for example, WO96/33735 and WO94/02602. Additional methods relating to transgenic mice for making human antibodies are described in United States Patent No. 5,545,807; No. 6,713,610; No. 6,673,986; No. 6,162,963; No. 5,545,807; No. 6,300,129; No. 6,255,458; No. 5,877,397; No. 5,874,299 and No. 5,545,806; in PCT publications WO91/10741, WO90/04036, and in EP 546073B1 and EP 546073A1.

The transgenic mice described above, referred to herein as "HuMab" mice, contain a human immunoglobulin gene minilocus that encodes unrearranged human heavy ([mu] and [gamma]) and [kappa] light chain immunoglobulin sequences, together with targeted mutations that inactivate the endogenous [mu] and [kappa] chain loci (Lonberg et al., 1994, Nature 368:856-859). Accordingly, the mice exhibit reduced expression of mouse IgM or [kappa] and in response to immunization, and the introduced human heavy and light chain transgenes undergo class switching and somatic mutation to generate high affinity human IgG [kappa] monoclonal antibodies (Lonberg *et al*., *supra.*; Lonberg and Huszar, 1995, Intern. Rev. Immunol. 13: 65-93; Harding and Lonberg, 1995, Ann. N.Y Acad. Sci. 764:536-546). The preparation of HuMab mice is described in detail in Taylor et al., 1992, Nucleic Acids Research 20:6287-6295; Chen et al., 1993, International Immunology 5:647-656; Tuaillon et al., 1994, J. Immunol. 152:2912-2920; Lonberg et al., 1994, Nature 368:856-859; Lonberg, 1994, Handbook of Exp. Pharmacology 113:49-101; Taylor et al., 1994, International Immunology 6:579-591; Lonberg and Huszar, 1995, Intern. Rev. Immunol. 13:65-93; Harding and Lonberg, 1995, Ann. N.Y Acad. Sci. 764:536-546; Fishwild et al., 1996, Nature Biotechnology 14:845-851. *See,* further United States Patent No. 5,545,806; No. 5,569,825; No. 5,625,126; No. 5,633,425; No. 5,789,650; No. 5,877,397; No. 5,661,016; No. 5,814,318; No. 5,874,299; and No. 5,770,429; as well as United States Patent No. 5,545,807; International Publication Nos. WO 93/1227; WO 92/22646; and WO 92/03918. Technologies utilized for producing human antibodies in these transgenic mice are disclosed also in WO 98/24893, and Mendez et al., 1997, Nature Genetics 15:146-156. For example, the HCo7 and HCo12 transgenic mice strains can be used to generate anti-c-LCAT antibodies. Further details regarding the production of human antibodies using transgenic mice are provided in the Examples below.

Using hybridoma technology, antigen-specific human mAbs with the desired specificity can be produced and selected from the transgenic mice such as those described above. Such antibodies may be cloned and expressed using a suitable vector and host cell, or the antibodies can be harvested from cultured hybridoma cells.

Fully human antibodies can also be derived from phage-display libraries (as disclosed in Hoogenboom et al., 1991, J. Mol. Biol. 227:381; and Marks et al., 1991, J. Mol. Biol. 222:581). Phage display techniques mimic immune selection through the display of antibody repertoires on the surface of filamentous bacteriophage, and subsequent selection of phage by their binding to an antigen of choice. One such technique is described in PCT Publication No. WO 99/10494.

**TABLE 4** includes sequence information for a number of fully human antigen binding proteins such as are provided herein.

### Bispecific or Bifunctional Antigen Binding Proteins

The antigen binding proteins that are provided also include bispecific and bifunctional antibodies that include one or more CDRs or one or more variable regions as described above. A bispecific or bifunctional antibody in some instances is an artificial hybrid antibody having two different heavy/light chain pairs and two different binding sites. Bispecific antibodies may be produced by a variety of methods including, but not limited to, fusion of hybridomas or linking of Fab' fragments. *See, e.g*., Songsivilai and Lachmann, 1990, Clin. Exp. Immunol. 79:315-321; Kostelny et al., 1992, J. Immunol. 148:1547-1553.

### Various Other Forms

The LCAT binding protein can also be a variant, mimetic, derivative or oligomer based upon the structure of LCAT antigen binding proteins have the CDRS, variable regions and/or full length chains as described above in **TABLES 1-4**.

### Variants

In one embodiment, for instance, an antigen binding protein is a variant form of the antigen binding proteins disclosed above (*e.g*., those having the sequences listed in **TABLES 1-4**). For instance, some of the antigen binding proteins have one or more conservative amino acid substitutions in one or more of the heavy or light chains, variable regions or CDRs listed in **TABLES 1-4**.

Naturally-occurring amino acids may be divided into classes based on common side chain properties:
1) hydrophobic: norleucine, Met, Ala, Val, Leu, Ile;
2) neutral hydrophilic: Cys, Ser, Thr, Asn, Gln;
3) acidic: Asp, Glu;
4) basic: His, Lys, Arg;
5) residues that influence chain orientation: Gly, Pro; and
6) aromatic: Trp, Tyr, Phe.

Conservative amino acid substitutions may involve exchange of a member of one of these classes with another member of the same class. Conservative amino acid substitutions may encompass non-naturally occurring amino acid residues, which are typically incorporated by chemical peptide synthesis rather than by synthesis in biological systems. These include peptidomimetics and other reversed or inverted forms of amino acid moieties.

Non-conservative substitutions may involve the exchange of a member of one of the above classes for a member from another class. Such substituted residues may be introduced into regions of the antibody that are homologous with human antibodies, or into the non-homologous regions of the molecule.

In making such changes, the hydropathic index of amino acids may be considered. The hydropathic profile of a protein is calculated by assigning each amino acid a numerical value ("hydropathy index") and then repetitively averaging these values along the peptide chain. Each amino acid has been assigned a hydropathic index on the basis of its hydrophobicity and charge characteristics. They are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic profile in conferring interactive biological function on a protein is understood in the art (*see, e.g*., Kyte et al., 1982, J. Mol. Biol. 157:105-131). It is known that certain amino acids may be substituted for other amino acids having a similar hydropathic index or score and still retain a similar biological activity. In making changes based upon the hydropathic index, in certain embodiments, the substitution of amino acids whose hydropathic indices are within ±2 is included. In some embodiments, those which are within ±1 are included, and in other embodiments, those within ±0.5 are included.

It is also understood in the art that the substitution of like amino acids can be made effectively on the basis of hydrophilicity, particularly where the biologically functional protein or peptide thereby created is intended for use in immunological embodiments, as in the present case. In certain embodiments, the greatest local average hydrophilicity of a protein, as governed by the hydrophilicity of its adjacent amino acids, correlates with its immunogenicity and antigen-binding or immunogenicity, that is, with a biological property of the protein.

The following hydrophilicity values have been assigned to these amino acid residues: arginine (+3.0); lysine (+3.0); aspartate (+3.0±1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5) and tryptophan (-3.4). In making changes based upon similar hydrophilicity values, in certain embodiments, the substitution of amino acids whose hydrophilicity values are within ±2 is included, in other embodiments, those which are within ±1 are included, and in still other embodiments, those within ±0.5 are included. In some instances, one may also identify epitopes from primary amino acid sequences on the basis of hydrophilicity. These regions are also referred to as "epitopic core regions."

Exemplary conservative amino acid substitutions are set forth in **TABLE 5**.

**Table 5: Conservative Amino Acid Substitutions**

| **Original Residue** | **Exemplary Substitutions** |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

A skilled artisan will be able to determine suitable variants of polypeptides as set forth herein using well-known techniques. One skilled in the art may identify suitable areas of the molecule that may be changed without destroying activity by targeting regions not believed to be important for activity. The skilled artisan also will be able to identify residues and portions of the molecules that are conserved among similar polypeptides. In further embodiments, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in a protein that correspond to amino acid residues important for activity or structure in similar proteins. One skilled in the art may opt for chemically similar amino acid substitutions for such predicted important amino acid residues.

One skilled in the art can also analyze the 3-dimensional structure and amino acid sequence in relation to that structure in similar polypeptides. In view of such information, one skilled in the art may predict the alignment of amino acid residues of an antibody with respect to its three dimensional structure. One skilled in the art may choose not to make radical changes to amino acid residues predicted to be on the surface of the protein, since such residues may be involved in important interactions with other molecules. Moreover, one skilled in the art may generate test variants containing a single amino acid substitution at each desired amino acid residue. These variants can then be screened using assays for LCAT activity, (*see* examples below) thus yielding information regarding which amino acids can be changed and which must not be changed. In other words, based on information gathered from such routine experiments, one skilled in the art can readily determine the amino acid positions where further substitutions should be avoided either alone or in combination with other mutations.

A number of scientific publications have been devoted to the prediction of secondary structure. *See,* Moult, 1996, Curr. Op. in Biotech. 7:422-427; Chou et al., 1974, Biochem. 13:222-245, Chou et al., 1974, Biochemistry 113:211-222; Chou et al., 1978, Adv. Enzymol. Relat. Areas Mol. Biol. 47:45-148; Chou et al., 1979, Ann. Rev. Biochem. 47:251-276; and Chou et al., 1979, Biophys. J. 26:367-384. Moreover, computer programs are currently available to assist with predicting secondary structure. One method of predicting secondary structure is based upon homology modeling. For example, two polypeptides or proteins that have a sequence identity of greater than 30%, or similarity greater than 40% can have similar structural topologies. The recent growth of the protein structural database (PDB) has provided enhanced predictability of secondary structure, including the potential number of folds within a polypeptide's or protein's structure. *See,* Holm et al., 1999, Nucl. Acid. Res. 27:244-247. It has been suggested (Brenner et al., 1997, Curr. Op. Struct. Biol. 7:369-376) that there are a limited number of folds in a given polypeptide or protein and that once a critical number of structures have been resolved, structural prediction will become dramatically more accurate.

Additional methods of predicting secondary structure include "threading" (Jones, 1997, Curr. Opin. Struct. Biol. 7:377-387; Sippl et al., 1996, Structure 4:15-19), "profile analysis" (Bowie et al., 1991, Science 253:164-170; Gribskov et al., 1990, Meth. Enzym. 183:146-159; Gribskov et al., 1987, Proc. Nat. Acad. Sci. 84:4355-4358), and "evolutionary linkage" (*See,* Holm, 1999, *supra*; and Brenner, 1997, *supra*).

In some embodiments, amino acid substitutions are made that: (1) reduce susceptibility to proteolysis, (2) reduce susceptibility to oxidation, (3) alter binding affinity for forming protein complexes, (4) alter ligand or antigen binding affinities, and/or (4) confer or modify other physicochemical or functional properties on such polypeptides. For example, single or multiple amino acid substitutions (in certain embodiments, conservative amino acid substitutions) may be made in the naturally-occurring sequence. Substitutions can be made in that portion of the antibody that lies outside the domain(s) forming intermolecular contacts). In such embodiments, conservative amino acid substitutions can be used that do not substantially change the structural characteristics of the parent sequence (*e.g*., one or more replacement amino acids that do not disrupt the secondary structure that characterizes the parent or native antigen binding protein). Examples of art-recognized polypeptide secondary and tertiary structures are described in Proteins, Structures and Molecular Principles (Creighton, Ed.), 1984, W. H. New York: Freeman and Company; Introduction to Protein Structure (Branden and Tooze, eds.), 1991, New York: Garland Publishing; and Thornton et al., 1991, Nature 354:105.

Additional preferred antibody variants include cysteine variants wherein one or more cysteine residues in the parent or native amino acid sequence are deleted from or substituted with another amino acid (*e.g.,* serine). Cysteine variants are useful, *inter alia* when antibodies must be refolded into a biologically active conformation. Cysteine variants may have fewer cysteine residues than the native antibody, and typically have an even number to minimize interactions resulting from unpaired cysteines.

The heavy and light chains, variable regions domains and CDRs that are disclosed can be used to prepare polypeptides that contain an antigen binding region that can specifically bind to LCAT. For example, one or more of the CDRs listed in **TABLES 3A, 3B and 4** can be incorporated into a molecule (*e.g*., a polypeptide) covalently or noncovalently to make an immunoadhesion. An immunoadhesion may incorporate the CDR(s) as part of a larger polypeptide chain, may covalently link the CDR(s) to another polypeptide chain, or may incorporate the CDR(s) noncovalently. The CDR(s) enable the immunoadhesion to bind specifically to a particular antigen of interest (*e.g*., an LCAT polypeptide or epitope thereof).

### Mimetics

Mimetics (*e.g*., "peptide mimetics" or "peptidomimetics") based upon the variable region domains and CDRs that are described herein are also provided. These analogs can be peptides, non-peptides or combinations of peptide and non-peptide regions. Fauchere, 1986, Adv. Drug Res. 15:29; Veber and Freidinger, 1985, TINS p. 392; and Evans et al., 1987, J. Med. Chem. 30:1229. Peptide mimetics that are structurally similar to therapeutically useful peptides may be used to produce a similar therapeutic or prophylactic effect. Such compounds are often developed with the aid of computerized molecular modeling. Generally, peptidomimetics are proteins that are structurally similar to an antibody displaying a desired biological activity, such as here the ability to specifically bind LCAT, but have one or more peptide linkages optionally replaced by a linkage selected from: -CH₂NH-, -CH₂S-, -CH₂-CH₂-, -CH-CH-(cis and trans),-COCH₂-, -CH(OH)CH₂-, and -CH₂SO-, by methods well known in the art. Systematic substitution of one or more amino acids of a consensus sequence with a D-amino acid of the same type (*e.g*., D-lysine in place of L-lysine) may be used in certain embodiments to generate more stable proteins. In addition, constrained peptides comprising a consensus sequence or a substantially identical consensus sequence variation may be generated by methods known in the art (Rizo and Gierasch, 1992, Ann. Rev. Biochem. 61:387)), for example, by adding internal cysteine residues capable of forming intramolecular disulfide bridges which cyclize the peptide.

### Derivatives

Derivatives of the antigen binding proteins that are described herein are also provided. The derivatized antigen binding proteins can comprise any molecule or substance that imparts a desired property to the antibody or fragment, such as increased half-life in a particular use. The derivatized antigen binding protein can comprise, for example, a detectable (or labeling) moiety (*e.g*., a radioactive, colorimetric, antigenic or enzymatic molecule, a detectable bead (such as a magnetic or electrodense (*e.g*., gold) bead), or a molecule that binds to another molecule *(e.g.,* biotin or streptavidin)), a therapeutic or diagnostic moiety (*e.g.,* a radioactive, cytotoxic, or pharmaceutically active moiety), or a molecule that increases the suitability of the antigen binding protein for a particular use (*e.g*., administration to a subject, such as a human subject, or other *in vivo* or *in vitro* uses). Examples of molecules that can be used to derivatize an antigen binding protein include albumin (*e.g*., human serum albumin) and polyethylene glycol (PEG). Albumin-linked and PEGylated derivatives of antigen binding proteins can be prepared using techniques well known in the art. Certain antigen binding proteins include a pegylated single chain polypeptide as described herein. In one embodiment, the antigen binding protein is conjugated or otherwise linked to transthyretin (TTR) or a TTR variant. The TTR or TTR variant can be chemically modified with, for example, a chemical selected from the group consisting of dextran, poly(n-vinyl pyrrolidone), polyethylene glycols, propropylene glycol homopolymers, polypropylene oxide/ethylene oxide co-polymers, polyoxyethylated polyols and polyvinyl alcohols.

Other derivatives include covalent or aggregative conjugates of LCAT antigen binding proteins with other proteins or polypeptides, such as by expression of recombinant fusion proteins comprising heterologous polypeptides fused to the N-terminus or C-terminus of an LCAT antigen binding protein. For example, the conjugated peptide may be a heterologous signal (or leader) polypeptide, *e.g*., the yeast alpha-factor leader, or a peptide such as an epitope tag. LCAT antigen binding protein-containing fusion proteins can comprise peptides added to facilitate purification or identification of the LCAT antigen binding protein (*e.g*., poly-His). An LCAT antigen binding protein also can be linked to the FLAG peptide as described in Hopp et al., 1988, Bio/Technology 6:1204; and United States Patent No. 5,011,912. The FLAG peptide is highly antigenic and provides an epitope reversibly bound by a specific monoclonal antibody (mAb), enabling rapid assay and facile purification of expressed recombinant protein. Reagents useful for preparing fusion proteins in which the FLAG peptide is fused to a given polypeptide are commercially available (Sigma, St. Louis, MO).

### Oligomers

Oligomers that contain one or more LCAT antigen binding proteins may be employed as LCAT agonists. Oligomers may be in the form of covalently-linked or noncovalently-linked dimers, trimers, or higher oligomers. Oligomers comprising two or more LCAT antigen binding proteins are contemplated for use, with one example being a homodimer. Other oligomers include heterodimers, homotrimers, heterotrimers, homotetramers, heterotetramers, etc.

to the application discloses oligomers comprising multiple LCAT-binding polypeptides joined *via* covalent or non-covalent interactions between peptide moieties fused to the LCAT antigen binding proteins. Such peptides may be peptide linkers (spacers), or peptides that have the property of promoting oligomerization. Leucine zippers and certain polypeptides derived from antibodies are among the peptides that can promote oligomerization of LCAT antigen binding proteins attached thereto, as described in more detail below.

In one disclosure, the oligomers comprise from two to four LCAT antigen binding proteins. The LCAT antigen binding protein moieties of the oligomer may be in any of the forms described above, *e.g*., variants or fragments. Preferably, the oligomers comprise LCAT antigen binding proteins that have agonist activity.

In one disclosure, an oligomer is prepared using polypeptides derived from immunoglobulins. Preparation of fusion proteins comprising certain heterologous polypeptides fused to various portions of antibody-derived polypeptides (including the Fc domain) has been described, *e.g.,* by Ashkenazi et al., 1991, Proc. Natl. Acad. Sci. USA 88:10535; Byrn et al., 1990, Nature 344:677; and Hollenbaugh et al., 1992 "Construction of Immunoglobulin Fusion Proteins", in Current Protocols in Immunology, Suppl. 4, pages 10.19.1-10.19.11.

One disclosure is directed to a dimer comprising two fusion proteins created by fusing an LCAT antigen binding protein to the Fc region of an antibody. The dimer can be made by, for example, inserting a gene fusion encoding the fusion protein into an appropriate expression vector, expressing the gene fusion in host cells transformed with the recombinant expression vector, and allowing the expressed fusion protein to assemble much like antibody molecules, whereupon interchain disulfide bonds form between the Fc moieties to yield the dimer.

The term "Fc polypeptide" as used herein includes native and mutein forms of polypeptides derived from the Fc region of an antibody. Truncated forms of such polypeptides containing the hinge region that promotes dimerization also are included. Fusion proteins comprising Fc moieties (and oligomers formed therefrom) offer the advantage of facile purification by affinity chromatography over Protein A or Protein G columns.

One suitable Fc polypeptide, described in PCT application WO 93/10151 and United States Patent. No. 5,426,048 and No. 5,262,522, is a single chain polypeptide extending from the N-terminal hinge region to the native C-terminus of the Fc region of a human IgG1 antibody. Another useful Fc polypeptide is the Fc mutein described in Baum et al., 1994, EMBO J. 13:3992-4001. The amino acid sequence of this mutein is identical to that of the native Fc sequence presented in WO 93/10151, except that amino acid 19 has been changed from Leu to Ala, amino acid 20 has been changed from Leu to Glu, and amino acid 22 has been changed from Gly to Ala. The mutein exhibits reduced affinity for Fc receptors.

Alternatively, the oligomer is a fusion protein comprising multiple LCAT antigen binding proteins, with or without peptide linkers (spacer peptides). Among the suitable peptide linkers are those described in United States Patent. No. 4,751,180 and No. 4,935,233.

Another method for preparing oligomeric LCAT antigen binding protein derivatives involves use of a leucine zipper. Leucine zipper domains are peptides that promote oligomerization of the proteins in which they are found. Leucine zippers were originally identified in several DNA-binding proteins (Landschulz et al., 1988, Science 240:1759), and have since been found in a variety of different proteins. Among the known leucine zippers are naturally occurring peptides and derivatives thereof that dimerize or trimerize. Examples of leucine zipper domains suitable for producing soluble oligomeric proteins are described in PCT application WO 94/10308, and the leucine zipper derived from lung surfactant protein D (SPD) described in Hoppe et al., 1994, FEBS Letters 344:191. The use of a modified leucine zipper that allows for stable trimerization of a heterologous protein fused thereto is described in Fanslow et al., 1994, Semin. Immunol. 6:267-278. In one approach, recombinant fusion proteins comprising an LCAT antigen binding protein fragment or derivative fused to a leucine zipper peptide are expressed in suitable host cells, and the soluble oligomeric LCAT antigen binding protein fragments or derivatives that form are recovered from the culture supernatant.

### Species Cross Reactivity

In one embodiment, the LCAT antigen binding protein (e.g., those with sequences as described in **TABLES 1- 4**) binds a human LCAT protein (SEQ ID NO:1) but does not bind rabbit LCAT (SEQ ID NO:5) or mouse LCAT protein (SEQ ID NO:4). In another embodiment, the LCAT antigen binding protein binds a human LCAT protein (SEQ ID NO:1) and cyno LCAT (SEQ ID NO:3) but does not bind rabbit LCAT (SEQ ID NO:5) or mouse LCAT protein (SEQ ID NO:4).

In another embodiment, the LCAT antigen binding protein binds a human LCAT protein but does not bind rabbit LCAT (SEQ ID NO:5), mouse LCAT protein (SEQ ID NO:4) or cyno-LCAT (SEQ ID NO:3).

In various embodiments, the LCAT antigen binding protein binds a human LCAT protein (e.g., SEQ ID NO:1) with a K_{D} of ≤ 1, 2, 5, 10, 20 or 50 nM and binds to a cyno LCAT protein (e.g., SEQ ID NO:3) with a K_{D} of ≤ 50, 100, 150, 200, 300, 400, 500, 600 or 700 nM. In an aspect, the binding to human and cyno LCAT is determined, for example, by BiaCore such as described in **Example 3**.

### Glycosylation State of LCAT Antigen Binding Proteins

The antigen-binding protein may have a glycosylation pattern that is different or altered from that found in the native species. As is known in the art, glycosylation patterns can depend on both the sequence of the protein (*e.g*., the presence or absence of particular glycosylation amino acid residues, discussed below), or the host cell or organism in which the protein is produced. Particular expression systems are discussed below.

Glycosylation of polypeptides is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.

Addition of glycosylation sites to the antigen binding protein is conveniently accomplished by altering the amino acid sequence such that it contains one or more of the above-described tri-peptide sequences (for N-linked glycosylation sites). The alteration may also be made by the addition of, or substitution by, one or more serine or threonine residues to the starting sequence (for O-linked glycosylation sites). For ease, the antigen binding protein amino acid sequence may be altered through changes at the DNA level, particularly by mutating the DNA encoding the target polypeptide at preselected bases such that codons are generated that will translate into the desired amino acids.

Another means of increasing the number of carbohydrate moieties on the antigen binding protein is by chemical or enzymatic coupling of glycosides to the protein. These procedures are advantageous in that they do not require production of the protein in a host cell that has glycosylation capabilities for N- and O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published Sep. 11, 1987, and in Aplin and Wriston, 1981, CRC Crit. Rev, Biochem., pp. 259-306.

Removal of carbohydrate moieties present on the starting antigen binding protein may be accomplished chemically or enzymatically. Chemical deglycosylation requires exposure of the protein to the compound trifluoromethanesulfonic acid, or an equivalent compound. This treatment results in the cleavage of most or all sugars except the linking sugar (N-acetylglucosamine or N-acetylgalactosamine), while leaving the polypeptide intact. Chemical deglycosylation is described by Hakimuddin et al., 1987, Arch. Biochem. Biophys. 259:52 and by Edge et al., 1981, Anal. Biochem. 118:131. Enzymatic cleavage of carbohydrate moieties on polypeptides can be achieved by the use of a variety of endo- and exo-glycosidases as described by Thotakura et al., 1987, Meth. Enzymol. 138:350. Glycosylation at potential glycosylation sites may be prevented by the use of the compound tunicamycin as described by Duskin et al., 1982, J. Biol. Chem. 257:3105. Tunicamycin blocks the formation of protein-N-glycoside linkages.

Hence, aspects include glycosylation variants of the antigen binding proteins wherein the number and/or type of glycosylation site(s) has been altered compared to the amino acid sequences of the parent polypeptide. In certain embodiments, antibody protein variants comprise a greater or a lesser number of N-linked glycosylation sites than the native antibody. An N-linked glycosylation site is characterized by the sequence: Asn-X-Ser or Asn-X-Thr, wherein the amino acid residue designated as X may be any amino acid residue except proline. The substitution of amino acid residues to create this sequence provides a potential new site for the addition of an N-linked carbohydrate chain. Alternatively, substitutions that eliminate or alter this sequence will prevent addition of an N-linked carbohydrate chain present in the native polypeptide. For example, the glycosylation can be reduced by the deletion of an Asn or by substituting the Asn with a different amino acid. In other embodiments, one or more new N-linked sites are created. Antibodies typically have a N-linked glycosylation site in the Fc region.

As described in the Examples, it was found that the glycosylation state of certain LCAT antigen binding proteins had a significant effect on activity. In particular, glycosylation of certain sites within a CDR significantly reduced the ability of the antigen binding protein to agonize LCAT. Thus, in one embodiment, the antibodies listed in **TABLE 4** are unglycosylated. In another embodiment, the antigen binding protein has the 1, 2, 3, 4, 5 or all 6 of the CDRs of antibody 27C3 as shown in **TABLE 4**, but the CDR1 of the heavy chain (i.e., **CDRH1**) has been mutated such that the CDR is unglycosylated. In one aspect, the CDRH1 sequence is mutated such that an N-linked glycosylation site has been removed. Examples of CDRH1 sequences with such mutations include those described in **Example 5**. In certain embodiments, the antigen binding protein comprises the CDRs for 27C3 as specified in **TABLE 4**, except that CDRH1 has the general sequence SGGYXWS (SEQ ID NO:121), where X is either A, D, E, Q, S, T, V or Y.

### Antigen Binding Proteins with Labels and Effector Groups

In some embodiments, the antigen-binding proteincomprises one or more labels. The term "labeling group" or "label" means any detectable label. Examples of suitable labeling groups include, but are not limited to, the following: radioisotopes or radionuclides (*e.g.*, ³H, ¹⁴C, ¹⁵N, ³⁵S, ⁹⁰Y, ⁹⁹Tc, ¹¹¹In, ¹²⁵I, ¹³¹I), fluorescent groups (*e.g.*, FITC, rhodamine, lanthanide phosphors), enzymatic groups (*e.g*., horseradish peroxidase, β-galactosidase, luciferase, alkaline phosphatase), chemiluminescent groups, biotinyl groups, or predetermined polypeptide epitopes recognized by a secondary reporter (*e.g*., leucine zipper pair sequences, binding sites for secondary antibodies, metal binding domains, epitope tags). In some embodiments, the labeling group is coupled to the antigen binding protein *via* spacer arms of various lengths to reduce potential steric hindrance. Various methods for labeling proteins are known in the art and may be used as is seen fit.

The term "effector group" means any group coupled to an antigen binding protein that acts as a cytotoxic agent. Examples for suitable effector groups are radioisotopes or radionuclides (*e.g*., ³H, ¹⁴C, ¹⁵N, ³⁵S, ⁹⁰Y, ⁹⁹Tc, ¹¹¹In, ¹²⁵I, ¹³¹I). Other suitable groups include toxins, therapeutic groups, or chemotherapeutic groups. Examples of suitable groups include calicheamicin, auristatins, geldanamycin and maytansine. In some embodiments, the effector group is coupled to the antigen binding protein *via* spacer arms of various lengths to reduce potential steric hindrance.

In general, labels fall into a variety of classes, depending on the assay in which they are to be detected: a) isotopic labels, which may be radioactive or heavy isotopes; b) magnetic labels (*e.g*., magnetic particles); c) redox active moieties; d) optical dyes; enzymatic groups (*e.g*. horseradish peroxidase, β-galactosidase, luciferase, alkaline phosphatase); e) biotinylated groups; and f) predetermined polypeptide epitopes recognized by a secondary reporter (*e.g*., leucine zipper pair sequences, binding sites for secondary antibodies, metal binding domains, epitope tags, etc.). In some embodiments, the labeling group is coupled to the antigen binding protein *via* spacer arms of various lengths to reduce potential steric hindrance. Various methods for labeling proteins are known in the art.

Specific labels include optical dyes, including, but not limited to, chromophores, phosphors and fluorophores, with the latter being specific in many instances. Fluorophores can be either "small molecule" fluores, or proteinaceous fluores.

By "fluorescent label" is meant any molecule that may be detected *via* its inherent fluorescent properties. Suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade BlueJ, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 660, Alexa Fluor 680), Cascade Blue, Cascade Yellow and R-phycoerythrin (PE) (Molecular Probes, Eugene, OR), FITC, Rhodamine, and Texas Red (Pierce, Rockford, IL), Cy5, Cy5.5, Cy7 (Amersham Life Science, Pittsburgh, PA). Suitable optical dyes, including fluorophores, are described in Molecular Probes Handbook by Richard P. Haugland.

Suitable proteinaceous fluorescent labels also include, but are not limited to, green fluorescent protein, including a Renilla, Ptilosarcus, or Aequorea species of GFP (Chalfie et al., 1994, Science 263:802-805), EGFP (Clontech Labs., Inc., Genbank Accession Number U55762), blue fluorescent protein (BFP, Quantum Biotechnologies, Inc., Quebec, Canada; Stauber, 1998, Biotechniques 24:462-471; Heim et al., 1996, Curr. Biol. 6:178-182), enhanced yellow fluorescent protein (EYFP, Clontech Labs., Inc.), luciferase (Ichiki et al., 1993, J. Immunol. 150:5408-5417), β galactosidase (Nolan et al., 1988, Proc. Natl. Acad. Sci. U.S.A. 85:2603-2607) and Renilla (WO92/15673, WO95/07463, WO98/14605, WO98/26277, WO99/49019, United States Patents No. 5292658, No. 5418155, No. 5683888, No. 5741668, No. 5777079, No. 5804387, No. 5874304, No. 5876995, No. 5925558).

### Nucleic Acids Encoding LCAT Antigen Binding Proteins

Nucleic acids that encode for the antigen binding proteins described herein, or portions thereof, are also provided, including nucleic acids encoding one or both chains of an antibody, or a fragment, derivative, mutein, or variant thereof, polynucleotides encoding heavy chain variable regions or only CDRs, polynucleotides sufficient for use as hybridization probes, PCR primers or sequencing primers for identifying, analyzing, mutating or amplifying a polynucleotide encoding a polypeptide, anti-sense nucleic acids for inhibiting expression of a polynucleotide, and complementary sequences of the foregoing. The nucleic acids can be any length. They can be, for example, 5, 10, 15, 20, 25, 30, 35, 40, 45, 50, 75, 100, 125, 150, 175, 200, 250, 300, 350, 400, 450, 500, 750, 1,000, 1,500, 3,000, 5,000 or more nucleotides in length, and/or can comprise one or more additional sequences, for example, regulatory sequences, and/or be part of a larger nucleic acid, for example, a vector. The nucleic acids can be single-stranded or double-stranded and can comprise RNA and/or DNA nucleotides, and artificial variants thereof (e.g., peptide nucleic acids). **TABLE 6** shows exemplary nucleic acid sequences encoding an IgG2 heavy chain constant region and IgG2 kappa and lambda light chain constant regions. Any variable region provided herein may be attached to these constant regions to form complete heavy and light chain sequences. However, it should be understood that these constant regions sequences are provided as specific examples only. In some embodiments, the variable region sequences are joined to other constant region sequences that are known in the art. Exemplary nucleic acid sequences encoding heavy and light chain variable regions are provided in **TABLE 7**.

**Table 6: Exemplary Nucleic Acid Sequences Encoding Heavy and Light Chain Constant Regions**

| Chain Type | Nucleic Acid Sequence |
|---|---|
| IgG2 Heavy Chain | |
| | |
| IgG2 lambda light chain constant region | |
| IgG2 kappa light chain constant region | |

**TABLE 7** shows exemplary nucleic acid sequences encoding heavy chain and light chain variable regions, in which the various CDRH1, CDRH2, CDRH3, CDRL1, CDRL2 and CDRL3 sequences are embedded.

**Table 7**

| SEQ ID NO: | Variable Sequence | Nucleic Acid Encoding Sequence |
|---|---|---|
| 125 | V_{H}1 | |
| 126 | V_{H}2 | |
| 127 | V_{H}3 | |
| 128 | V_{H}4 | |
| 129 | V_{H}5 | |
| 130 | V_{H}6 | |
| 131 | V_{H}7 | |
| 132 | V_{H}8 | |
| 133 | V_{H}9 | |
| 134 | V_{H}10 | |
| 135 | V_{H}11 | |
| 136 | V_{H}12 | |
| 137 | V_{H}13 | |
| 138 | V_{H}14 | |
| 139 | V_{H}15 | |
| 140 | V_{H}16 | |
| 141 | V_{H}17 | |
| 142 | V_{H}18 | |
| 143 | V_{H}19 | |
| 144 | V_{H}20 | |
| 145 | V_{H}21 | |
| 146 | V_{H}22 | |
| 147 | V_{H}23 | |
| 148 | V_{H}24 | |
| 149 | V_{H}25 | |
| 150 | V_{H}26 | |
| 151 | V_{H}27 | |
| 152 | V_{H}28 | |
| 153 | V_{H}29 | |
| 154 | V_{H}30 | |
| 155 | V_{H}31 | |
| 156 | V_{L}1 | |
| 157 | V_{L}2 | |
| 158 | V_{L}3 | |
| 159 | V_{L}4 | |
| 160 | V_{L}5 | |

Nucleic acids encoding certain antigen binding proteins, or portions thereof (e.g., full length antibody, heavy or light chain, variable domain, or CDRH1, CDRH2, CDRH3, CDRL1, CDRL2, or CDRL3) may be isolated from B-cells of mice that have been immunized with LCAT or an immunogenic fragment thereof. The nucleic acid may be isolated by conventional procedures such as polymerase chain reaction (PCR). Phage display is another example of a known technique whereby derivatives of antibodies and other antigen binding proteins may be prepared. In one approach, polypeptides that are components of an antigen binding protein of interest are expressed in any suitable recombinant expression system, and the expressed polypeptides are allowed to assemble to form antigen binding proteins.

The nucleic acids provided in **TABLES 6** and **7** are exemplary only. Due to the degeneracy of the genetic code, each of the polypeptide sequences listed in **TABLES 1-4** or otherwise depicted herein are also encoded by a large number of other nucleic acid sequences besides those provided. One of ordinary skill in the art will appreciate that the present application thus provides adequate written description and enablement for each degenerate nucleotide sequence encoding each antigen binding protein.

An aspect further provides nucleic acids that hybridize to other nucleic acids (*e.g.,* nucleic acids comprising a nucleotide sequence listed in **TABLE 6 and TABLE 7**) under particular hybridization conditions. Methods for hybridizing nucleic acids are well-known in the art. *See, e.g.,* Current Protocols in Molecular Biology, John Wiley & Sons, N.Y. (1989), 6.3.1-6.3.6. As defined herein, a moderately stringent hybridization condition uses a prewashing solution containing 5x sodium chloride/sodium citrate (SSC), 0.5% SDS, 1.0 mM EDTA (pH 8.0), hybridization buffer of about 50% formamide, 6x SSC, and a hybridization temperature of 55°C (or other similar hybridization solutions, such as one containing about 50% formamide, with a hybridization temperature of 42°C), and washing conditions of 60°C, in 0.5x SSC, 0.1% SDS. A stringent hybridization condition hybridizes in 6x SSC at 45°C, followed by one or more washes in 0.1x SSC, 0.2% SDS at 68°C. Furthermore, one of skill in the art can manipulate the hybridization and/or washing conditions to increase or decrease the stringency of hybridization such that nucleic acids comprising nucleotide sequences that are at least 65%, 70%, 75%, 80%, 85%, 90%, 95%, 98% or 99% identical to each other typically remain hybridized to each other.

The basic parameters affecting the choice of hybridization conditions and guidance for devising suitable conditions are set forth by, for example, Sambrook, Fritsch, and Maniatis (2001, Molecular Cloning: A Laboratory Manual, Cold Spring Harbor Laboratory Press, Cold Spring Harbor, N.Y., *supra*; and Current Protocols in Molecular Biology, 1995, Ausubel et al., eds., John Wiley & Sons, Inc., sections 2.10 and 6.3-6.4), and can be readily determined by those having ordinary skill in the art based on, *e.g.,* the length and/or base composition of the nucleic acid.

Changes can be introduced by mutation into a nucleic acid, thereby leading to changes in the amino acid sequence of a polypeptide (e.g., an antibody or antibody derivative) that it encodes. Mutations can be introduced using any technique known in the art. In one embodiment, one or more particular amino acid residues are changed using, for example, a site-directed mutagenesis protocol. In another embodiment, one or more randomly selected residues is changed using, for example, a random mutagenesis protocol. However it is made, a mutant polypeptide can be expressed and screened for a desired property.

Mutations can be introduced into a nucleic acid without significantly altering the biological activity of a polypeptide that it encodes. For example, one can make nucleotide substitutions leading to amino acid substitutions at non-essential amino acid residues. Alternatively, one or more mutations can be introduced into a nucleic acid that selectively changes the biological activity of a polypeptide that it encodes. For example, the mutation can quantitatively or qualitatively change the biological activity. Examples of quantitative changes include increasing, reducing or eliminating the activity. Examples of qualitative changes include changing the antigen specificity of an antibody. In one embodiment, a nucleic acid encoding any antigen binding protein described herein can be mutated to alter the amino acid sequence using molecular biology techniques that are well-established in the art.

Another aspect providess nucleic acid molecules that are suitable for use as primers or hybridization probes for the detection of nucleic acid sequences. A nucleic acid molecule can comprise only a portion of a nucleic acid sequence encoding a full-length polypeptide, for example, a fragment that can be used as a probe or primer or a fragment encoding an active portion (e.g., LCAT binding portion) of a polypeptide.

Probes based on the sequence of a nucleic acid can be used to detect the nucleic acid or similar nucleic acids, for example, transcripts encoding a polypeptide. The probe can comprise a label group, *e.g.,* a radioisotope, a fluorescent compound, an enzyme, or an enzyme co-factor. Such probes can be used to identify a cell that expresses the polypeptide.

Another aspect provides vectors comprising a nucleic acid encoding a polypeptide or a portion thereof (e.g., a fragment containing one or more CDRs or one or more variable region domains). Examples of vectors include, but are not limited to, plasmids, viral vectors, non-episomal mammalian vectors and expression vectors, for example, recombinant expression vectors. The recombinant expression vectors can comprise a nucleic acid in a form suitable for expression of the nucleic acid in a host cell. The recombinant expression vectors include one or more regulatory sequences, selected on the basis of the host cells to be used for expression, which is operably linked to the nucleic acid sequence to be expressed. Regulatory sequences include those that direct constitutive expression of a nucleotide sequence in many types of host cells *(e.g.,* SV40 early gene enhancer, Rous sarcoma virus promoter and cytomegalovirus promoter), those that direct expression of the nucleotide sequence only in certain host cells (*e.g.,* tissue-specific regulatory sequences, *see,* Voss et al., 1986, Trends Biochem. Sci. 11:287, Maniatis et al., 1987, Science 236:1237), and those that direct inducible expression of a nucleotide sequence in response to particular treatment or condition (*e.g*., the metallothionin promoter in mammalian cells and the tet-responsive and/or streptomycin responsive promoter in both prokaryotic and eukaryotic systems (*see, id*.). It will be appreciated by those skilled in the art that the design of the expression vector can depend on such factors as the choice of the host cell to be transformed, the level of expression of protein desired, etc. The expression vectors can be introduced into host cells to thereby produce proteins or peptides, including fusion proteins or peptides, encoded by nucleic acids as described herein.

Another aspect provides host cells into which a recombinant expression vector has been introduced. A host cell can be any prokaryotic cell (for example, *E. coli*) or eukaryotic cell (for example, yeast, insect, or mammalian cells *(e.g.,* CHO cells)). Vector DNA can be introduced into prokaryotic or eukaryotic cells *via* conventional transformation or transfection techniques. For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., for resistance to antibiotics) is generally introduced into the host cells along with the gene of interest. Preferred selectable markers include those which confer resistance to drugs, such as G418, hygromycin and methotrexate. Cells stably transfected with the introduced nucleic acid can be identified by drug selection (e.g., cells that have incorporated the selectable marker gene will survive, while the other cells die), among other methods.

### Preparing LCAT Antigen Binding Proteins

Non-human antibodies that are provided can be, for example, derived from any antibody-producing animal, such as mouse, rat, rabbit, goat, donkey, or non-human primate (such as monkey *(e.g.,* cynomolgus or rhesus monkey) or ape *(e.g.,* chimpanzee)). Non-human antibodies can be used, for instance, in *in vitro* cell culture and cell-culture based applications, or any other application where an immune response to the antibody does not occur or is insignificant, can be prevented, is not a concern, or is desired. The antibodies may be produced by immunizing with full-length LCAT or a fragment thereof. Alternatively, the certain non-human antibodies may be raised by immunizing with amino acids which are segments of LCAT that form part of the epitope to which certain antibodies provided herein bind (see infra). The antibodies may be polyclonal, monoclonal, or may be synthesized in host cells by expressing recombinant DNA.

Fully human antibodies may be prepared as described above by immunizing transgenic animals containing human immunoglobulin loci or by selecting a phage display library that is expressing a repertoire of human antibodies.

The monoclonal antibodies (mAbs) can be produced by a variety of techniques, including conventional monoclonal antibody methodology, *e.g*., the standard somatic cell hybridization technique of Kohler and Milstein, 1975, Nature 256:495. Alternatively, other techniques for producing monoclonal antibodies can be employed, for example, the viral or oncogenic transformation of B-lymphocytes. One suitable animal system for preparing hybridomas is the murine system, which is a very well established procedure. Immunization protocols and techniques for isolation of immunized splenocytes for fusion are known in the art. For such procedures, B cells from immunized mice are fused with a suitable immortalized fusion partner, such as a murine myeloma cell line. If desired, rats or other mammals besides can be immunized instead of mice and B cells from such animals can be fused with the murine myeloma cell line to form hybridomas. Alternatively, a myeloma cell line from a source other than mouse may be used. Fusion procedures for making hybridomas also are well known.

As described in greater detail in the Examples, in one aspect, methods for generating agonist LCAT antigen binding proteins such as agonist monoclonal antibodies are provided in which an antibody-producing animal (e.g., mouse, rat, rabbit, goat, donkey, non-human primate and transgenic animals (e.g., mice) containing human immunological loci) are immunized with an activated form of an LCAT protein (i.e., a protein with higher enzymatic activity than native LCAT such as shown in SEQ ID NO: 1). A number of examples of activated LCAT proteins or fragments thereof that can be utilized for immunogens are described in WO 09/015314. In one embodiment, an animal is immunized with an LCAT containing an amino acid substitution at C31 of SEQ ID NO:1 or a fragment of such a mutant. In certain embodiments, the LCAT contains a C31Y mutation, i.e., cysteine 31 of SEQ ID NO:1 is substituted with tyrosine.

The single chain antibodies that are provided may be formed by linking heavy and light chain variable domain (Fv region) fragments *via* an amino acid bridge (short peptide linker), resulting in a single polypeptide chain. Such single-chain Fvs (scFvs) may be prepared by fusing DNA encoding a peptide linker between DNAs encoding the two variable domain polypeptides (V_{L} and V_{H}). The resulting polypeptides can fold back on themselves to form antigen-binding monomers, or they can form multimers (*e.g*., dimers, trimers, or tetramers), depending on the length of a flexible linker between the two variable domains (Kortt et al., 1997, Prot. Eng. 10:423; Kortt et al., 2001, Biomol. Eng. 18:95-108). By combining different V_{L} and V_{H} - comprising polypeptides, one can form multimeric scFvs that bind to different epitopes (Kriangkum et al., 2001, Biomol. Eng. 18:31-40). Techniques developed for the production of single chain antibodies include those described in U.S. Pat. No. 4,946,778; Bird, 1988, Science 242:423; Huston et al., 1988, Proc. Natl. Acad. Sci. U.S.A. 85:5879; Ward et al., 1989, Nature 334:544, de Graaf et al., 2002, Methods Mol Biol. 178:379-387. Single chain antibodies derived from antibodies provided herein include, but are not limited to scFvs comprising the variable domain combinations of the heavy and light chain variable regions depicted in **TABLE 2,** or combinations of light and heavy chain variable domains which include CDRs depicted in **TABLES 3A, 3B and 4.**

Antibodies provided herein that are of one subclass can be changed to antibodies from a different subclass using subclass switching methods. Thus, IgG antibodies may be derived from an IgM antibody, for example, and *vice versa.* Such techniques allow the preparation of new antibodies that possess the antigen binding properties of a given antibody (the parent antibody), but also exhibit biological properties associated with an antibody isotype or subclass different from that of the parent antibody. Recombinant DNA techniques may be employed. Cloned DNA encoding particular antibody polypeptides may be employed in such procedures, *e.g.,* DNA encoding the constant domain of an antibody of the desired isotype. *See, e.g.,* Lantto et al., 2002, Methods Mol. Biol. 178:303-316.

Accordingly, the antibodies that are provided include those comprising, for example, the variable domain combinations described, *supra*., having a desired isotype (for example, IgA, IgG1, IgG2, IgG3, IgG4, IgE, and IgD) as well as Fab or F(ab')₂ fragments thereof. Moreover, if an IgG4 is desired, it may also be desired to introduce a point mutation (CPSCP->CPPCP) (SEQ ID NOS 167 and 168, respectively) in the hinge region as described in Bloom et al., 1997, Protein Science 6:407, incorporated by reference herein) to alleviate a tendency to form intra-H chain disulfide bonds that can lead to heterogeneity in the IgG4 antibodies.

Moreover, techniques for deriving antibodies having different properties (*i.e.,* varying affinities for the antigen to which they bind) are also known. One such technique, referred to as chain shuffling, involves displaying immunoglobulin variable domain gene repertoires on the surface of filamentous bacteriophage, often referred to as phage display. Chain shuffling has been used to prepare high affinity antibodies to the hapten 2-phenyloxazol-5-one, as described by Marks et al., 1992, BioTechnology 10:779.

Conservative modifications may be made to the heavy and light chain variable regions described in **TABLE 2,** or the CDRs described in **TABLES 3A, 3B and 4** (and corresponding modifications to the encoding nucleic acids) to produce an LCAT antigen binding protein having functional and biochemical characteristics. Methods for achieving such modifications are described above.

LCAT antigen binding proteins may be further modified in various ways. For example, if they are to be used for therapeutic purposes, they may be conjugated with polyethylene glycol (pegylated) to prolong the serum half-life or to enhance protein delivery. Alternatively, the V region of the subject antibodies or fragments thereof may be fused with the Fc region of a different antibody molecule. The Fc region used for this purpose may be modified so that it does not bind complement, thus reducing the likelihood of inducing cell lysis in the patient when the fusion protein is used as a therapeutic agent. In addition, the subject antibodies or functional fragments thereof may be conjugated with human serum albumin to enhance the serum half-life of the antibody or fragment thereof. Another useful fusion partner for the antigen binding proteins or fragments thereof is transthyretin (TTR). TTR has the capacity to form a tetramer, thus an antibody-TTR fusion protein can form a multivalent antibody which may increase its binding avidity.

Alternatively, substantial modifications in the functional and/or biochemical characteristics of the antigen binding proteins described herein may be achieved by creating substitutions in the amino acid sequence of the heavy and light chains that differ significantly in their effect on maintaining (a) the structure of the molecular backbone in the area of the substitution, for example, as a sheet or helical conformation, (b) the charge or hydrophobicity of the molecule at the target site, or (c) the bulkiness of the side chain. A "conservative amino acid substitution" may involve a substitution of a native amino acid residue with a nonnative residue that has little or no effect on the polarity or charge of the amino acid residue at that position. *See,* **TABLE 3,** *supra.* Furthermore, any native residue in the polypeptide may also be substituted with alanine, as has been previously described for alanine scanning mutagenesis.

Amino acid substitutions (whether conservative or non-conservative) of the subject antibodies can be implemented by those skilled in the art by applying routine techniques. Amino acid substitutions can be used to identify important residues of the antibodies provided herein, or to increase or decrease the affinity of these antibodies for LCAT.

### Methods of Expressing Antigen Binding Proteins

Expression systems and constructs in the form of plasmids, expression vectors, transcription or expression cassettes that comprise at least one polynucleotide as described above are also provided herein, as well host cells comprising such expression systems or constructs.

The antigen binding proteins provided herein may be prepared by any of a number of conventional techniques. For example, LCAT antigen binding proteins may be produced by recombinant expression systems, using any technique known in the art. *See, e.g.,* Monoclonal Antibodies, Hybridomas: A New Dimension in Biological Analyses, Kennet et al. (eds.) Plenum Press, New York (1980); and Antibodies: A Laboratory Manual, Harlow and Lane (eds.), Cold Spring Harbor Laboratory Press, Cold Spring Harbor, N.Y. (1988).

Antigen binding proteins can be expressed in hybridoma cell lines *(e.g.,* in particular antibodies may be expressed in hybridomas) or in cell lines other than hybridomas. Expression constructs encoding the antibodies can be used to transform a mammalian, insect or microbial host cell. Transformation can be performed using any known method for introducing polynucleotides into a host cell, including, for example packaging the polynucleotide in a virus or bacteriophage and transducing a host cell with the construct by transfection procedures known in the art, as exemplified by United States Patent No. 4,399,216; No. 4,912,040; No. 4,740,461; No. 4,959,455. The optimal transformation procedure used will depend upon which type of host cell is being transformed. Methods for introduction of heterologous polynucleotides into mammalian cells are well known in the art and include, but are not limited to, dextran-mediated transfection, calcium phosphate precipitation, polybrene mediated transfection, protoplast fusion, electroporation, encapsulation of the polynucleotide(s) in liposomes, mixing nucleic acid with positively-charged lipids, and direct microinjection of the DNA into nuclei.

Recombinant expression constructs typically comprise a nucleic acid molecule encoding a polypeptide comprising one or more of the following: one or more CDRs provided herein; a light chain constant region; a light chain variable region; a heavy chain constant region *(e.g.,* C_{H}1, C_{H}2 and/or C_{H}3); and/or another scaffold portion of an LCAT antigen binding protein. These nucleic acid sequences are inserted into an appropriate expression vector using standard ligation techniques. In one embodiment, the heavy or light chain constant region is appended to the C-terminus of the anti-LCAT specific heavy or light chain variable region and is ligated into an expression vector. The vector is typically selected to be functional in the particular host cell employed (*i.e.,* the vector is compatible with the host cell machinery, permitting amplification and/or expression of the gene can occur). In some embodiments, vectors are used that employ protein-fragment complementation assays using protein reporters, such as dihydrofolate reductase (*see,* for example, U.S. Pat. No. 6,270,964). Suitable expression vectors can be purchased, for example, from Invitrogen Life Technologies or BD Biosciences (formerly "Clontech"). Other useful vectors for cloning and expressing the antibodies and fragments include those described in Bianchi and McGrew, 2003, Biotech. Biotechnol. Bioeng. 84:439-44. Additional suitable expression vectors are discussed, for example, in Methods Enzymol., vol. 185 (D. V. Goeddel, ed.), 1990, New York: Academic Press.

Typically, expression vectors used in any of the host cells will contain sequences for plasmid maintenance and for cloning and expression of exogenous nucleotide sequences. Such sequences, collectively referred to as "flanking sequences" in certain embodiments will typically include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element. Each of these sequences is discussed below.

Optionally, the vector may contain a "tag"-encoding sequence, *i.e.,* an oligonucleotide molecule located at the 5' or 3' end of the LCAT antigen binding protein coding sequence; the oligonucleotide sequence encodes polyHis (such as hexaHis (SEQ ID NO:169)), or another "tag" such as FLAG^{®}, HA (hemaglutinin influenza virus), or myc, for which commercially available antibodies exist. This tag is typically fused to the polypeptide upon expression of the polypeptide, and can serve as a means for affinity purification or detection of the LCAT antigen binding protein from the host cell. Affinity purification can be accomplished, for example, by column chromatography using antibodies against the tag as an affinity matrix. Optionally, the tag can subsequently be removed from the purified LCAT antigen binding protein by various means such as using certain peptidases for cleavage.

Flanking sequences may be homologous *(i.e.,* from the same species and/or strain as the host cell), heterologous (*i.e*., from a species other than the host cell species or strain), hybrid *(i.e.,* a combination of flanking sequences from more than one source), synthetic or native. As such, the source of a flanking sequence may be any prokaryotic or eukaryotic organism, any vertebrate or invertebrate organism, or any plant, provided that the flanking sequence is functional in, and can be activated by, the host cell machinery.

Flanking sequences useful in the vectors may be obtained by any of several methods well known in the art. Typically, flanking sequences useful herein will have been previously identified by mapping and/or by restriction endonuclease digestion and can thus be isolated from the proper tissue source using the appropriate restriction endonucleases. In some cases, the full nucleotide sequence of a flanking sequence may be known. Here, the flanking sequence may be synthesized using the methods described herein for nucleic acid synthesis or cloning.

Whether all or only a portion of the flanking sequence is known, it may be obtained using polymerase chain reaction (PCR) and/or by screening a genomic library with a suitable probe such as an oligonucleotide and/or flanking sequence fragment from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen^{®} column chromatography (Chatsworth, CA), or other methods known to the skilled artisan. The selection of suitable enzymes to accomplish this purpose will be readily apparent to one of ordinary skill in the art.

An origin of replication is typically a part of those prokaryotic expression vectors purchased commercially, and the origin aids in the amplification of the vector in a host cell. If the vector of choice does not contain an origin of replication site, one may be chemically synthesized based on a known sequence, and ligated into the vector. For example, the origin of replication from the plasmid pBR322 (New England Biolabs, Beverly, MA) is suitable for most gram-negative bacteria, and various viral origins (e.g., SV40, polyoma, adenovirus, vesicular stomatitus virus (VSV), or papillomaviruses such as HPV or BPV) are useful for cloning vectors in mammalian cells. Generally, the origin of replication component is not needed for mammalian expression vectors (for example, the SV40 origin is often used only because it also contains the virus early promoter).

A transcription termination sequence is typically located 3' to the end of a polypeptide coding region and serves to terminate transcription. Usually, a transcription termination sequence in prokaryotic cells is a G-C rich fragment followed by a poly-T sequence. While the sequence is easily cloned from a library or even purchased commercially as part of a vector, it can also be readily synthesized using methods for nucleic acid synthesis such as those described herein.

A selectable marker gene encodes a protein necessary for the survival and growth of a host cell grown in a selective culture medium. Typical selection marker genes encode proteins that (a) confer resistance to antibiotics or other toxins, *e.g*., ampicillin, tetracycline, or kanamycin for prokaryotic host cells; (b) complement auxotrophic deficiencies of the cell; or (c) supply critical nutrients not available from complex or defined media. Specific selectable markers are the kanamycin resistance gene, the ampicillin resistance gene, and the tetracycline resistance gene. Advantageously, a neomycin resistance gene may also be used for selection in both prokaryotic and eukaryotic host cells.

Other selectable genes may be used to amplify the gene that will be expressed. Amplification is the process wherein genes that are required for production of a protein critical for growth or cell survival are reiterated in tandem within the chromosomes of successive generations of recombinant cells. Examples of suitable selectable markers for mammalian cells include dihydrofolate reductase (DHFR) and promoterless thymidine kinase genes. Mammalian cell transformants are placed under selection pressure wherein only the transformants are uniquely adapted to survive by virtue of the selectable gene present in the vector. Selection pressure is imposed by culturing the transformed cells under conditions in which the concentration of selection agent in the medium is successively increased, thereby leading to the amplification of both the selectable gene and the DNA that encodes another gene, such as an antigen binding protein that binds LCAT polypeptide. As a result, increased quantities of a polypeptide such as an antigen binding protein are synthesized from the amplified DNA.

A ribosome-binding site is usually necessary for translation initiation of mRNA and is characterized by a Shine-Dalgarno sequence (prokaryotes) or a Kozak sequence (eukaryotes). The element is typically located 3' to the promoter and 5' to the coding sequence of the polypeptide to be expressed.

In some cases, such as where glycosylation is desired in a eukaryotic host cell expression system, one may manipulate the various pre- or pro-sequences to improve glycosylation or yield. For example, one may alter the peptidase cleavage site of a particular signal peptide, or add prosequences, which also may affect glycosylation. The final protein product may have, in the -1 position (relative to the first amino acid of the mature protein), one or more additional amino acids incident to expression, which may not have been totally removed. For example, the final protein product may have one or two amino acid residues found in the peptidase cleavage site, attached to the amino-terminus. Alternatively, use of some enzyme cleavage sites may result in a slightly truncated form of the desired polypeptide, if the enzyme cuts at such area within the mature polypeptide.

Expression and cloning will typically contain a promoter that is recognized by the host organism and operably linked to the molecule encoding the LCAT antigen binding protein. Promoters are untranscribed sequences located upstream *(i.e.,* 5') to the start codon of a structural gene (generally within about 100 to 1000 bp) that control transcription of the structural gene. Promoters are conventionally grouped into one of two classes: inducible promoters and constitutive promoters. Inducible promoters initiate increased levels of transcription from DNA under their control in response to some change in culture conditions, such as the presence or absence of a nutrient or a change in temperature. Constitutive promoters, on the other hand, uniformly transcribe a gene to which they are operably linked, that is, with little or no control over gene expression. A large number of promoters, recognized by a variety of potential host cells, are well known. A suitable promoter is operably linked to the DNA encoding heavy chain or light chain comprising a an LCAT antigen binding protein by removing the promoter from the source DNA by restriction enzyme digestion and inserting the desired promoter sequence into the vector.

Suitable promoters for use with yeast hosts are also well known in the art. Yeast enhancers are advantageously used with yeast promoters. Suitable promoters for use with mammalian host cells are well known and include, but are not limited to, those obtained from the genomes of viruses such as polyoma virus, fowlpox virus, adenovirus (such as Adenovirus 2), bovine papilloma virus, avian sarcoma virus, cytomegalovirus, retroviruses, hepatitis-B virus, and Simian Virus 40 (SV40). Other suitable mammalian promoters include heterologous mammalian promoters, for example, heat-shock promoters and the actin promoter.

An enhancer sequence may be inserted into the vector to increase transcription of DNA encoding light chain or heavy chain comprising an LCAT antigen binding protein by higher eukaryotes. Enhancers are cis-acting elements of DNA, usually about 10-300 bp in length, that act on the promoter to increase transcription. Enhancers are relatively orientation and position independent, having been found at positions both 5' and 3' to the transcription unit. Several enhancer sequences available from mammalian genes are known (e.g., globin, elastase, albumin, alpha-feto-protein and insulin). Typically, however, an enhancer from a virus is used. The SV40 enhancer, the cytomegalovirus early promoter enhancer, the polyoma enhancer, and adenovirus enhancers known in the art are exemplary enhancing elements for the activation of eukaryotic promoters. While an enhancer may be positioned in the vector either 5' or 3' to a coding sequence, it is typically located at a site 5' from the promoter. A sequence encoding an appropriate native or heterologous signal sequence (leader sequence or signal peptide) can be incorporated into an expression vector, to promote extracellular secretion of the antibody. The choice of signal peptide or leader depends on the type of host cells in which the antibody is to be produced, and a heterologous signal sequence can replace the native signal sequence. Examples of signal peptides that are functional in mammalian host cells include the following: the signal sequence for interleukin-7 (IL-7) described in US Patent No. 4,965,195; the signal sequence for interleukin-2 receptor described in Cosman et al., 1984, Nature 312:768; the interleukin-4 receptor signal peptide described in EP Patent No. 0367 566; the type I interleukin-1 receptor signal peptide described in U.S. Patent No. 4,968,607; the type II interleukin-1 receptor signal peptide described in EP Patent No. 0 460 846.

The expression vectors that are provided may be constructed from a starting vector such as a commercially available vector. Such vectors may or may not contain all of the desired flanking sequences. Where one or more of the flanking sequences described herein are not already present in the vector, they may be individually obtained and ligated into the vector. Methods used for obtaining each of the flanking sequences are well known to one skilled in the art.

After the vector has been constructed and a nucleic acid molecule encoding light chain, a heavy chain, or a light chain and a heavy chain comprising an LCAT antigen binding sequence has been inserted into the proper site of the vector, the completed vector may be inserted into a suitable host cell for amplification and/or polypeptide expression. The transformation of an expression vector for an antigen-binding protein into a selected host cell may be accomplished by well known methods including transfection, infection, calcium phosphate co-precipitation, electroporation, microinjection, lipofection, DEAE-dextran mediated transfection, or other known techniques. The method selected will in part be a function of the type of host cell to be used. These methods and other suitable methods are well known to the skilled artisan, and are set forth, for example, in Sambrook *et al*., 2001, *supra.*

A host cell, when cultured under appropriate conditions, synthesizes an antigen binding protein that can subsequently be collected from the culture medium (if the host cell secretes it into the medium) or directly from the host cell producing it (if it is not secreted). The selection of an appropriate host cell will depend upon various factors, such as desired expression levels, polypeptide modifications that are desirable or necessary for activity (such as glycosylation or phosphorylation) and ease of folding into a biologically active molecule.

Mammalian cell lines available as hosts for expression are well known in the art and include, but are not limited to, immortalized cell lines available from the American Type Culture Collection (ATCC), including but not limited to Chinese hamster ovary (CHO) cells, HeLa cells, baby hamster kidney (BHK) cells, monkey kidney cells (COS), human hepatocellular carcinoma cells *(e.g.,* Hep G2), and a number of other cell lines. In certain embodiments, cell lines may be selected through determining which cell lines have high expression levels and constitutively produce antigen binding proteins with LCAT binding properties. In another embodiment, a cell line from the B cell lineage that does not make its own antibody but has a capacity to make and secrete a heterologous antibody can be selected.

### Use of LCAT Antigen Binding Proteins in Therapy

### General Considerations

The antigen binding proteins that are provided can be used to decrease (e.g., slow or reverse) the accumulation of cholesterol in the arteries of a mammalian subject by increasing the level of LCAT activity and modulating HDL metabolism in the serum of the subject. The LCAT antigen binding proteins can also be used to increase the level of HDL-C and to decrease the level of triglyceride in a patient's serum relative to the level prior to administration of an LCAT binding protein. In some therapeutic applications, an LCAT antigen binding protein is administered to maintain a ratio of total serum cholesterol to serum high density lipoproteins in a mammal at below 5:1, which is considered to be a profile of average risk for heart disease. (W. P. Castelli et al. (1986) JAMA 256:2835.) By helping to maintain healthy serum cholesterol levels, the LCAT antigen binding proteins provided herein have utility in preventing or treating a variety of conditions, including: atherosclerosis, cardiovascular diseases, cholesterol-related disorders, metabolic syndrome, diabetes, insulin-resistance, inflammatory conditions, thrombosis-related conditions, blood disorders, anemia and conditions involving LCAT deficiencies such as Fish Eye Syndrome, Classic LCAT Deficiency Disease, and chronic kidney disease.

When the LCAT binding proteins are administered prophylactically to prevent diseases associated with low LCAT activity, low HDL levels, or insufficient reverse cholesterol transport, the binding protein is administered to a subject that is in need of prevention of disorders caused by insufficient LCAT activity and/or low levels of HDL-C and/or insufficient reverse cholesterol transport. The subject may be a human subject who is at risk of disorders such as those described herein. The subject may be at risk due to genetic predisposition, sedentary lifestyle, diet, exposure to disorder-causing agents, and/or exposure to pathogens. To determine whether a subject is at risk of, for example atherosclerosis, an atherogenic lipoprotein profile can be assessed. For example, a ratio of serum cholesterol to HDLs of 5:1 or above indicates a higher than average risk of developing a disease associated with high cholesterol levels. Other factors include a serum cholesterol level of 240 mg/dL or above, an HDL level 35 mg/dL or below, or an LDL level 190 mg/dL or above, a plasma LCAT protein level lower than normal (<5 .mu.g/ml), and/or a decreased plasma cholesterol esterification rate (<60 nmol/ml/hr).

In humans, the normal cholesterol esterification rate ranges from about 60 nmol/ml/hr to about 130 nmol/mL per hour. The effective treatment of atherosclerosis in humans can involve administration of an LCAT antigen binding protein to achieve a cholesterol esterification rate of about 130 nmol/ml/hr.

In one embodiment, an LCAT binding protein is administered for chronic treatment. In another embodiment, the binding proteins are administered for acute therapy.

### Treatment of Cardiovascular Disease, Cholesterol-Related Disorders and Atherosclerosis

In certain embodiments, the LCAT binding proteins can be utilized in the treatment, prevention, or management of a cardiovascular disease. As used herein, the term "cardiovascular disease" refers to diseases of the heart and circulatory system. Cardiovascular diseases which the LCAT binding proteins are useful for preventing or treating include, but are not limited to, arteriosclerosis; atherosclerosis; stroke; ischemia; endothelium dysfunctions, in particular those dysfunctions affecting blood vessel elasticity; peripheral vascular disease; coronary artery disease, coronary heart disease; myocardial infarction; cerebral infarction and restenosis; thrombosis; high blood pressure and angina. Other cardiovascular diseases that can be treated or prevented with an LCAT antigen binding protein include, acute cardiac ischemic events, arrhythmia, arterial fibrulation, atrial fibrillation, cardiac insufficiency, chronic heart failure, congestive heart failure, deep vein thrombosis, diabetic neuropathy, diastolic dysfunction in subjects with diabetes mellitus, edema, essential hypertension, eventual pulmonary embolism, fatty liver disease, heart disease, heart failure, homozygous familial hypercholesterolemia (HoFH), homozygous familial sitosterolemia, hypercholesterolemia, hyperlipidemia, hyperlipidemia in HIV positive subjects, hypertension, hypertriglyceridemia, ischemic complications in unstable angina and myocardial infarction, low blood pressure, mixed dyslipidemia, moderate to mild heart failure, obesity management, paroxysmal atrial fibrillation/flutter (PAF), paroxysmal supraventricular tachycardias (PSVT), platelet aggregation, primary hypercholesterolemia, primary hyperlipidemia, pulmonary arterial hypertension, pulmonary hypertension, recurrent hemodynamically unstable ventricular tachycardia (VT), recurrent ventricular arrhythmias, recurrent ventricular fibrillation (VF), ruptured aneurysm, sitisterolemia, stroke, supraventricular tachycardia, symptomatic atrial fibrillation/flutter, tachycardia, venous thromboembolism, and ventricular arrhythmias.

In other embodiments, the antigen binding proteins are used in the treatment, prevention, or management of a "cholesterol-related disorder", which includes any one or more of the following: hypercholesterolemia; heart disease; metabolic syndrome; diabetes; coronary heart disease; stroke; cardiovascular diseases; Alzheimer's disease and dyslipidemias, which can be manifested, for example, by an elevated total serum cholesterol, elevated LDL, elevated triglycerides, elevated VLDL, and/or low HDL and /or circulating LCAT activity. Non-limiting examples of primary and secondary dyslipidemias that can be treated include metabolic syndrome; diabetes mellitus; familial combined hyperlipidemia; familial hypertriglyceridemia; familial hypercholesterolemias, including heterozygous hypercholesterolemia; homozygous hypercholesterolemia; familial defective apoplipoprotein B-100; polygenic hypercholesterolemia; remnant removal disease, hepatic lipase deficiency; and dyslipidemia secondary to any of the following: dietary indiscretion; hypothyroidism; drug treatment (including estrogen and progestin therapy and treatment with beta-blockers, and thiazide diuretics); nephrotic syndrome; chronic renal failure; Cushing's syndrome; primary biliary cirrhosis; glycogen storage diseases; hepatoma; cholestasis; acromegaly; insulinoma; isolated growth hormone deficiency; and alcohol-induced hypertriglyceridemia.

In another embodiment, the LCAT antigen binding protein is useful in preventing or treating atherosclerotic diseases, such as, for example, coronary heart disease; coronary artery disease; peripheral arterial disease; stroke (ischaemic and hemorrhagic); angina pectoris; cerebrovascular disease; acute coronary syndrome; and myocardial infarction. In some embodiments, an antigen binding protein is useful in reducing the risk of: nonfatal heart attacks; fatal and non-fatal strokes; heart surgery; hospitalization for heart failure; chest pain in patients with heart disease; and/or cardiovascular events because of established heart disease such as prior heart attack, prior heart surgery, and/or chest pain with evidence of clogged arteries. In some embodiments, the antigen binding proteins are used to reduce the risk of recurrent cardiovascular events.

### Treatment and Prevention of Inflammatory Conditions

The LCAT binding proteins are also useful in preventing and treating various inflammatory and autoimmune diseases, including, but not limited to: arthritic diseases (such as rheumatoid arthritis), osteoarthritis, gouty arthritis, spondylitis, thyroid-associated opthalmopathy, Behcet disease, sepsis, septic shock, endotoxic shock, gram negative sepsis, gram positive sepsis, toxic shock syndrome, asthma, chronic bronchitis, allergic rhinitis, allergic conjunctivitis, vernal conjunctivitis, eosinophilic granuloma, adult (acute) respiratory distress syndrome (ARDS), chronic pulmonary inflammatory disease (such as chronic obstructive pulmonary disease), silicosis, pulmonary sarcoidosis, reperfusion injury of the myocardium, brain or extremities, brain or spinal cord injury due to minor trauma, fibrosis including cystic fibrosis, keloid formation, scar tissue formation, atherosclerosis, autoimmune diseases, such as systemic lupus erythematosus (SLE) and transplant rejection disorders (e.g., graft vs. host (GvH) reaction and allograft rejection), chronic glomerulonephritis, inflammatory bowel diseases, such as Crohn's disease and ulcerative colitis, proliferative lymphocytic diseases, such as leukemias (e.g. chronic lymphocytic leukemia; CLL) (see Munoz et al., J. Exp. Med. 172:95-103 (1990); Mentz et al., Blood 88:2172-2182 (1996)), and inflammatory dermatoses, such as atopic dermatitis, psoriasis, or urticaria.

### Treatment and Prevention of Thrombosis Related Conditions

In a further embodiment, an LCAT antigen binding protein is used in the prevention or treatment of a variety of disorders in which there is a need to prevent or treat thrombosis and subsequent decrease or loss of blood flow. Examples of thrombotic disorders, include, but are not limited to, atherosclerosis, myocardial infarction, stroke, and kidney ischemia, and thrombosis in any part of the mammalian body. LCAT antigen binding proteins can also be used in the prevention and treatment of microangiopathy in which formation of microthrombi or von Willebrand factor (VWF) binding to platelets causes excessive consumption of platelets and/or VWF leading to subsequent bleeding diathesis. Examples of the latter disorders include, but not limited to, thrombotic thrombocytopenic purpura, type II and platelet type von Willebrand disease (VWD). Additionally, the binding proteins are useful in prolonging bleed time in a mammal and as such, are useful as anti-thrombotic agents, both in therapeutic and prophylactic methods. As such, the antigen binding proteins are useful as anticoagulant agents and/or anti-platelet agents.

The LCAT antigen binding proteins also have utility in the treatment of any disorder that is presently treated using anticoagulant therapy. Such disorders include pulmonary embolism, unstable angina, myocardial infarction, deep vein thrombosis, atrial fibrillation with embolization, acute and chronic coagulopathies (disseminated intravascular coagulation), for prevention of clotting in arterial and cardiac surgery, for prophylaxis and treatment of peripheral arterial embolism. The antigen binding protein can also used to treat thrombotic thrombocytopic purpura, other types of microangiopathy that are mediated by spontaneous interaction between VWF and platelets, platelet type or type IIb von Willebrand diseases in which there is an increased binding of VWF to platelets (either caused by a defect in GPIb or in VWF). LCAT antigen binding proteins are also useful as anti-platelet agents in blood transfusions, extracorporeal circulation, dialysis procedures as well as blood sampling for laboratory procedures. The LCAT antigen binding proteins also have value in surgical procedures to prevent the formation of blood clots. Such indications are particularly desirable for patients undergoing abdominal surgery to reduce the risk of thromboemolic complications, patients undergoing knee or hip replacement therapy during and following the replacement procedure, as well as a general prophylactic to prevent clot formation at a later stage. The compounds, compositions and methods are further useful in the treatment of subjects that are under risk of thromboembolic complications due to severely restricted mobility e.g., during acute illness.

### Treatment and Prevention of Blood Disorders

Increasing LCAT levels causes the net transfer of free cholesterol from red blood cells (RBC) to HDL, thus changing the composition of the RBC membrane to a more fluid state This action increases the oxygenation of the RBC and improves the rheology of RBCs (by increasing RBC deformability and flow, decreasing phosphatidylserine externalization, and by decreasing the propensity for RBC adhesion and aggregation), thereby decreasing anemia and increasing the ability of the RBC to oxygenate tissue, especially peripheral tissues (see, e.g., WO 2010/144904). LCAT antigen binding proteins can thus be administered to a subject to treat or prevent a condition characterized by red blood cells with reduced deformability, reduced oxygenation, reduced nitric oxide function, increased adhesion and/or aggregation, or decreased life span.

Thus, in one emodiment, the LCAT antigen binding proteins can be used to modulate lipid content of red blood cells membranes. There are many conditions in which the cell membranes of the RBC have increased levels of free cholesterol (FC) in relation to phospholipid. Accordingly, the LCAT binding proteins can be utilized to treat or prevent such conditions, including, but not limited to, sickle cell disease, diabetes, thalassemia, liver disease, cirrhosis, hepatitis, acanthosytosis, sepsis, dementia, anemia, or microvascular disorders, parasitic disease, erectile dysfunction, cancer, and pre-eclampsia.

In liver disease, RBC cholesterol is increased and anemia often occurs. Hence, in another embodiment, an LCAT antigen binding protein is administered to treat anemia. Administering an LCAT antigen binding protein is expected to aid in normalizing RBC cholesterol levels, restoring normal shape and function of the effected RBCs, and increasing RBC life-span, thereby reducing the risk for anemia.

An increase in free cholesterol levels in RBC is also associated with various microvascular disorders which cause rigidity and increase the tendency of RBCs to adhere and aggregate. These changes are magnified in the low flow (or low pressure) found in capillaries and venules, thus increasing the risk of blockages in the peripheral vessels. In organs where the microvasculature is critical for normal function (e.g., eyes, ears, brain, kidney, penis, lungs), repeated ischemic events in these vessels can lead to loss of function (e g., blindness, hearing loss, kidney failure, ischemic microvascular brain disease such as dementia and Alzheimer's), and erectile dysfunction). Accordingly, treatment with LCAT antigen binding proteins can be useful in treating such microvascular disorders.

### Pharmaceutical Formulations and Administration

Pharmaceutical compositions that comprise an LCAT antigen binding protein are also provided and can be utilized in any of the preventive and therapeutic methods disclosed herein. In an embodiment, a therapeutically effective amount of one or a plurality of the antigen binding proteins and a pharmaceutically acceptable diluent, carrier, solubilizer, emulsifier, preservative, and/or adjuvant are also provided. Acceptable formulation materials are nontoxic to recipients at the dosages and concentrations employed.

In certain embodiments, the pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. REMINGTON'S PHARMACEUTICAL SCIENCES, 18" Edition, (A.R. Genrmo, ed.), 1990, Mack Publishing Company provides additional details and options for suitable agents that can be incorporated into the pharmaceutical compositions.

In certain embodiments, the optimal pharmaceutical composition will be determined by one skilled in the art depending upon, for example, the intended route of administration, delivery format and desired dosage. *See,* for example, REMINGTON'S PHARMACEUTICAL SCIENCES, *supra.* In certain embodiments, such compositions may influence the physical state, stability, rate of *in vivo* release and rate *of in vivo* clearance of the antigen binding proteins disclosed. In certain embodiments, the primary vehicle or carrier in a pharmaceutical composition may be either aqueous or non-aqueous in nature. For example, a suitable vehicle or carrier may be water for injection or physiological saline solution. In certain embodiments, LCAT antigen binding protein compositions may be prepared for storage by mixing the selected composition having the desired degree of purity with optional formulation agents (REMINGTON'S PHARMACEUTICAL SCIENCES, *supra*) in the form of a lyophilized cake or an aqueous solution. Further, in certain embodiments, the LCAT antigen binding protein may be formulated as a lyophilizate using appropriate excipients such as sucrose.

The pharmaceutical compositions can be selected for parenteral delivery. Alternatively, the compositions may be selected for inhalation or for delivery through the digestive tract, such as orally. Preparation of such pharmaceutically acceptable compositions is within the skill of the art.

The formulation components are present preferably in concentrations that are acceptable to the site of administration. In certain embodiments, buffers are used to maintain the composition at physiological pH or at a slightly lower pH, typically within a pH range of from about 5 to about 8.

When parenteral administration is contemplated, the therapeutic compositions may be provided in the form of a pyrogen-free, parenterally acceptable aqueous solution comprising the desired human LCAT antigen binding protein in a pharmaceutically acceptable vehicle. A particularly suitable vehicle for parenteral injection is sterile distilled water in which the LCAT antigen binding protein is formulated as a sterile, isotonic solution, properly preserved. In certain embodiments, the preparation can involve the formulation of the desired molecule with an agent, such as injectable microspheres, bio-erodible particles, polymeric compounds (such as polylactic acid or polyglycolic acid), beads or liposomes, that may provide controlled or sustained release of the product which can be delivered *via* depot injection. In certain embodiments, hyaluronic acid may also be used, having the effect of promoting sustained duration in the circulation. In certain embodiments, implantable drug delivery devices may be used to introduce the desired antigen binding protein.

Certain pharmaceutical compositions are formulated for inhalation. In some embodiments, LCAT antigen binding proteins are formulated as a dry, inhalable powder. In specific embodiments, LCAT antigen binding protein inhalation solutions may also be formulated with a propellant for aerosol delivery. In certain embodiments, solutions may be nebulized. Pulmonary administration and formulation methods therefore are further described in WO 9420069, which describes pulmonary delivery of chemically modified proteins. Some formulations can be administered orally. LCAT antigen binding proteins that are administered in this fashion can be formulated with or without carriers customarily used in the compounding of solid dosage forms such as tablets and capsules. In certain embodiments, a capsule may be designed to release the active portion of the formulation at the point in the gastrointestinal tract when bioavailability is maximized and pre-systemic degradation is minimized. Additional agents can be included to facilitate absorption of the LCAT antigen binding protein. Diluents, flavorings, low melting point waxes, vegetable oils, lubricants, suspending agents, tablet disintegrating agents, and binders may also be employed.

Some pharmaceutical compositions comprise an effective quantity of one or a plurality of LCAT antigen binding proteins in a mixture with non-toxic excipients that are suitable for the manufacture of tablets. By dissolving the tablets in sterile water, or another appropriate vehicle, solutions may be prepared in unit-dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional pharmaceutical compositions will be evident to those skilled in the art, including formulations involving LCAT binding proteins in sustained- or controlled-delivery formulations. Techniques for formulating a variety of other sustained- or controlled-delivery means, such as liposome carriers, bio-erodible microparticles or porous beads and depot injections, are also known to those skilled in the art. *See,* for example, WO 9315722 International Patent Application No. PCT/US93/00829, which describes controlled release of porous polymeric microparticles for delivery of pharmaceutical compositions. Sustained-release preparations may include semipermeable polymer matrices in the form of shaped articles, *e.g.,* films, or microcapsules. Sustained release matrices may include polyesters, hydrogels, polylactides (as disclosed in U.S. Patent No. 3,773,919 and European Patent Application Publication No. EP 058481), copolymers of L-glutamic acid and gamma ethyl-L-glutamate (Sidman et al., 1983, Biopolymers 2:547-556), poly (2-hydroxyethyl-inethacrylate) (Langer et al., 1981, J. Biomed. Mater. Res. 15:167-277 and Langer, 1982, Chem. Tech. 12:98-105), ethylene vinyl acetate (Langer *et al*., 1981, *supra*) or poly-D(-)-3-hydroxybutyric acid (European Patent Application Publication No. EP 133,988). Sustained release compositions may also include liposomes that can be prepared by any of several methods known in the art. *See, e.g.,* Eppstein et al., 1985, Proc. Natl. Acad. Sci. U.S.A. 82:3688-3692; European Patent Application Publication Nos. EP 036,676; EP 088,046 and EP 143,949.

Pharmaceutical compositions used for *in vivo* administration are typically provided as sterile preparations. Sterilization can be accomplished by filtration through sterile filtration membranes. When the composition is lyophilized, sterilization using this method may be conducted either prior to or following lyophilization and reconstitution. Compositions for parenteral administration can be stored in lyophilized form or in a solution. Parenteral compositions generally are placed into a container having a sterile access port, for example, an intravenous solution bag or vial having a stopper pierceable by a hypodermic injection needle.

In certain formulations, an antigen binding protein has a concentration of at least 10 mg/ml, 20 mg/ml, 30 mg/ml, 40 mg/ml, 50 mg/ml, 60 mg/ml, 70 mg/ml, 80 mg/ml, 90 mg/ml, 100 mg/ml or 150 mg/ml. In one embodiment, a pharmaceutical composition comprises the antigen binding protein, a buffer and polysorbate. In other embodiments, the pharmaceutical composition comprises an antigen binding protein, a buffer, sucrose and polysorbate. An example of a pharmaceutical composition is one containing 50-100 mg/ml of antigen binding protein, 5-20 mM sodium acetate, 5-10% w/v sucrose, and 0.002-0.008% w/v polysorbate. Certain, compositions, for instance, contain 65-75 mg/ml of an antigen binding protein in 9-11 mM sodium acetate buffer, 8-10% w/v sucrose, and 0.005-0.006% w/v polysorbate. The pH of certain such formulations is in the range of 4.5-6. Other formulations have a pH of 5.0-5.5 (e.g., pH of 5.0, 5.2 or 5.4).

Once the pharmaceutical composition has been formulated, it may be stored in sterile vials as a solution, suspension, gel, emulsion, solid, crystal, or as a dehydrated or lyophilized powder. Such formulations may be stored either in a ready-to-use form or in a form (e.g., lyophilized) that is reconstituted prior to administration. Kits for producing a single-dose administration unit are also provided. Certain kits contain a first container having a dried protein and a second container having an aqueous formulation Kits containing single and multi-chambered pre-filled syringes (e.g., liquid syringes and lyosyringes) are provided. The therapeutically effective amount of a LCAT antigen binding protein-containing pharmaceutical composition to be employed will depend, for example, upon the therapeutic context and objectives. One skilled in the art will appreciate that the appropriate dosage levels for treatment will vary depending, in part, upon the molecule delivered, the indication for which the LCAT antigen binding protein is being used, the route of administration, and the size (body weight, body surface or organ size) and/or condition (the age and general health) of the patient. In certain embodiments, the clinician may titer the dosage and modify the route of administration to obtain the optimal therapeutic effect.

Dosing frequency will depend upon the pharmacokinetic parameters of the particular LCAT antigen binding protein in the formulation used. Typically, a clinician administers the composition until a dosage is reached that achieves the desired effect. The composition may therefore be administered as a single dose, or as two or more doses (which may or may not contain the same amount of the desired molecule) over time, or as a continuous infusion *via* an implantation device or catheter. Appropriate dosages may be ascertained through use of appropriate dose-response data. In certain embodiments, the antigen binding proteins can be administered to patients throughout an extended time period. In certain embodiments, the antigen binding protein is dosed every two weeks, every month, every two months, every three months, every four months, every five months, or every six months.

The route of administration of the pharmaceutical composition is in accord with known methods, *e.g.*, orally, through injection by intravenous, intraperitoneal, intracerebral (intra-parenchymal), intracerebroventricular, intramuscular, intra-ocular, intraarterial, intraportal, or intralesional routes; by sustained release systems or by implantation devices. In certain embodiments, the compositions may be administered by bolus injection or continuously by infusion, or by implantation device.

The composition also may be administered locally *via* implantation of a membrane, sponge or another appropriate material onto which the desired molecule has been absorbed or encapsulated. In certain embodiments, where an implantation device is used, the device may be implanted into any suitable tissue or organ, and delivery of the desired molecule may be *via* diffusion, timed-release bolus, or continuous administration.

It also may be desirable to use LCAT antigen binding protein pharmaceutical compositions according to the disclosed *ex vivo.* In such instances, cells, tissues or organs that have been removed from the patient are exposed to LCAT antigen binding protein pharmaceutical compositions after which the cells, tissues and/or organs are subsequently implanted back into the patient.

A physician will be able to select an appropriate treatment indication and target lipid levels depending on the individual profile of a particular patient. One well-accepted standard for guiding treatment of hyperlipidemia is the Third Report of the National Cholesterol Education Program (NCEP) Expert Panel on Detection, Evaluation, and Treatment of the High Blood Cholesterol in Adults (Adult Treatment Panel III) Final Report, National Institutes of Health, NIH Publication No. 02-5215 (2002).

The efficacy of a particular dose can be assessed by reference to biomarkers or improvement in certain physiological parameters. Examples of suitable biomarkers include, the ratio of free cholesterol to plasma lipid, free cholesterol to membrane protein, phospatidylcholine to sphingomyelin, or HDL-C levels.

### Combination Therapy

Also provided herein are compositions comprising an LCAT antigen binding protein and one or more additional therapeutic agents, as well as methods in which such agents are administered concurrently or sequentially with an LCAT antigen binding protein for use in the preventive and therapeutic methods disclosed herein. The one or more additional agents can be co-formulated with an LCAT antigen binding protein or can be co-administered with an LCAT antigen binding protein. In general, the therapeutic methods, compositions and compounds may also be employed in combination with other therapeutics in the treatment of various disease states, with the additional agents being administered concurrently. Examples of additional agents that can be so administered include, but are not limited to, other drugs that treat cardiovascular disease, cholesterol-related disorders and atherosclerosis, anti-inflammatory drugs, anti-thrombosis drugs, anti-diabetic drugs and cytokines.

### Agents for Treating Cardiovascular Disease, Cholesterol-Related Disorders and Atherosclerosis

Additional active agents may act in complementary or synergistic ways with the LCAT antigen binding protein when used to treat, and prevent atherosclerosis or manage cholesterol, or related disorders such as cardiovascular disease. The interchangeable terms "cardiovascular agent" or "cardiovascular drug" herein refer to a drug or agent that is capable of treating, preventing, or reducing the risk of developing a cardiovascular disease or a cholesterol-related disorder (e.g., atherosclerosis) or a risk factor or symptom thereof. Such cardiovascular agents include, but are not limited to, cholesterol and triglyceride modulating agents, agents that treat coronary artery disease, agents that treat hypertension or pulmonary arterial hypertension, agents that treat arterial fibrillation or arrhythmia, agents that treat stroke, agents that treat myocardial ischemia and/or agents that treat thrombosis. Specific examples, include: statins, fibrates, Acyl-coenzyme A: cholesterol acyltransferase (ACAT) inhibitors, alpha-adrenergic blocking drugs (alpha-blockers), alpha/beta blockers, angiotensin-converting enzyme (ACE) inhibitors, aldosterone antagonists, angiotensin II receptor antagonists, anti-arrhythmics, anticoagulants, antiplatelet agents, apolipoprotein A-I (apoA-1) mimetics, beta-blockers, bile acid sequestrants, calcium-channel blockers, ApoB cholesteryl ester transfer protein (CETP) inhibitors, cholesterol absorption inhibitors, diuretics, dyslipidemia agents, endothelin receptor antagonists, fibrates, 3-hydroxy-3-methyl-glutaryl-coenzyme A (HMG-CoA) reductase inhibitors, LCAT activators, LDL receptor inducers, lipase inhibitors, lipoprotein- associated phospholipase A2 (Lp-PLA2) inhibitors, microsomal triglyceride transfer protein (MTP) inhibitors, platelet aggregation inhibitors, PPAR agonists and activators including PPARγ; agonists, PPARα agonists and PPAR dual α/γ agonists, PCSK9 antisense or RNAi, squalene epoxidase inhibitors, squalene synthetase inhibitors, thrombolytics, and thyroid receptor beta activators.

One class of agents that can be used are PCSK9 neutralizing antibodies. Examples of such antibodies are disclosed, for instance, in WO 2008/057457, WO 2008/057458, WO 2008/057459, WO 2008/063382, WO 2008/133647, WO 2009/100297, WO 2009/100318, WO 2011/037791, WO 2011/053759, WO 2011/053783, WO 2008/125623, WO 2011/072263, WO 2009/055783, WO 2010/029513, WO 2011/111007, WO 2010/077854. One specific example is AMG 145 developed by Amgen.

Another class of agents that can be used are anti- endothelial lipase antibodies.

### Statins (HMG-CoA Reductase Inhibitors)

In one embodiment, LCAT antigen binding proteins can be used with statins. Statins are drugs that competitively inhibit 3-hydroxy-3-methylglutaryl coenzyme A reductase "HMG-CoA reductase," which is the enzyme that catalyzes an early, rate-limiting step in cholesterol biosynthesis. Hebert et al., JAMA 1997, 278: 313-21. This combination, in addition to raising HDL levels and lowering LDL levels may also lower triglyceride levels and reduce inflammation. The combination can lower blood pressure; protect against heart disease, for example, by reducing smooth muscle proliferation, reduce heart attacks, reduce platelet aggregation, and to reduce strokes, as well as peripheral arterial disease (clogging of the arteries to the legs).

Examples of suitable statins that can be used with an LCAT antigen binding protein include, but are not limited to, mevastatin, pitavastatin, rosuvastatin, pentostatin (Nipent®), nystatin, lovastatin (Mevacor®), simvastatin (Zocor®), pravastatin (Pravachol®), fluvastatin (Lescol®), atorvastatin (Lipitor®), cerivastatin (Baycol®), or combinations thereof. Statins suitable for use in the compositions and methods described herein are disclosed in U.S. Pat. Nos. 4,681,893; 5,273,995; 5,356,896; 5,354,772; 5,686,104; 5,969,156; and 6,126,971. As some statins may exist in an inactive form, such as a lactone (e.g., simvastatin), the methods encompasses using the active form (e.g., b-hydroxy acid form) of them. See Physicians Desk Reference, 54th Ed. (2000) pp. 1917-1920.

### CETP Inhibitors

Combination compositions and methods may also include agents that inhibit cholesteryl ester transfer protein ("CETP"). CETP is involved in the transfer of cholesteryl esters and triglycerides. Inhhibitors of CETP are thus useful in preventing or treating atherosclerosis and other cardiovascular diseases. Non- limiting examples of CETP inhibitors include, for example, Torcetrapib, and S-(2-[([1-(2-ethylbutyl)cyclohexyl]carbonyl)amino]phenyl)-2-methylpropane- thioate and anacetrapib and combinations thereof.

### Cardiovascular Drugs

Cardiovascular drugs for use in combination with an LCAT antigen binding protein include peripheral antiadrenergic drugs, centrally acting antihypertensive drugs (e.g., methyldopa, methyldopa HCl), antihypertensive direct vasodilators (e.g., diazoxide, hydralazine HCl), drugs affecting renin-angiotensin system, peripheral vasodilators, phentolamine, antianginal drugs, cardiac glycosides, inodilators (e.g., aminone, milrinone, enoximone, fenoximone, imazodan, sulmazole), antidysrhythmic drugs, calcium entry blockers, ranitine, bosentan, and rezulin.

### Cholesterol Lowering Drugs

Some of the common types of cholesterol-lowering drugs that can be used in combination with an LCAT antigen binding protein include statins, resins and nicotinic acid (niacin), gemfibrozil and clofibrate. Thus, combination therapy is contemplated utilizing, for example, clofibrate (Atromid-S, which raises the HDL cholesterol levels and lowers triglyceride levels), gemfibrozil (Lopid, which raises HDL cholesterol levels) and nicotinic acid (which works in the liver by affecting the production of blood fats and is used to lower triglycerides and LDL cholesterol, and raise HDL ("good") cholesterol), resins (which are also called bile acid-binding drugs and work in the intestines by promoting increased disposal of cholesterol), including cholestyramine (Questran, Prevalite, Lo-Cholest), colestipol (Colestid) and colesevelam (WelChol).

### ACE Inhibitors

An LCAT antigen binding protein can also be used in combination with an ACE inhibitor. Angiotensin II causes blood vessels to contract and thereby narrows the blood vessels. The narrowing of the vessels increases the pressure within the vessels and can cause high blood pressure (hypertension). Angiotensin II is formed from angiotensin I in the blood by the enzyme, angiotensin converting enzyme (ACE). ACE inhibitors decrease the production of angiotensin II. As a result, the blood vessels enlarge or dilate, and the blood pressure is reduced. ACE inhibitors that are available for use with an LCAT antigen binding protein include captopril (Capoten), benazepril (Lotensin), enalapril (Vasotec), enalapril, enaliprilat, lisinopril (Prinivil, Zestril) fosinopril (Monopril), ramipril (Altace), perindopril (Aceon), quinapril (Accupril), moexipril (Univasc), perindopril, and trandolapril (Mavik), or combinations thereof.

### ACAT Inhibitors

Acyl-CoA cholesteryl acyl transferase ("ACAT") is an acyltransferase enzyme that catalyzes the intracellular formation of cholesterol esters from cholesterol during bile acid biosynthesis. ACAT promotes accumulation of cholesterol esters in vascular tissues. Accordingly, LCAT antigen binding proteins can be used with agents that inhibit ACAT, can be sued to prevent and treat atherosclerosis. Examples of suitable ACAT inhibitors include, but are not limited to, CI-1011 (Avasimibe, Pfizer), CS-505 (Pactimibe sulfate, Sankyo Pharma), or combinations thereof.

### Aldosterone Antagonists

Aldosterone is a steroidal hormone that contributes to hypertension by inhibiting kidney function. Agents that compete with aldosterone for mineralo-corticoid receptors are therefore useful in preventing or treating hypertension. Suitable aldosterone agents for use in combination with an LCAT antigen binding protein include eplerenone and aldactone, or combinations thereof.

### Alpha Blockers

Adrenergic alpha-antagonists (alpha-blockers), compete with adrenaline binding at α-adrenoreceptors. Binding of adrenaline at such receptors results in vasoconstriction and therefore hypertension. Agents that compete with adrenaline or block α-adrenoreceptors are therefore useful in preventing or treating hypertension. Examples of alpha blockers for use with an LCAT antigen binding protein include, but are not limited to, doxazosin, methyldopa, clonidine, prazosin, terazosin, or combinations thereof.

### Angiotensin II Receptor Antagonists

These compounds are also known as angiotensin receptor blockers, or simply ARBs, ATi-receptor antagonists, or sartans. They are useful in treating hypertension, congestive heart failure, and various other diseases and disorders. Suitable examples of angiotensin II receptor antagonists for use with an LCAT antigen binding protein include, but are not limited to, candesartan, irbesartan, olmesartan, losartan, valsartan, telmisartan, eprosartan, or combinations thereof.

### Anti-arrhythmic Drugs

Drugs of this class function by correcting an irregular heartbeat and/or to slow a heart that is beating too rapidly. Drugs that can be combined with an LCAT antigen-binding protein include, but are not limited to, adenosine, amiodarone, digoxin, disopyramide, flecainide, lidocaine, mexiletine, procainamide, quinidine gluconate, propafenone hydrochloride, tocainide, or combinations thereof

### ApoA-1 Mimetics

Apolipoprotein A-I ("apoA-1") is the primary protein component of serum HDL cholesterol. Suitable examples of apoA-1 mimetics for use with an LCAT antigen binding protein include, but are not limited to, ETC-216, ETC-588- liposome, ETC-642, trimeric apoA-1, CSL-111, APPO 18, reverse D-4F, or combinations thereof.

### Beta Blockers

Beta blockers block responses to the beta nerve receptor, thereby slowing slow heart rate and lowering blood pressure. Non-limiting examples of suitable beta blockers include acebutolol, atenolol, metoprolol, nadolol, nebivolol, pindolol, propranolol, or combinations thereof.

### Bile Acid Sequestrants

This class of compounds interrupts the enterohepatic circulation of bile acids by binding bile acid components in the gastrointestinal tract such that they cannot be absorbed. This property makes bile acid sequestrants useful in preventing or treating hyperlipidemia and related disorders. Examples of sequestrants that can be used with an LCAT antigen binding protein include, but are not limited to, colesevelam HcI, colestipol, locholest and cholestyramine or combinations thereof.

### Calcium-Channel Blockers

These molecules cause vasodilation and are useful in preventing or treating hypertension. Examples of suitable calcium channel blockers for use with the antigen binding proteins provided herein include, but are not limited to, nicardipine, diltiazem, clevidipine butyrate, isradipine, nimodipine, nisoldipine, verapamil, amlodipine besylate, amlodipine, olmesartan, valsartan, or combinations thereof.

### Dyslipidemia Agents

Dyslipidemia is a class of diseases that characterized by elevated cholesterol levels. Examples of dyslipidemia agents that can be utilized in combination with an LCAT antigen binding protein, include, but are not limited to, Angptl4 antibody, APA-01 (Phosphagenics), CRD-5 (ImaSight), NCX6560 (McOx), PCSK9 RNAi (Alnylam), recombinant apoA-1 (SemBioSys Genetics), anti-oxLDL (Genentech), APL1 80 (Novartis), APPO 18 (D4F) (Novartis), CER-002 (Cerenis Therapeutics), CP-800,569 (Pfizer), GSK- 256073 (GlaxoSmithKline), MB07811 (Metabasis), PF-3, 185,043 (Pfizer), R7232 (Roche), rilapladib (GlaxoSmithKline), RVX-208 (Resverlogix), Sobetirome (QRX-431 (QuatRx)), anacetrapib (Merk), CSL1 I1 (CSL Limited), darapladib (GlaxoSmithKline), eprotirome (Kara Bio), GFT505 (Genfit), MAHDLO1 (Marzal Plant Pharma), MBX-8025 (Metabolex), PLX204 (Wyeth/Plexxikon), aleglitezar (Roche), dalcetrapib (Roche), SLx4090 (Surface Logix), verespladib (Anthera Pharmaceuticals), AEGR-733 (Aegerion), ABT-335 (Abbott Laboratories), AVE5530 (Sanofi-Aventis), LCP-AtorFen (LifeCycle Pharma), TRIA-662 (Cortria), fenofibrate, choline fenofibrate, ezetimibe, colsevelam, laropiprant, or combinations of any of the foregoing.

### Endothelin Receptor Antagonists

When endothelin- 1 binds to endothelin-A (ETA) or endothelin-B (ETB) receptors, the binding causes pulmonary vasoconstriction. Antagonists are thus useful in attenuating pulmonary vasoconstriction and, as such, are useful in treating pulmonary hypertension. Examples of endothelin receptor antagonists that can be utilized in combination with an LCAT antigen binding protein include, but are not limited to, ambrisentan, bosentan, volibris, thelin, or combinations thereof.

### LCAT Activators

The LCAT antigen binding proteins disclosed herein can be used in combination with another LCAT activator. Examples of suitable activators include native and modified forms of LCAT as described, for example, in U.S. Patent No. 6,635,614 and WO 09/015314. Small molecule activators are described in WO 08/002591.

### Lp-PLA2 Inhibitors

Lp-PLA2 hydrolyzes oxidized phospholipids in LDL cholesterols. High levels of Lp-PLA2 seem to trigger a cascade of inflammatory events in atherosclerosis and an increased risk of stroke. Lp-PLA2 inhibitors, therefore, are useful in slowing or preventing development of atherosclerosis. Examples of suitable Lp-PLA2 inhibitors include, but are not limited to, rilapladib, darapladib, and combinations thereof.

### Fibrates and PPAR Agonists

Fibrates or fibric acid derivatives are regarded as broad-spectrum lipid-modulating agents in that although their main action is to decrease serum triglycerides they also tend to reduce LDL-cholesterol and to raise HDL-cholesterol. The combined use of an LCAT antigen binding protein and a fibrate can reduce the risk of coronary heart disease events in those with low HDL-cholesterol or with raised triglycerides by speeding up the chemical breakdown (i.e., catabolism) of triglyceride-rich lipoproteins that circulate in the body.

Fibrates include, but are not limited to, bezafibrate, ciprofibrate, fenofibrate, gemfibrozil, clofibrate, CER-002, rosiglitazone, GW501516, RWJ 800025, KD-3010, and combinations thereof. Fibrates suitable for inclusion in the compositions or administration in the methods of the invention are disclosed in U.S. Pat. Nos. 4,895,762; 6,074,670; and 6,277,405.

### Squalene Epoxidase Inhibitors

These inhibitors, also called squalene monooxygenase inhibitors, inhibit the oxidation of squalene in the cholesterol biosynthesis pathway and are useful in preventing or slowing the cholesterol production. Examples of squalene epoxidase inhibitors that can be used as part of a co-therapy include, but are not limited to, terbinafme, naftifine, amorolfme, butenafine, FR194738, NB-598, resveratrol (trans-3,4',5-trihydroxystilbene), epigallocatechin-3-O-gallate, S-allylcysteine, selenocysteine, alliin, diallyl trisulfide, diallyl disulfide, and combinations thereof.

### Anti-Inflammatory Drugs

In prevention and treatment of inflammation, LCAT antigen binding proteins can be used in combination with, for example, acetylsalicylic acid (Aspirin, Ecotrin), choline magnesium salicylate (Trilisate), diclofenac (Voltaren, Cataflam, Voltaren-XR), diflunisal (Dolobid), etodolac (Lodine), fenoprofen (Nalfon), flurbiprofen (Ansaid), ibuprofen (Advil, Motrin, Medipren, Nuprin), indomethacin (Indocin, Indocin-SR), ketoprofen (Orudis, Oruvail), meclofenamate (Meclomen), nabumetone (Relafen), naproxen (Naprosyn, Naprelan, Anaprox, Aleve), oxaprozin (Daypro), phenylbutazone (Butazolidine), piroxicam (Feldene), salsalate (Disalcid, Salflex), tolmetin (Tolectin), valdecoxib (Bextra), and COX-2 selective non-steroidal anti-inflammatory drugs (NSAIDs) including Bextra, Celebrex, Naproxen, and Vioxx. Prescription-only NSAIDs include ibuprofen (Brufen), aceclofenac (Preservex), acemetacin (Emflex), azapropazone (Rheumox), celecoxib (Celebrex), dexketoprofen (Keral), diclofenac (Voltarol, Diclomax, Arthrotec), diflusinal (Dolobid), etodolac (Lodine), fenbufen (Lederfen), fenoprofen (Fenopron), flurbiprofen (Froben), indometacin, ketoprofen (Orudis, Oruvail), mefenamic acid, meloxicam (Mobic), nabumetone (Relifex), naproxen (Naprosyn, Synflex), phenylbutazone (Butacote), piroxicam (Feldene), sulindac (Clinoril), tenoxicam (Mobiflex) and tiaprofenic acid (Surgam).

### Anti-Thrombosis Drugs

In methods for prevention and treatment of thrombosis-related conditions, an LCAT antigen binding protein can be used in combination with anti-thrombosis drugs such as anticoagulant drugs, which inhibit the ability of blood to clot, or coagulate and include dalteparin (Fragmin), danaparoid (Orgaran), enoxaparin (Lovenox), heparin (various), tinzaparin (Innohep), warfarin (Coumadin), and lepirudin (Refludan), and antiplatelet drugs such as aspirin, ticlopidine (Ticlid), clopidogrel (Plavix), tirofiban (Aggrastat) and eptifibatide (Integrilin). Still other methods include the use of bivalirudin (selective and reversible thrombin inhibitor), argatroban (reversible inhibitor of thrombin), and low molecular weight heparins (LMWHs), including enoxaparin (Lovenox), dalteparin (Fragmin), ardeparin (Normiflo) fondaparinux and idraparinux. Still other anti-thrombosis drugs contemplated for use with the LCAT antigen binding proteins include fragmin (dalteparin sodium injection) lovenox (enoxaparin sodium), Normiflo (ardeparin sodium), Orgaran (danaparoid sodium), indirect (Antithrombin-Dependent) FXa inhibitors such as fondaparinux (Arixtra®) and idraparinux, direct (Antithrombin-Independent) FXa inhibitors such as BAY 59-7939 [Bayer], DPC-423 [Bristol-Myers Squibb], DX-9065a [Daiichi], LY517717, razaxaban (DPC906), lepirudin (Refludan.RTM.), desirudin (Revasc®), bivalirudin (Hirulog®, Angiomax®), argatroban (Novastan®), melagatran, and ximelagatran (Exanta®).

### Anti-Diabetic Drugs

Combination therapy using anti-diabetic drugs that lower blood glucose levels are also provided. Except for insulin, exenatide and pramlintide, antidiabetics are administered orally and are thus also called oral hypoglycemic agents or oral antihyperglycemic agents. Antidiabetic drugs divided into the following groups: insulin, sulfonylureas, alpha-glucosidase inhibitors, biguanides, meglitinides, glitazones, thiazolidinediones and incretin peptides such as GLP-1 and exendin and analogs thereof. Drugs from any of these classes can be combined with an LCAT antigen binding protein for use in therapy.

Insulin (Humulin, Novolin) controls blood glucose levels. Forms include isophane insulin suspension, insulin zinc suspension, and other formulations that extend the duration of insulin action. Inhaled forms of insulin can also be utilized.

Sulfonylureas increase insulin release from the beta cells of the pancreas, and include chlorpropamide [Diabinese], tolazamide [Tolinase], glipizide [Glucotrol], glimepiride (Amaryl), tolbutamide (Orinase), acetohexamide (Dymelor), glyburide (Diabeta, Micronase, Glynase), glisoxepid, glyburide, acetohexamide, glibomuride, gliquidone, glyhexamide, phenbutamide, tolcyclamide, and gliclazide (Diamicron).

Alpha-glucosidase inhibitors inhibit the conversion of disaccharides and complex carbohydrates to glucose, and are used in combination therapy with sulfonylureas or other hypoglycemic agents. This type of anti-diabetic agent includes acarbose [Precose] and miglitol [Glyset].

The biguanide class of compounds mentioned above decreases hepatic glucose production, decreases intestinal absorption of glucose and increases peripheral glucose uptake and use. Biguanides for use in the compositions and methods provided herein include, but are not limited to, metformin, phenformin, buformin, or combinations thereof. Suitable biguanides suitable are also disclosed in U.S. Pat. No. 6,303,146. The combined use of a bigaunide and an antigen binding protein may improve glycemic control by enhancing insulin sensitivity in the liver and in muscle. The combination may reduce or avoid cardiovascular risk factors such as dyslipidemia, elevated plasminogen activator inhibitor I levels, other fibrinolytic abnormalities, hyperinsulinemia, insulin resistance, and is an effective and safe therapeutic agent for the treatment of type 2 diabetes.

The meglitinide class of compounds stimulates insulin production and may be used in combination with metformin. This class includes repaglinide (Prandin) and nateglitinide (Starlix). Thiazolidinedione agents reduce glucose production in the liver and increase insulin-dependent glucose uptake in muscle cells. These agents may be used in combination with metformin or a sulfonylurea, and include rosiglitazone (Avandia) and pioglitazone (Actos).

Glitazones, which may increase glucose uptake in muscle and reduce endogenous glucose production. Glitazones include 5-((4-(2-(methyl-2-pyridinyl amino)ethoxy)-phenyl)methyl)-2,4-thiazolidinedione, troglitazone, pioglitazone, ciglitazone, WAY-120,744, englitazone, AD 5075, darglitazone, rosiglitazone, combinations thereof, or a pharmaceutically acceptable salt, solvate, clathrate, polymorph, prodrug, or pharmacologically active metabolite thereof. Glitazones suitable for use in the compositions or methods described herein are disclosed in U.S. Pat. Nos. 4,687,777; 5,002,953; 5,741,803; 5,965,584; 6,150,383; 6,150,384; 6,166,042; 6,166,043; 6,172,090; 6,211,205; 6,271,243; 6,288,095; 6,303,640; and 6,329,404.

Anti-diabetic peptide analogs include incretins which are insulin secretagogues, including glucagon-like peptide-1 (GLP-1) and gastric inhibitory peptide (also known as glucose-dependent insulinotropic peptide or GIP). Both GLP-1 and GIP are inactivated by the dipeptidyl peptidase-4 (DPP-4). Other peptides include Exenatide (also Exendin-4, sold as Byetta®) which is a GLP agonist. and is more resistant to degradation by DPP-4; dipeptidyl peptidase-4 (DPP-4) inhibitors which maintain blood concentration of GLP-1 by inhibiting its degradation by dipeptidyl peptidase-4 (DPP-4), this class of peptides including vildagliptin and sitagliptin, and amylin agonist analogues which slow gastric emptying and suppress glucagons, this type including pramlintide.

### Cytokines

Exemplary cytokines or hematopoietic factors for co-administration include IL-1 alpha, IL-1 beta, IL-2, IL-3, IL-4, IL-5, IL-6, IL-11, colony stimulating factor-1 (CSF-1), M-CSF, SCF, GM-CSF, granulocyte colony stimulating factor (G-CSF), EPO, interferon-alpha (IFN-alpha), consensus interferon, IFN-beta, IFN-gamma, IFN-omega, IL-7, IL-8, IL-9, IL-10, IL-12, IL-13, IL-14, IL-15, IL-16, IL-17, IL-18, IL-19, IL-20, IL-21, IL-22, IL-23, IL-24, IL-31, IL-32 alpha, IL-33, thrombopoietin (TPO), angiopoietins, for example Ang-1, Ang-2, Ang-4, Ang-Y, the human angiopoietin-like polypeptides ANGPTL1 through 7, vitronectin, vascular endothelial growth factor (VEGF), angiogenin, activin A, activin B, activin C, bone morphogenic protein-1, bone morphogenic protein-2, bone morphogenic protein-3, bone morphogenic protein-4, bone morphogenic protein-5, bone morphogenic protein-6, bone morphogenic protein-7, bone morphogenic protein-S, bone morphogenic protein-9, bone morphogenic protein-10, bone morphogenic protein-11, bone morphogenic protein-12, bone morphogenic protein-13, bone morphogenic protein-14, bone morphogenic protein-15, bone morphogenic protein receptor IA, bone morphogenic protein receptor IB, bone morphogenic protein receptor II, brain derived neurotrophic factor, cardiotrophin-1, ciliary neutrophic factor, ciliary neutrophic factor receptor, cripto, cryptic, cytokine-induced neutrophil chemotactic factor 1, cytokine-induced neutrophil, chemotactic factor 2a, cytokine-induced neutrophil chemotactic factor 2.beta., .beta. endothelial cell growth factor, endothelin 1, epidermal growth factor, epigen, epiregulin, epithelial-derived neutrophil attractant, fibroblast growth factor 4, fibroblast growth factor 5, fibroblast growth factor 6, fibroblast growth factor 7, fibroblast growth factor 8, fibroblast growth factor 8b, fibroblast growth factor 8c, fibroblast growth factor 9, fibroblast growth factor 10, fibroblast growth factor 11, fibroblast growth factor 12, fibroblast growth factor 13, fibroblast growth factor 16, fibroblast growth factor 17, fibroblast growth factor 19, fibroblast growth factor 20, fibroblast growth factor 21, fibroblast growth factor acidic, fibroblast growth factor basic, glial cell line-derived neutrophic factor receptor .alpha.1, glial cell line-derived neutrophic factor receptor .alpha.2, growth related protein, growth related protein .alpha., growth related protein .beta., growth related protein .gamma., heparin binding epidermal growth factor, hepatocyte growth factor, hepatocyte growth factor receptor, hepatoma-derived growth factor, insulin-like growth factor I, insulin-like growth factor receptor, insulin-like growth factor II, insulin-like growth factor binding protein, keratinocyte growth factor, leukemia inhibitory factor, leukemia inhibitory factor receptor .alpha., nerve growth factor nerve growth factor receptor, neuropoietin, neurotrophin-3, neurotrophin-4, oncostatin M (OSM), placenta growth factor, placenta growth factor 2, platelet-derived endothelial cell growth factor, platelet derived growth factor, platelet derived growth factor A chain, platelet derived growth factor AA, platelet derived growth factor AB, platelet derived growth factor B chain, platelet derived growth factor BB, platelet derived growth factor receptor .alpha., platelet derived growth factor receptor .beta., pre-B cell growth stimulating factor, stem cell factor (SCF), stem cell factor receptor, TNF, including TNF0, TNF1, TNF2, transforming growth factor .alpha., transforming growth factor .beta., transforming growth factor .beta.1, transforming growth factor .beta.1.2, transforming growth factor .beta.2, transforming growth factor .beta.3, transforming growth factor .beta.5, latent transforming growth factor .beta.1, transforming growth factor .beta. binding protein I, transforming growth factor .beta. binding protein II, transforming growth factor .beta.3 binding protein III, thymic stromal lymphopoietin (TSLP), tumor necrosis factor receptor type I, tumor necrosis factor receptor type II, urokinase-type plasminogen activator receptor, vascular endothelial growth factor, and chimeric proteins and biologically or immunologically active fragments thereof.

### Use of LCAT Antigen Binding Proteins for Diagnosis

The LCAT antigen binding proteins that are provided herein are useful for detecting LCAT in biological samples. For instance, the LCAT antigen binding proteins can be used in diagnostic assays, *e.g*., binding assays to detect and/or quantify LCAT expressed in serum.

The antigen binding proteins of the described can be used for diagnostic purposes to detect, diagnose, or monitor diseases and/or conditions associated with LCAT. The disclosed antigen binding proteins provide a means for the detection of the presence of LCAT in a sample using classical immunohistological methods known to those of skill in the art (e.g., Tijssen, 1993, Practice and Theory of Enzyme Immunoassays, Vol 15 (Eds R.H. Burdon and P.H. van Knippenberg, Elsevier, Amsterdam); Zola, 1987, Monoclonal Antibodies: A Manual of Techniques, pp. 147-158 (CRC Press, Inc.); Jalkanen et al., 1985, J. Cell. Biol. 101:976-985; Jalkanen et al., 1987, J. Cell Biol. 105:3087-3096). The detection of LCAT can be performed *in vivo* or *in vitro.*

Diagnostic applications disclosed herein include use of the antigen binding proteins to detect expression of LCAT. Examples of methods useful in the detection of the presence of LCAT include immunoassays, such as the enzyme linked immunosorbent assay (ELISA) and the radioimmunoassay (RIA).

For diagnostic applications, the antigen binding protein typically will be labeled with a detectable labeling group. Suitable labeling groups include, but are not limited to, the following: radioisotopes or radionuclides (*e*.*g*., ³H, ¹⁴C, ¹⁵N, ³⁵S, ⁹⁰Y, ⁹⁹Tc, ¹¹¹In, ¹²⁵I, ¹³¹I), fluorescent groups (*e.g.,* FITC, rhodamine, lanthanide phosphors), enzymatic groups (*e.g.,* horseradish peroxidase, β-galactosidase, luciferase, alkaline phosphatase), chemiluminescent groups, biotinyl groups, or predetermined polypeptide epitopes recognized by a secondary reporter (*e.g*., leucine zipper pair sequences, binding sites for secondary antibodies, metal binding domains, epitope tags). The labeling group is coupled to the antigen binding protein *via* spacer arms of various lengths to reduce potential steric hindrance. Various methods for labeling proteins are known in the art and may be used.

The LCAT antigen binding protein is isolated and measured using techniques known in the art. *See,* for example, Harlow and Lane, 1988, Antibodies: A Laboratory Manual, New York: Cold Spring Harbor (ed. 1991 and periodic supplements); John E. Coligan, ed., 1993, Current Protocols In Immunology New York: John Wiley & Sons.

Another aspect of the disclosure provides for detecting the presence of a test molecule that competes for binding to LCAT with the antigen binding proteins provided. An example of one such assay would involve detecting the amount of free antigen binding protein in a solution containing an amount of LCAT in the presence or absence of the test molecule. An increase in the amount of free antigen binding protein *(i.e.,* the antigen binding protein not bound to LCAT) would indicate that the test molecule is capable of competing for LCAT binding with the antigen binding protein. In one dislcosure, the antigen binding protein is labeled with a labeling group. Alternatively, the test molecule is labeled and the amount of free test molecule is monitored in the presence and absence of an antigen binding protein.

### EXAMPLES

The following examples, including the experiments conducted and the results achieved, are provided for illustrative purposes only.

### EXAMPLE 1

### Assays

### 1. LCAT Enzyme Activity Assays

Two assay methods for determining LCAT activity were developed. The first method used human apoA-I liposomes as the substrates for the LCAT reaction. ApoA-I proteoliposomes were prepared by standard cholate-dialysis procedure (Chen and Albert., J Lipid Res 1982; 23: 680). Initial proteoliposome mixtures contained egg phosphatidylcholine (PC)/³H-unesterfied cholesterol/human apoA-I in a molar ratio of 250:12.5:0.8. After dialysis, the proteoliposomes were incorporated with an aliquot of LCAT samples (e.g., from purified material, plasma, compounds under test, etc.) at 37°C for 60 minutes and LCAT activity was determined by measuring the conversion of radiolabeled free cholesterol (FC) to esterified cholesterol (CE) using either scintillation proximity assay (SPA) format or the thin-layer chromagraphy (TLC) formats. This method is an *in vitro* assay protocol to measure LCAT enzyme activity of the samples/compounds under standardized substrate condition. The SPA format was performed to identify non-inhibitors for LCAT.

The second assay for LCAT activity measured the cholesterol esterification rate (CER) of plasma from test animals or subjects, and is thus referred to as an *"ex vivo"* assay format. CER reflects the LCAT activity of both the endogenous plasma enzyme and/or the spiked exogenous rLCAT protein on native plasma lipoprotein substrates. This assay also reflects the bioavailability of substrate molecules and apoA-I activity of the plasma samples. Aliquots of pooled plasma samples from healthy subjects were equilibrated with a trace amount of radiolabeled FC at 4°C for 4 hours and the effect of added samples or test compounds on CER was quantified by TLC analysis after incubation at 37°C for 60 minutes. This plasma CER method was used for lead confirmation and characterization.

### 2. Methods for Analyzing Plasma Lipoproteins

Plasma lipids and lipoprotein were measured by using clinical analyzer (Cobas Integra 400 system) and Cobas HDL-C plus 2^{nd} Generation kit (Roche). Fast performance liquid chromatography (FPLC) was used to measure the lipoprotein profile and changes of HDL particle size in plasma from animals treated with rLCAT. Plasma samples (individual or pooled) were loaded onto a Sepharose 6 PC column connected to an FPLC system (Amersham Biosciences) and eluted with PBS. Fractions of 50 µL volume were collected. Total cholesterol (TC) and triglyceride content in each FPLC fraction were determined using Infinity kits (Thermo DMA) according to manufacturer's protocols.

### 3. Methods of Western Blot Analysis

Western blot analysis of plasma apoA-I, apoB and apoE was performed using goat anti-human antibodies (abcam). Bands were quantified by densitometry using ImageJ (National Institutes of Health).

### EXAMPLE 2

### Preparation of anti-LCAT Monoclonal Antibodies

Immunizations were conducted using one or more suitable forms of LCAT antigen, including soluble recombinant human wild-type LCAT and soluble recombinant human mutant C31Y LCAT (i.e., human LCAT in which C31 of SEQ ID NO:1 was substituted with tyrosine), or combinations thereof.

A suitable amount of immunogen (i.e., ten µg/mouse of soluble LCAT) was used for initial immunization in XenoMouse™ according to the methods disclosed in U.S. Patent Application Serial No. 08/759,620, filed December 3, 1996 and International Patent Application Nos. WO 98/24893, published June 11, 1998 and WO 00/76310, published December 21, 2000. Following the initial immunization, subsequent boost immunizations of immunogen (five µg/mouse of soluble LCAT) were administered on a schedule and for the duration necessary to induce a suitable titer of anti-LCAT antibody in the mice. Titers were determined by various suitable methods, such as enzyme immunoassay.

Animals exhibiting suitable titers were identified, and lymphocytes were obtained from draining lymph nodes and spleen and, if necessary, pooled for each cohort. Lymphocytes were dissociated from lymphoid tissue by grinding in a suitable medium (for example, Dulbecco's Modified Eagle Medium; DMEM; obtainable from Invitrogen, Carlsbad, CA) to release the cells from the tissues, and suspended in DMEM. B cells were selected and/or expanded and fused with suitable fusion partner, for example, nonsecretory myeloma P3X63Ag8.653 cells (American Type Culture Collection CRL 1580; Kearney et al, J. Immunol. 123, 1979, 1548-1550), using techniques that are known in the art.

In one suitable fusion method, lymphocytes are mixed with fusion partner cells at a ratio of 1:4. The cell mixture is gently pelleted by centrifugation at 400 x g for 4 minutes, the supernatant decanted, and the cell mixture gently mixed. Fusion is induced with PEG/DMSO (polyethylene glycol/dimethyl sulfoxide; obtainable from Sigma-Aldrich, St. Louis MO; 1 ml per million of lymphocytes). PEG/DMSO is slowly added with gentle agitation over one minute followed, by one minute of mixing. IDMEM (DMEM without glutamine; 2 ml per million of B cells), is then added over 2 minutes with gentle agitation, followed by additional IDMEM (8 ml per million B-cells) which is added over 3 minutes.

The fused cells are gently pelleted (400 x g 6 minutes) and resuspended in 20 ml Selection media (for example, DMEM containing Azaserine and Hypoxanthine [HA] and other supplemental materials as necessary) per million B-cells. Cells re incubated for 20-30 minutes at 37° C and then resuspended in 200 ml Selection media and cultured for three to four days in T175 flasks prior to 96 well plating.

Cells were distributed into 96-well plates using standard techniques to maximize clonality of the resulting colonies. After several days of culture, supernatants were collected and subjected to screening assays, including confirmation of binding to human LCAT and evaluation of cross-reactivity with other species of LCAT (for example, cynomolgus monkey and/or murine LCAT). Positive cells were further selected and subjected to standard cloning and subcloning techniques. Clonal lines were expanded *in vitro,* and the secreted human antibodies obtained for analysis.

A total of 49760 hybridomas were screened for binding to human wild type LCAT; 1748 hybridomas were identified as capable of binding. Based upon further characterization studies, 3 different antibodies (25B7, 18E5 and 27C3) were identified as capable of binding recombinant wild-type human LCAT, and 3 antibodies (14F11, 25B7 and 27C3) were identified as capable of binding recombinant wild-type cynomolgus (cyno) LCAT. These antibodies were selected for further characterization.

### EXAMPLE 3

### Antibody Binding Affinities to Human and Cynomolgus LCAT

BIAcore was used to determine the affinity binding constants to full-length wild type recombinant cynomolgus LCAT and full-length wild type recombinant human LCAT proteins for the four antibodies demonstrated to bind human LCAT (i.e., antibodies 14F11, 18E5, 25B7, and 27C3).

Biosensor analysis was conducted at 25 °C in a HBS-EP buffer system (10 mM HEPES pH 7.4, 150 mM NaCl, 3 mM EDTA, 0.005% Surfactant P20) using a Biacore 3000 optical biosensor equipped with a CM5 sensor chip. All reagents were kept at 4 °C prior to injection.

*Surface Preparation.* Goat anti-human IgG (30 mg/mL) (abcam) was immobilized (10000 RU) over flow cells 1-4 via standard amine coupling and followed by ethanolamine blocking. Each antibody (150 ng/mL) was captured (∼100 RU) on a separate flow cell and analyzed simultaneously in sets of three. Flow cell 1 was used as the reference flow cell.

*Analyte Preparation.* Triplicate samples of LCAT were prepared in running buffer. The association rate was monitored for three minutes at 50 mL/min and the dissociation rate for either five minutes (cyno studies) or ten minutes (human studies).

*Surface Regeneration.* The surface was regenerated at 50 mL/min with 10 mM glycine (pH 1.5, 25 mL).

*Model*/*Fit.* The data were fit to a 1:1 binding model (global Rmax) via Scrubber2 software. In some cases the highest concentrations were omitted to provide a better fit to the 1:1 binding model.

The binding results of the antibodies to the two different species of LCAT are summarized in **TABLE 8** below. As indicated in **TABLE 8,** the top binders to human LCAT were 27C3, 18E5 and 25B7. Each bound human LCAT in the 1-2 nM (K_{D}) range. Only one antibody (25B7, K_{D} = 1.2 nM) bound cyno LCAT with comparable affinity.

**TABLE 8**

| Antibody | Binding Affinity to Human LCAT | Binding Affinity to Cyno LCAT |
|---|---|---|
| | (K_{D}, nM) | (K_{D}, nM) |
| 14F11 | (-) | 70.6 |
| 18E5 | 1.01 | (-) |
| 25B7 | 2.24 | 1.02 |
| 27C3 | 1.15 | 128 |

### EXAMPLE 4

### Characterization of anti-LCAT Monoclonal Antibodies as

### Binders, Activators and/or Inhibitors

The antibodies confirmed as LCAT protein binders were further investigated for their effects on activation or inhibition of human LCAT enzyme using the LCAT assays described in the LCAT Enzyme Activity Assay section above.

For the activation assay 10ul hybridoma media samples were incubated with full-length wild type recombinant human LCAT protein that carries a His and Flag tag at the C-terminus (HuLCAT-HF) at a reaction concentration of 0.8ug/ml. This assay also contained 1% Human Serum Albumin (Fatty Acid Free), 7mM Tris pH7.4, 4mM EDTA, 100mM NaCl, and 2mM beta-mercaptoethanol. LCAT reaction substrate consisted of human Apolipoprotein-AI proteoliposomes containing 67uM L-alpha-Phosphatidylcholine Type XVI-E (Sigma P3556), 8uM Cholesterol (Sigma C8667), 4uM [4-14C]-cholesterol (Perkin Elmer NEC018250UC (stock 48mCi/mMol), and 12ug/ml Apolipoprotein-AI (Meridian Life Sciences). Reactions were run in triplicate and incubated in a 37 °C water bath for 1 hour. Following the reaction, lipids were extracted with a 10-fold volume addition of 100% EtOH. Lipid-containing supernatants were obtained by centrifugation and then dried under a stream of nitrogen gas, resuspended in chloroform, and spotted on thin-layer chromatography plates (Silica gel 60A, Whatman 4865-821). Cholesterol and Cholesteryl ester were separated by running plates in a TLC chamber with Petroleum Ether : Ether : Acetic Acid (100 : 20 : 0.5 by volume). Cholesterol and Cholesterylester bands were detected by exposing to image plates and subsequent reading on a Fujifilm FLA-5100 image reader. Ratios of cholesterol to cholesteryl ester were determined using ImageQuant software (Fujifilm). Activation was confirmed in select samples by a volumetric dose response using 0ul, 0.4ul, 2ul, and 10ul with the same assay conditions.

**TABLE 9** summarizes the results of these studies and lists LCAT activity as fold over the vehicle control buffer activity against full-length wild type recombinant LCAT enzyme with use of human apoA-I liposome as the substrate at 37°C for 60 min reaction.

**TABLE 9**

| Antibody | LCAT Activity (fold the vehicle control activity) | Type of Activity |
|---|---|---|
| 14F11 | 1 | Binder |
| 18E5 | 2.5 | Activator |
| 25B7 | 0.1 | Inhibitor |
| 27C3 | 3.0 | Activator |

### EXAMPLE 5

### Antibody Mutants - Glycosylation Site Mutants

Glycosylation occurs on asparagines (N) in the motif N-X-S/T, where X is anything but proline. N-linked glycosylation sites were investigated on antibody 27C3 (see **TABLE 4** for relevant sequence information) because glycosylation can cause variability in the product produced through the manufacturing process. In the case of antibody 27C3, an N-linked glycosylation site was identified at Asn40 (using AHo numbering; see reference below) in the CDR1 of the heavy chain. This glycosylation site was determined to be incompletely glycosylated. When the glycosylated and unglycosylated forms were separated, the glycosylated material was found to neither bind nor activate LCAT. Thus, to explore options for improving manufacturing and activity, standard directed mutagenesis techniques were employed to generate different mutations at the N-linked glycosylation site of CDR1 of the heavy chain of antibody 27C3.

TABLE 10 provides a summary of the different mutants tested and the resulting activity and binding affinities. The different mutants are indicated in the form XPosition numberY, where X is the amino acid that occurs at the listed position number in the variable heavy chain of antibody 27C3 and Y is the amino acid that is substituted for amino acid X. Thus, for instance, N40Q means that glutamine (Q) has been substituted for asparagine (N) at position 40. As shown in Table 11, the positioning numbering for the different mutants can be expressed in two formats: (a) as determined using a numbering system based on the AHo numbering convention (see, e.g., Honegger, A. and Pluckthun, A. (2001) J. Mol. Biol. 309:657-670) and (b) the actual position within the variable heavy chain of antibody 27C3 as shown in SEQ ID NO:45. Mutant forms of antibody 27C3 containing a mutation as just described are referred to in either the short hand form described above or alternatively as 27C3(XPosition numberY) and as shown in TABLE 10, where X and Y have the meaning just described above, and where the position number is determined using the numbering system referred to above that is based on the AHo convention. As an example, 27C3(S42A) refers to an antibody in which serine (S) at position 42 (using the numbering system based on the AHo convention; corresponding to position 37 of SEQ ID NO:45) of the variable heavy domain of 27C3 is replaced with alanine (A).

**TABLE 10**

| **LCAT Activity for Different Mutations of 27C3** | | | |
|---|---|---|---|
| Antibody Ref. | Binding Affinity (nM) | | Activity |
| | Human | Cyno | |
| 27C3 | 1.15 | 128 | 100% |
| 27C3(N40Q) | 1.4 | 87 | 100% |
| 27C3(N40D) | 168 | 2400 | 60% |
| 27C3(N40E) | 5.6 | 347 | 70% |
| 27C3(N40A) | 89 | 730 | 60% |
| 27C3(N40S) | 42 | 2160 | 60% |
| 27C3(N40T) | 4.5 | 180 | 100% |
| 27C3(N40V) | 8.5 | 390 | 100% |
| 27C3(N40Y) | 13.1 | 684 | |
| 27C3(S42V) | 64 | >2500 | 40% |
| 27C3(S42A) | 4.5 | 325 | 100% |

**TABLE 11**

| **Location of Different Mutations of VH Domain of 27C3** | | |
|---|---|---|
| Antibody Ref. | Location of Mutation (using numbering system based on AHo convention) | Location of Mutation (SEQ ID NO: ) |
| 27C3 | NA | NA |
| 27C3(N40Q) | 40 | 35 |
| 27C3(N40D) | 40 | 35 |
| 27C3(N40E) | 40 | 35 |
| 27C3(N40A) | 40 | 35 |
| 27C3(N40S) | 40 | 35 |
| 27C3(N40T) | 40 | 35 |
| 27C3(N40V) | 40 | 35 |
| 27C3(N40Y) | 40 | 35 |
| 27C3(S42V) | 42 | 37 |
| 27C3(S42A) | 42 | 37 |

### EXAMPLE 6

### LCAT Activation by LCAT Activating Antibodies

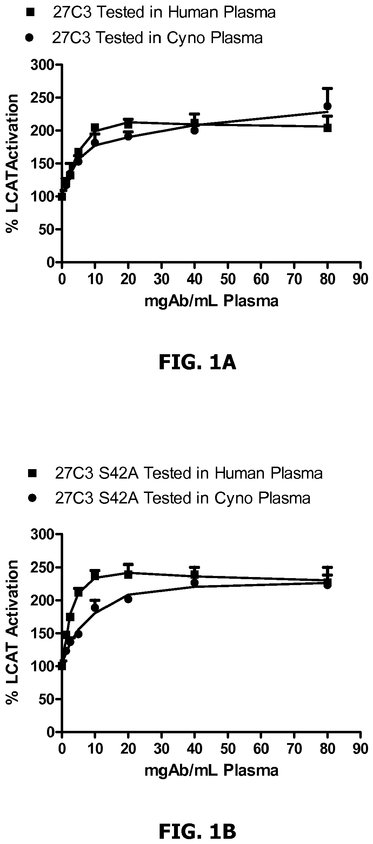

The antibodies 27C3 and the 27C3 mutant, 27C3(S42A) as described in **Example 5,** were tested ex vivo in cyno plasma and human plasma by dosing various amounts of antibody material into isolated plasma samples. **FIGS. 1A and 1B** show that both the 27C3 and the 27C3(S42A) antibodies were able to activate cyno and human LCAT in the plasma compartments in a dose-dependent manner.

### EXAMPLE 7

### In Vivo Elevation of HDL Levels in Cynomolgus Monkeys

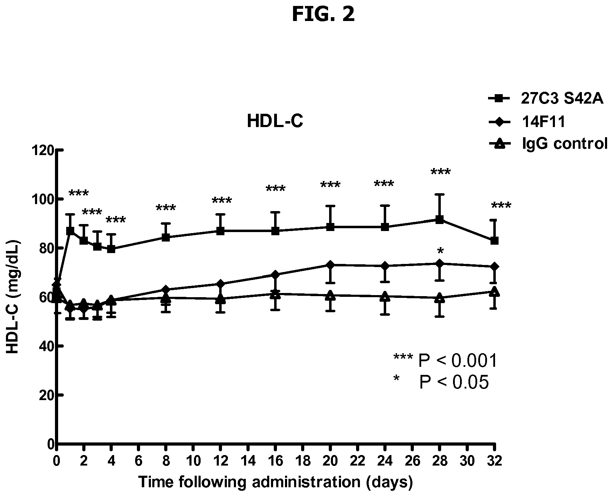

A study in cynomolgus monkeys (cynos) was undertaken to determine and confirm the in vivo effect of antibody 27C3(S42A). Animals (N=6) were treated with either antibody 27C3(S42A), or control IgG2, or antibody 14F11, which binds cyno LCAT but lacks activating activity. Blood samples were collected from single dosing up to 32 days. The results are shown in **FIG. 2** and demonstrate that 27C3(S42A) treatment increased cyno plasma levels of HDL-C about 45% above the control group, with the HDL-raising efficacy lasting up to 30 days.

### EXAMPLE 8

### Epitope Binding Determination from Competition Assays using BIAcore

A set of binding studies were conducted via Biacore analysis to determine whether antibodies 27C3, 7G8, 18E5, 27C3(S42A), 25B7, 14F121 and 23G2 bound the same epitopes.

Biosensor analysis was conducted at 25 °C in a HBS-EP+ (1X) buffer system (10 mM HEPES pH 7.4, 150 mM NaCl, 3.0 mM EDTA, 0.05% Surfactant P20) using a BIAcore 4000 optical biosensor equipped with a CM5 sensor chip. The autosampler kept each of the reagents at 8 °C prior to use. Each antibody was immobilized (5000±600 RU) to the sensor chip via standard amine coupling to a pair of spots (either 1 and 2 or 4 and 5) in one of the four available flow cells. This was followed with ethanolamine blocking. The antigen (huLCAT, 300 nM) was then captured to spots 1 and 5 of each flow cell. Finally one of the antibodies was injected (10 ug/mL) over each spot in each flow cell. After a short dissociation period (2 min) the surface was regenerated (10 mM glycine, pH 1.5) so that antigen could be captured again and a different secondary antibody analyzed.

The acquired data was analyzed with Biacore 4000 Evaluation Software. The data was cropped to isolate the interaction between the antigen/primary antibody complex and the secondary antibody. The sensorgrams from the reference spots (2 and 4) were then subtracted from their adjacent assay spots (1 and 5). A difference between the reference spots (2 or 4) and their respective assay spots (1 or 5) was an indication that the secondary molecule binds to a different epitope on the antigen.

The results from this epitope mapping experiment showed that activator antibodies 27C3 and 27C3(S42A) bind to the same epitope. Activator antibody 18E5 competes for binding with both 27C3 and 27C3(S42A). Inhibitor antibody 25B7 binds to a different epitope than 18E5 and both versions of 27C3 (i.e., 27C3 and 27C3(S42A).

### EXAMPLE 9

### Determination of Epitope for Antibody 27C3 by X-ray Crystallography

The three dimensional X-ray crystal structure of a Fab fragment of the activating antibody, 27C3, in complex with human LCAT was solved to determine the specific amino acid residues involved in this interaction.

### Methods

### Expression and Purification of Protein Samples

Human LCAT (L4F, N5D LCAT) was expressed in 293S cells with a C-terminal His₆ tag (SEQ ID NO: 169). The LCAT protein was purified first by nickel affinity chromatography and ion exchange chromatography. TEV protease was used to remove the His₆ tag (SEQ ID NO: 169). LCAT was then further purified with size exclusion chromatography and ion exchange chromatography. The sequence of the resulting LCAT molecule used in the crystallization is set forth in SEQ ID NO: 161

The 22A9 Fab fragment was expressed in *E. coli.* This protein was purified by streptavidin affinity chromatography, size exclusion chromatography and ion exchange chromatography. The 22A9 Fab is an inhibitory Fab and served as a "tool Fab" that was useful in promoting crystal formation. The 22A9 Fab light chain sequence and heavy chain sequences are set forth in SEQ ID NOs: 162 and 163, respectively.
antiLCAT 22A9 Fab light chain
antiLCAT 22A9 Fab heavy chain

The 27C3 Fab was expressed in 293-6E cells on a caspase cleavable IgG1 scaffold. Following caspase cleavage, the Fab fragment was purified by MAb select and nickel affinity chromatography. Further purification was performed with size exclusion chromatography and ion exchange chromatography.

### Complex Formation and Crystallization

The LCAT/22A9 Fab complex was made by mixing a molar excess of 22A9 Fab with LCAT. The complex was separated from excess 22A9 Fab by purification on a size exclusion chromatography column. The LCAT/27C3 Fab/22A9 Fab complex was made by mixing a molar excess of 27C3 Fab with LCAT/22A9 Fab complex. The ternary complex was separated from excess 27C3 Fab by purification on a size exclusion chromatography column. The LCAT/27C3 Fab/22A9 Fab complex crystallizes in 0.1 M Hepes pH 7, 5% PEG 20000.

### Data Collection and Structure Determination

The highest resolution dataset for the LCAT/27C3 Fab/22A9 Fab crystal was collected at the Berkeley ALS on beamline 5.0.2. and processed with imosflm/scala (CCP4, The CCP4 suite: programs for protein crystallography. Acta Crystallogr D Biol Crystallogr, 1994. 50(Pt 5): p. 760-3).

LCAT/27C3 Fab/22A9 Fab crystals grow in the P2₁2₁2₁ space group with unit cell dimensions a=57, b=127, c=256 Å with one complex per asymmetric unit, and diffract up to 2.5 Å resolution. The LCAT/27C3 Fab/22A9 Fab structure was solved by molecular replacement with Phaser (CCP4, The CCP4 suite: programs for protein crystallography. Acta Crystallogr D Biol Crystallogr, 1994. 50(Pt 5): p. 760-3) using LCAT, 22A9 variable domain, 27C3 variable domain, 22A9 constant domain, 27C3 constant domain as the search models in sequence. The complete structure was improved with multiple rounds of model building with Quanta and refinement with cnx (Brunger, A.T., et al., Crystallography & NMR system: A new software suite for macromolecular structure determination. Acta Crystallogr D Biol Crystallogr, 1998. 54(Pt 5): p. 905-21).

Interaction interface amino acids were determined as being all amino acid residues with at least one atom less than or equal to 5 Å from the LCAT partner protein. 5 Å was chosen as the cutoff distance to allow for atoms within a van der Waals radius plus a possible water-mediated hydrogen bond. Amino acids that met these distance criteria were calculated with the program PyMOL (DeLano, W.L., The PyMOL Molecular Graphics System. 2002: Palo Alto).

### Results

Ternary complexes with LCAT/27C3 Fab and an additional tool Fab were required for crystallization. The complex between LCAT and the Fabs of 27C3 and 22A9 (tool Fab) was formed and purified. Protein crystals of the LCAT/27C3 Fab /22A9 Fab complex were grown. The crystal structure of this complex was determined at 2.5 Å resolution. This structure shows that 27C3 and 22A9 bind to opposing sides of the LCAT protein.

### LCAT Interacting Amino Acids

LCAT amino acid residues of the interaction interface with 27C3 were defined as LCAT residues that are within 5 Å of the 27C3 protein. The amino acid residues of human LCAT (SEQ ID NO:1) satisfying this criterion include the following:
S255, R256, M257, A258, W259, P260, Y315, V317, G318, L319, P320, T321, Y341, E342, D343, T350, R351, E354, L355, C356, G357, L358, Q360, R362, V367, H368, L369, P371, H373 and G374

### 27C3 Interacting Amino Acids

27C3 amino acid residues of the interaction interface with LCAT were defined as 27C3 residues that are within 5 Å of the LCAT protein. The amino acid residues are listed below.
27C3 variable heavy domain (SEQ ID NO:45): S30, S31, G32, G33, Y52, Y54, Y55, S56, G57, S58, T59, Y60, Y61, K66, C104, S105, S106, T107, S108, C109
27C3 variable light domain (SEQ ID NO:76): Y30, R90, D91, N92, I93, G94, N95

### EXAMPLE 10

### Determination of Epitope for Antibody 18E5 by X-ray Crystallography

Experiments were also undertaken to determine the three dimensional X-ray crystal structure of a Fab fragment from a second activating antibody, 18E5, to determine the specific amino acid residues or this antibody which are involved at the interface with LCAT and to evaluate how these interactions compare with those identified as being involved in the binding of 27C3 (see **Example 9**).

### Methods

### Expression and Purification of Protein Samples

Human LCAT (C31Y LCAT) was expressed in 293F cells in the presence of 5 µM kifunensine with a C-terminal His₆ tag (SEQ ID NO: 169). The LCAT protein was purified first by nickel affinity chromatography and ion exchange chromatography. Endo H treatment was used to deglycosylate the protein. TEV protease was used to remove the His₆ tag (SEQ ID NO: 169). LCAT was then further purified with size exclusion chromatography and ion exchange chromatography. The sequence of the resulting LCAT molecule used in the crystallization is set forth in SEQ ID NO:164

The 25B7 Fab fragment was expressed in *E. coli.* This protein was purified by nickel affinity chromatography, size exclusion chromatography and ion exchange chromatography. Inclusion of this inhibitor Fab as a "tool Fab" promoted crystal formation. The 25B7 Fab light chain sequence and heavy chain sequences are set forth in SEQ ID NOs: 165 and 166, respectively
antiLCAT 25B7 Fab light chain
antiLCAT 25B7 Fab heavy chain

The 18E5 Fab was expressed in 293-6E cells on a caspase cleavable IgG1 scaffold. Following caspase cleavage, the Fab fragment was purified by MAb select affinity chromatography. Further purification was performed with size exclusion chromatography and ion exchange chromatography.

### Complex Formation and Crystallization

The LCAT/18E5 Fab complex was made by mixing a molar excess of 18E5 Fab with LCAT. The complex was separated from excess 18E5 Fab by purification on a size exclusion chromatography column. The LCAT/18E5 Fab/25B7 Fab complex was made by mixing a molar excess of 25B7 Fab with LCAT/18E5 Fab complex. The ternary complex was separated from excess 25B7 Fab by purification on a size exclusion chromatography column. The LCAT/18E5 Fab/25B7 Fab complex crystallizes in 0.1 M sodium acetate pH 4.6, 24% PEG 600.

### Data Collection and Structure Determination

The dataset for the LCAT/18E5 Fab/25B7 Fab crystal was collected at the APS at Argonne National Lab on beamline 211DF and processed with imosflm/scala (CCP4, The CCP4 suite: programs for protein crystallography. Acta Crystallogr D Biol Crystallogr, 1994. 50(Pt 5): p. 760-3).

LCAT/18E5 Fab/25B7 Fab crystals grow in the P2₁ space group with unit cell dimensions a=53.45, b=270.67, c=111.29 A, and β=92.22 with two complexes per asymmetric unit, and diffract up to 2.75 Å resolution. The LCAT/18E5 Fab/25B7 Fab structure was solved by molecular replacement with Phaser(CCP4, The CCP4 suite: programs for protein crystallography. Acta Crystallogr D Biol Crystallogr, 1994. 50(Pt 5): p. 760-3) using 2 each of LCAT, 25B7 variable domain, 18E5 variable domain, 25B7 constant domain, 18E5 constant domain as the search models in sequence. The complete structure was improved with multiple rounds of model building with Quanta and refinement with cnx (Brunger, A.T., et al., Crystallography & NMR system: A new software suite for macromolecular structure determination. Acta Crystallogr D Biol Crystallogr, 1998. 54(Pt 5): p. 905-21).

Interaction interface amino acids were determined as being all amino acid residues with at least one atom less than or equal to 5 Å from the LCAT partner protein. 5 Å was chosen as the cutoff distance to allow for atoms within a van der Waals radius plus a possible water-mediated hydrogen bond. Amino acids that met these distance criteria were calculated with the program PyMOL (DeLano, W.L., The PyMOL Molecular Graphics System. 2002: Palo Alto).

### Results

Ternary complexes with LCAT/18E5 Fab and an additional tool Fab were used for crystallization. The complex between LCAT and the Fabs of 18E5 and 25B7 (tool Fab) was formed and purified. Protein crystals of the LCAT/18E5 Fab /25B7 Fab complex were grown. The crystal structure of this complex was determined at 2.75 Å resolution. This structure shows that 18E5 and 25B7 bind to opposing sides of the LCAT protein.

### LCAT Interacting Amino Acids

LCAT amino acid residues of the interaction interface with 18E5 were defined as LCAT residues that are within 5 Å of the 18E5 protein. The amino acid residues of human LCAT (SEQ ID NO:1) satisfying this criterion include the following:
Y315, V317, D343, T350, R351, E354, G357, Q360, G361, Q365, P366, V367, H368, L369, L370, P371, L372, H373, I375, L385, E388, H389, A392, L395, G396, A397, Y398, R399

### 18E5 Interacting Amino Acids

18E5 amino acid residues of the interaction interface with LCAT were defined as 18E5 residues that are within 5 Å of the LCAT protein. The amino acid residues are listed below:
18E5 variable heavy domain (SEQ ID NO:75): Y33, W50, N52, N54, S55, G57, T58, N59, Y60, Q62, Q65, W101, E102, Y104
18E5 variable light domain (SEQ ID NO:80): E1, I2, Q27, V29, S30, G31, Y33, Y92, G93, G94, S95, P97

### EXAMPLE 11

### Determination of LCAT Antibody Complex Contact Residues through Solvent Accessible Surface Area Differences

The residue contacts in the paratope (the portion of the antibody that recognizes the antigen) and the portion of the antigen that is bound by the paratope in a complex between the 27C3 Fab or 18E5 Fab and human LCAT (see **Examples 9 and 10**) were determined using solvent accessable surface area differences. The solvent accessible surface area calculations were performed using Molecular Operating Environment (Chemical Computing Group, Montreal, Quebec).

### 27C3 Results

The solvent accessible surface area differences of the paratope residues in the 27C3 Fab complex were calculated by setting the 27C3 Fab residues as the desired set. The structural information obtained in **Example 9** for the 27C3 Fab-LCAT complex was used and the residue solvent accessible surface area of the amino acid residues of the 27C3 Fab in the presence of human LCAT were calculated and represent the "bound areas" for the set.

The residue solvent accessible surface area of each of the 27C3 Fab residues in the absence of the LCAT antigen were calculated and represent the "free areas" of the set.

The "bound areas" were then subtracted from the "free areas" resulting in the "solvent exposed surface area difference" for each residue in the set. The 27C3 Fab residues that had no change in surface area, or a zero difference, had no contact with the residues of the LCAT antigen when complexed. The 27C3 Fab residues that had a difference value ≥10 Å² were considered to be in significant contact with residues in the LCAT antigen such that these 27C3 residues were at least partially to completely occluded when the 27C3 Fab was bound to human LCAT. This set of 27C3 Fab residues make up the "covered patch", the residues involved in the structure of the interface when the 27C3 Fab is bound to human LCAT.

The residues of the 27C3 Fab at the interface as determined using this approach include the following:
27C3 variable heavy domain (SEQ ID NO:45): S30, S31, G32, G33, Y52, I53, Y54, Y55, S56, G57, S58, T59, Y60, Y61, K66, T70, I71, S72, V73, G102, C104, S105, S106, T107, S108, C109, S110 and R111
27C3 variable light domain (SEQ ID NO:76): S28, Y29, R89, D90, N91, I92, G93 and N94

The residues identified as being in the paratope based upon this method are very similar to those identified as described in **Example 9.**

The solvent accessible surface area differences of the portion of human LCAT bound by the paratope of the 27C3 Fab were also calculated. This was done by setting the LCAT residues as the desired set. The structural information obtained in **Example 9** for the 27C3 Fab-LCAT complex was used and the residue solvent accessible surface area of the amino acid residues of LCAT in the presence of the 27C3 Fab were calculated and represent the bound areas for the set. The residue solvent accessible surface area of each of the LCAT residues in the absence of the 27C3 Fab were calculated and represent the free areas of the set.

As described above, the bound areas were subtracted from the free areas resulting in the solvent exposed surface area difference for each LCAT residue. The LCAT residues that had no change in surface area, or a zero difference, had no contact with the residues of the 27C3 Fab when complexed. The LCAT residues that had a difference value ≥10 Å² were considered to be in significant contact with residues of the 27C3 Fab and these LCAT residues were at least partially to completely occluded when the human LCAT was bound to the 27C3 Fab. This set of LCAT residues make up the covered patch, the residues involved in the structure of the interface when the human LCAT is bound to the 27C3 Fab.

The human LCAT residues (SEQ ID NO:1) determined to be at the interface with the 27C3 Fab in this approach include the following: R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 and H389.

The residues identified by this approach are very similar to those identified as described in **Example 9.**

### 18E5 Results

The solvent accessible surface area differences of the paratope residues in the 18E5 Fab complex were calculated as described above for the 27C3 Fab, except that the 18E5 Fab residues were used as the desired set and the structural information as obtained in Example 10 for the 18E5 Fab-LCAT complex were used.

The residues of the 18E5 Fab at the interface as determined using this approach include the following:
18E5 heavy chain domain (SEQ ID NO:75): Y33, W50, N52, N54, S55, G57, T58, N59, Y60, Q62, Q65, W101, E102 and Y104.
18E5 light chain domain (SEQ ID NO:80): E1, I2, Q27, S28, V29, S30, G31, Y33, Y92, G93, G94, S95 and P97.

These results are consistent with those obtained in **Example 10.**

The solvent accessible surface area differences of the portion of human LCAT bound by the paratope of the 18E5 Fab were also calculated as described above for the 27C3 Fab-LCAT complex, except that the structural information obtained in **Example 10** for the 18E5 Fab-LCAT complex was used and the residue solvent accessible surface area of the amino acid residues of LCAT in the presence of the 18E5 Fab were calculated and represent the bound areas for the set. The residue solvent accessible surface area of each of the LCAT residues in the absence of the 18E5 Fab were calculated and represent the free areas of the set.

The human LCAT (SEQ ID NO: 1) residues determined to be at the interface with the 18E5 Fab in this approach include the following: Y315, V317, E342, D343, T350, R351, E354, G357, Q360, G361, P364, P366, V367, H368, L369, L370, P371, L372, H373, L385, E388, H389, A392, L395, G396, A397, Y398 and R399.

The residues identified by this methodology compare well with those identified according to the approach taken in **Example 10.**

### EXAMPLE 12

### Epitope Binding Comparison Between Agonist and Antagonist Anti-LCAT Antibodies as Determined by X-ray Crystallography

The 18E5 binding site on LCAT overlaps with the 27C3 binding site on LCAT, sharing about 13 amino acids. Both activating antibodies bind on the surface of LCAT behind the D345 of the catalytic triad.

Amino acids to which both the 27C3 and 18E5 antibodies bind include: Y315, V317, D343, T350, R351, E354, G357, Q360, V367, H368, L369, P371, and H373. These residues thus appear to play an important role in binding for these agonist antibodies.

As mentioned above and as shown **FIGS. 3A and 3B**, the inhibitory Fab fragments 22A9 and 25B7 bind on opposite sides of the LCAT protein and on opposite sides of the catalytic triad, thus indicating that agonist and inhibitory antibodies bind distinct regions of the protein.

### EXAMPLE 13

### Antibody Mutants - Cross-Reactivity

To explore options for improving cyno LCAT binding while retaining or improving human LCAT activity, standard directed metagenesis techniques were employed to generate one or more mutations within CDR3 of the heavy chain of antibody 27C3 and/or one or more mutations within CDR3 of the light chain of antibody 27C3.

**TABLE 12** provides a summary of the different mutants tested and the resulting activity and binding affinities. The different mutants are indicated in the form XPosition numberY, where X is the amino acid that occurs at the listed position number in the variable heavy chain or variable light chain, as applicable, of antibody 27C3 and Y is the amino acid that is substituted for amino acid X. Thus, for instance, S131T means that threonine (T) has been substituted for serine (S) at position 131. As shown in **TABLE 13**, the position number for a mutation in the variable heavy chain can be expressed in two formats: (a) as determined using a numbering system based on the AHo numbering convention, see reference above, and (b) the actual position within the variable heavy chain or of antibody 27C3 as shown in SEQ ID NO:45. Likewise, as shown in **TABLE 14**, the position number for a mutation in the variable light chain also can be expressed in two formats: (a) as determined using a numbering system based on the AHo numbering convention and (b) the actual position within the variable light chain of antibody 27C3 as shown in SEQ ID NO:76.

Mutant forms of antibody 27C3 containing a mutation in the variable heavy chain as just described are referred to in either the short hand form described above or alternatively as 27C3(XPosition numberY), where X and Y have the meaning just described above, and where the position number is determined using the numbering system referred to above that is based on the AHo convention. If a mutant form of antibody 27C3 comprises multiple mutations in the variable heavy chain, references to the mutations are separated by commas. As an example, 27C3(S42A,M136I) refers to an antibody in which serine (S) at position 42 (using the numbering system based on the AHo convention; corresponding to position 37 of SEQ ID NO:45) of the variable heavy domain of 27C3 is replaced with alanine (A) and the methionine (M) at position 136 (using the numbering system based on the AHo convention; corresponding to position 113 of SEQ ID NO:45) of the variable heavy domain of 27C3 is replaced with isoleucine (I).

If a mutant form of antibody 27C3 comprises mutations in both the variable heavy chain and the variable light chain, references to the mutation(s) in the variable light chain follow references to the mutations(s) in the variable heavy chain, and are separated by a slash. If there are multiple mutations in the variable light chain, references to the mutations are separated by commas. As an example, 27C3(S42A,M136I/N107G,I112V) refers to an antibody in which serine (S) at position 42 (using the numbering system based on the AHo convention; corresponding to position 37 of SEQ ID NO:45) of the variable heavy domain of 27C3 is replaced with alanine (A), methionine (M) at position 136 (using the numbering system based on the AHo convention; corresponding to position 113 of SEQ ID NO:45) of the variable heavy domain of 27C3 is replaced with isoleucine (I), asparagine (N) at position 107 (using the numbering system based on the AHo convention; corresponding to position 88 of SEQ ID NO:76) of the variable light domain of 27C3 is replaced with glycine (G) and isoleucine (I) at position 112 (using the numbering system based on the AHo convention; corresponding to position 93 of SEQ ID NO:76) is replaced with valine (V).

**TABLE 12**

| **LCAT Activity for Different Mutations of 27C3** | | | |
|---|---|---|---|
| Antibody Ref. | Binding Affinity (nM) | | Activity |
| | Human | Cyno | |
| 27C3(S42A) | 1.2 | 179 | 100% |
| 27C3(S42A,S133T,M136I/I112V) | 0.3 | 88 | 46% |
| 27C3(S42A,S131T,M136I/I112V) | 3 | 198 | 65% |
| 27C3(S42A,M136I/I112V) | 0.8 | 132 | 79% |
| 27C3(S42A/I112V) | 1 | 172 | 77% |
| 27C3(S42A,S131T,V135I,M136I/I112V) | 3 | 198 | 69% |
| 27C3(S42A,S133T,M136I/N107G) | 0.4 | 62 | 73% |
| 27C3(S42A,S31T,M136I/N107G) | 2.8 | 226 | 65% |
| 27C3(S42A,M136I/N107G) | 0.7 | 149 | 81% |
| 27C3(S42A/N107G) | 1.1 | 225 | 64% |
| 27C3(S42A,S131T,V135I,M136I/N107G) | 2.8 | 226 | 70% |
| 27C3(S42A,S131T,V135I,M136I/N107G,I112V) | 34 | 88 | 70% |
| 27C3(S42A,S 133T,M136I/N107G,I112V) | 0.5 | 8 | 83% |
| 27C3(S42A,M136I/N107G,I112V) | 1.2 | 190 | 70% |
| 27C3(S42A,S 131T,M136I/N107G,I112V) | 1.3 | 181 | 89% |

**TABLE 13**

| **Location of Different Mutations of VH Domain of 27C3** | | |
|---|---|---|
| Antibody Ref. | Location of Mutation (using numbering system based on AHo convention) | Location of Mutation (SEQ ID NO: ) |
| 27C3 | NA | NA |
| 27C3(S42A) | 42 | 37 |
| 27C3(S131T) | 131 | 108 |
| 27C3(S133T) | 133 | 110 |
| 27C3(V135I) | 135 | 112 |
| 27C3(M136I) | 136 | 113 |

**TABLE 14**

| **Location of Different Mutations of VL Domain of 27C3** | | |
|---|---|---|
| Antibody Ref. | Location of Mutation (using numbering system based on AHo convention) | Location of Mutation (SEQ ID NO: ) |
| 27C3 | NA | NA |
| 27C3(N107G) | 107 | 88 |
| 27C3(I112V) | 112 | 93 |

### EXAMPLE 14

### Treatment of Atherosclerosis Using an LCAT Antigen Binding Protein

A human patient is diagnosed as having or being at risk for atherosclerosis. This is done, for instance, by clinic assessment by a physician, and/or obtaining an atherogenic lipoprotein profile. For example, a ratio of serum cholesterol to HDLs of 5:1 or above indicates a higher than average risk of developing atherosclerosis. Other factors include a serum cholesterol level of 240 mg/dL or above, an HDL level 35 mg/dL or below, or an LDL level 190 mg/dL or above, a plasma LCAT protein level lower than normal (<5 µg/ml), and/or a decreased plasma cholesterol esterification rate (<60 nmol/ml/hr).

The patient is administered an effective amount of an LCAT antigen binding protein as described herein in combination with standard treatment, for instance, statins, and/or with other therapeutics, for instance, PCSK9-antibodies. The LCAT antigen binding protein continues to be administered to reach and maintain desired serum cholesterol levels, HDL levels, LDL levels and/or LCAT levels. Significant increase in plasma level of LCAT enzyme activity and/or HDL-C levels are found in response to administration of the LCAT antigen binding protein.

## Claims

1. An agonist antigen binding protein that specifically binds to a human Lecithin-cholesterol acyltransferase polypeptide, wherein the antigen binding protein is a human antibody.

2. The antigen binding protein of any one of claim 1 that binds a human Lecithin-cholesterol acyltransferase polypeptide consisting of SEQ ID NO:1.

3. The antigen binding protein of claims 1 or 2, wherein the antigen binding protein has one or more of the following characteristics:
a) binds to the human Lecithin-cholesterol acyltransferase polypeptide of SEQ ID NO:1 with a K_{D} of less than 50 nM, as determined by BiaCore; and
b) binds to human Lecithin-cholesterol acyltransferase with K_{D} of less than 50 nM and cyno Lecithin-cholesterol acyltransferase with K_{D} of less than 500 nM, as determined by BiaCore.

4. The antigen binding protein of any one of claims 1 to 3, wherein the antigen binding protein comprises:
I.
a. one or more heavy chain complementary determining regions (CDRHs) selected from the group consisting of:
i. a CDRH1 selected from the group consisting of SEQ ID NO:81-92;
ii. a CDRH2 selected from the group consisting of SEQ ID NO:93-94;
iii. a CDRH3 selected from the group consisting of SEQ ID NO:95-111;
iv. a CDRH of (i), (ii) and (iii) that contains one or more amino acid substitutions, deletions or insertions totaling no more than 4 amino acids;
b. one or more light chain complementary determining regions (CDRLs) selected from the group consisting of:
i. a CDRL1 selected from the group consisting of SEQ ID NO:112-113;
ii. a CDRL2 selected from the group consisting of SEQ ID NO:114-115;
iii. a CDRL3 selected from the group consisting of SEQ ID NO:116-120;
iv. a CDRL of (i), (ii) and (iii) that contains one or more amino acid substitutions, deletions or insertions totaling no more than 4 amino acids; or
c. one or more CDRHs or (a) and one or more CDRLs of (b), or
II. a heavy chain variable region (VH) and/or a light chain variable region (VL), wherein
(i)
a. the VH has at least 90% sequence identity with the amino acid sequence selected from the group consisting of SEQ ID NO:45-75; and
b. the VL has at least 90% sequence identity with the amino acid sequence selected from the group consisting of SEQ ID NO:76-80; or
(ii)
a. the VH has at least 90% sequence identity with the amino acid sequence of SEQ ID NO:45 and the VL has at least 90% sequence identity with the amino acid sequence of SEQ ID NO:76; or
b. the VH has at least 90% sequence identity with the amino acid sequence of SEQ ID NO:75 and the VL has at least 90% sequence identity with the amino acid sequence of SEQ ID NO:80, or
III. a heavy chain (HC) and a light chain (LC), wherein
(i)
a. the HC has at least 90% sequence identity with the amino acid sequence of SEQ ID NO:9 and the LC has at least 90% sequence identity with the amino acid sequence of SEQ ID NO:40; or
b. the HC has at least 90% sequence identity with the amino acid sequence of SEQ ID NO:39 and the LC has at least 90% sequence identity with the amino acid sequence of SEQ ID NO:44, or
(ii)
a. the HC comprises the amino acid sequence of SEQ ID NO:9 and the LC comprises the amino acid sequence of SEQ ID NO:40;
b. the HC comprises the amino acid sequence of SEQ ID NO:19 and the LC comprises the amino acid sequence of SEQ ID NO:40;
c. the HC comprises the amino acid sequence of SEQ ID NO:27 and the LC comprises the amino acid sequence of SEQ ID NO:40;
d. the HC comprises the amino acid sequence of SEQ ID NO:30 and the LC comprises the amino acid sequence of SEQ ID NO:40;
e. the HC comprises the amino acid sequence of SEQ ID NO:32 and the LC comprises the amino acid sequence of SEQ ID NO:40;
f. the HC comprises the amino acid sequence of SEQ ID NO:36 and the LC comprises the amino acid sequence of SEQ ID NO:40;
g. the HC comprises the amino acid sequence of SEQ ID NO:19 and the LC comprises the amino acid sequence of SEQ ID NO:41;
h. the HC comprises the amino acid sequence of SEQ ID NO:27 and the LC comprises the amino acid sequence of SEQ ID NO:41;
i. the HC comprises the amino acid sequence of SEQ ID NO:30 and the LC comprises the amino acid sequence of SEQ ID NO:41;
j. the HC comprises the amino acid sequence of SEQ ID NO:32 and the LC comprises the amino acid sequence of SEQ ID NO:41;
k. the HC comprises the amino acid sequence of SEQ ID NO:36 and the LC comprises the amino acid sequence of SEQ ID NO:41;
l. the HC comprises the amino acid sequence of SEQ ID NO:19 and the LC comprises the amino acid sequence of SEQ ID NO:42;
m. the HC comprises the amino acid sequence of SEQ ID NO:27 and the LC comprises the amino acid sequence of SEQ ID NO:42;
n. the HC comprises the amino acid sequence of SEQ ID NO:30 and the LC comprises the amino acid sequence of SEQ ID NO:42;
o. the HC comprises the amino acid sequence of SEQ ID NO:32 and the LC comprises the amino acid sequence of SEQ ID NO:42;
p. the HC comprises the amino acid sequence of SEQ ID NO:36 and the LC comprises the amino acid sequence of SEQ ID NO:42;
q. the HC comprises the amino acid sequence of SEQ ID NO:19 and the LC comprises the amino acid sequence of SEQ ID NO:43;
r. the HC comprises the amino acid sequence of SEQ ID NO:27 and the LC comprises the amino acid sequence of SEQ ID NO:43;
s. the HC comprises the amino acid sequence of SEQ ID NO:30 and the LC comprises the amino acid sequence of SEQ ID NO:43;
t. the HC comprises the amino acid sequence of SEQ ID NO:32 and the LC comprises the amino acid sequence of SEQ ID NO:43;
u. the HC comprises the amino acid sequence of SEQ ID NO:36 and the LC comprises the amino acid sequence of SEQ ID NO:43; or
the HC has comprises the amino acid sequence of SEQ ID NO:39 and the LC comprises the amino acid sequence of SEQ ID NO:44.

5. The antigen binding protein of claim 4 (I) that comprises a CDRH3 and a CDRL3.

6. The antigen binding protein of claim 5, wherein
a. the CDRH3 comprises SEQ ID NO:95 and the CDRL3 comprises SEQ ID NO:116; or
b. the CDRH3 comprises SEQ ID NO:111 and the CDRL3 comprises SEQ ID NO:120.

7. The isolated antigen binding protein of any one of claims 1 to 3, wherein said antigen binding protein comprises:
I.
a. a CDRH selected from the group consisting of
i. a CDRH1 selected from the group consisting of SEQ ID NO:81-92;
ii. a CDRH2 selected from the group consisting of SEQ ID NO:93-94; and
iii. a CDRH3 selected from the group consisting of SEQ ID NO:95-111;
b. a CDRL selected from the group consisting of
i. a CDRL1 selected from the group consisting of SEQ ID NO:112-113;
ii. a CDRL2 selected from the group consisting of SEQ ID NO:114-115; and
iii. a CDRL3 selected from the group consisting of SEQ ID NO:116-120 or
c. one or more CDRHs of a) and one or more CDRLs of b); or
II.
a. a CDRH1 of SEQ ID NO:81, a CDRH2 of SEQ ID NO:93, a CDRH3 of SEQ ID NO:95, a CDRL1 of SEQ ID NO:112, a CDRL2 of SEQ ID NO:114, and a CDRL3 of SEQ ID NO:116;
b. a CDRH1 of SEQ ID NO:91, a CDRH2 of SEQ ID NO:93, a CDRH3 of SEQ ID NO:95, a CDRL1 of SEQ ID NO:112, a CDRL2 of SEQ ID NO:114, and a CDRL3 of SEQ ID NO:116;
c. a CDRH1 of SEQ ID NO:91, a CDRH2 of SEQ ID NO:93, a CDRH3 of SEQ ID NO:99, a CDRL1 of SEQ ID NO:112, a CDRL2 of SEQ ID NO:114, and a CDRL3 of SEQ ID NO:116;
d. a CDRH1 of SEQ ID NO:91, a CDRH2 of SEQ ID NO:93, a CDRH3 of SEQ ID NO:102, a CDRL1 of SEQ ID NO:112, a CDRL2 of SEQ ID NO:114, and a CDRL3 of SEQ ID NO:116;
e. a CDRH1 of SEQ ID NO:91, a CDRH2 of SEQ ID NO:93, a CDRH3 of SEQ ID NO:104, a CDRL1 of SEQ ID NO:112, a CDRL2 of SEQ ID NO:114, and a CDRL3 of SEQ ID NO:116;
f. a CDRH1 of SEQ ID NO:91, a CDRH2 of SEQ ID NO:93, a CDRH3 of SEQ ID NO:108, a CDRL1 of SEQ ID NO:112, a CDRL2 of SEQ ID NO:114, and a CDRL3 of SEQ ID NO:116;
g. a CDRH1 of SEQ ID NO:91, a CDRH2 of SEQ ID NO:93, a CDRH3 of SEQ ID NO:95, a CDRL1 of SEQ ID NO:112, a CDRL2 of SEQ ID NO:114, and a CDRL3 of SEQ ID NO:117;
h. a CDRH1 of SEQ ID NO:91, a CDRH2 of SEQ ID NO:93, a CDRH3 of SEQ ID NO:99, a CDRL1 of SEQ ID NO:112, a CDRL2 of SEQ ID NO:114, and a CDRL3 of SEQ ID NO:117;
i. a CDRH1 of SEQ ID NO:91, a CDRH2 of SEQ ID NO:93, a CDRH3 of SEQ ID NO:102, a CDRL1 of SEQ ID NO:112, a CDRL2 of SEQ ID NO:114, and a CDRL3 of SEQ ID NO:117;
j. a CDRH1 of SEQ ID NO:91, a CDRH2 of SEQ ID NO:93, a CDRH3 of SEQ ID NO:104, a CDRL1 of SEQ ID NO:112, a CDRL2 of SEQ ID NO:114, and a CDRL3 of SEQ ID NO:117;
k. a CDRH1 of SEQ ID NO:91, a CDRH2 of SEQ ID NO:93, a CDRH3 of SEQ ID NO:108, a CDRL1 of SEQ ID NO:112, a CDRL2 of SEQ ID NO:114, and a CDRL3 of SEQ ID NO:117;
l. a CDRH1 of SEQ ID NO:91, a CDRH2 of SEQ ID NO:93, a CDRH3 of SEQ ID NO:95, a CDRL1 of SEQ ID NO:112, a CDRL2 of SEQ ID NO:114, and a CDRL3 of SEQ ID NO:118;
m. a CDRH1 of SEQ ID NO:91, a CDRH2 of SEQ ID NO:93, a CDRH3 of SEQ ID NO:99, a CDRL1 of SEQ ID NO:112, a CDRL2 of SEQ ID NO:114, and a CDRL3 of SEQ ID NO:118;
n. a CDRH1 of SEQ ID NO:91, a CDRH2 of SEQ ID NO:93, a CDRH3 of SEQ ID NO:102, a CDRL1 of SEQ ID NO:112, a CDRL2 of SEQ ID NO:114, and a CDRL3 of SEQ ID NO:118;
o. a CDRH1 of SEQ ID NO:91, a CDRH2 of SEQ ID NO:93, a CDRH3 of SEQ ID NO:104, a CDRL1 of SEQ ID NO:112, a CDRL2 of SEQ ID NO:114, and a CDRL3 of SEQ ID NO:118;
p. a CDRH1 of SEQ ID NO:91, a CDRH2 of SEQ ID NO:93, a CDRH3 of SEQ ID NO:108, a CDRL1 of SEQ ID NO:112, a CDRL2 of SEQ ID NO:114, and a CDRL3 of SEQ ID NO:118;
q. a CDRH1 of SEQ ID NO:91, a CDRH2 of SEQ ID NO:93, a CDRH3 of SEQ ID NO:95, a CDRL1 of SEQ ID NO:112, a CDRL2 of SEQ ID NO:114, and a CDRL3 of SEQ ID NO:119;
r. a CDRH1 of SEQ ID NO:91, a CDRH2 of SEQ ID NO:93, a CDRH3 of SEQ ID NO:99, a CDRL1 of SEQ ID NO:112, a CDRL2 of SEQ ID NO:114, and a CDRL3 of SEQ ID NO:119;
s. a CDRH1 of SEQ ID NO:91, a CDRH2 of SEQ ID NO:93, a CDRH3 of SEQ ID NO:102, a CDRL1 of SEQ ID NO:112, a CDRL2 of SEQ ID NO:114, and a CDRL3 of SEQ ID NO:119;
t. a CDRH1 of SEQ ID NO:91, a CDRH2 of SEQ ID NO:93, a CDRH3 of SEQ ID NO:104, a CDRL1 of SEQ ID NO:112, a CDRL2 of SEQ ID NO:114, and a CDRL3 of SEQ ID NO:119;
u. a CDRH1 of SEQ ID NO:91, a CDRH2 of SEQ ID NO:93, a CDRH3 of SEQ ID NO:108, a CDRL1 of SEQ ID NO:112, a CDRL2 of SEQ ID NO:114, and a CDRL3 of SEQ ID NO:119; or
a CDRH1 of SEQ ID NO:92, a CDRH2 of SEQ ID NO:94, a CDRH3 of SEQ ID NO:111, a CDRL1 of SEQ ID NO:113, a CDRL2 of SEQ ID NO:115, and a CDRL3 of SEQ ID NO:120.

8. The antigen binding protein of claim 4 (II) (i), wherein said antigen binding protein comprises a heavy chain variable region (VH) and a light chain variable region (VL), wherein
a. the VH comprises the amino acid sequence of SEQ ID NO:45 and the VL comprises the amino acid sequence of SEQ ID NO:76;
b. the VH comprises the amino acid sequence of SEQ ID NO:55 and the VL comprises the amino acid sequence of SEQ ID NO:76;
c. the VH comprises the amino acid sequence of SEQ ID NO:63 and the VL comprises the amino acid sequence of SEQ ID NO:76;
d. the VH comprises the amino acid sequence of SEQ ID NO:66 and the VL comprises the amino acid sequence of SEQ ID NO:76;
e. the VH comprises the amino acid sequence of SEQ ID NO:68 and the VL comprises the amino acid sequence of SEQ ID NO:76;
f. the VH comprises the amino acid sequence of SEQ ID NO:72 and the VL comprises the amino acid sequence of SEQ ID NO:76;
g. the VH comprises the amino acid sequence of SEQ ID NO:55 and the VL comprises the amino acid sequence of SEQ ID NO:77;
h. the VH comprises the amino acid sequence of SEQ ID NO:63 and the VL comprises the amino acid sequence of SEQ ID NO:77;
i. the VH comprises the amino acid sequence of SEQ ID NO:66 and the VL comprises the amino acid sequence of SEQ ID NO:77;
j. the VH comprises the amino acid sequence of SEQ ID NO:68 and the VL comprises the amino acid sequence of SEQ ID NO:77;
k. the VH comprises the amino acid sequence of SEQ ID NO:72 and the VL comprises the amino acid sequence of SEQ ID NO:77;
l. the VH comprises the amino acid sequence of SEQ ID NO:55 and the VL comprises the amino acid sequence of SEQ ID NO:78;
m. the VH comprises the amino acid sequence of SEQ ID NO:63 and the VL comprises the amino acid sequence of SEQ ID NO:78;
n. the VH comprises the amino acid sequence of SEQ ID NO:66 and the VL comprises the amino acid sequence of SEQ ID NO:78;
o. the VH comprises the amino acid sequence of SEQ ID NO:68 and the VL comprises the amino acid sequence of SEQ ID NO:78;
p. the VH comprises the amino acid sequence of SEQ ID NO:72 and the VL comprises the amino acid sequence of SEQ ID NO:78;
q. the VH comprises the amino acid sequence of SEQ ID NO:55 and the VL comprises the amino acid sequence of SEQ ID NO:79;
r. the VH comprises the amino acid sequence of SEQ ID NO:63 and the VL comprises the amino acid sequence of SEQ ID NO:79;
s. the VH comprises the amino acid sequence of SEQ ID NO:66 and the VL comprises the amino acid sequence of SEQ ID NO:79;
t. the VH comprises the amino acid sequence of SEQ ID NO:68 and the VL comprises the amino acid sequence of SEQ ID NO:79;
u. the VH comprises the amino acid sequence of SEQ ID NO:72 and the VL comprises the amino acid sequence of SEQ ID NO:79; or
the VH comprises the amino acid sequence of SEQ ID NO:75 and the VL comprises the amino acid sequence of SEQ ID NO:80.

9. An agonist antigen binding protein that competes with the antigen binding protein of any one of claims 1 to 8 for binding to a human Lecithin-cholesterol acyltransferase polypeptide of SEQ ID NO:1.

10. The agonist antigen binding protein of claim 9, that
a. competes with an antigen binding protein comprising: a CDRH1 of SEQ ID NO:81, a CDRH2 of SEQ ID NO:93 a CDRH3 of SEQ ID NO:95, a CDRL1 of SEQ ID NO:112, a CDRL2 of SEQ ID NO:114, and a CDRL3 of SEQ ID NO:116;
b. competes with an antigen binding protein comprising: a CDRH1 of SEQ ID NO:92, a CDRH2 of SEQ ID NO:94, a CDRH3 of SEQ ID NO:111, a CDRL1 of SEQ ID NO:113, a CDRL2 of SEQ ID NO:115, and a CDRL3 of SEQ ID NO:120;
c. competes with an antigen binding protein comprising a VH of SEQ ID NO:45 and a VL of SEQ ID NO:76; or
d. competes with an antigen binding protein comprising a VH of SEQ ID NO:75 and a VL of SEQ ID NO:80.

11. The antigen binding protein of any one of claims 1 to 3 that binds to amino acids located within a region of an Lecithin-cholesterol acyltransferase polypeptide consisting of **SEQ ID NO:1**, wherein
a) the region is from amino acid 255 to amino acid 389 of **SEQ ID NO:1**, inclusive; or
b) wherein the region is from amino acid 255 to amino acid 374 of **SEQ ID NO:1**, inclusive.

12. The antigen binding protein of claim 11 (b) that binds to amino acids within one or more of the following regions:
I.
a. the region from amino acid 255 to amino acid 262 of **SEQ ID NO:1**, inclusive;
b. the region from amino acid 315 to amino acid 321 of **SEQ ID NO:1**, inclusive; and
c. the region from amino acid 341 to amino acid 374 of **SEQ ID NO:1**, inclusive; or.
II.
a. the region from amino acid 255 to amino acid 262 of **SEQ ID NO:1**, inclusive;
b. the region from amino acid 315 to amino acid 321 of **SEQ ID NO:1**, inclusive;
c. the region from amino acid 341 to amino acid 343 of **SEQ ID NO:1**, inclusive; and
d. the region from amino acid 350 to amino acid 374 of **SEQ ID NO:1**, inclusive.

13. The antigen binding protein of claim 12 (I) that binds to amino acids with regions (a), (b) and (c).

14. The antigen binding protein of claim 12 (II) that binds to amino acids within regions (a), (b), (c) and (d).

15. The antigen binding protein of claim 11 (a) that binds to
I.
(i) at least 12 of the amino acids selected from the group consisting of S255, R256, M257, A258, W259, P260, Y315, V317, G318, L319, P320, T321, Y341, E342, D343, T350, R351, E354, L355, C356, G357, L358, Q360, R362, V367, H368, L369, P371, H373 and G374 of **SEQ ID NO:1**, or
(ii) to amino acids S255, R256, M257, A258, W259, P260, Y315, V317, G318, L319, P320, T321, Y341, E342, D343, T350, R351, E354, L355, C356, G357, L358, Q360, R362, V367, H368, L369, P371, H373, and G374 of **SEQ ID NO:1**; or
II.
(i) to at least 15 of the amino acids selected from the group consisting of amino acids R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 and H389 of **SEQ ID NO:1**; or
(ii) to amino acids R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 and H389 of **SEQ ID NO:1.**

16. The antigen binding protein of any one of claims 1 to 3 that binds to amino acids located within a region of an Lecithin-cholesterol acyltransferase polypeptide consisting of **SEQ ID NO:1**, wherein
a. the region is from amino acid 315 to amino acid 399, inclusive, or
b. the region is from amino acid 343 to amino acid 399, inclusive.

17. The antigen binding protein of claim 16 that binds to amino acids within one or more of the following regions:
I.
a. the region from amino acid 350 to amino acid 375 of **SEQ ID NO:1**, inclusive;
b. the region from amino acid 385 to amino acid 399 of **SEQ ID NO:1**, inclusive; or
II.
a. the region from amino acid 315 to amino acid 317 of **SEQ ID NO:1**, inclusive;
b. the region from amino acid 350 to amino acid 375 of **SEQ ID NO:1**, inclusive; and
c. the region from amino acid 385 to amino acid 399 of **SEQ ID NO:1**, inclusive.

18. The antigen binding protein of claim 17 (I) that binds to amino acids within regions (a) and (b).

19. The antigen binding protein of claim 17 (II) that binds to amino acids within regions (a), (b) and (c).

20. The antigen binding protein of claim 16 that binds to
I.
(i) at least 12 of the amino acids selected from the group consisting of Y315, V317, D343, T350, R351, E354, G357, Q360, G361, Q365, P366, V367, H368, L369, L370, P371, L372, H373, I375, L385, E388, H389, A392, L395, G396, A397, Y398 and R399 of **SEQ ID NO:1**, or
(ii) to amino acids Y315, V317, D343, T350, R351, E354, G357, Q360, G361, Q365, P366, V367, H368, L369, L370, P371, L372, H373, I375, L385, E388, H389, A392, L395, G396, A397, Y398 and R399 of **SEQ ID NO:1**; or
II.
(i) at least 12 of the amino acids selected from the group consisting of Y315, V317, E342, D343, T350, R351, E354, G357, Q360, G361, P364, P366, V367, H368, L369, L370, P371, L372, H373, L385, E388, H389, A392, L395, G396, A397, Y398 and R399 of **SEQ ID NO:1**, or
(ii) to amino acids Y315, V317, E342, D343, T350, R351, E354, G357, Q360, G361, P364, P366, V367, H368, L369, L370, P371, L372, H373, L385, E388, H389, A392, L395, G396, A397, Y398 and R399 of **SEQ ID NO:1.**

21. The antigen binding protein of any one of claims 1 to 3 that binds to an epitope, wherein the epitope is located within
a. amino acid 255 to amino acid 389 of **SEQ ID NO:1**;
b. amino acids 315 to amino acid 399 of **SEQ ID NO:1**; or
c. amino acids 342 to amino acid 399 of **SEQ ID NO:1.**

22. The antigen binding protein of claim 21, wherein the epitope is a conformational and/or discontinuous epitope.

23. The antigen binding protein of any one of claims 1 to 21, wherein the antigen binding protein has one or more of the following characteristics:
a. is a monoclonal antibody, a human antibody, a humanized antibody, a chimeric antibody, or a multispecific antibody;
b. is of the IgG1, IgG2, IgG3, or the IgG4 type;
c. is a Fab fragment, a Fab' fragment, a F(ab')2 fragment, or an Fv fragment;
d. is a diabody, a single chain antibody, a domain antibody, or a nanobody;
e. is derived from a mammalian source selected from mouse, rat, camelid or rabbit; or
f. is labeled.

24. A pharmaceutical composition comprising at least one antigen binding protein according to any one of claims 1 to 23.

25. The pharmaceutical composition of claim 24 further comprising a further active agent selected from the group consisting of an agent used to treat cardiovascular disease, an agent used to treat cholesterol-related disorders, an agent used to treat atherosclerosis, an anti-inflammatory agent, an anti-thrombosis agent, an anti-diabetic agent and a cytokine.

26. A nucleic acid molecule encoding the antigen binding protein of any one of claims 1 to 23.

27. A method of making the antigen binding protein according to any one of claims 1 to 23, the method comprising preparing the antigen binding protein from a host cell that secretes the antigen binding protein.

28. The antigen binding protein of any one of claims 1 to 23 or the pharmaceutical composition of claims 24 or 25 for use in therapy.

29. The antigen binding protein of any one of claims 1 to 23 or the pharmaceutical composition of claims 24 or 25 for use in
a) decreasing the cholesterol level in a subject or increasing HDL-C serum levels in a subject; or
b) treating or preventing a condition selected from the group consisting of a cholesterol-related disorder, a cardiovascular disease, an inflammatory condition, a thrombosis-related condition, a blood disorder, and a condition associated with Lecithin-cholesterol acyltransferase deficiency.

30. The antigen binding protein of any one of claims 1 to 23 or the pharmaceutical composition of claims 24 or 25 for use according to claims 28 or 29, wherein
a. the cardiovascular disease or cholesterol related disorder is selected from the group consisting of arteriosclerosis, atherosclerosis, stroke, ischemia, peripheral vascular disease, coronary artery disease, coronary heart disease, myocardial infarction, high blood pressure, hypercholerolemia, metabolic syndrome, dyslipidemia, diabetes and insulin-resistance;
b. the inflammatory disease is selected from the group consisting of arthritis, sepsis, septic shock, asthma, a chronic pulmonary inflammatory disease, and an inflammatory bowel disease; and
c. the thrombosis related condition is selected from the group consisting of a myocardial infarction, deep vein thrombosis, embolism, thrombocytopic purpura and microangiopathy.

31. The antigen binding protein of any one of claims 1 to 23 or the pharmaceutical composition of claims 24 or 25 for use according to claims 28 or 29, wherein the antigen binding protein or pharmaceutical composition is administered in combination with a further active agent selected from the group consisting of a statin, a CETP inhibitor, a cardiovascular agent, a cholesterol lowering agent, an ACE inhibitor, an ACAT inhibitor, an aldosterone antagonist, an alpha blocker, an angiotensin II receptor antagonist, an anti-arrhythmic agent, an apoA-1 agent, a beta blocker, a bile acid sequesterant, a calcium-channel blocker, a dyslipidemia agent, and endothelin receptor antagonist, an Lecithin-cholesterol acyltransferase activator, a fibrate, an PPAR agonist, a squalene epoxidase inhibitor, an anti-inflammatory agent, an anti-thrombosis agent, an anti-diabetic agent and a cytokine.

## Patentansprüche

1. Ein agonistisches antigenbindendes Protein, das spezifisch an ein humanes Lecithin-Cholesterin Acyltransferase Polypeptid bindet, wobei das antigenbindende Protein ein humaner Antikörper ist.

2. Das antigenbindende Protein gemäß Anspruch 1, das ein humanes Lecithin-Cholesterin Acyltransferase Polypeptid bestehend aus SEQ ID NO: 1 bindet.

3. Das antigenbindende Protein gemäß der Ansprüche 1 oder 2, wobei das antigenbindende Protein eine oder mehrere der folgenden Eigenschaften besitzt:
a) bindet an das humane Lecithin-Cholesterin Acyltransferase Polypeptid von SEQ ID NO: 1 mit einer KD von weniger als 50 nM, wie durch BiaCore bestimmt; und
b) bindet an humaner Lecithin-Cholesterin Acyltransferase mit einer KD von weniger als 50 nM und cyno Lecithin-Cholesterin Acyltransferase mit einer KD von weniger als 500 nM, wie durch BiaCore bestimmt.

4. Das antigenbindende Protein gemäß irgendeinem der Ansprüche 1 bis 3, wobei das antigenbindende Protein umfasst:
I.
a. eine oder mehrere Complementary Determining Regionen einer schweren Kette (CDRHs) ausgewählt aus der Gruppe bestehend aus:
i. eine CDRH1 ausgewählt aus der Gruppe bestehend aus SEQ ID NO: 81-92;
ii. eine CDRH2 ausgewählt aus der Gruppe bestehend aus SEQ ID NO: 93-94;
iii. eine CDRH3 ausgewählt aus der Gruppe bestehend aus SEQ ID NO: 95-111;
iv. eine CDRH von (i), (ii), (iii), die eine oder mehrere Aminosäure Substitutionen, Deletionen oder Insertionen in insgesamt nicht mehr als 4 Aminosäuren beinhaltet;
b. eine oder mehrere Complementary Determining Regionen einer leichten Kette (CDRL) ausgewählt aus der Gruppe bestehend aus:
i. eine CDRL1 ausgewählt aus der Gruppe bestehend aus SEQ ID NO: 112-113;
ii. eine CDRL2 ausgewählt aus der Gruppe bestehend aus SEQ ID NO: 114-115;
iii. eine CDRL3 ausgewählt aus der Gruppe bestehend aus SEQ ID NO: 116-120;
iv. eine CDRL von (i), (ii), (iii), die eine oder mehrere Aminosäure Substitutionen, Deletionen oder Insertionen in insgesamt nicht mehr als 4 Aminosäuren beinhaltet; oder
c. eine oder mehrere CDRHs von (a) und eine oder mehrere CDRLs von (b), oder
II. eine variable Region einer schweren Kette (VH) und/oder eine variable Region einer leichten Kette (VL), wobei
(i)
a. die VH mindestens 90% Sequenzidentität zur Aminosäuresequenz ausgewählt aus der Gruppe bestehend aus SEO ID NO. 45-75 aufweist; und
b. die VL mindestens 90% Sequenzidentität zur Aminosäuresequenz ausgewählt aus der Gruppe bestehend aus SEQ ID NO: 76-80 aufweist; oder
(ii)
a. die VH mindestens 90% Sequenzidentität zur Aminosäuresequenz von SEQ ID NO: 45 aufweist und die VL mindestens 90% Sequenzidentität zur Aminosäuresequenz von SEQ ID NO: 76 aufweist; oder
b. die VH mindestens 90% Sequenzidentität zur Aminosäuresequenz von SEQ ID NO: 75 aufweist und die VL mindestens 90% Sequenzidentität zur Aminosäuresequenz von SEQ ID NO: 80 aufweist; oder
III: eine schwere Kette (HC) und eine leichte Kette (LC), wobei
(i)
a. die HC mindestens 90% Sequenzidentität zur Aminosäuresequenz von SEQ ID NO: 9 aufweist und die LC mindestens 90% Sequenzidentität zur Aminosäuresequenz von SEQ ID NO: 40 aufweist; oder
b. die HC mindestens 90% Sequenzidentität zur Aminosäuresequenz von SEQ ID NO: 39 aufweist und die LC mindestens 90% Sequenzidentität zur Aminosäuresequenz von SEQ ID NO: 44 aufweist; oder
(ii)
a. die HC umfasst die Aminosäuresequenz von SEQ ID NO: 9 und die LC umfasst die Aminosäuresequenz von SEQ ID NO: 40;
b. die HC umfasst die Aminosäuresequenz von SEQ ID NO: 19 und die LC umfasst die Aminosäuresequenz von SEQ ID NO: 40;
c. die HC umfasst die Aminosäuresequenz von SEQ ID NO: 27 und die LC umfasst die Aminosäuresequenz von SEQ ID NO: 40;
d. die HC umfasst die Aminosäuresequenz von SEQ ID NO: 30 und die LC umfasst die Aminosäuresequenz von SEQ ID NO: 40;
e. die HC umfasst die Aminosäuresequenz von SEQ ID NO: 32 und die LC umfasst die Aminosäuresequenz von SEQ ID NO: 40;
f. die HC umfasst die Aminosäuresequenz von SEQ ID NO: 36 und die LC umfasst die Aminosäuresequenz von SEQ ID NO: 40;
g. die HC umfasst die Aminosäuresequenz von SEQ ID NO: 19 und die LC umfasst die Aminosäuresequenz von SEQ ID NO: 41;
h. die HC umfasst die Aminosäuresequenz von SEQ ID NO: 27 und die LC umfasst die Aminosäuresequenz von SEQ ID NO: 41;
i. die HC umfasst die Aminosäuresequenz von SEQ ID NO: 30 und die LC umfasst die Aminosäuresequenz von SEQ ID NO: 41;
j. die HC umfasst die Aminosäuresequenz von SEQ ID NO: 32 und die LC umfasst die Aminosäuresequenz von SEQ ID NO: 41;
k. die HC umfasst die Aminosäuresequenz von SEQ ID NO: 36 und die LC umfasst die Aminosäuresequenz von SEQ ID NO: 41;
l. die HC umfasst die Aminosäuresequenz von SEQ ID NO: 19 und die LC umfasst die Aminosäuresequenz von SEQ ID NO: 42;
m. die HC umfasst die Aminosäuresequenz von SEQ ID NO: 27 und die LC umfasst die Aminosäuresequenz von SEQ ID NO: 42;
n. die HC umfasst die Aminosäuresequenz von SEQ ID NO: 30 und die LC umfasst die Aminosäuresequenz von SEQ ID NO: 42;
o. die HC umfasst die Aminosäuresequenz von SEQ ID NO: 32 und die LC umfasst die Aminosäuresequenz von SEQ ID NO: 42;
p. die HC umfasst die Aminosäuresequenz von SEQ ID NO: 36 und die LC umfasst die Aminosäuresequenz von SEQ ID NO: 42;
q. die HC umfasst die Aminosäuresequenz von SEQ ID NO: 19 und die LC umfasst die Aminosäuresequenz von SEQ ID NO: 43;
r. die HC umfasst die Aminosäuresequenz von SEQ ID NO: 27 und die LC umfasst die Aminosäuresequenz von SEQ ID NO: 43;
s. die HC umfasst die Aminosäuresequenz von SEQ ID NO: 30 und die LC umfasst die Aminosäuresequenz von SEQ ID NO: 43;
t. die HC umfasst die Aminosäuresequenz von SEQ ID NO: 32 und die LC umfasst die Aminosäuresequenz von SEQ ID NO: 43;
u. die HC umfasst die Aminosäuresequenz von SEQ ID NO: 36 und die LC umfasst die Aminosäuresequenz von SEQ ID NO: 43; oder
die HC umfasst die Aminosäuresequenz von SEQ ID NO: 39 und die LC umfasst die Aminosäuresequenz von SEQ ID NO: 44.

5. Das antigenbindende Protein gemäß Anspruch 4 (I), dass eine CDRH3 und eine CDRL3 umfasst.

6. Das antigenbindende Protein gemäß Anspruch 5, wobei
a. die CDRH3 SEQ ID NO: 95 umfasst und die CDRL3 SEQ ID NO: 116 umfasst; oder
b. die CDRH3 SEQ ID NO: 111 umfasst und die CDRL3 SEQ ID NO: 120 umfasst.

7. Das isolierte antigenbindende Protein gemäß irgendeinem der Ansprüche 1 bis 3, wobei das besagte antigenbindende Protein umfasst:
I.
a. eine CDRH ausgewählt aus der Gruppe bestehend aus
i. einer CDRH1 ausgewählt aus der Gruppe bestehend aus SEQ ID NO: 81-92;
ii. einer CDRH2 ausgewählt aus der Gruppe bestehend aus SEQ ID NO: 93-94; und
iii. einer CDRH3 ausgewählt aus der Gruppe bestehend aus SEQ ID NO: 95-111;
b. einer CDRL ausgewählt aus der Gruppe bestehend aus
i. einer CDRL1 ausgewählt aus der Gruppe bestehend aus SEQ ID NO: 112-113;
ii. einer CDRL2 ausgewählt aus der Gruppe bestehend aus SEO ID NO: 114-115; und
iii. einer CDRL3 ausgewählt aus der Gruppe bestehend aus SEQ ID NO: 116-120; oder
c. eine oder mehrere CDRHs von (a) und eine oder mehrere CDRLs von (b); oder
II.
a. eine CDRH1 von SEQ ID NO: 81, eine CDRH2 von SEQ ID NO: 93, eine CDRH3 von SEQ ID NO: 95, eine CDRL1 von SEQ ID NO: 112, eine CDRL2 von SEQ ID NO: 114, und eine CDRL3 von SEQ ID NO: 116;
b. eine CDRH1 von SEQ ID NO: 91, eine CDRH2 von SEQ ID NO: 93, eine CDRH3 von SEQ ID NO: 95, eine CDRL1 von SEQ ID NO: 112, eine CDRL2 von SEO ID NO: 114, und eine CDRL3 von SEQ ID NO: 116;
c. eine CDRH1 von SEQ ID NO: 91, eine CDRH2 von SEQ ID NO: 93, eine CDRH3 von SEQ ID NO: 99, eine CDRL1 von SEQ ID NO: 112, eine CDRL2 von SEQ ID NO: 114, und eine CDRL3 von SEQ ID NO: 116;
d. eine CDRH1 von SEQ ID NO: 91, eine CDRH2 von SEQ ID NO: 93, eine CDRH3 von SEQ ID NO: 102, eine CDRL1 von SEQ ID NO: 112, eine CDRL2 von SEO ID NO: 114, und eine CDRL3 von SEQ ID NO: 116;
e. eine CDRH1 von SEQ ID NO: 91, eine CDRH2 von SEO ID NO: 93, eine CDRH3 von SEQ ID NO: 104, eine CDRL1 von SEQ ID NO: 112, eine CDRL2 von SEQ ID NO: 114, und eine CDRL3 von SEQ ID NO: 116;
f. eine CDRH1 von SEQ ID NO: 91, eine CDRH2 von SEQ ID NO: 93, eine CDRH3 von SEQ ID NO: 108, eine CDRL1 von SEQ ID NO: 112, eine CDRL2 von SEQ ID NO: 114, und eine CDRL3 von SEQ ID NO: 116;
g. eine CDRH1 von SEQ ID NO: 91, eine CDRH2 von SEQ ID NO: 93, eine CDRH3 von SEQ ID NO: 95, eine CDRL1 von SEQ ID NO: 112, eine CDRL2 von SEQ ID NO: 114, und eine CDRL3 von SEQ ID NO: 117;
h. eine CDRH1 von SEQ ID NO: 91, eine CDRH2 von SEQ ID NO: 93, eine CDRH3 von SEQ ID NO: 99, eine CDRL1 von SEQ ID NO: 112, eine CDRL2 von SEQ ID NO: 114, und eine CDRL3 von SEQ ID NO: 117;
i. eine CDRH1 von SEQ ID NO: 91, eine CDRH2 von SEQ ID NO: 93, eine CDRH3 von SEO ID NO: 102, eine CDRL1 von SEQ ID NO: 112, eine CDRL2 von SEQ ID NO: 114, und eine CDRL3 von SEQ ID NO: 117;
j. eine CDRH1 von SEQ ID NO: 91, eine CDRH2 von SEQ ID NO: 93, eine CDRH3 von SEQ ID NO: 104, eine CDRL1 von SEQ ID NO: 112, eine CDRL2 von SEQ ID NO: 114, und eine CDRL3 von SEQ ID NO: 117;
k. eine CDRH1 von SEQ ID NO: 91, eine CDRH2 von SEQ ID NO: 93, eine CDRH3 von SEQ ID NO: 108, eine CDRL1 von SEQ ID NO: 112, eine CDRL2 von SEQ ID NO: 114, und eine CDRL3 von SEQ ID NO: 117;
l. eine CDRH1 von SEQ ID NO: 91, eine CDRH2 von SEQ ID NO: 93, eine CDRH3 von SEQ ID NO: 95, eine CDRL1 von SEQ ID NO: 112, eine CDRL2 von SEQ ID NO: 114, und eine CDRL3 von SEQ ID NO: 118;
m. eine CDRH1 von SEQ ID NO: 91, eine CDRH2 von SEQ ID NO: 93, eine CDRH3 von SEQ ID NO: 99, eine CDRL1 von SEQ ID NO: 112, eine CDRL2 von SEQ ID NO: 114, und eine CDRL3 von SEQ ID NO: 118;
n. eine CDRH1 von SEQ ID NO: 91, eine CDRH2 von SEQ ID NO: 93, eine CDRH3 von SEQ ID NO: 102, eine CDRL1 von SEQ ID NO: 112, eine CDRL2 von SEQ ID NO: 114, und eine CDRL3 von SEQ ID NO: 118;
o. eine CDRH1 von SEQ ID NO: 91, eine CDRH2 von SEQ ID NO: 93, eine CDRH3 von SEQ ID NO: 104, eine CDRL1 von SEQ ID NO: 112, eine CDRL2 von SEO ID NO: 114, und eine CDRL3 von SEQ ID NO: 118;
p. eine CDRH1 von SEQ ID NO: 91, eine CDRH2 von SEQ ID NO: 93, eine CDRH3 von SEQ ID NO: 108, eine CDRL1 von SEQ ID NO: 112, eine CDRL2 von SEQ ID NO: 114, und eine CDRL3 von SEQ ID NO: 118;
q. eine CDRH1 von SEQ ID NO: 91, eine CDRH2 von SEQ ID NO: 93, eine CDRH3 von SEQ ID NO: 95, eine CDRL1 von SEQ ID NO: 112, eine CDRL2 von SEQ ID NO: 114, und eine CDRL3 von SEQ ID NO: 119;
r. eine CDRH1 von SEQ ID NO: 91, eine CDRH2 von SEQ ID NO: 93, eine CDRH3 von SEQ ID NO: 99, eine CDRL1 von SEQ ID NO: 112, eine CDRL2 von SEQ ID NO: 114, und eine CDRL3 von SEQ ID NO: 119;
s. eine CDRH1 von SEQ ID NO: 91, eine CDRH2 von SEQ ID NO: 93, eine CDRH3 von SEQ ID NO: 102, eine CDRL1 von SEQ ID NO: 112, eine CDRL2 von SEQ ID NO: 114, und eine CDRL3 von SEQ ID NO: 119;
t. eine CDRH1 von SEQ ID NO: 91, eine CDRH2 von SEQ ID NO: 93, eine CDRH3 von SEQ ID NO: 104, eine CDRL1 von SEQ ID NO: 112, eine CDRL2 von SEQ ID NO: 114, und eine CDRL3 von SEQ ID NO: 119;
u. eine CDRH1 von SEQ ID NO: 91, eine CDRH2 von SEQ ID NO: 93, eine CDRH3 von SEQ ID NO: 108, eine CDRL1 von SEQ ID NO: 112, eine CDRL2 von SEQ ID NO: 114, und eine CDRL3 von SEQ ID NO: 119; oder
eine CDRH1 von SEQ ID NO: 92, eine CDRH2 von SEQ ID NO: 94, eine CDRH3 von SEQ ID NO: 111, eine CDRL1 von SEQ ID NO: 113, eine CDRL2 von SEQ ID NO: 115, und eine CDRL3 von SEQ ID NO: 120.

8. Das antigenbindende Protein gemäß Anspruch 4 (II) (i), wobei das besagte antigenbindende Protein eine variable Region einer schweren Kette (VH) und eine variable Region einer leichten Kette (VL) umfasst, wobei
a. die VH die Aminosäuresequenz von SEQ ID NO: 45 umfasst und die VL die Aminosäuresequenz von SEQ ID NO: 76 umfasst;
b. die VH die Aminosäuresequenz von SEQ ID NO: 55 umfasst und die VL die Aminosäuresequenz von SEQ ID NO: 76 umfasst;
c. die VH die Aminosäuresequenz von SEQ ID NO: 63 umfasst und die VL die Aminosäuresequenz von SEQ ID NO: 76 umfasst;
d. die VH die Aminosäuresequenz von SEQ ID NO: 66 umfasst und die VL die Aminosäuresequenz von SEQ ID NO: 76 umfasst;
e. die VH die Aminosäuresequenz von SEQ ID NO: 68 umfasst und die VL die Aminosäuresequenz von SEQ ID NO: 76 umfasst;
f. die VH die Aminosäuresequenz von SEQ ID NO: 72 umfasst und die VL die Aminosäuresequenz von SEQ ID NO: 76 umfasst;
g. die VH die Aminosäuresequenz von SEQ ID NO: 55 umfasst und die VL die Aminosäuresequenz von SEQ ID NO: 77 umfasst;
h. die VH die Aminosäuresequenz von SEQ ID NO: 63 umfasst und die VL die Aminosäuresequenz von SEQ ID NO: 77 umfasst;
i. die VH die Aminosäuresequenz von SEQ ID NO: 66 umfasst und die VL die Aminosäuresequenz von SEQ ID NO: 77 umfasst;
j. die VH die Aminosäuresequenz von SEQ ID NO: 68 umfasst und die VL die Aminosäuresequenz von SEQ ID NO: 77 umfasst;
k. die VH die Aminosäuresequenz von SEQ ID NO: 72 umfasst und die VL die Aminosäuresequenz von SEQ ID NO: 77 umfasst;
l. die VH die Aminosäuresequenz von SEQ ID NO: 55 umfasst und die VL die Aminosäuresequenz von SEQ ID NO: 78 umfasst;
m. die VH die Aminosäuresequenz von SEQ ID NO: 63 umfasst und die VL die Aminosäuresequenz von SEQ ID NO: 78 umfasst;
n. die VH die Aminosäuresequenz von SEQ ID NO: 66 umfasst und die VL die Aminosäuresequenz von SEQ ID NO: 78 umfasst;
o. die VH die Aminosäuresequenz von SEQ ID NO: 68 umfasst und die VL die Aminosäuresequenz von SEQ ID NO: 78 umfasst;
p. die VH die Aminosäuresequenz von SEQ ID NO: 72 umfasst und die VL die Aminosäuresequenz von SEQ ID NO: 78 umfasst;
q. die VH die Aminosäuresequenz von SEQ ID NO: 55 umfasst und die VL die Aminosäuresequenz von SEQ ID NO: 79 umfasst;
r. die VH die Aminosäuresequenz von SEQ ID NO: 63 umfasst und die VL die Aminosäuresequenz von SEQ ID NO: 79 umfasst;
s. die VH die Aminosäuresequenz von SEQ ID NO: 66 umfasst und die VL die Aminosäuresequenz von SEQ ID NO: 79 umfasst;
t. die VH die Aminosäuresequenz von SEQ ID NO: 68 umfasst und die VL die Aminosäuresequenz von SEQ ID NO: 79 umfasst;
u. die VH die Aminosäuresequenz von SEQ ID NO: 72 umfasst und die VL die Aminosäuresequenz von SEQ ID NO: 79 umfasst; oder
die VH die Aminosäuresequenz von SEQ ID NO: 75 umfasst und die VL die Aminosäuresequenz von SEQ ID NO: 80 umfasst.

9. Ein agonistisches antigenbindendes Protein das mit dem antigenbindenden Protein gemäß irgendeinem der Ansprüche 1 bis 8 um die Bindung an ein humanes Lecithin-Cholesterin Acyltransferase Polypeptid der SEQ ID NO: 1 konkurriert.

10. Das agonistische antigenbindende Protein gemäß Anspruch 9, das
a. mit einem antigenbindenden Protein umfassend einer CDRH1 von SEQ ID NO: 81, einer CDRH2 von SEQ ID NO: 93, einer CDRH3 von SEQ ID NO: 95, einer CDRL1 von SEQ ID NO: 112, einer CDRL2 von SEQ ID NO: 114 und einer CDRL3 von SEQ ID NO: 116 konkurriert;
b. mit einem antigenbindenden Protein umfassend einer CDRH1 von SEQ ID NO: 92, einer CDRH2 von SEQ ID NO: 94, einer CDRH3 von SEQ ID NO: 111, einer CDRL1 von SEQ ID NO: 113, einer CDRL2 von SEQ ID NO: 115 und einer CDRL3 von SEQ ID NO: 120 konkurriert;
c. mit einem antigenbindenden Protein umfassend einer VH von SEQ ID NO: 45 und einer VL von SEQ ID NO: 76 konkurriert; oder
d. mit einem antigenbindenden Protein umfassend einer VH von SEQ ID NO: 75 und einer VL von SEO ID NO: 80 konkurriert.

11. Das antigenbindende Protein gemäß irgendeinem der Ansprüche 1 bis 3, das die Aminosäuren bindet, die innerhalb einer Region eines Lecithin-Cholesterin Acyltransferase Polypeptids bestehend aus SEQ ID NO: 1 lokalisiert sind, wobei
a. die Region von Aminosäure 255 bis Aminosäure 389 von SEQ ID NO: 1 reicht, inklusive; öder
b. wobei die Region von Aminosäure 255 bis Aminosäure 374 von SEQ ID NO: 1 reicht, inklusive.

12. Das antigenbindende Protein gemäß Anspruch 11 (b), das an die Aminosäuren innerhalb einer oder mehrerer der folgenden Regionen bindet:
I.
a. die Region von Aminosäure 255 bis Aminosäure 262 von SEQ ID NO: 1, inklusive;
b. die Region von Aminosäure 315 bis Aminosäure 321 von SEQ ID NO: 1, inklusive; und
c. die Region von Aminosäure 341 bis Aminosäure 374 von SEQ ID NO: 1, inklusive; oder
II.
a. die Region von Aminosäure 255 bis Aminosäure 262 von SEQ ID NO: 1, inklusive;
b. die Region von Aminosäure 315 bis Aminosäure 321 von SEQ ID NO: 1, inklusive;
c. die Region von Aminosäure 341 bis Aminosäure 343 von SEQ ID NO: 1, inklusive; und
d. die Region von Aminosäure 350 bis Aminosäure 374 von SEQ ID NO: 1, inklusive.

13. Das antigenbindende Protein gemäß Anspruch 12 (I), das an die Aminosäuren der Regionen (a), (b) und (c) bindet.

14. Das antigenbindende Protein gemäß Anspruch 12 (II), das an die Aminosäuren innerhalb der Regionen (a), (b), (c) und (d) bindet.

15. Das antigenbindende Protein gemäß Anspruch 11 (a), das an
I.
(i) mindestens 12 Aminosäuren ausgewählt aus der Gruppe bestehend aus S255, R256, M257, A258, W259, P260, Y315, V317, G318, L319, P320, T321, Y341, E342, D343, T350, R351, E354, L355, C356, G357, L358, Q360, R362, V367, H368, L369, P371, H373 und G374 von SEQ ID NO: 1; oder
(ii) die Aminosäuren S255, R256, M257, A258, W259, P260, Y315, V317, G318, L319, P320, T321, Y341, E342, D343, T350, R351, E354, L355, C356, G357, L358, Q360, R362, V367, H368, L369, P371, H373, und G374 von SEQ ID NO: 1; oder
II.
(i) mindestens 15 Aminosäuren ausgewählt aus der Gruppe bestehend aus R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 und H389 von SEQ ID NO: 1; oder
(ii) die Aminosäuren R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 und H389 von SEQ ID NO: 1 bindet.

16. Das antigenbindende Protein gemäß irgendeinem der Ansprüche 1 bis 3, das an Aminosäuren bindet, die innerhalb einer Region eines Lecithin-Cholesterin Acyltransferase Polypeptids bestehend aus SEQ ID NO: 1 lokalisiert sind, wobei
a. die Region von Aminosäure 315 bis Aminosäure 399 reicht, inklusive, oder
b. die Region von Aminosäure 343 bis Aminosäure 399 reicht, inklusive.

17. Das antigenbindende Protein gemäß Anspruch 16, das an die Aminosäuren innerhalb einer oder mehrerer der folgenden Regionen bindet:
I.
a. die Region von Aminosäure 350 bis Aminosäure 375 von SEQ ID NO: 1, inklusive;
b. die Region von Aminosäure 385 bis Aminosäure 399 von SEQ ID NO: 1, inklusive; oder
II.
a. die Region von Aminosäure 315 bis Aminosäure 317 von SEQ ID NO: 1, inklusive;
b. die Region von Aminosäure 350 bis Aminosäure 375 von SEQ ID NO: 1, inklusive; und
c. die Region von Aminosäure 385 bis Aminosäure 399 von SEQ ID NO: 1, inklusive.

18. Das antigenbindende Protein gemäß Anspruch 17 (I), das an Aminosäuren innerhalb der Regionen (a) und (b) bindet.

19. Das antigenbindende Protein gemäß Anspruch 17 (II), das an Aminosäuren innerhalb der Regionen (a), (b) und (c) bindet.

20. Das antigenbindende Protein gemäß Anspruch 16, das an
I.
(i) mindestens 12 Aminosäuren ausgewählt aus der Gruppe bestehend aus Y315, V317, D343, T350, R351, E354, G357, Q360, G361, Q365, P366, V367, H368, L369, L370, P371, L372, H373, I375, L385, E388, H389, A392, L395, G396, A397, Y398 und R399 von SEQ ID NO: 1, oder
(ii) Aminosäuren Y315, V317, D343, T350, R351, E354, G357, Q360, G361, Q365, P366, V367, H368, L369, L370, P371, L372, H373, I375, L385, E388, H389, A392, L395, G396, A397, Y398 und R399 von SEQ ID NO: 1; oder
II.
(i) mindestens 12 Aminosäuren ausgewählt aus der Gruppe bestehend aus Y315, V317, E342, D343, T350, R351, E354, G357, Q360, G361, P364, P366, V367, H368, L369, L370, P371, L372, H373, L385, E388, H389, A392, L395, G396, A397, Y398 und R399 von SEQ ID NO: 1, oder
(ii) Aminosäuren Y315, V317, E342, D343, T350, R351, E354, G357, Q360, G361, P364, P366, V367, H368, L369, L370, P371, L372, H373, L385, E388, H389, A392, L395, G396, A397, Y398 und R399 von SEQ ID NO: 1 bindet.

21. Das antigenbindende Protein gemäß irgendeinem der Ansprüche 1 bis 3, das an ein Epitop bindet, wobei das Epitop zwischen
a. Aminosäure 255 bis Aminosäure 389 von SEQ ID NO: 1;
b. Aminosäure 315 bis Aminosäure 399 von SEQ ID NO: 1; oder
c. Aminosäure 342 bis Aminosäure 399 von SEQ ID NO: 1 lokalisiert ist.

22. Das antigenbindende Protein gemäß Anspruch 21, wobei das Epitop ein konformatives und/oder ein diskontinuierliches Epitop ist.

23. Das antigenbindende Protein gemäß irgendeinem der Ansprüche 1 bis 21, wobei das antigenbindende Protein eine oder mehrere der folgenden Eigenschaften besitzt:
a. ist ein monoklonaler Antikörper, ein humaner Antikörper, ein humanisierter Antikörper, ein chimärer Antikörper, öder ein multispezifischer Antikörper;
b. ist vom IgG1, IgG2, IgG3 oder vom IgG4 Typ;
c. ist ein Fab Fragment, ein Fab' Fragment, ein(Fab')2 Fragment, oder ein Fv Fragment;
d. ist ein Diabody, ein einzelkettiger Antikörper, ein Domänen-Antikörper, oder ein Nanobody;
e. ist von einem Säugetier Ursprung ausgewählt von Maus, Ratte, Kamel oder Hase, oder
f. ist markiert.

24. Eine pharmazeutische Zusammensetzung, die mindestens ein antigenbindendes Protein gemäß irgendeinem der Ansprüche 1-23 umfasst.

25. Die pharmazeutische Zusammensetzung gemäß Anspruch 24, die des Weiteren ein weiteres aktives Agens umfasst, das ausgewählt ist aus der Gruppe bestehend aus einem Agens verwendet zur Behandlung einer kardiovaskulären Erkrankung, einem Agens verwendet zur Behandlung einer Cholesterin-bezogenen Störung, einem Agens verwendet zur Behandlung von Atherosklerose, einem entzündungshemmenden Agens, einem thrombosehemmenden Agens, einem antidiabetischen Agens und einem Zytokin.

26. Ein Nukleinsäuremolekül, das das antigenbindende Protein gemäß irgendeinem der Ansprüche 1 bis 23 kodiert.

27. Ein Verfahren zur Herstellung des antigenbindenden Proteins gemäß irgendeinem der Ansprüche 1 bis 23, das Verfahren umfasst die Bereitstellung des antigenbindenden Proteins aus einer Wirtszelle, die das antigenbindende Protein sezerniert.

28. Das antigenbindende Protein gemäß irgendeinem der Ansprüche 1 bis 23 oder die pharmazeutische Zusammensetzung gemäß der Ansprüche 24 oder 25 zur therapeutischen Verwendung.

29. Das antigenbindende Protein gemäß irgendeinem der Ansprüche 1-23 oder die pharmazeutische Zusammensetzung gemäß der Ansprüche 24 oder 25 zur Verwendung in
a) Verminderung des Cholesterinspiegels in einem Subjekt oder Erhöhung des HDL-C Serumspiegels in einem Subjekt; oder
b) Behandlung oder Vorbeugung eines Leidens ausgewählt aus der Gruppe bestehend aus einer Cholesterin-bezogenen Störung, einer kardiovaskulären Erkrankung, eines entzündlichen Leidens, eines Thrombose-bezogenen Leidens, einer Blutstörung, und einem Leiden, das mit Lecithin-Cholesterin Acyltransferase Mangel assoziiert ist.

30. Das antigenbindende Protein gemäß irgendeinem der Ansprüche 1 bis 23 oder die pharmazeutische Zusammensetzung gemäß der Ansprüche 24 oder 25 zur Verwendung gemäß der Ansprüche 28 oder 29, wobei
a. die kardiovaskuläre Erkrankung oder Cholesterin-bezogene Störung ausgewählt ist aus der Gruppe bestehend aus Arteriosklerose, Atherosklerose, Schlaganfall, Ischämie, peripherere vaskuläre Erkrankung, koronare Gefäßerkrankung, koronare Herzerkrankung, Herzinfarkt, Bluthochdruck, Hypercholesterinämie, metabolisches Syndrom, Dyslipidämie, Diabetis und Insulinresistenz;
b. die entzündliche Erkrankung ausgewählt ist aus der Gruppe bestehend aus Arthritis, Sepsis, septischer Schock, Asthma, chronische entzündliche Lungenerkrankung, und einer entzündlichen Darmerkrankung; und
c. das Thrombose-bezogene Leiden ausgewählt ist aus der Gruppe bestehend aus Herzinfarkt, tiefe Venenthrombose, Embolie, thrombocytopenische Purpura und Mikroangiopathie.

31. Das antigenbindende Protein gemäß irgendeinem der Ansprüche 1 bis 23 oder die pharmazeutische Zusammensetzung gemäß der Ansprüche 24 oder 25 zur Verwendung gemäß der Ansprüche 28 oder 29, wobei das antigenbindende Protein oder die pharmazeutische Zusammensetzung in Kombination mit einem weiteren aktiven Agens verabreicht wird, das ausgewählt ist aus der Gruppe bestehend aus einem Statin, einem CETP Inhibitor, einem kardiovaskulären Agens, einem cholesterinsenkenden Agens, einem ACE Inhibitor, einem ACAT Inhibitor, einem Aldosteron-Antagonisten, einem Alphablocker, einem Angiotensin II Rezeptor-Antagonisten, einem antiarrhythmischen Agens, einem apoA-1 Agens, einem Betablocker, einem Gallensäurebinder, einem Kalziumkanalblocker, einem dyslipidämischen Agens, und einem Endothelin-Rezeptor-Antagonisten, einem Lecithin-Cholesterin-Acyltransferase-Aktivator, einem Fibrat, einem PPAR Agonisten, Squalen-Epoxidase-Inhibitor, einem entzündungshemmenden Agens, einem thrombosehemmenden Agens, einem antidiabetischen Agens und einem Zytokin.

## Revendications

1. Protéine agoniste de liaison à l'antigène, qui se lie spécifiquement à un polypeptide de lécithine-cholestérol acyltransférase humaine, laquelle protéine de liaison à l'antigène est un anticorps humain.

2. Protéine de liaison à l'antigène selon la revendication 1 qui se lie à un polypeptide de lécithine-cholestérol acyltransférase humaine constitué de la SEQ ID NO:1.

3. Protéine de liaison à l'antigène selon les revendications 1 ou 2, laquelle protéine de liaison à l'antigène présente une ou plusieurs des caractéristiques suivantes :
a) se lie au polypeptide de lécithine-cholestérol acyltransférase humaine de SEQ ID NO:1 avec un K_{D} inférieur à 50 nm, tel que déterminé par BiaCore ; et
b) se lie à la lécithine-cholestérol acyltransférase humaine avec un K_{D} inférieur à 50 nm et à la lécithine-cholestérol acyltransférase de cyno avec un K_{D} inférieur à 500 nm, tel que déterminé par BiaCore.

4. Protéine de liaison à l'antigène selon l'une quelconque des revendications 1 à 3, laquelle protéine de liaison à l'antigène comprend :
I.
a. une ou plusieurs régions déterminant la complémentarité de chaîne lourde (CDRH) choisies dans le groupe constitué par :
i. une CDRH1 choisie dans le groupe constitué par les SEQ ID NO:81-92 ;
ii. une CDRH2 choisie dans le groupe constitué par les SEQ ID NO:93-94 ;
iii. une CDRH3 choisie dans le groupe constitué par les SEQ ID NO:95-111 ;
iv. une CDRH de (i), (ii) et (iii) qui contient une ou plusieurs substitutions, délétions ou insertions d'acides aminés ne totalisant pas plus de 4 acides aminés ;
b. une ou plusieurs régions déterminant la complémentarité de chaîne légère (CDRL) choisies dans le groupe constitué par :
i. une CDRL1 choisie dans le groupe constitué par les SEQ ID NO:112-113 ;
ii. une CDRL2 choisie dans le groupe constitué par les SEQ ID NO:114-115 ;
iii. une CDRL3 choisie dans le groupe constitué par les SEQ ID NO:116-120 ;
iv. une CDRL de (i), (ii) et (iii) qui contient une ou plusieurs substitutions, délétions ou insertions d'acides aminés ne totalisant pas plus de 4 acides aminés ;
c. une ou plusieurs CDRH de (a) et une ou plusieurs CDRL de (b), ou
II. une région variable de chaîne lourde (VH) et/ou une région variable de chaîne légère (VL), où
(i)
a. la VH a au moins 90 % d'identité de séquence avec la séquence d'acides aminés choisie dans le groupe constitué par les SEQ ID NO:45-75 ; et
b. la VL a au moins 90 % d'identité de séquence avec la séquence d'acides aminés choisie dans le groupe constitué par les SEQ ID NO:76-80 ; ou
(ii)
a. la VH a au moins 90 % d'identité de séquence avec la séquence d'acides aminés de SEQ ID NO:45 et la VL a au moins 90 % d'identité de séquence avec la séquence d'acides aminés de SEQ ID NO:76 ; ou
b. la VH a au moins 90 % d'identité de séquence avec la séquence d'acides aminés de SEQ ID NO:75 et la VL a au moins 90 % d'identité de séquence avec la séquence d'acides aminés de SEQ ID NO:80, ou
III. une chaîne lourde (HC) et une chaîne légère (LC), où
(i)
a. la HC a au moins 90 % d'identité de séquence avec la séquence d'acides aminés de SEQ ID NO:9 et la LC a au moins 90 % d'identité de séquence avec la séquence d'acides aminés de SEQ ID NO:40 ; ou
b. la HC a au moins 90 % d'identité de séquence avec la séquence d'acides aminés de SEQ ID NO:39 et la LC a au moins 90 % d'identité de séquence avec la séquence d'acides aminés de SEQ ID NO:44, ou
(ii)
a. la HC comprend la séquence d'acides aminés de SEQ ID NO:9 et la LC comprend la séquence d'acides aminés de SEQ ID NO:40 ;
b. la HC comprend la séquence d'acides aminés de SEQ ID NO:19 et la LC comprend la séquence d'acides aminés de SEQ ID NO:40 ;
c. la HC comprend la séquence d'acides aminés de SEQ ID NO:27 et la .LC comprend la séquence d'acides aminés de SEQ ID NO:40 ;
d. la HC comprend la séquence d'acides aminés de SEQ ID NO:30 et la LC comprend la séquence d'acides aminés de SEQ ID NO:40 ;
e. la HC comprend la séquence d'acides aminés de SEQ ID NO:32 et la LC comprend la séquence d'acides aminés de SEQ ID NO:40 ;
f. la HC comprend la séquence d'acides aminés de SEQ ID NO:36 et la LC comprend la séquence d'acides aminés de SEQ ID NO:40 ;
g. la HC comprend la séquence d'acides aminés de SEQ ID NO:19 et la LC comprend la séquence d'acides aminés de SEQ ID NO:41 ;
h. la HC comprend la séquence d'acides aminés de SEQ ID NO:27 et la LC comprend la séquence d'acides aminés de SEQ ID NO:41 ;
i. la HC comprend la séquence d'acides aminés de SEQ ID NO:30 et la LC comprend la séquence d'acides aminés de SEQ ID NO:41 ;
j. la HC comprend la séquence d'acides aminés de SEQ ID NO:32 et la LC comprend la séquence d'acides aminés de SEQ ID NO:41 ;
k. la HC comprend la séquence d'acides aminés de SEQ ID NO:36 et la LC comprend la séquence d'acides aminés de SEQ ID NO:41 ;
l. la HC comprend la séquence d'acides aminés de SEQ ID NO:19 et la LC comprend la séquence d'acides aminés de SEQ ID NO:42 ;
m. la HC comprend la séquence d'acides aminés de SEQ ID NO:27 et la LC comprend la séquence d'acides aminés de SEQ ID NO:42 ;
n. la HC comprend la séquence d'acides aminés de SEQ ID NO:30 et la LC comprend la séquence d'acides aminés de SEQ ID NO:42 ;
o. la HC comprend la séquence d'acides aminés de SEQ ID NO:32 et la LC comprend la séquence d'acides aminés de SEQ ID NO:42 ;
p. la HC comprend la séquence d'acides aminés de SEQ ID NO:36 et la LC comprend la séquence d'acides aminés de SEQ ID NO:42 ;
q. la HC comprend la séquence d'acides aminés de SEQ ID NO:19 et la LC comprend la séquence d'acides aminés de SEQ ID NO:43 ;
r. la HC comprend la séquence d'acides aminés de SEQ ID NO:27 et la LC comprend la séquence d'acides aminés de SEQ ID NO:43 ;
s. la HC comprend la séquence d'acides aminés de SEQ ID NO:30 et la LC comprend la séquence d'acides aminés de SEQ ID NO:43 ;
t. la HC comprend la séquence d'acides aminés de SEQ ID NO:32 et la LC comprend la séquence d'acides aminés de SEQ ID NO:43 ;
u. la HC comprend la séquence d'acides aminés de SEQ ID NO:36 et la LC comprend la séquence d'acides aminés de SEQ ID NO:43 ; ou
la HC comprend la séquence d'acides aminés de SEQ ID NO:39 et la LC comprend la séquence d'acides aminés de SEQ ID NO:44.

5. Protéine de liaison à l'antigène selon la revendication 4 (I) qui comprend une CDRH3 et une CDRL3.

6. Protéine de liaison à l'antigène selon la revendication 5, dans laquelle
a. la CDRH3 comprend la SEQ ID NO:95 et la CDRL3 comprend la SEQ ID NO:116 ; ou
b. la CDRH3 comprend la SEQ ID NO:111 et la CDRL3 comprend la SEQ ID NO:120.

7. Protéine de liaison à l'antigène isolée selon l'une quelconque des revendications 1 à 3, ladite protéine de liaison à l'antigène comprenant :
I.
a. une CDRH choisie dans le groupe constitué par
i. une CDRH1 choisie dans le groupe constitué par les SEQ ID NO:81-92 ;
ii. une CDRH2 choisie dans le groupe constitué par les SEQ ID NO:93-94 ; et
iii. une CDRH3 choisie dans le groupe constitué par les SEQ ID NO:95-111 ;
b. une CDRL choisie dans le groupe constitué par
i. une CDRL1 choisie dans le groupe constitué par les SEQ ID NO:112-113 ;
ii. une CDRL2 choisie dans le groupe constitué par les SEQ ID NO:114-115 ; et
iii. une CDRL3 choisie dans le groupe constitué par les SEQ ID NO:116-120 ou
c. une ou plusieurs CDRH de a) et une ou plusieurs CDRL de b) ; ou
II.
a. une CDRH1 de SEQ ID NO:81, une CDRH2 de SEQ ID NO:93, une CDRH3 de SEQ ID NO:95, une CDRL1 de SEQ ID NO:112, une CDRL2 de SEQ ID NO:114, et une CDRL3 de SEQ ID NO:116 ;
b. une CDRH1 de SEQ ID NO:91, une CDRH2 de SEQ ID NO:93, une CDRH3 de SEQ ID NO:95, une CDRL1 de SEQ ID NO:112, une CDRL2 de SEQ ID NO:114, et une CDRL3 de SEQ ID NO:116 ;
c. une CDRH1 de SEQ ID NO:91, une CDRH2 de SEQ ID NO:93, une CDRH3 de SEQ ID NO:99, une CDRL1 de SEQ ID NO:112, une CDRL2 de SEQ ID NO:114, et une CDRL3 de SEQ ID NO:116 ;
d. une CDRH1 de SEQ ID NO:91, une CDRH2 de SEQ ID NO:93, une CDRH3 de SEQ ID NO:102, une CDRL1 de SEQ ID NO:112, une CDRL2 de SEQ ID NO:114, et une CDRL3 de SEQ ID NO:116 ;
e. une CDRH1 de SEQ ID NO:91, une CDRH2 de SEQ ID NO:93, une CDRH3 de SEQ ID NO:104, une CDRL1 de SEQ ID NO:112, une CDRL2 de SEQ ID NO:114, et une CDRL3 de SEQ ID NO:116 ;
f. une CDRH1 de SEQ ID NO:91, une CDRH2 de SEQ ID NO:93, une CDRH3 de SEQ ID NO:108, une CDRL1 de SEQ ID NO:112, une CDRL2 de SEQ ID NO:114, et une CDRL3 de SEQ ID NO:116 ;
g. une CDRH1 de SEQ ID NO:91, une CDRH2 de SEQ ID NO:93, une CDRH3 de SEQ ID NO:95, une CDRL1 de SEQ ID NO:112, une CDRL2 de SEQ ID NO:114, et une CDRL3 de SEQ ID N0:117 ;
h. une CDRH1 de SEQ ID NO:91, une CDRH2 de SEQ ID NO:93, une CDRH3 de SEQ ID NO:99, une CDRL1 de SEQ ID NO:112, une CDRL2 de SEQ ID NO:114, et une CDRL3 de SEQ ID NO:117 ;
i. une CDRH1 de SEQ ID NO:91, une CDRH2 de SEQ ID. NO:93, une CDRH3 de SEQ ID NO:102, une CDRL1 de SEQ ID NO:112, une CDRL2 de SEQ ID NO:114, et une CDRL3 de SEQ ID NO:117 ;
j. une CDRH1 de SEQ ID NO:91, une CDRH2 de SEQ ID NO:93, une CDRH3 de SEQ ID NO:104, une CDRL1 de SEQ ID NO:112, une CDRL2 de SEQ ID NO:114, et une CDRL3 de SEQ ID NO:117 ;
k. une CDRH1 de SEQ ID NO:91, une CDRH2 de SEQ ID NO:93, une CDRH3 de SEQ ID NO:108, une CDRL1 de SEQ ID NO:112, une CDRL2 de SEQ ID NO:114 et une CDRL3 de SEQ ID NO:117 ;
l. une CDRH1 de SEQ ID NO:91, une CDRH2 de SEQ ID NO:93, une CDRH3 de SEQ ID NO:95, une CDRL1 de SEQ ID NO:112, une CDRL2 de SEQ ID NO:114, et une CDRL3 de SEQ ID NO:118 ;
m. une CDRH1 de SEQ ID NO:91, une CDRH2 de SEQ ID NO:93, une CDRH3 de SEQ ID NO:99, une CDRL1 de SEQ ID NO:112, une CDRL2 de SEQ ID NO:114, et une CDRL3 de SEQ ID NO:118 ;
n. une CDRH1 de SEQ ID NO:91, une CDRH2 de SEQ ID NO:93, une CDRH3 de SEQ ID NO:102, une CDRL1 de SEQ ID NO:112, une CDRL2 de SEQ ID NO:114, et une CDRL3 de SEQ ID NO:118 ;
o. une CDRH1 de SEQ ID NO:91, une CDRH2 de SEQ ID NO:93, une CDRH3 de SEQ ID NO:104, une CDRL1 de SEQ ID NO:112, une CDRL2 de SEQ ID NO:114, et une CDRL3 de SEQ ID NO:118 ;
p. une CDRH1 de SEQ ID NO:91, une CDRH2 de SEQ ID NO:93, une CDRH3 de SEQ ID NO:108, une CDRL1 de SEQ ID NO:112, une CDRL2 de SEQ ID NO:114, et une CDRL3 de SEQ ID NO:118 ;
q. une CDRH1 de SEQ ID NO:91, une CDRH2 de SEQ ID NO:93, une CDRH3 de SEQ ID NO:95, une CDRL1 de SEQ ID NO:112, une CDRL2 de SEQ ID NO:114, et une CDRL3 de SEQ ID NO:119 ;
r. une CDRH1 de SEQ ID NO:91, une CDRH2 de SEQ ID NO:93, une CDRH3 de SEQ ID NO:99, une CDRL1 de SEQ ID NO:112, une CDRL2 de SEQ ID NO:114, et une CDRL3 de SEQ ID NO:119 ;
s. une CDRH1 de SEQ ID NO:91, une CDRH2 de SEQ ID NO:93, une CDRH3 de SEQ ID NO:102, une CDRL1 de SEQ ID NO:112 une CDRL2 de SEQ ID NO:114 et une CDRL3 de SEQ ID NO:119 ;
t. une CDRH1 de SEQ ID NO:91, une CDRH2 de SEQ ID NO:93, une CDRH3 de SEQ ID NO:104, une CDRL1 de SEQ ID NO:112, une CDRL2 de SEQ ID NO:114, et une CDRL3 de SEQ ID NO:119 ;
u. une CDRH1 de SEQ ID NO:91, une CDRH2 de SEQ ID NO:93, une CDRH3 de SEQ ID NO:108, une CDRL1 de SEQ ID NO:112, une CDRL2 de SEQ ID NO:114, et une CDRL3 de SEQ ID NO:119 ; ou
une CDRH1 de SEQ ID NO:92, une CDRH2 de SEQ ID NO:94, une CDRH3 de SEQ ID NO:111, une CDRL1 de SEQ ID NO:113, une CDRL2 de SEQ ID NO:115, et une CDRL3 de SEQ ID NO:120.

8. Protéine de liaison à l'antigène selon la revendication 4 (II) (i), ladite protéine de liaison à l'antigène comprenant une région variable de chaîne lourde (VH) et une région variable de chaîne légère (VL), où
a. la VH comprend la séquence d'acides aminés de SEQ ID NO:45 et la VL comprend la séquence d'acides aminés de SEQ ID NO:76 ;
b. la VH comprend la séquence d'acides aminés de SEQ ID NO:55 et la VL comprend la séquence d'acides aminés de SEQ ID NO:76 ;
c. la VH comprend la séquence d'acides aminés de SEQ ID NO:63 et la VL comprend la séquence d'acides aminés de SEQ ID NO:76 ;
d. la VH comprend la séquence d'acides aminés de SEQ ID NO:66 et la VL comprend la séquence d'acides aminés de SEQ ID NO:76 ;
e. la VH comprend la séquence d'acides aminés de SEQ ID NO:68 et la VL comprend la séquence d'acides aminés de SEQ ID NO:76 ;
f. la VH comprend la séquence d'acides aminés de SEQ ID NO:72 et la VL comprend la séquence d'acides aminés de SEQ ID NO:76 ;
g. la VH comprend la séquence d'acides aminés de SEQ ID NO:55 et la VL comprend la séquence d'acides aminés de SEQ ID NO:77 ;
h. la VH comprend la séquence d'acides aminés de SEQ ID NO:63 et la VL comprend la séquence d'acides aminés de SEQ ID NO:77 ;
i. la VH comprend la séquence d'acides aminés de SEQ ID NO:66 et la VL comprend la séquence d'acides aminés de SEQ ID NO:77 ;
j. la VH comprend la séquence d'acides aminés de SEQ ID NO:68 et la VL comprend la séquence d'acides aminés de SEQ ID NO:77 ;
k. la VH comprend la séquence d'acides aminés de SEQ ID NO:72 et la VL comprend la séquence d'acides aminés de SEQ ID NO:77 ;
l. la VH comprend la séquence d'acides aminés de SEQ ID NO:55 et la VL comprend la séquence d'acides aminés de SEQ ID NO:78 ;
m. la VH comprend la séquence d'acides aminés de SEQ ID NO:63 et la VL comprend la séquence d'acides aminés de SEQ ID NO:78 ;
n. la VH comprend la séquence d'acides aminés de SEQ ID NO:66 et la VL comprend la séquence d'acides aminés de SEQ ID NO:78 ;
o. la VH comprend la séquence d'acides aminés de SEQ ID NO:68 et la VL comprend la séquence d'acides aminés de SEQ ID NO:78 ;
p. la VH comprend la séquence d'acides aminés de SEQ ID NO:72 et la VL comprend la séquence d'acides aminés de SEQ ID NO:78 ;
q. la VH comprend la séquence d'acides aminés de SEQ ID NO:55 et la VL comprend la séquence d'acides aminés de SEQ ID NO:79 ;
r. la VH comprend la séquence d'acides aminés de SEQ ID NO:63 et la VL comprend la séquence d'acides aminés de SEQ ID NO:79 ;
s. la VH comprend la séquence d'acides aminés de SEQ ID NO:66 et la VL comprend la séquence d'acides aminés de SEQ ID NO:79 ;
t. la VH comprend la séquence d'acides aminés de SEQ ID NO:68 et la VL comprend la séquence d'acides aminés de SEQ ID NO:79 ;
u. la VH comprend la séquence d'acides aminés de SEQ ID NO:72 et la VL comprend la séquence d'acides aminés de SEQ ID NO:79 ; ou
la VH comprend la séquence d'acides aminés de SEQ ID NO:75 et la VL comprend la séquence d'acides aminés de SEQ ID NO:80.

9. Protéine agoniste de liaison à l'antigène, qui entre en compétition avec la protéine de liaison à l'antigène selon l'une quelconque des revendications 1 à 8 pour la liaison à un polypeptide de lécithine-cholestérol acyltransférase humaine de SEQ ID NO:1.

10. Protéine agoniste de liaison à l'antigène selon la revendication 9, qui
a. entre en compétition avec une protéine de liaison à l'antigène comprenant : une CDRH1 de SEQ ID NO:81, une CDRH2 de SEQ ID NO:93, une CDRH3 de SEQ ID NO:95, une CDRL1 de SEQ ID NO:112, une CDRL2 de SEQ ID NO:114, et une CDRL3 de SEQ ID NO: 116 ;
b. entre en compétition avec une protéine de liaison à l'antigène comprenant : une CDRH1 de SEQ ID NO:92, une CDRH2 de SEQ ID NO:94, une CDRH3 de SEQ ID NO:111, une CDRL1 de SEQ ID NO:113, une CDRL2 de SEQ ID NO:115, et une CDRL3 de SEQ ID NO:120 ;
c. entre en compétition avec une protéine de liaison à l'antigène comprenant une VH de SEQ ID NO:45 et une VL de SEQ ID NO:76 ; ou
d. entre en compétition avec une protéine de liaison à l'antigène comprenant une VH de SEQ ID NO:75 et une VL de SEQ ID NO:80.

11. Protéine de liaison à l'antigène selon l'une quelconque des revendications 1 à 3 qui se lie à des acides aminés situés dans une région d'un polypeptide de lécithine-cholestérol acyltransférase constitué de la **SEQ ID NO:1,** où
a) la région va de l'acide aminé 255 à l'acide aminé 389 de la **SEQ ID NO:1,** bornes incluses ; ou
b) où la région va de l'acide aminé 255 à l'acide aminé 374 de la **SEQ ID NO:1,** bornes incluses.

12. Protéine de liaison à l'antigène selon la revendication 11 (b) qui se lie à des acides aminés présents dans une ou plusieurs des régions suivantes :
I.
a. la région allant de l'acide aminé 255 à l'acide aminé 262 de la **SEQ ID NO:1,** bornes incluses ;
b. la région allant de l'acide aminé 315 à l'acide aminé 321 de la **SEQ ID NO:1,** bornes incluses ; et
c. la région allant de l'acide aminé 341 à l'acide aminé 374 de la **SEQ ID NO:1,** bornes incluses ; ou
II.
a. la région allant de l'acide aminé 255 à l'acide aminé 262 de la **SEQ ID NO:1,** bornes incluses ;
b. la région allant de l'acide aminé 315 à l'acide aminé 321 de la **SEQ ID NO:1,** bornes incluses ;
c. la région allant de l'acide aminé 341 à l'acide aminé 343 de la **SEQ ID NO:1,** bornes incluses ; et
d. la région allant de l'acide aminé 350 à l'acide aminé 374 de la **SEQ ID NO:1,** bornes incluses.

13. Protéine de liaison à l'antigène selon la revendication 12 (I) qui se lie à des acides aminés présents dans les régions (a), (b) et (c).

14. Protéine de liaison à l'antigène selon la revendication 12 (II) qui se lie à des acides aminés présents dans les régions (a), (b), (c) et (d).

15. Protéine de liaison à l'antigène selon la revendication 11 (a) qui se lie
I.
(i) à au moins 12 des acides aminés choisis dans le groupe constitué par S255, R256, M257, A258, W259, P260, Y315, V317, G318, L319, P320, T321, Y341, E342, D343, T350, R351, E354, L355, C356, G357, L358, Q360, R362, V367, H368, L369, P371, H373 et G374 de la **SEQ ID NO:1,** ou
(ii) aux acides aminés S255, R256, M257, A258, W259, P260, Y315, V317, G318, L319, P320, T321, Y341, E342, D343, T350, R351, E354, L355, C356, G357, L358, Q360, R362, V367, H368, L369, P371, H373 et G374 de la **SEQ ID NO:1 ;** ou
II.
(i) à au moins 15 des acides aminés choisis dans le groupe constitué par les acides aminés R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 et H389 de la **SEQ ID NO:1** ; ou
(ii) aux acides aminés R256, M257, A258, P260, D262, Y315, V317, G318, L319, P320, Y341, E342, D343, T350, R351, E354, L355, G357, L358, Q360, G361, R362, P366, V367, H368, L369, P371, H373, G374 et H389 de la **SEQ ID NO:1.**

16. Protéine de liaison à l'antigène selon l'une quelconque des revendications 1 à 3 qui se lie à des acides aminés situés dans une région d'un polypeptide de lécithine-cholestérol acyltransférase constitué de la **SEQ ID NO:1,** où
a. la région va de l'acide aminé 315 à l'acide aminé 399, bornes incluses, ou
b. la région va de l'acide aminé 343 à l'acide aminé 399, bornes incluses.

17. Protéine de liaison à l'antigène selon la revendication 16 qui se lie à des acides aminés présents dans une ou plusieurs des régions suivantes :
I.
a. la région allant de l'acide aminé 350 à l'acide aminé 375 de la **SEQ ID NO:1,** bornes incluses ;
b. la région allant de l'acide aminé 385 à l'acide aminé 399 de la **SEQ ID NO:1,** bornes incluses ; ou
II.
a. la région allant de l'acide aminé 315 à l'acide aminé 317 de la **SEQ ID NO:1,** bornes incluses ;
b. la région allant de l'acide aminé 350 à l'acide aminé 375 de la **SEQ ID NO:1**, bornes incluses ; et
c. la région allant de l'acide aminé 385 à l'acide aminé 399 de la **SEQ ID NO:1**, bornes incluses.

18. Protéine de liaison à l'antigène selon la revendication 17 (I) qui se lie à des acides aminés présents dans les régions (a) et (b).

19. Protéine de liaison à l'antigène selon la revendication 17 (II) qui se lie à des acides aminés présents dans les régions (a), (b) et (c).

20. Protéine de liaison à l'antigène selon la revendication 16 qui se lie
I.
(i) à au moins 12 des acides aminés choisis dans le groupe constitué par Y315, V317, D343, T350, R351, E354, G357, Q360, G361, Q365, P366, V367, H368, L369, L370, P371, L372, H373, I375, L385, E388, H389, A392, L395, G396, A397, Y398 et R399 de la **SEQ ID NO:1,** ou
(ii) aux acides aminés Y315, V317, D343, T350, R351, E354, G357, Q360, G361, Q365, P366, V367, H368, L369, L370, P371, L372, H373, I375, L385, E388, H389, A392, L395, G396, A397, Y398 et R399 de la **SEQ ID NO:1** ; ou
II.
(i) à au moins 12 des acides aminés choisis dans le groupe constitué par Y315, V317, E342, D343, T350, R351, E354, G357, Q360, G361, P364, P366, V367, H368, L369, L370, P371, L372, H373, L385, E388, H389, A392, L395, G396, A397, Y398 et R399 de la **SEQ ID NO:1,** ou
(ii) aux acides aminés, Y315, V317, E342, D343, T350, R351, E354, G357, Q360, G361, P364, P366, V367, H368, L369, L370, P371, L372, H373, L385, E388, H389, A392, L395, G396, A397, Y398 et R399 de la **SEQ ID NO:1.**

21. Protéine de liaison à l'antigène selon l'une quelconque des revendications 1 à 3 qui se lie à un épitope, lequel épitope est situé
a. entre l'acide aminé 255 et l'acide aminé 389 de la **SEQ ID NO:1 ;**
b. entre l'acide aminé 315 et l'acide aminé 399 de la **SEQ ID NO:1** ; ou
c. entre l'acide aminé 342 et l'acide aminé 399 de la **SEQ ID NO:1.**

22. Protéine de liaison à l'antigène selon la revendication 21, où l'épitope est un épitope conformationnel et/ou discontinu.

23. Protéine de liaison à l'antigène selon les revendications 1 à 21, laquelle protéine de liaison à l'antigène présente une ou plusieurs des caractéristiques suivantes :
a. est un anticorps monoclonal, un anticorps humain, un anticorps humanisé, un anticorps chimérique, ou un anticorps multispécifique ;
b. est du type IgG1, IgG2, IgG3, ou IgG4 ;
c. est un fragment Fab, un fragment Fab', un fragment F(ab')2, ou un fragment Fv ;
d. est un diabody, un anticorps simple chaîne, un anticorps à domaine, ou un nanobody ;
e. provient d'une source mammifère choisie parmi souris, rat, camélidé ou lapin ; ou
f. est marquée.

24. Composition pharmaceutique comprenant au moins une protéine de liaison à l'antigène selon l'une quelconque des revendications 1 à 23.

25. Composition pharmaceutique selon la revendication 24 comprenant en outre un agent actif supplémentaire choisi dans le groupe constitué par un agent utilisé pour traiter une maladie cardiovasculaire, un agent utilisé pour traiter des troubles liés au cholestérol, un agent utilisé pour traiter l'athérosclérose, un agent anti-inflammatoire, un agent antithrombotique, un agent antidiabétique et une cytokine.

26. Molécule d'acide nucléique codant la protéine de liaison à l'antigène selon l'une quelconque des revendications 1 à 23.

27. Procédé d'obtention de la protéine de liaison à l'antigène selon l'une quelconque des revendications 1 à 23, le procédé comprenant la préparation de la protéine de liaison à l'antigène à partir d'une cellule hôte qui sécrète la protéine de liaison à l'antigène.

28. Protéine de liaison à l'antigène selon l'une quelconque des revendications 1 à 23 ou composition pharmaceutique selon les revendications 24 ou 25 destinée à être utilisée en thérapeutique.

29. Protéine de liaison à l'antigène selon l'une quelconque des revendications 1 à 23 ou composition pharmaceutique selon les revendications 24 ou 25 destinée à être utilisée pour
a) diminuer le taux de cholestérol chez un sujet ou augmenter les taux sériques de HDL-C chez un sujet ; ou
b) traiter ou prévenir une affection choisie dans le groupe constitué par un trouble lié au cholestérol, une maladie cardiovasculaire, une affection inflammatoire, une affection liée à une thrombose, un trouble sanguin, et une affection associée un déficit en lécithine-cholestérol acyltransférase.

30. Protéine de liaison à l'antigène selon l'une quelconque des revendications 1 à 23 ou composition pharmaceutique selon les revendications 24 ou 25 destinée à être utilisée selon les revendications 28 ou 29, où
a. la maladie cardiovasculaire ou le trouble lié au cholestérol est choisi(e) dans le groupe constitué par l'artériosclérose, l'athérosclérose, l'accident vasculaire cérébral, l'ischémie, les maladies vasculaires périphériques, la maladie des artères coronaires, la cardiopathie coronarienne, l'infarctus du myocarde, l'hypertension artérielle, l'hypercholestérolémie, le syndrome métabolique, la dyslipidémie, le diabète et l'insulinorésistance ;
b. la maladie inflammatoire est choisie dans le groupe constitué par l'arthrite, le sepsis, le choc septique, l'asthme, une maladie inflammatoire pulmonaire chronique, et une maladie intestinale inflammatoire ; et
c. l'affection liée à une thrombose est choisie dans le groupe constitué par un infarctus du myocarde, une thrombose veineuse profonde, une embolie, un purpura thrombocytopénique et une microangiopathie.

31. Protéine de liaison à l'antigène selon l'une quelconque des revendications 1 à 23 ou composition pharmaceutique selon les revendications 24 ou 25 destinée à être utilisée selon les revendications 28 ou 29, laquelle protéine de liaison à l'antigène ou composition pharmaceutique est administrée en association avec un agent actif supplémentaire choisi dans le groupe constitué par une statine, un inhibiteur de CETP, un agent cardiovasculaire, un agent hypocholestérolémiant, un inhibiteur d'ACE, un inhibiteur d'ACAT, un antagoniste de l'aldostérone, un alpha-bloquant, un antagoniste des récepteurs de l'angiotensine II, un agent antiarythmique, un agent apoA-1, un bêta-bloquant, un séquestrant des acides biliaires, un inhibiteur calcique, un agent contre la dyslipidémie, et un antagoniste des récepteurs de l'endothéline, un activateur de la lécithine-cholestérol acyltransférase, un fibrate, un agoniste de PPAR, un inhibiteur de la squalène époxydase, un agent anti-inflammatoire, un agent antithrombotique, un agent antidiabétique et une cytokine.
